(19) Europäisches Patentamt
European Patent Office
Office européen des brevets

(11) EP 0 901 788 A2

(12) # EUROPÄISCHE PATENTANMELDUNG

(43) Veröffentlichungstag:
**17.03.1999 Patentblatt 1999/11**

(51) Int. Cl.$^6$: **A61K 31/00**, A61K 31/155, A61K 31/40, A61K 31/415

(21) Anmeldenummer: **98115902.3**

(22) Anmeldetag: **24.08.1998**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **28.08.1997 DE 19737463**

(71) Anmelder:
**Hoechst Marion Roussel Deutschland GmbH**
**65929 Frankfurt am Main (DE)**

(72) Erfinder:
**Die Erfindernennung liegt noch nicht vor**

(54) **Verwendung von Inhibitoren des Natrium-Wasserstoff-Austauschers zur Herstellung eines Arzneimittels zur Behandlung von Erkrankungen, die durch Protozoen verursacht werden**

(57) Inhibitoren des Natrium-Wasserstoff-Austauschers eignen sich allein oder in Kombination mit anderen Wirkstoffklassen zur Herstellung eines Medikaments zur Behandlung von Krankheiten, die durch Protozoen verursacht werden; so von Krankheiten, welche durch veterinär- und humanpathogene Protozoen verursacht werden, wie durch intrazellulär aktive Parasiten der Klassen Apicomlexa und Zoomastigophora, insbesondere Trypanosomen, Plasmodien (Malaria-Erreger), Leishmanien, Babesien und Theilerien, Cryptosporidiidae, Sarcocystidae, Amöben, Coccidien und Trichomonaden.

Besonders bevorzugt eignen sich NHE-Inhibitoren zur Herstellung eines Medikaments zur Behandlung der Malaria tropica, der Malaria tertiana, der Malaria quartana, sowie der Toxoplasmose, der Coccidiose, der Darmsarcosporidose, der Cryptosporidose, der Chargaskrankheit und der kutanen und visceralen sowie anderer Formen von Leishmaniosen;
sowie zur Herstellung eines Medikaments zur Behandlung von Tieren, die durch veterinärpathogene Protozoen infiziert wurden, wie durch Theileria parva, dem Erreger des Ostküstenfiebers von Rindern, Babesia begemina, dem Erreger des Texasfiebers bei Rindern und Büffeln, Babesia bovis, dem Erreger der europäischen Rinderbabesiose, sowie Babesiosen bei Hund, Katze und Schaf, Sarcocystidae, den Erregern von Sarkozystosen bei Schaf, Rind und Schwein, Cryptosporidiidae, den Erregern der Cryptosporidose bei Rind und Vögeln, Coccidien, den Erregern von Coccidiosen von Kaninchen, Rindern, Schafen, Ziegen und Schweinen, insbesondere aber von Hühnern und Truthühnern.

EP 0 901 788 A2

**Beschreibung**

Die Erfindung betrifft die Verwendung von Inhibitoren des zellulären Natrium-Wasserstoff-Austauschers zur Herstellung eines Arzneimittels zur Behandlung von Krankheiten, die durch Befall von Menschen und Tieren mit Protozoen zustande kommen.

Inhibitoren des Natrium-Wasserstoffaustauschers (NHE) sind im Verlauf der letzten Jahre in zahlreichen präklinischen Studien als Substanzen charakterisiert worden, die bei Minderdurchblutung des Herzens in überlegener Weise geeignet sind, das gefährdete Herzgewebe vor dem Untergang zu schützen. Der Schutz des Herzgewebes umfaßt dabei alle Formen der durch Mangeldurchblutung hervorgerufenen Herzschädigungen, angefangen bei Herz-Rhythmusstörungen über Hyperkontraktur des Herzmuskels und vorübergehenden Funktionsverlust bis hin zum Absterben des Herzgewebes und damit verbundenen dauerhaften Schäden.

Der Wirkmechanismus der NHE-Inhibitoren besteht darin, daß sie den verstärkten Einstrom von Natriumionen, der in mangeldurchbluteten Geweben infolge intrazellulärer Ansäuerung und nachfolgender Aktivierung des NHE entsteht, vermindern. Da in Säugetiergewebe Natrium- und Calciumionentransport miteinander gekoppelt sind, wird damit auch die lebensbedrohliche Calciumüberladung der Zellen verhindert. Dieser einzigartige Mechanismus bezieht sich relativ spezifisch insbesondere auf Herzgewebe.

Es war deshalb überraschend, als nunmehr gefunden werden konnte, daß NHE-Inhibitoren in der Lage sind, Protozoen mit pathogener Wirkung auf Mensch und Tier zu töten bzw. die Überlebensfähigkeit von Protozoen signifikant einzuschränken. Deshalb eignen sich Inhibitoren des Natrium-Wasserstoff-Austauschers allein oder in Kombination mit anderen Wirkstoffklassen zur Behandlung von Krankheiten, die durch Protozoen verursacht werden.

Derartige veterinär- und humanpathogene Protozoen sind bevorzugt intrazellulär aktive Parasiten der Klassen Apicomlexa und Zoomastigophora, insbesondere Trypanosomen, Plasmodien (Malaria-Erreger), Leishmanien, Babesien und Theilerien, Cryptosporidiidae, Sarcocystidae, Amöben, Coccidien und Trichomonaden.

Besonders bevorzugt eignen sich NHE-Inhibitoren

zur Behandlung der Malaria tropica, die durch Plasmodium falciparum verursacht wird,
zur Behandlung der Malaria tertiana, ausgelöst durch Plasmodium vivax und Plasmodium ovale,
zur Behandlung der Malaria quartana, verursacht durch Plasmodium malariae, sowie zur Behandlung
der Toxoplasmose, ausgelöst durch Toxoplasma gondii,
der Coccidiose, ausgelöst durch Isospora belli,
der Darmsarcosporidose, verursacht durch Sarcocystis suihominis,
der Cryptosporidose, verursacht durch Cryptosporidium parvum,
der Chargaskrankheit, die durch Trypanosoma cruzi verursacht wird,
der kutanen und visceralen sowie anderen Formen von Leishmaniosen,
sowie zur Behandlung von Tieren, die durch veterinärpathogene Protozoen infiziert wurden, wie durch
Theileria parva, dem Erreger des Ostküstenfiebers von Rindern,
Babesia begemina, dem Erreger des Texasfiebers bei Rindern und Büffeln,
Babesia bovis, dem Erreger der europäischen Rinderbabesiose, sowie Babesiosen bei Hund, Katze und Schaf,
Sarcocystidae, den Erregern von Sarkozystosen bei Schaf, Rind und Schwein,
Cryptosporidiidae, den Erregern der Cryptosporidose bei Rind und Vögeln,
Coccidien, den Erregern von Coccidiosen von Kaninchen, Rindern, Schafen,
Ziegen und Schweinen, insbesondere aber von Hühnern und Truthühnern.

Bei der Behandlung von Krankheiten, die durch human- und veterinärpathogene Protozoen verursacht werden, können die NHE-inhibitoren sowohl oral wie auch parenteral verabreicht werden. Dabei kommen NHE-Inhibitoren als Monosubstanzen und in Kombination mit anderen Wirkstoffen zur Anwendung. Da es sich bei den NHE um ein neues Wirkprinzip handelt, sind auch Kombination mit Arzneimitteln vorteilhaft anwendbar, die bereits zur Behandlung der jeweiligen protozoenbedingten Erkrankung verwendet werden, wobei eine günstige, additiv verstärkende Wirkung beobachtet wird.

Ganz besonders bevorzugt eignen sich die NHE-Inhibitoren zur Behandlung der Malariaformen, insbesondere zur Behandlung der Malaria tropica. Zur Zeit sind zwischen 300 - 500 Millionen Menschen an Malaria erkrankt, und jede zwölfte Sekunde stirbt ein Mensch an den Folgen dieser Infektionskrankheit. Die Zahl der Malariafälle wird sich aller Voraussicht nach in den nächsten Jahren noch weiter erhöhen, da wirksame Methoden zur Bekämpfung der Malaria derzeit fehlen. Medikamente, die bislang gegen Malaria eingesetzt wurden, haben weitgehend ihre Wirksamkeit verloren. Die Malaria-Erreger, Protozoen der Gattung Plasmodium, sind resistent geworden. Um die sich anbahnende Katastrophe abzuwenden, bedarf es laut Weltgesundheitsorganisation dringend neuer Arzneimittel zur prophylaktischen und kurativen Behandlung der Malaria. Dabei hat sich die Entwicklung neuer Medikamente gegen Malaria als sehr schwierig erwiesen.

Es war deshalb überraschend, daß sich NHE-Inhibitoren sich nicht nur gegen Chloroquin-sensitive sondern auch gegen Chloroquin-resistente Plasmodium falciparum-Stämme als wirksam erwiesen. Neben der bislang üblichen Behandlung des späteren erythrocytären Stadiums des Malariaerregers erscheint mit NHE-Inhibitoren die Behandlung der Malariafrühform auch durch Abtötung der Erreger bereits in der Leber ganz besonders vorteilhaft zu sein.

Im folgenden wird die Wirksamkeit von NHE-Inhibitoren gegen Protozoen beschrieben:

Kultur von Plasmodium falciparum

Erythrocytäre Stadien des humanen Malariaparasiten *Plasmodium falciparum* wurden in RPMI 1640-Medium, supplimentiert mit L-Glutamin, 25 mM HEPES, 4 µg/ml Gentamycin, 10% humanem Serum und 5 % humanen Erythrozyten, nach Trager und Jensen (1976) kultiviert. Die Kulturen wurden in Petrischalen (10 ml pro Schale mit 10 cm Durchmesser) gehalten und bei einer Athmosphäre von 92 % Stickstoff, 3 % Kohlendioxid und 5 % Sauerstoff bei 37°C inkubiert. Das Medium der Kulturen wurde täglich gewechselt. Bei einer Parasitämie von maximal 10% wurden die Kulturen verdünnt. Um die Kulturen zu synchronisieren, wurden die infizierten Erythrozyten in 5 %iger Sorbitollösung nach Lambros und Vanderberg (1979) gewaschen. Diese Behandlung überleben nur die Ringstadien.

Bestimmung der $IC_{50}$-Werte verschiedener NHE-Inhibitoren

Die halbmaximale inhibitorische Konzentration verschiedener NHE-Inhibitoren für das Wachstum von Plasmodium falciparum wurde auf 96 well-Mikrotiterplatten durch Einbau von radioaktivem Hypoxanthin nach Desjardins et al. (1979) gemessen. Dabei wurden die Parasiten für einen 48 Stunden dauernden vollständigen erythrozytären Zyklus in Gegenwart der entsprechenden NHE-Inhibitoren kultiviert.

Die NHE-Inhibitoren wurden in DMSO gelöst und in physiologischer Salzlösung (150 mM NaCl) vorverdünnt. Auf den Mikrititerplatten wurde eine Verdünnungsreihe der NHE-Inhibitoren in 10fach konzentrierten 20 µl-Aliquots vorgelegt. Dann wurde zu jedem well 180 µl Parasitensuspension in Kulturmedium zugefügt. Die Parasiten befanden sich im Ring-Stadium bei einer Parasitämie von 1 %. Anschließend wurden die Mikrititerplatten für 24 Stunden inkubiert. Um das Kulturmedium zu wechseln, wurden je 150 µl Überstand von jedem weil abgenommen und durch 135 µl neues Medium, zusätzlich supplementiert mit 2 µCi / ml [3H]-Hypoxanthin, ersetzt. Die Konzentration der NHE-inhibitoren wurde durch Zufügen 10fach konzentrierter Stammlösungen in 15 µl-Aliquots wieder eingestellt. Nach weiterer 24 stündiger Inkubation wurden die Kulturen durch Filtration auf Glasfaserfilter geerntet und die inkorporierte Radioaktivität gemessen. Die prozentuale Inhibition der Tritiuminkorporation wurde auf eine Kontrolle ohne NHE-Inhibitor bezogen.

Die $IC_{50}$ Werte der verschiedenen NHE-Inhibitoren wurden an einem Chloroquinsensitiven (HB3) und einem Chloroquin-resistenten (Dd2) Stamm gemessen.

| Substanz | HB3 $IC_{50}$ [µm] | Dd2 $IC_{50}$ [µm] |
|---|---|---|
| 3-Trifluormethyl-5-N,N-dimethylaminobenzoyl-guanidin Dihydrochlorid | 0,97 ± 0.12 | 1,3 ± 0,10 |
| 3-Trifluormethyl-4-(3-pyridyloxy)benzoylguanidin Dihydrochlorid | 12 ± 1,7 | 6,5 ± 1,5 |
| 4-[4-(2-Aminoethyl)phenoxy]-3-methylsulfonylbenzoyl-guanidin Dihydrochlorid | 2,9 ± 0,79 | 3,5 ± 0,04 |

Literatur:

Desjardins, R. E., Canfield, C. J., Haynes, J. D. and Chulay, J. D. (1979) Quantitativement assessment of antimalarial activity in vitro by semiautomated microdilution technique, Antimicrob. Agents Chemother. 16: 710 - 718.

Lambros, C.J. and Vanderberg, J. (1979) Synchronization of Plasmodium falciparum erythrocytic stages in culture, J. Parasitol. 65: 418 - 420.

Trager, W. und Jensen, J.B. (1976) Human malaria parasites in continously culture. Science 193: 673 - 675

Bei den bekannten und als NHE-Inhibitoren identifizierten Wirkstoffen handelt es sich um Guanidin-Derivate, vorzugsweise um Acylguanidine, unter anderem wie sie in folgenden Publikationen und Patentveröffentlichungen

beschrieben: Edward J. Cragoe, Jr., "DIURETICS, Chemistry, Pharmacology and Medicine", J. WILEY & Sons (1983), 303 - 341, außerdem um Verbindungen der folgenden Formeln:

I. (HOE 89/F 288 - US 5 292 755)

a) Benzoylguanidine der Formel I

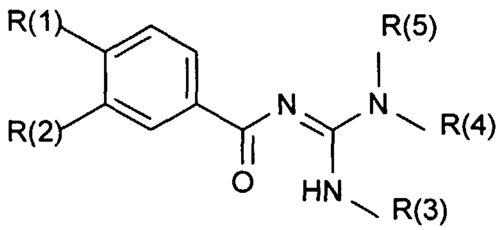

I

worin bedeuten:

R(1) oder R(2)

$R(6)-S(O)_n-$ oder $R(7)R(8)N-O_2S-$;

und der jeweils andere Substituent R(1) oder R(2)

H, F, Cl, Br, $(C_1-C_4)$-Alkyl, $(C_1-C_4)$-Alkoxy oder Phenoxy,

das unsubstituiert ist oder substituiert mit 1 - 3 Substituenten ausgewählt aus der Gruppe bestehend aus Fluor, Chlor, Methyl und Methoxy;

oder der jeweils andere Substituent R(1) oder R(2)

$R(6)-S(O)_n$ oder $R(7)R(8)N-$;

n Null, 1 oder 2;

R(6) $(C_1-C_6)$-Alkyl, $(C_5-C_7)$-Cycloalkyl, Cyclopentylmethyl, Cyclohexylmethyl oder Phenyl,

das unsubstituiert ist oder substituiert mit 1 - 3 Substituenten ausgewählt aus der Gruppe bestehend aus Fluor, Chlor, Methyl und Methoxy;

R(7) und R(8)

gleich oder verschieden H oder $(C_1-C_6)$-Alkyl;

oder

R(7) Phenyl-$(CH_2)_m$;

m 1 - 4;

oder

R(7) Phenyl,

das unsubstituiert ist oder substituiert mit 1 - 2 Substituenten ausgewählt aus der Gruppe bestehend aus Fluor, Chlor, Methyl und Methoxy;

oder

R(7) und R(8)

gemeinsam eine geradkettige oder verzweigte $(C_4-C_7)$-Kette, wobei die Kette zusätzlich durch O, S oder NR(9) unterbrochen sein kann;

R(9) H oder Methyl;

oder
R(7) und R(8)

gemeinsam mit dem Stickstoffatom, an das sie gebunden sind, ein Dihydroindol-, Tetrahydrochinolin- oder Tetrahydroisochinolin-System;

R(3), R(4) und R(5)

unabhängig voneinander H oder ($C_1$-$C_2$)-Alkyl,

oder
R(3) und R(4)

gemeinsam eine ($C_2$-$C_4$)-Alkylenkette;

oder
R(4) und R(5)

gemeinsam eine ($C_4$-$C_7$)-Alkylenkette;

sowie deren pharmazeutisch verträgliche Salze;

(HOE 92/F 34 - US 5 373 924)
b) Benzoylguanidine der Formel I

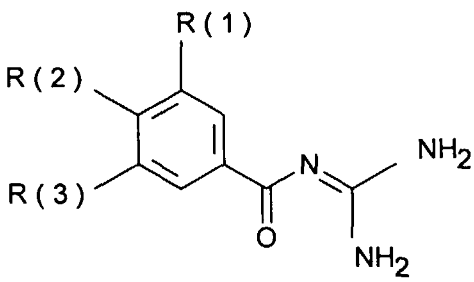

(I)

worin bedeuten:

R(1) R(4)-$SO_m$ oder R(5)R(6)N-$SO_2$-;

m Null, 1 oder 2;
R(4) und R(5)

$C_1$-$C_8$-Alkyl, $C_3$-$C_6$-Alkenyl oder -$C_nH_{2n}$-R(7);
n Null, 1, 2, 3 oder 4;
R(7) $C_5$-$C_7$-Cycloalkyl oder Phenyl,

welches unsubstituiert ist oder substituiert mit 1 - 3 Substituenten ausgewählt aus der Gruppe bestehend aus F, Cl, $CF_3$, Methyl, Methoxy und NR(8)R(9);

R(8) und R(9)

H oder $C_1$-$C_4$-Alkyl;

oder
R(5) H;
R(6) H oder $C_1$-$C_4$-Alkyl,
oder

R(5) und R(6)

gemeinsam 4 oder 5 Methylengruppen, von denen eine $CH_2$-Gruppe durch ein O, S, NH, N-$CH_3$ oder N-Benzyl ersetzt sein kann;

R(2) Wasserstoff, F, Cl, Br, ($C_1$-$C_4$)-Alkyl-, O-$(CH_2)_mC_pF_{2p+1}$ oder-X-R(10);

m Null oder 1;
p 1, 2 oder 3;
X O, S oder NR(11);
R(10) H, $C_1$-$C_6$-Alkyl, $C_5$-$C_7$-Cycloalkyl, Cyclohexylmethyl, Cyclopentylmethyl oder -$C_nH_{2n}$-R(12);

n Null, 1, 2, 3 oder 4;
R(12) Phenyl,

das unsubstituiert oder substituiert ist mit 1 - 3 Substituenten ausgewählt aus der Gruppe bestehend aus F, Cl, $CF_3$, Methyl, Methoxy und NR(8)R(9);
R(8) und R(9)

gleich H oder $C_1$-$C_4$-Alkyl;

R(11) Wasserstoff oder $C_1$-$C_3$-Alkyl;
oder
R(10) und R(11)

gemeinsam 4 oder 5 Methylengruppen, von denen eine $CH_2$-Gruppe durch O, S, NH, N-$CH_3$ oder N-Benzyl ersetzt sein kann;

R(3) wie R(1) definiert, oder $C_1$-$C_6$-Alkyl, Nitro, Cyano, Trifluormethyl, F, Cl, Br, J oder -X-R(10);

X O, S oder NR(11);

R(10) gleich H, $C_1$-$C_6$-Alkyl, $C_5$-$C_7$-Cycloalkyl, Cyclohexylmethyl, Cyclopentylmethyl oder -$C_nH_{2n}$-R(12);
n null bis 4;
R(12) Phenyl,

das unsubstituiert ist oder substituiert mit 1 - 3 Substituenten ausgewählt aus der Gruppe bestehend aus F, Cl, $CF_3$, Methyl, Methoxy und NR(8)R(9);
R(8) und R(9)

H oder $C_1$-$C_4$-Alkyl;

R(11) $C_1$-$C_3$-Alkyl, oder

R(10) und R(11)

gemeinsam 4 oder 5 Methylengruppen, von denen eine $CH_2$-Gruppe durch O, S, NH, N-$CH_3$ oder N-Benzyl ersetzt sein kann;

sowie deren pharmazeutisch verträgliche Salze;

(HOE 92/F 035 EP-OS 556 673)
c) Ortho-substituierte Benzoylguanidine der Formel I

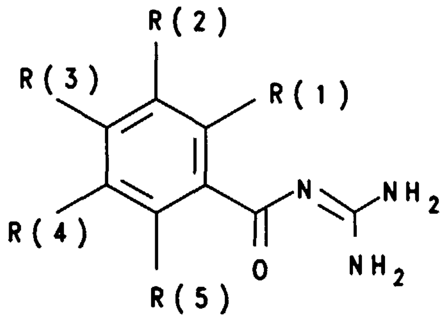

worin bedeuten:

R(1) F, Cl, Br, J, $C_1$-$C_6$-Alkyl oder

-X-R(6);

X O, S, NR(7) oder Y-ZO;

Y O oder NR(7);
Z C oder SO;

R(6) H, $C_1$-$C_6$-Alkyl, $C_5$-$C_7$-Cycloalkyl, Cyclohexylmethyl, Cyclopentylmethyl, $-(CH_2)_mC_pF_{2p+1}$ oder $-C_nH_{2n}$-R(8);

m Null oder 1;
p 1 - 3;
n Null bis 4;
R(8) Phenyl,

das unsubstituiert ist oder substituiert mit 1 - 3 Substituenten ausgewählt aus der Gruppe bestehend aus den Gruppen F, Cl, $CF_3$, Methyl, Methoxy und NR(9)R(10);
R(9) und R(10)

H oder $C_1$-$C_4$-Alkyl;

R(7) H oder $C_1$-$C_3$-Alkyl;

oder
R(6) und R(7)

gemeinsam 4 oder 5 Methylengruppen, von denen eine $CH_2$-Gruppe durch ein O, S, NH, $N$-$CH_3$ oder N-Benzyl ersetzt sein kann;

R(3) H oder -X-R(6);

X O, S, NR(7) oder Y-ZO;

R(7) H oder $C_1$-$C_3$-Alkyl;
Y O oder NR(7);

wobei Y am Phenylrest der Formel I gebunden ist,

Z C oder SO;

R(6) H, $C_1$-$C_6$-Alkyl, $C_5$-$C_7$-Cycloalkyl, Cyclohexylmethyl, Cyclopentylmethyl, $-(CH_2)_mC_pF_{2p+1}$ oder $-C_nH_{2n}$-R(8);

m null oder 1;
p 1 - 3;
n null bis 4;
R(8) Phenyl,

das unsubstituiert ist oder substituiert mit 1 - 3 Substituenten ausgewählt aus der Gruppe bestehend aus F, Cl, $CF_3$, Methyl, Methoxy und NR(9)R(10);
R(9) und R(10)

H oder $C_1$-$C_4$-Alkyl;

oder

R(6) und R(7)

gemeinsam 4 oder 5 Methylengruppen, von denen eine $CH_2$-Gruppe durch ein O, S, NH, N-$CH_3$ oder N-Benzyl ersetzt sein kann;

R(2) und R(4)

gleich oder verschieden R(11)-$SO_q$- oder R(12)R(13)N-$SO_2$-;
q null - 2;
R(11) $C_1$-$C_4$-Alkyl,

das unsubstituiert ist oder Phenyl als Substituenten trägt, wobei Phenyl unsubstituiert ist oder substituiert mit 1 - 3 Substituenten ausgewählt aus der Gruppe bestehend aus F, Cl, $CF_3$, Methyl, Methoxy und NR(9)R(10);
R(9) und R(10)

H oder $C_1$-$C_4$-Alkyl;

R(12) und R(13)

wie R(6) und R(7) definiert;

oder
einer der beiden Reste R(2) oder R(4)

Wasserstoff oder wie R(1) definiert;

R(5) H, Methyl, F, Cl oder Methoxy,
sowie deren pharmazeutisch verträgliche Salze.

(HOE 92/F 036 - US 5 364 868)
d) Benzoylguanidine der Formel I

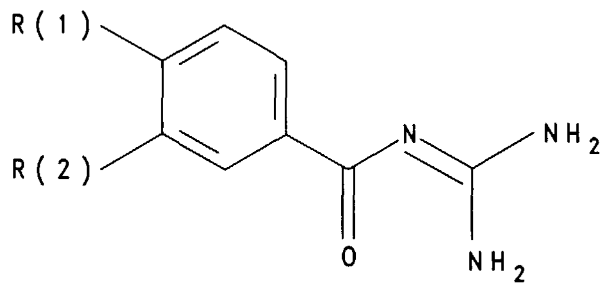

(I)

worin bedeuten:

R(1) oder R(2)

eine Aminogruppe -NR(3)R(4);
R(3) und R(4)

gleich oder verschieden H, $C_1$-$C_6$-Alkyl oder $C_3$-$C_7$-Cycloalkyl;

oder
R(3) Phenyl-$(CH_2)_p$-;

p 0, 1, 2, 3 oder 4;

oder
R(3) Phenyl,
wobei das Phenyl jeweils unsubstituiert ist oder einen bis zwei Substituenten ausgewählt aus der Gruppe bestehend aus Fluor, Chlor, Methyl und Methoxy trägt;
oder
R(3) und R(4)

gemeinsam eine geradkettige oder verzweigte $C_4$-$C_7$-Methylenkette sein können, wobei ein $-CH_2$-Glied der Methylenkette durch Sauerstoff, S oder NR(5) ersetzt sein kann;
R(5) H oder niedriges Alkyl;

der jeweils andere Substituent R(1) oder R(2)

H, F, Cl, $C_1$-$C_4$-Alkyl, $C_1$-$C_4$-Alkoxy, $CF_3$, $C_mF_{2m+1}$-$CH_2$-, Benzyl oder Phenoxy,

wobei der jeweilige Phenylrest unsubstituiert ist oder einen bis zwei Substituenten ausgewählt aus der Gruppe bestehend aus Methyl, Methoxy, Fluor und Chlor trägt;

m 1, 2 oder 3;

sowie deren pharmazeutisch verträgliche Salze;

(92/F 197 K - NZ 248 013)
e) Benzoylguanidine der Formel I

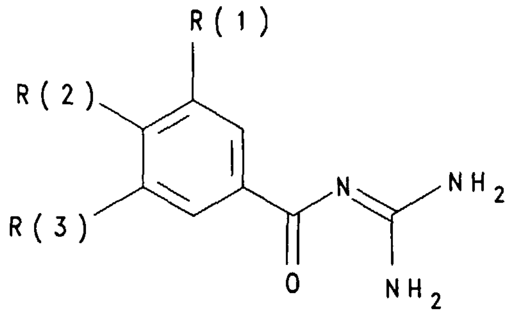

(I)

worin bedeuten:

R(1) R(4)-$SO_m$ oder R(5)R(6)N-$SO_2$-;

m Null, 1 oder 2;
R(4) und R(5)

$C_1$-$C_8$-Alkyl, $C_3$-$C_6$-Alkenyl oder -$C_nH_{2n}$-R(7);
n Null, 1, 2, 3 oder 4;
R(7) $C_5$-$C_7$-Cycloalkyl oder Phenyl,

welches unsubstituiert ist oder substituiert mit 1 - 3 Substituenten ausgewählt aus der Gruppe bestehend aus F, Cl, $CF_3$, Methyl, Methoxy und NR(8)R(9);
R(8) und R(9)

H oder $C_1$-$C_4$-Alkyl;

oder
R(5) H;
R(6) H oder $C_1$-$C_4$-Alkyl;
oder
R(5) und R(6)

gemeinsam 4 oder 5 Methylengruppen, von denen eine $CH_2$-Gruppe durch ein O, S, NH, N-$CH_3$ oder N-Benzyl ersetzt sein kann;

R(2) Wasserstoff, geradkettiges oder verzweigtes ($C_5$-$C_8$)-Alkyl, -CR(13)=CHR(12) oder -C≡-CR(12);

R(12) Phenyl,

das unsubstituiert oder substituiert ist mit 1 - 3 Substituenten ausgewählt aus der Gruppe bestehend aus F, Cl, $CF_3$, Methyl, Methoxy und NR(14)R(15);
R(14) und R(15)

H oder ($C_1$-$C_4$)-Alkyl;

oder
R(12) ($C_1$-$C_9$)-Heteroaryl,

das unsubstituiert oder wie Phenyl substituiert ist,

oder
R(12) ($C_1$-$C_6$)-Alkyl,

das unsubstituiert oder mit 1 - 3 OH substituiert ist,

oder

R(12) ($C_3$-$C_8$)-Cycloalkyl;

R(13) Wasserstoff oder Methyl,

oder

R(12) ($C_3$-$C_8$)-Cycloalkyl, ($C_3$-$C_8$)-Cycloalkyl-($C_1$-$C_4$)-alkyl, Phenyl, $C_6H_5$-($C_1$-$C_4$)-Alkyl, Naphthyl, Biphenylyl, 1,1-Diphenyl-($C_1$-$C_4$)-Alkyl, Cyclopentadienyl, Pyridyl, Pyrrolyl, Furanyl, Thienyl, Thiazolyl, Oxazolyl, Indenyl, Chinolyl, Indolyl, Benzofuranyl, Benzothienyl, Benzothiazolyl, Benzoxazolyl, Imidazolyl, Pyrazolyl, Triazolyl, Tetrazolyl, Isoxazolyl, Isothiazolyl, Pyrazinyl, Pyrimidinyl, Pyridazinyl, Indazolyl, Isochinolyl, Phthalazinyl, Chinoxalinyl, Chinazolinyl oder Cinnolinyl;

R(3) wie R(2) definiert;

und wobei die aromatischen Substituenten R(2) bzw. R(3) unsubstituiert oder mit 1-3 Substituenten aus den Gruppen F, Cl, $CF_3$, ($C_1$-$C_4$)-Alkyl, -Alkoxy, oder NR(10)R(11) mit R(10) und R(11) in der Bedeutung von H oder ($C_1$-$C_4$)-Alkyl substituiert sind;

sowie deren pharmazeutisch verträgliche Salze.

(HOE 92/F 303 K - EP-OS589 336, NZ 248 703)

f) Benzoylguanidine der Formel I

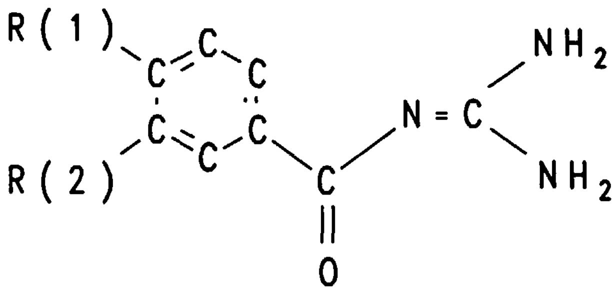

I

worin bedeuten:

R(1) oder R(2)

R(3)-S(O)$_n$- oder R(4)R(5)N-$SO_2$-

der jeweils andere Substituent R(1) oder R(2)

H, OH, F, Cl, Br, J, $C_1$-$C_4$-Alkyl, $C_1$-$C_4$-Alkoxy, Benzyloxy oder Phenoxy,

das unsubstituiert ist oder einen bis drei Substituenten ausgewählt aus der Gruppe bestehend aus Fluor, Chlor, Methyl, Methoxy, Hydroxy oder Benzyloxy trägt,

R(3)-S(O)$_n$, -NR4)R(5) oder 3,4-Dehydropiperidin

R(3) $C_1$-$C_6$-Alkyl, $C_5$-$C_7$-Cycloalkyl, Cyclopentylmethyl, Cyclohexylmethyl oder Phenyl,

das unsubstituiert ist oder einen bis drei Substituenten ausgewählt aus der Gruppe bestehend aus Fluor, Chlor, Methyl und Methoxy trägt;

R(4) und R(5)

gleich oder verschieden, H oder $C_1$-$C_6$-Alkyl;

oder

R(4) Phenyl-($CH_2$)$_m$-;

m 1, 2, 3 oder 4;

oder

R(4) Phenyl,

das unsubstituiert ist oder einen bis zwei Substituenten ausgewählt aus der Gruppe bestehend aus Fluor, Chlor, Methyl und Methoxy trägt;

oder

R(4) und R(5)

gemeinsam eine geradkettige oder verzweigte $C_4$-$C_7$-Kette, wobei die Kette zusätzlich durch O, S oder NR(6) unterbrochen sein kann,

R(6) H oder Methyl;

oder

R(4) und R(5)

gemeinsam mit dem Stickstoffatom, an das sie gebunden sind, ein Dihydroindol, Tetrahydrochinolin oder Tetrahydroisochinolin-System;

n Null, 1 oder 2;

sowie deren pharmazeutisch verträgliche Salze.

(92/F 304 - US 5 416 094)

g) Isochinoline der Formel I

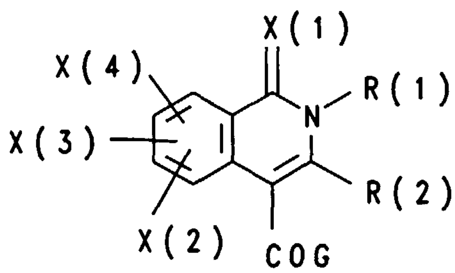

I

worin bedeuten:

R(1) Wasserstoff, Alkyl, Cycloalkyl, Arylalkyl, Alkenyl, substituiertes Aminoalkyl oder ein Aryl- oder Heteroarylring;

wobei die ringe unsubstituiert oder substituiert sind mit 1 - 3 Gruppen ausgewählt aus der Gruppe bestehend aus Halogen, Nitro, Amino, Mono(niederalkyl)amino, Di(niederalkyl)amino, Nieder alkyl, Niederalkoxy, Benzyloxy, Phenoxy, Hydroxy, Trifluoromethyl,

R(2) Wasserstoff, Halogen, Alkyl, oder Aryl;

welches unsubstituiert ist oder substituiert mit 1 - 3 Gruppen ausgewählt aus der Gruppe bestehend aus Halogen, Nitro, Amino, Mono(niederalkyl)amino, Di(niederalkyl)amino, Niederalkyl, Niederalkoxy, Benzyloxy, Phenoxy, Hydroxy,

G

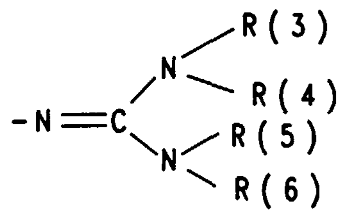

VII

X(2), X(3) and X(4)

unabhängig voneinander Wasserstoff, Halogen, Nitro, Amino, Alkyl, Sulfonamid, Mono(niederalkyl)amino, Di(niederalkyl)amino, Niederalkyl, Benzyloxy, Hydroxy;

X(1) Wasserstoff, Sauerstoff, Schwefel oder NR(7);

R(7) Wasserstoff, Alkyl, Cycloalkyl, Arylalkyl, Alkenyl, substituiertes Aminoalkyl oder ein aryl oder ein Heteroarylring;

welche ringe unsubstituiert oder substituiert sind mit 1 - 3 Gruppen ausgewählt aus der Gruppe bestehend aus Halogen, Nitro, Amino, Mono(niederalkyl)amino, Di(niederalkyl)amino, Niederalkyl, Niederalkoxy, Benzyloxy, Phenoxy, Hydroxy und Trifluoromethyl;

in welchen Substituenten jede Alkylkette oder Alkenylkette durch Sauerstoff, Schwefel oder NR(8) unterbrochen sein kann;
R(8) Wasserstoff, Alkyl, Cycloalkyl, Arylalkyl, Alkenyl,

substituiertes Aminoalkyl oder ein Aryl- oder Heteroarylring,

welche Ringe unsubstituiert oder substituiert sind mit 1 - 3 Gruppen ausgewählt aus der Gruppe bestehend aus Halogen, Nitro, Amino, Mono(niederalkyl)amino, Di(niederalkyl)amino, Niederalkyl, Niederalkoxy, Benzyloxy, Phenoxy, Hydroxy und Trifluoromethyl;

und ihre pharmazeutisch akzeptablen Salze;

92/F 404 - EP 602 522, NZ 250 438)
h) Verbindungen der Formel I

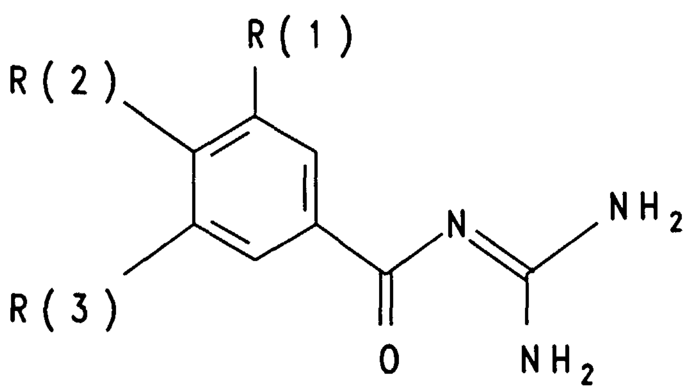

(I)

worin bedeuten:

R(1) Wasserstoff, F, Cl, Br, I, $-NO_2$, -C≡N, $-CF_3$, R(4)-$SO_m$ oder R(5)R(6)N-$SO_2$-;

m Null, 1 oder 2;

R(4) und R(5)

$(C_1-C_8)$-Alkyl, $(C_3-C_6)$-Alkenyl, $-C_nH_{2n}$-R(7) oder $CF_3$;

n Null, 1, 2, 3 oder 4;

R(7) $(C_3-C_7)$-Cycloalkyl oder Phenyl,

welches nicht substituiert ist oder substituiert mit 1 - 3 Substituenten ausgewählt aus der Gruppe bestehend aus F, Cl, $CF_3$, Methyl, Methoxy und NR(8)R(9);

R(8) und R(9)

H oder $C_1-C_4$-Alkyl;

oder

R(5) H;

R(6) H oder $(C_1-C_4)$-Alkyl;

oder

R(5) und R(6)

gemeinsam 4 oder 5 Methylengruppen, von denen eine $CH_2$-Gruppe durch Sauerstoff, S, NH, $N-CH_3$ oder N-Benzyl ersetzt sein kann;

R(2) -SR(10), -OR(10), -NHR(10), -NR(10)R(11), -CHR(10)R(12), -[CR(12)R(13)OR(13')], -{C-[$CH_2$-OR(13')]R(12)(R(13)} oder $-[CR(18)R(17)]_p$-(CO)-$[CR(19)R(20)]_q$-R(14);

R(10) und R(11)

gleich oder verschieden $-[CHR(16)]_s-(CH_2)_p-(CHOH)_q-(CH_2)_r-(CHOH)_t$-R(21) oder $-(CH_2)_p-O-(CH_2-CH_2O)_q$-R(21),

R(21) Wasserstoff, Methyl,

p, q, r gleich oder verschieden

Null, 1, 2, 3 oder 4;

s Null oder 1;

t 1, 2, 3 oder 4;

R(12) und R(13)

gleich oder verschieden Wasserstoff, $(C_1-C_6)$-Alkyl oder zusammen mit dem sie tragenden Kohlenstoff-Atom ein $(C_3-C_8)$-Cycloalkyl,

R(13') Wasserstoff oder $(C_1-C_4)$-Alkyl;

R(14) H, $(C_1-C_6)$-Alkyl, $(C_3-C_8)$-Cycloalkyl oder $-C_aH_{2a}$-R(15);

a Null, 1, 2, 3 oder 4;

R(15) Phenyl,

das unsubstituiert oder substituiert ist mit 1 - 3 Substituenten ausgewählt aus der Gruppe bestehend aus F, Cl, $CF_3$, Methyl, Methoxy und NR(8)R(9);

R(8) und R(9)

H oder $(C_1-C_4)$-Alkyl;

oder

R(15) $(C_1-C_9)$-Heteroaryl,

das unsubstituiert oder wie Phenyl substituiert ist,

oder
R(15) ($C_1$-$C_6$)-Alkyl,

das unsubstituiert oder mit 1 - 3 OH substituiert ist;

R(16), R(17), R(18), R(19) und R(20)

Wasserstoff oder ($C_1$-$C_3$)-Alkyl;

R(3) wie R(1) definiert,
oder
R(3) ($C_1$-$C_6$)-Alkyl oder -X-R(22);

X Sauerstoff, S oder NR(16);

R(16) H oder ($C_1$-$C_3$)-Alkyl;
oder
R(22) und R(16)

gemeinsam 4 oder 5 Methylengruppen, von denen eine $CH_2$-Gruppe durch Sauerstoff, S, NH, N-$CH_3$ oder N-Benzyl ersetzt sein kann;

R(22) wie R(14) definiert;

sowie deren pharmazeutisch verträgliche Salze.

(HOE 92/F 405 - EP 602 523, NZ 250 437)
i) Benzoylguanidine der Formel I

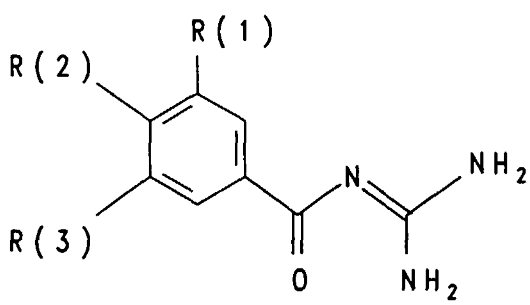

(I)

worin bedeuten:

R(1) Wasserstoff, F, Cl, Br, I, -$NO_2$, -C≡N, R(16)-$C_pH_{2p}$-$O_q$, R(4)-$SO_m$ oder R(5)R(6)N-$SO_2$-;

m Null, 1 oder 2;
p Null oder 1;
q Null, 1, 2 oder 3;
R(16) $C_rF_{2r+1}$;

r 1, 2 oder 3;

R(4) und R(5)

($C_1$-$C_8$)-Alkyl, ($C_3$-$C_6$)-Alkenyl, -$C_nH_{2n}$-R(7) oder $CF_3$;
n Null, 1, 2, 3 oder 4;

R(7) ($C_3$-$C_7$)-Cycloalkyl oder Phenyl,

welches nicht substituiert ist oder substituiert mit 1 - 3 Substituenten ausgewählt aus der Gruppe bestehend aus F, Cl, $CF_3$, Methyl, Methoxy und NR(8)R(9);
R(8) und R(9)

H oder $C_1$-$C_4$-Alkyl;

oder
R(5) H;
R(6) H oder ($C_1$-$C_4$)-Alkyl;
oder
R(5) und R(6)

gemeinsam 4 oder 5 Methylengruppen, von denen eine $CH_2$-Gruppe durch Sauerstoff, S, NH, N-$CH_3$ oder N-Benzyl ersetzt sein kann,

R(2) ($C_1$-$C_9$)-Heteroaryl,

das über C oder N verknüpft ist und das unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;

oder

R(2) -SR(10), -OR(10), -NR(10)R(11), -CR(10)R(11)R(12);

R(10) -$C_aH_{2a}$-($C_1$-$C_9$)-Heteroaryl,

das unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;

a Null, 1 oder 2;
R(11) und R(12)

unabhängig voneinander wie R(10) definiert oder Wasserstoff oder ($C_1$-$C_4$)-Alkyl;

R(3) wie R(1) definiert, oder ($C_1$-$C_6$)-Alkyl oder -X-R(13);

X Sauerstoff, S, oder NR(14);

R(14) H oder ($C_1$-$C_3$)-Alkyl;

R(13) H, ($C_1$-$C_6$)-Alkyl, ($C_3$-$C_8$)-Cycloalkyl oder -$C_bH_{2b}$-R(15);

b Null, 1, 2, 3 oder 4;

oder
R(13) und R(14)

gemeinsam 4 oder 5 Methylengruppen, von denen $CH_2$-Gruppe durch Sauerstoff, S, NH, N-$CH_3$ oder N-Benzyl ersetzt sein kann;
R(15) Phenyl,

das unsubstituiert oder substituiert ist mit 1 - 3 Substituenten ausgewählt aus der Gruppe bestehend aus F, Cl, $CF_3$, Methyl, Methoxy und NR(8)R(9);
R(8) und R(9)

H oder ($C_1$-$C_4$)-Alkyl;

sowie deren pharmazeutisch verträgliche Salze;

(HOE 92/F 411 - NZ 250 450, EP 603 650)
k) Benzoylguanidine der Formel I

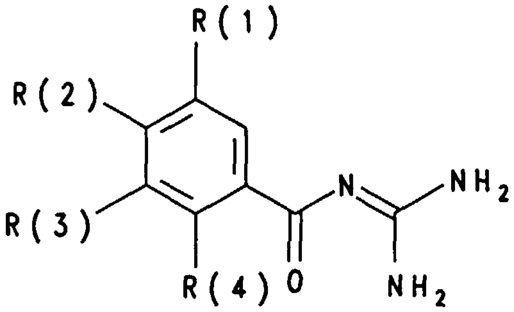

(I)

worin bedeuten:

einer der Substituenten R(1), R(2), R(3) oder R(4):

eine Aminogruppe

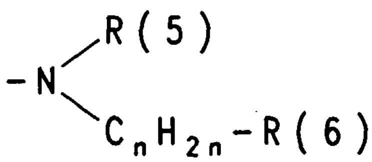

;

R(5) Wasserstoff oder $C_{(1-6)}$-Alkyl;
n Null, 1, 2, 3 oder 4;
R(6) H oder $C_{(1-4)}$-Alkyl;
worin eine $CH_2$-Gruppe durch 1 S-Atom oder eine Gruppe NR(7) ersetzt sein kann;
R(7) Wasserstoff, Methyl oder Ethyl;
oder
R(6) $C_{(3-8)}$-Cycloalkyl oder Phenyl,

das unsubstituiert ist oder 1, 2 oder 3 Substituenten ausgewählt aus der Gruppe bestehend aus F, Cl, Br, Methyl, Methoxy, -NR(8)R(9) trägt;
R(8) und R(9)

H, Methyl oder Ethyl;

oder
R(5) und R(6)

gemeinsam mit dem Stickstoffatom einen 5-, 6- oder 7-gliedrigen Ring, in welchem 1 C-Atom durch Sauerstoff, S oder NR(10) ersetzt sein kann;
R(10) H, $C_{(1-3)}$-Alkyl oder Benzyl;

und die jeweils anderen Substituenten R(1), R(2), R(3), R(4):

Wasserstoff, F, Cl, Br, I, CN, $CF_3$, $NO_2$, $CF_3$-O-, $C_mF_{2m+1}$-$CH_2$-O- oder R(11)-$C_qH_{2q}$-$X_p$-;
m 1, 2 oder 3;

q Null, 1, 2, 3 oder 4;
p Null oder 1;
X Sauerstoff oder NR(12);

R(12) H oder $C_{(1-3)}$-Alkyl;

R(11) Wasserstoff, $C_{(1-6)}$-Alkyl, $C_{(3-8)}$-Cycloalkyl oder Phenyl,

das unsubstituiert oder substituiert ist mit 1, 2 oder 3 Substituenten ausgewählt aus der Gruppe bestehend aus F, Cl, $CH_3$, $CH_3$-O- und NR(13)R(14);
R(13), R(14)

H, Methyl oder Ethyl;

sowie deren pharmazeutisch verträgliche Salze.

(HOE 92/F 422 - EP 604 852)
I) Benzoylguanidine der Formel I

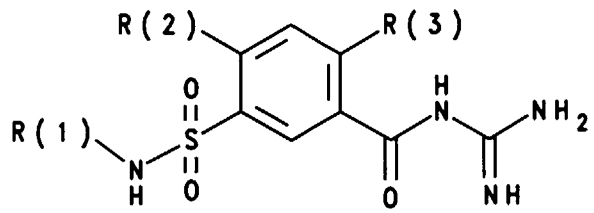

I

worin bedeuten

R(1) R(4)R(5)N-C(X)-;

X Sauerstoff, S oder N-R(6);
R(4) und R(5)

gleich oder verschieden, H, $(C_1-C_8)$-Alkyl, $(C_3-C_6)$-Alkenyl oder $-C_nH_{2n}$-R(7);
n Null, 1, 2, 3 oder 4;
R(7) $(C_5-C_7)$-Cycloalkyl oder Phenyl,

welches unsubstituiert ist oder substituiert mit 1 - 3 Substituenten ausgewählt aus der Gruppe bestehend aus F, Cl, $CF_3$, Methoxy und $(C_1-C_4)$-Alkyl;

oder
R(4) und R(5)

gemeinsam 4 oder 5 Methylengruppen, von denen eine $CH_2$-Gruppe durch Sauerstoff, S, NH, N-$CH_3$ oder N-Benzyl ersetzt sein kann; R(6) wie R(4) definiert oder gleich Amidin;

R(2) H, F, Cl, Br, I, $(C_1-C_8)$-Alkyl, 1-Alkenyl oder 1-Alkinyl, $(C_3-C_8)$-Cycloalkyl, $(C_3-C_8)$-Cycloalkyl-$(C_1-C_4)$-Alkyl, Phenyl, $C_6H_5$-$(C_1-C_4)$-Alkyl, Naphthyl, Biphenylyl, 1,1-Diphenyl-$(C_1-C_4)$-Alkyl, Cyclopentadienyl, Pyridyl, Thiopyridyl, Pyrrolyl, Furanyl, Thienyl, Thiazolyl, Oxazolyl, Indenyl, Chinolyl, Indolyl, Benzofuranyl, Benzothienyl, Benzothiazolyl, Benzoxazolyl oder -W-R(8);

W Sauerstoff, S oder NR(9);

R(8) H, $(C_1-C_6)$-Alkyl, $(C_5-C_7)$-Cycloalkyl, Cyclohexylmethyl, Cyclopentylmethyl, $-(CH_2)_mC_pF_{2p+1}$ oder $-C_qH_{2q}$-R(10);

m Null oder 1;
p 1, 2 oder 3;
q Null, 1, 2, 3 oder 4;
R(10) Phenyl,

das unsubstituiert oder substituiert ist mit 1 - 3 Substituenten ausgewählt aus der Gruppe bestehend aus F, Cl, $CF_3$, Methyl, Methoxy und NR(11)R(12);
R(11) und R(12)

H oder ($C_1$-$C_4$)-Alkyl;

R(9) H oder ($C_1$-$C_3$)-Alkyl;
oder
R(8) und R(9)

gemeinsam 4 oder 5 Methylengruppen, von denen eine $CH_2$-Gruppe durch Sauerstoff, S, NH, N-$CH_3$ oder N-Benzyl ersetzt sein kann;

R(3) H, F, Cl, Br, I, ($C_1$-$C_6$)-Alkyl oder -W-R(8) wie für R(2) definiert,
sowie deren pharmazeutisch akzeptable Salze;

(93/F 054 - NZ 250 919, EP-OS 612 723)
m) Benzoylguanidine der Formel I

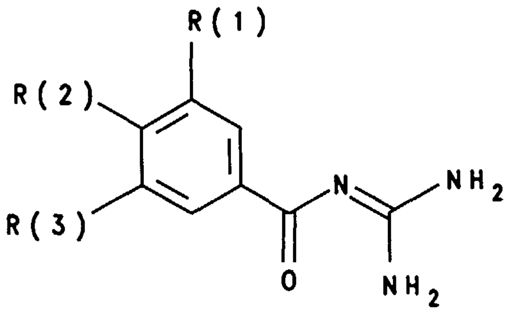

(I)

worin bedeuten:

R(1), R(2), R(3)

Wasserstoff, F, Cl, Br, I oder ($C_1$-$C_{12}$)-Alkyl;

einer der Substituenten R(1), R(2) oder R(3)

$N_3$, CN, OH oder ($C_1$-$C_{10}$)-Alkyloxy, wenn mindestens einer der restlichen Substituenten R(1), R(2) oder R(3) einen hinreichend lipophilen Alkylrest mit 3 bis 12 Kohlenstoff-Atomen bedeutet;

oder
einer der Substituenten R(1), R(2) oder R(3)

R(4)-$C_nH_{2n}$-$O_m$-;
m Null oder 1;
n Null, 1, 2 oder 3;
R(4) $C_pF_{2p+1}$;

p 1, 2 oder 3, sofern n gleich Null oder 1 ist;

oder

R(4) ($C_3$-$C_{12}$)-Cycloalkyl, Phenyl, Pyridyl, Chinolyl oder Isochinolyl, wobei

die aromatischen und heteroaromatischen Ringsysteme unsubstituiert sind oder substituiert mit einem Substituenten ausgewählt aus der Gruppe bestehend aus F, Cl, $CF_3$, Methyl, Methoxy und NR(5)R(6);

R(5) und R(6)

Wasserstoff oder ($C_1$-$C_4$)-Alkyl;

oder einer der Substituenten R(1), R(2) oder R(3)

-C≡CR(5) oder -C[R(6)]=CR(5);

R(5) Phenyl,

das unsubstituiert oder substituiert ist mit 1 - 3 Substituenten ausgewählt aus der Gruppe bestehend aus F, Cl, $CF_3$, Methyl, Methoxy, Hydroxy, Amino, Methylamino und Dimethylamino,

($C_1$-$C_9$)-Heteroaryl,

das unsubstituiert oder wie Phenyl substituiert ist,

oder

R(5) ($C_1$-$C_6$)-Alkyl,

das unsubstituiert oder mit 1 - 3 OH substituiert ist;

oder

R(5) ($C_3$-$C_8$)-Cycloalkyl,

R(6) Wasserstoff oder Methyl;

sowie deren pharmakologisch akzeptable Salze;

(93/F 153 - EP-OS 627 413, NZ 260 660)

o) Benzoylguanidine der Formel I

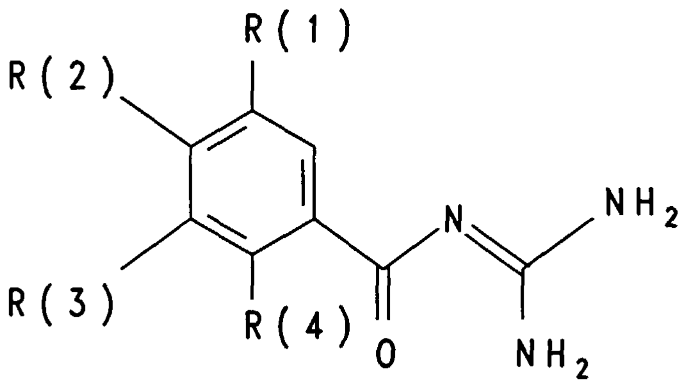

(I)

worin bedeuten:

R(1) Wasserstoff, F, Cl, Br, I, $-NO_2$, -C≡N, $X_o$-$(CH_2)_p$-$(CF_2)_q$-$CF_3$, R(5)-$SO_m$, R(6)-CO- oder R(6)R(7)N-$SO_2$-, mit

X Sauerstoff, S oder NR(14);

m Null, 1 oder 2;

o Null oder 1;

p Null, 1 oder 2;

q Null, 1, 2, 3, 4, 5 oder 6;

R(5) und R(6)

($C_1$-$C_8$)-Alkyl, ($C_3$-$C_6$)-Alkenyl, -$C_nH_{2n}$-R(8) oder $CF_3$;
n Null, 1, 2, 3 oder 4;
R(8) ($C_3$-$C_7$)-Cycloalkyl oder Phenyl,

welches nicht substituiert ist oder substituiert mit 1 - 3 Substituenten ausgewählt aus der Gruppe bestehend aus F, Cl, $CF_3$, Methyl, Methoxy und NR(9)R(10);
R(9) und R(10)

H oder $C_1$-$C_4$-Alkyl;

oder
R(6) H;
R(7) H oder ($C_1$-$C_4$)-Alkyl;
oder
R(6) und R(7)

gemeinsam 4 oder 5 Methylengruppen, von denen eine $CH_2$-Gruppe durch Sauerstoff, S, NH, N-$CH_3$ oder N-Benzyl ersetzt sein kann;

R(2)

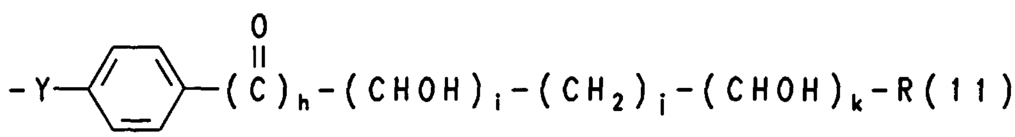

oder 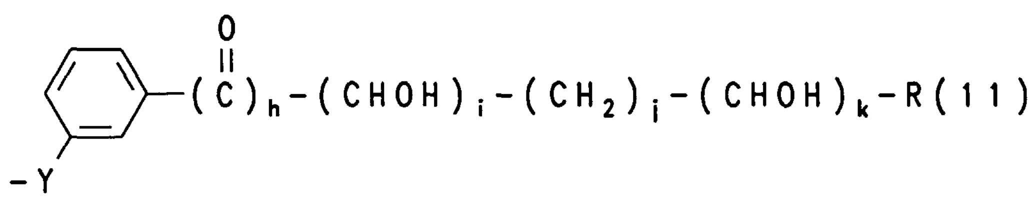

oder 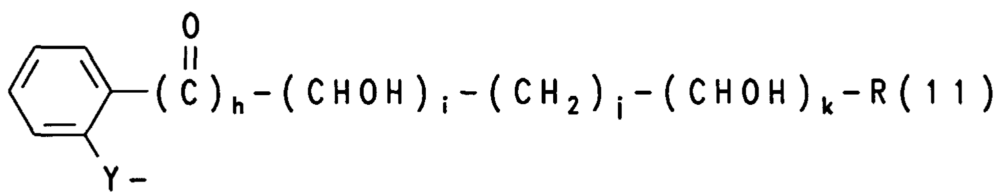

Y Sauerstoff, -S- oder -NR(12)-;
R(11) und R(12)

Wasserstoff oder ($C_1$-$C_3$)-Alkyl;

h Null oder 1;
i, j und k

unabhängig Null, 1, 2, 3 oder 4;

wobei jedoch nicht h, i und k gleichzeitig Null sind,

R(3) wie R(1) definiert, oder $(C_1-C_6)$-Alkyl oder -X-R(13);

X Sauerstoff, S oder NR(14);

R(14) H oder $(C_1-C_3)$-Alkyl;
R(13) H, $(C_1-C_6)$-Alkyl, $(C_3-C_8)$-Cycloalkyl oder $-C_bH_{2b}$-R(15);
b Null, 1, 2, 3 oder 4;
oder
R(13) und R(14)

gemeinsam 4 oder 5 Methylengruppen, wobei eine $CH_2$-Gruppe durch Sauerstoff, S, NH, $N-CH_3$ oder N-Benzyl ersetzt sein kann;
R(15) Phenyl,

das unsubstituiert oder substituiert ist mit 1 - 3 Substituenten ausgewählt aus der Gruppe bestehend aus F, Cl, $CF_3$, Methyl, Methoxy und NR(9)R(10);
R(9) und R(10)

H oder $(C_1-C_4)$-Alkyl;

R(4) Wasserstoff, -OR(16) oder -NR(16)R(17);

R(16) und R(17)

unabhängig Wasserstoff oder $(C_1-C_3)$-Alkyl;

sowie deren pharmazeutisch verträgliche Salze;

(HOE 93/F 154 - EP-OS 628 543, NZ 260 681)
p) Benzoylguanidine der Formel I

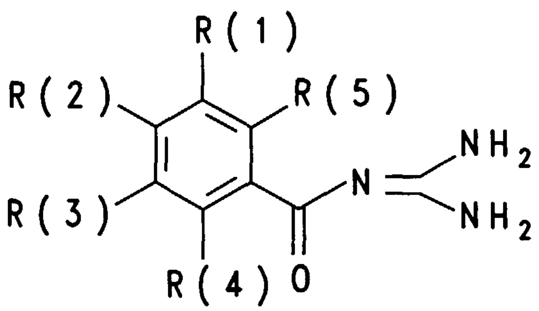

I

worin bedeuten:

R(1) R(6)-CO oder R(7)R(8)N-CO;

R(6) $(C_1-C_8)$-Alkyl, $(C_1-C_8)$-Perfluoralkyl, $(C_3-C_8)$-Alkenyl oder $-C_nH_{2n}$-R(9);

n Null, 1, 2, 3 oder 4;
R(9) $(C_3-C_8)$-Cycloalkyl, Phenyl, Biphenylyl oder Naphthyl,

wobei die Aromaten nicht substituiert oder substituiert sind mit 1 - 3 Substituenten ausgewählt aus der Gruppe bestehend aus F, Cl, $CF_3$, Methyl, Methoxy und NR(10)R(11);
R(10) und R(11)

H, $(C_1-C_4)$-Alkyl oder $(C_1-C_4)$-Perfuoralkyl;

R(7) H, ($C_1$-$C_8$)-Alkyl, ($C_1$-$C_8$)-Perfluoralkyl, ($C_3$-$C_8$)-Alkenyl oder - $C_nH_{2n}$-R(12);

n Null, 1, 2, 3 oder 4;
R(12) ($C_3$-$C_8$)-Cycloalkyl, Phenyl, Biphenylyl oder Naphthyl,

wobei die Aromaten nicht substituiert oder substituiert sind mit 1 - 3 Substituenten ausgewählt aus der Gruppe bestehend aus F, Cl, $CF_3$, Methyl, Methoxy und NR(13)R(14);
R(13) und R(14)

H, ($C_1$-$C_4$)-Alkyl oder ($C_1$-$C_4$)-Perfuoralkyl;

R(8) H, ($C_1$-$C_4$)-Alkyl oder ($C_1$-$C_4$)-Perfluoralkyl;
oder

R(7) und R(8)

gemeinsam 4 oder 5 Methylengruppen, von denen eine $CH_2$-Gruppe durch Sauerstoff, S, NH, N-$CH_3$ oder N-Benzyl ersetzt sein kann;

R(2) R(1) definiert, oder H, F, Cl, Br, I, CN, $NO_2$, ($C_1$-$C_8$)-Alkyl, ($C_1$-$C_8$)-Perfluoralkyl, ($C_3$-$C_8$)-Alkenyl oder -$C_nH_{2n}$R(15);

n Null, 1, 2, 3, 4;
R(15) ($C_3$-$C_8$)-Cycloalkyl, Phenyl, Biphenylyl oder Naphthyl,

wobei die Aromaten nicht substituiert oder substituiert sind mit 1 - 3 Substituenten ausgewählt aus der Gruppe bestehend aus F, Cl, $CF_3$, Methyl, Methoxy und NR(16)R(17);

R(16) und R(17)

H, ($C_1$-$C_4$)-Alkyl oder ($C_1$-$C_4$)-Perfuoralkyl;

oder
R(2) ($C_1$-$C_9$)-Heteroaryl,

das über C oder N verknüpft ist und das unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;

oder
R(2) SR(18), -OR(18), -NR(18)R(19), -CR(18)R(19)R(20);

R(18) -$C_aH_{2a}$-($C_1$-$C_9$)-Heteroaryl,

das unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;

a Null, 1 oder 2;
R(19) und R(20)

unabhängig voneinander wie R(18) definiert oder Wasserstoff, ($C_1$-$C_4$)-Alkyl oder ($C_1$-$C_4$)-Perfluoralkyl;

oder
R(2) R(21)-$SO_m$ oder R(22)R(23)N-$SO_2$-;

m 1 oder 2;
R(21) ($C_1$-$C_8$)-Alkyl, ($C_1$-$C_8$)-Perfluoralkyl, ($C_3$-$C_8$)-Alkenyl, -$C_nH_{2n}$-R(24),

n Null, 1, 2, 3 oder 4;

R(24) ($C_3$-$C_8$)-Cycloalkyl, Phenyl, Biphenylyl oder Naphthyl,

wobei die Aromaten nicht substituiert oder substituiert sind mit 1 - 3 Substituenten ausgewählt aus der Gruppe bestehend aus F, Cl, $CF_3$, Methyl, Methoxy und NR(27)R(28);
R(27) und R(28)

H, ($C_1$-$C_4$)-Alkyl oder ($C_1$-$C_4$)-Perfuoralkyl;

R(22) H, ($C_1$-$C_8$)-Alkyl, ($C_1$-$C_8$)-Perfluoralkyl, ($C_3$-$C_8$)-Alkenyl, -$C_nH_{2n}$-R(29);

n Null, 1, 2, 3 oder 4;
R(29) ($C_3$-$C_8$)-Cycloalkyl, Phenyl, Biphenylyl oder Naphthyl,

wobei die Aromaten nicht substituiert oder substituiert sind mit 1 - 3 Substituenten ausgewählt aus der Gruppe bestehend aus F, Cl, $CF_3$, Methyl, Methoxy und NR(30)R(31);
R(30) und R(31)

H, ($C_1$-$C_4$)-Alkyl oder ($C_1$-$C_4$)-Perfuoralkyl;

R(23) H, ($C_1$-$C_4$)-Alkyl oder ($C_1$-$C_4$)-Perfluoralkyl;
oder
R(22) und R(23)

gemeinsam 4 oder 5 Methylengruppen, von denen eine $CH_2$-Gruppe durch Sauerstoff, S, NH, N-$CH_3$ oder N-Benzyl ersetzt sein kann;

oder
R(2) R(33)X-;

X Sauerstoff, S, NR(34), (D=O)A-, NR(34)C=MN$^{(*)}$R(35)-;

M Sauerstoff oder S;
A Sauerstoff oder NR(34);
D C oder SO;

R(33) ($C_1$-$C_8$)-Alkyl, ($C_3$-$C_8$)-Alkenyl, $(CH_2)_bC_dF_{2d+1}$, -$C_nH_{2n}$-R(36),

b Null oder 1;
d 1, 2, 3, 4, 5, 6 oder 7;
n Null, 1, 2, 3, oder 4;
R(36) ($C_3$-$C_8$)-Cycloalkyl, Phenyl, Biphenylyl oder Naphthyl,

wobei die Aromaten nicht substituiert oder substituiert sind mit 1 - 3 Substituenten ausgewählt aus der Gruppe bestehend aus F, Cl, $CF_3$, Methyl, Methoxy und NR(37)R(38);
R(37) und R(38)

H, ($C_1$-$C_4$)-Alkyl oder ($C_1$-$C_4$)-Perfuoralkyl;

R(34) H, ($C_1$-$C_4$)-Alkyl oder ($C_1$-$C_4$)-Perfuoralkyl;
R(35) definiert wie R(33);
oder
R(33) und R(34)

gemeinsam 4 oder 5 Methylengruppen, von denen eine $CH_2$-Gruppe durch Sauerstoff, S, NH, N-$CH_3$ oder N-Benzyl ersetzt sein kann;

wobei A und N$^{(*)}$ an den Phenylkern des Benzoylguanidin-Grundkörpers gebunden sind;

oder

R(2) -SR(40), -OR(40), -NHR(40), -NR(40)R(41), -CHR(40)R(42), -C[R(42)R(43)OH], -C≡CR(45), -CR(46)=CHR(45), -[CR(47)R(48)]$_u$-(CO)-[CR49)R(50)]$_v$-R(44);

R(40), R(41)

gleich oder verschieden -$(CH_2)_p$-$(CHOH)_q$-$(CH_2)_r$-$(CHOH)_t$-R(51) oder - $(CH_2)_p$-O-$(CH_2$-$CH_2O)_q$-R(51);
R(51) Wasserstoff oder Methyl;

u 1, 2, 3 oder 4;
v Null, 1, 2, 3 oder 4;

p, q, r

gleich oder verschieden Null, 1, 2, 3 oder 4;

t 1, 2, 3 oder 4;

R(42) und R(43)

gleich oder verschieden Wasserstoff oder $(C_1$-$C_6)$-Alkyl;

oder
R(42) und R(43)

zusammen mit dem sie tragenden Kohlenstoff-Atom ein $(C_3$-$C_8)$-Cycloalkyl bilden;

R(44) H, $(C_1$-$C_6)$-Alkyl, $(C_3$-$C_8)$-Cycloalkyl oder -$C_eH_{2e}$-R(45);

e Null, 1, 2, 3 oder 4;
R(45) gleich Phenyl,

das unsubstituiert oder substituiert ist mit 1 - 3 Substituenten aus der Gruppe bestehend aus F, Cl, $CF_3$, Methyl, Methoxy und NR(52)R(53) mit

R(52) und R(53) gleich H oder $(C_1$-$C_4)$-Alkyl, oder

R(45) $(C_1$-$C_9)$-Heteroaryl,

das unsubstituiert oder wie Phenyl substituiert ist;

oder
R(45) $(C_1$-$C_6)$-Alkyl,

das unsubstituiert oder mit 1 - 3 OH substituiert ist;

R(46), R(47), R(48), R(49) und R(50)

Wasserstoff oder Methyl;

oder
R(2) R(55)-NH-$SO_2$-;

R(55) R(56)R(57)N-(C=Y)-;

Y Sauerstoff, S oder N-R(58);
R(56) und R(57)

gleich oder verschieden H, ($C_1$-$C_8$)-Alkyl, ($C_3$-$C_6$)-Alkenyl oder $-C_fH_{2f}$-R(59);
f Null, 1, 2, 3 oder 4;
R(59) ($C_5$-$C_7$)-Cycloalkyl oder Phenyl,

welches unsubstituiert ist oder substituiert mit 1 - 3 Substituenten ausgewählt aus der Gruppe bestehend aus F, Cl, $CF_3$, Methoxy und ($C_1$-$C_4$)-Alkyl;

oder
R(56) und R(57)

gemeinsam 4 oder 5 Methylengruppen, von denen eine $CH_2$-Gruppe durch Sauerstoff, S, NH, N-$CH_3$ oder N-Benzyl ersetzt sein kann;
R(58) wie R(56) definiert oder Amidin;

R(3), R(4) und R(5)

unabhängig voneinander wie R(1) oder R(2) definiert;

sowie deren pharmazeutisch verträgliche Salze;

(HOE 93/F 220 - EP-OS 640 593, NZ 264 117)
q) Benzoylguanidine der Formel I

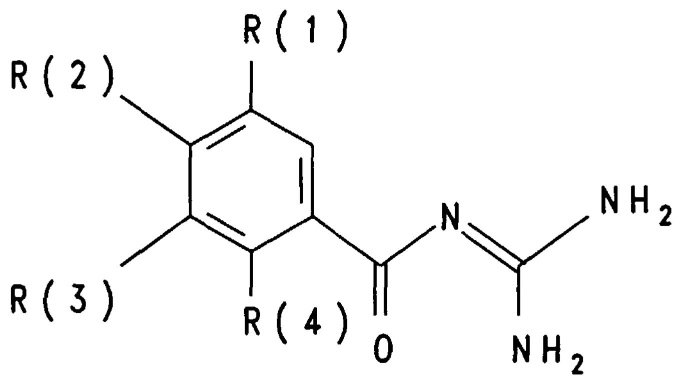

(I)

worin bedeuten:

R(1) Wasserstoff, F, Cl, Br, I, $-NO_2$, -C≡N, $-X_o-(CH_2)_p-(CF_2)_q-CF_3$, R(5)-$SO_m$-, R(6)-CO-, R(6)R(7)N-CO- oder R(6)R(7)N-$SO_2$-;

X Sauerstoff, -S- oder NR(14);
m Null, 1 oder 2;
o Null oder 1;
p Null, 1 oder 2;
q Null, 1, 2, 3, 4, 5 oder 6;
R(5) und R(6)

($C_1$-$C_8$)-Alkyl, ($C_3$-$C_6$)-Alkenyl, $-C_nH_{2n}$-R(8) oder $CF_3$;
n Null, 1, 2, 3 oder 4;
R(8) ($C_3$-$C_7$)-Cycloalkyl, Phenyl,

welches nicht substituiert ist oder substituiert mit 1 bis 3 Substituenten ausgewählt aus der Gruppe bestehend aus F, Cl, $CF_3$, Methyl, Methoxy und NR(9)R(10);
R(9) und R(10)

H oder ($C_1$-$C_4$)-Alkyl;

oder

R(6) Wasserstoff;

R(7) Wasserstoff oder $(C_1-C_4)$-Alkyl;

oder

R(6) und R(7)

gemeinsam 4 oder 5 Methylengruppen, von denen eine $CH_2$-Gruppe durch Sauerstoff, S, NH, $N-CH_3$ oder N-Benzyl ersetzt sein kann;

R(2)

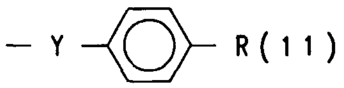 , 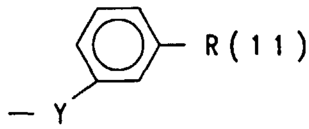 oder 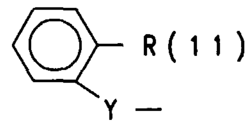

R(11) $(C_1-C_9)$-Heteroaryl,

das über C oder N verknüpft ist und das unsubstituiert oder substituiert ist mit 1 bis 3 Substituenten ausgewählt aus der Gruppe bestehend aus F, Cl, $CF_3$, $CH_3$, Methoxy, Hydroxy, Amino, Methylamino, Dimethylamino und Benzyl;

Y Sauerstoff, -S- oder NR(12);

R(12) H oder $(C_1-C_4)$-Alkyl;

R(3) wie R(1) definiert;

oder

R(3) $(C_1-C_6)$-Alkyl oder -X-R(13);

X Sauerstoff, -S- oder NR(14);

R(14) H oder $(C_1-C_3)$-Alkyl;

R(13) H, $(C_1-C_6)$-Alkyl, $(C_3-C_8)$-Cycloalkyl oder $-C_bH_{2b}$-R(15);

b Null, 1, 2, 3 oder 4;

oder

R(13) und R(14)

gemeinsam 4 oder 5 Methylengruppen, von denen eine $CH_2$-Gruppe durch Sauerstoff, S, NH, $N-CH_3$ oder N-Benzyl ersetzt sein kann;

R(15) Phenyl,

das unsubstituiert oder substituiert ist mit 1 - 3 Substituenten ausgewählt aus der Gruppe bestehend aus F, Cl, $CF_3$, Methyl, Methoxy und NR(9)R(10);

R(9) und R(10)

H oder $(C_1-C_4)$-Alkyl;

R(4) Wasserstoff, -OR(16), -NR(16)R(17) oder $C_rF_{2r+1}$;

R(16) und R(17)

unabhängig Wasserstoff oder $(C_1-C_3)$-Alkyl;

r 1, 2, 3 oder 4;

sowie deren pharmazeutisch verträgliche Salze;

(HOE 93/F 223 K - EP 639 573, NZ 264 130)
r) Benzokondensierte 5-Ringheterocyclen der Formel I

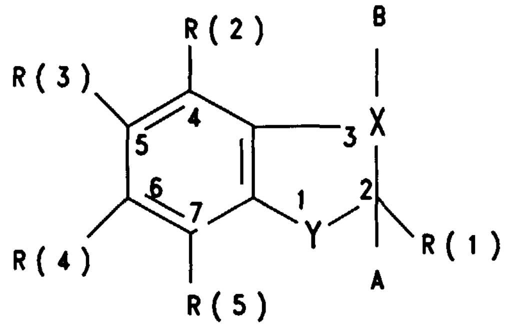

(I),

worin bedeuten:

X N oder CR(6);
Y Sauerstoff, S oder NR(7);
A, B gemeinsam eine Bindung
oder
A, B beide Wasserstoff, sofern gleichzeitig X gleich CR(6) und Y gleich NR(7) sind;
einer der Substituenten R(1) bis R(6) eine -CO-N=C$(NH_2)_2$-Gruppe;
die jeweils anderen Substituenten R(1) bis R(6)

Wasserstoff, F, Cl, Br, I oder ($C_1$-$C_6$)-Alkyl;

bis zu zwei der anderen Substituenten R(1) bis R(6)

CN, $NO_2$, $N_3$, ($C_1$-$C_4$)-Alkyloxy oder $CF_3$;

bis zu einem der anderen Substituenten

R(8)-$C_nH_{2n}$-Z-;
n Null bis 10;

wobei die Alkylenkette -$C_nH_{2n}$- geradkettig oder verzweigt ist und wobei ein C-Atom durch ein Sauerstoff- oder S-Atom oder durch ein N-Atom ersetzt sein kann;

R(8) Wasserstoff, ($C_2$-$C_6$)-Alkenyl oder ($C_3$-$C_{10}$)-Cycloalkyl,

das unsubstituiert oder durch 1 bis 4 Methylgruppen oder eine OH-Gruppe substituiert ist, oder eine Ethylengruppe - CH=CH- enthalten kann, und worin eine Methylengruppe durch ein Sauerstoff- oder S-Atom oder durch ein N-Atom ersetzt sein kann;

oder
R(8) Phenyl,

unsubstituiert oder substituiert durch 1 bis 3 Substituenten ausgewählt aus der Gruppe bestehend aus F, Cl, Br, I, $CF_3$, $CH_3$-S$(O)_s$- oder R(9)-$W_y$-;
s Null, 1 oder 2;
R(9) H, Methyl, Ethyl,
W Sauerstoff oder NR(10);

R(10) H oder Methyl;

y Null oder 1;

oder
R(8) $C_mF_{2m+1}$;

m 1 bis 3;

oder
R(8) 1- oder 2-Naphthyl, Pyridyl, Chinolyl oder Isochinolyl;

Z -CO-, $-CH_2-$ oder $-[CR(11)(OH)]_q-$;

q 1, 2 oder 3;
R(11) H oder Methyl;

oder
Z Sauerstoff oder -NR(12)-;

R(12) H oder Methyl;

oder
Z $-S(O)_s-$;

s Null, 1 oder 2;

oder
Z $-SO_2-NR(13)-$;

R(13) H oder $(C_1-C_4)$-Alkyl;

R(7) Wasserstoff, $(C_1-C_{10})$-Alkyl, $(C_2-C_{10})$-Alkenyl oder R(8)-$C_nH_{2n}$-;

sowie deren pharmazeutisch verträgliche Salze;

(HOE 93/F 236 - EP-OS 638 548, NZ 264 216)
s) Benzoylguanidine der Formel I

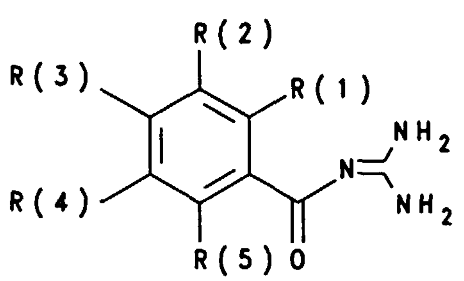

(I)

worin bedeuten:

R(1), R(3) oder R(4)

-NR(6) C=X NR(7)R(8);

X Sauerstoff oder S;
R(6) Wasserstoff, $(C_1-C_8)$-Alkyl, $(C_1-C_8)$-Perfluoralkyl, $(C_3-C_8)$-Alkenyl oder $-C_nH_{2n}$-R(9);

n Null, 1, 2, 3 oder 4;
R(9) ($C_3$-$C_8$)-Cycloalkyl, Phenyl, Biphenylyl oder Naphthyl,

wobei die Aromaten nicht substituiert oder substituiert sind mit 1 - 3 Substituenten ausgewählt aus der Gruppe bestehend aus F, Cl, $CF_3$, Methyl, Methoxy und NR(10)R(11);
R(10) und R(11)

H, ($C_1$-$C_4$)-Alkyl oder ($C_1$-$C_4$)-Perfluoralkyl;

R(7) Wasserstoff, ($C_1$-$C_8$)-Alkyl, ($C_1$-$C_8$)-Perfluoralkyl, ($C_3$-$C_8$)-Alkenyl oder -$C_oH_{2o}$-R(12);

o Null, 1, 2, 3 oder 4;
R(12) ($C_3$-$C_8$)-Cycloalkyl, Phenyl, Biphenylyl oder Naphthyl,

wobei die Aromaten nicht substituiert oder substituiert sind mit 1 - 3 Substituenten ausgewählt aus der Gruppe bestehend aus F, Cl, $CF_3$, Methyl, Methoxy und NR(13)R(14);
R(13) und R(14)

H, ($C_1$-$C_4$)-Alkyl oder ($C_1$-$C_4$)-Perfluoralkyl;

R(8) definiert wie R(7);
oder
R(7) und R(8)

gemeinsam 4 oder 5 Methylengruppen, von denen eine $CH_2$-Gruppe durch Sauerstoff, S, NH, N-$CH_3$ oder N-Benzyl ersetzt sein kann;

die jeweils verbleibenden Substituenten R(2), R(3), R(4), R(5) oder R(1), R(2), R(4), R(5) oder R(1), R(2), R(3), R(5)

unabhängig voneinander Wasserstoff, F, Cl, Br, I, -$O_{ta}$($C_1$-$C_8$)-Alkyl, -$O_{tb}$($C_3$-$C_8$)-Alkenyl, -$O_{tc}(CH_2)_bC_dF_{2d+1}$, -$O_{td}C_pH_{2p}$R(18),
oder bis zu 2 Gruppen CN, $NO_2$, NR(16)R(17),
b Null oder 1;
d 1, 2, 3, 4, 5, 6 oder 7;
ta Null oder 1;
tb Null oder 1;
tc Null oder 1;
td Null oder 1;
p Null, 1, 2, 3 oder 4;
R(18) ($C_3$-$C_8$)-Cycloalkyl, Phenyl, Biphenylyl oder Naphthyl,

wobei die Aromaten nicht substituiert oder substituiert sind mit 1 - 3 Substituenten ausgewählt aus der Gruppe bestehend aus F, Cl, $CF_3$, Methyl, Methoxy und NR(19)R(20);
R(19) und R(20)

Wasserstoff oder ($C_1$-$C_4$)-Alkyl oder ($C_1$-$C_4$)-Perfluoralkyl;

R(16) Wasserstoff, ($C_1$-$C_8$)-Alkyl, ($C_1$-$C_8$)-Perfluoralkyl, ($C_3$-$C_8$)-Alkenyl, - $C_qH_{2q}$-R(21),

q Null, 1, 2, 3 oder 4;
R(21) ($C_3$-$C_8$)-Cycloalkyl, Phenyl, Biphenylyl oder Naphthyl,

wobei die Aromaten nicht substituiert oder substituiert sind mit 1 - 3 Substituenten aus der Gruppe F, Cl, $CF_3$, Methyl, Methoxy oder NR(22)R(23),
R(22) und R(23) Wasserstoff, ($C_1$-$C_4$)-Alkyl oder ($C_1$-$C_4$)-Perfluoralkyl;

R(17) Wasserstoff, ($C_1$-$C_8$)-Alkyl, ($C_1$-$C_8$)-Perfluoralkyl, ($C_3$-$C_8$)-Alkenyl, - $C_rH_{2r}$-R(24);

r Null, 1, 2, 3 oder 4;
R(24) ($C_3$-$C_8$)-Cycloalkyl, Phenyl, Biphenylyl oder Naphthyl,

wobei die Aromaten nicht substituiert oder substituiert sind mit 1 - 3 Substituenten ausgewählt aus der Gruppe bestehend aus F, Cl, $CF_3$, Methyl, Methoxy und NR(25)R(26);
R(25) und R(26)

Wasserstoff, ($C_1$-$C_4$)-Alkyl oder ($C_1$-$C_4$)-Perfluoralkyl;

oder
R(16) und R(17)

gemeinsam 4 oder 5 Methylengruppen, von denen eine $CH_2$-Gruppe durch Sauerstoff, S, NH, N-$CH_3$ oder N-Benzyl ersetzt sein kann;

sowie deren pharmazeutisch verträgliche Salze;

(HOE 93/F 249 - EP-OS 640 587, NZ 264 282)
t) Diacylsubstituierte Guanidine der Formel I

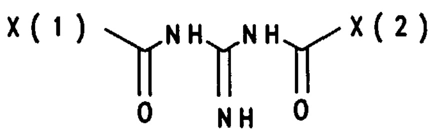

I

worin bedeuten:

X(1) und X(2)

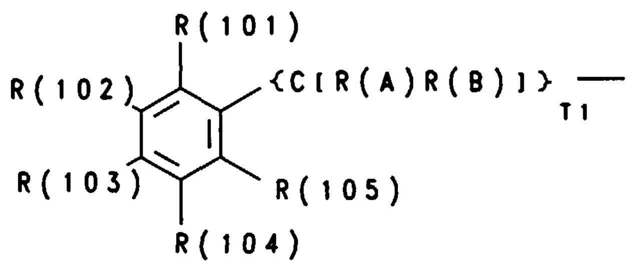

T1 Null, 1, 2, 3 oder 4;
R(A) und R(B)

unabhängig Wasserstoff, F, Cl, Br, I, CN, OR(106), ($C_1$-$C_8$)-Alkyl, ($C_3$-$C_8$)-Cycloalkyl, $O_{zk}(CH_2)_{zl}C_{zm}F_{2zm+1}$, NR(107)R(108), Phenyl oder Benzyl,

wobei die Aromaten nicht substituiert oder substituiert sind mit 1 - 3 Substituenten ausgewählt aus der Gruppe bestehend aus F, Cl, $CF_3$, Methyl, Methoxy und NR(109)R(110);
R(109) und R(110)

Wasserstoff, ($C_1$-$C_4$)-Alkyl oder ($C_1$-$C_4$)-Perfluoralkyl;

zl Null, 1, 2, 3 oder 4;
zk Null oder 1;
zm 1, 2, 3, 4, 5, 6, 7 oder 8;

R(106)

Wasserstoff, $(C_1-C_8)$-Alkyl, $(C_1-C_8)$-Perfluoralkyl, $(C_3-C_8)$-Alkenyl, $(C_3-C_8)$-Cycloalkyl, Phenyl oder Benzyl,

wobei die Aromaten nicht substituiert oder substituiert sind mit 1 - 3 Substituenten ausgewählt aus der Gruppe bestehend aus F, Cl, $CF_3$, Methyl, Methoxy und NR(111)R(112);
R(111) und R(112)

Wasserstoff, $(C_1-C_4)$-Alkyl oder $(C_1-C_4)$-Perfluoralkyl;

R(107) und R(108)

unabhängig voneinander wie R(106) definiert,

oder
R(107) und R(108)

gemeinsam 4 oder 5 Methylengruppen, von denen eine $CH_2$-Gruppe durch Sauerstoff, S, NH, N-$CH_3$ oder N-Benzyl ersetzt sein kann;

oder
X(1) und X(2)

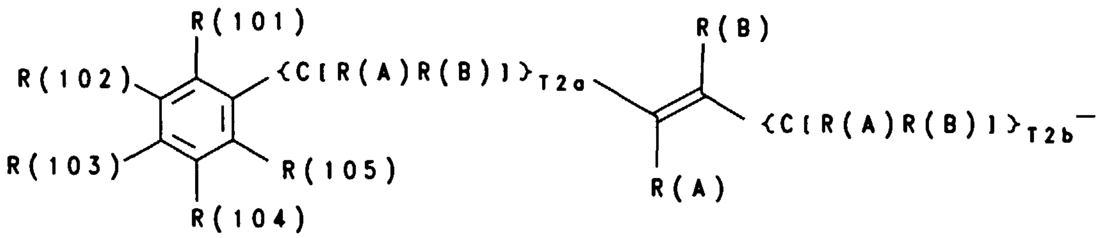

T2a und T2b

unabhängig voneinander Null, 1 oder 2; wobei die Doppelbindung (E)- oder (Z)-konfiguriert sein kann;

oder
X(1) und X(2)

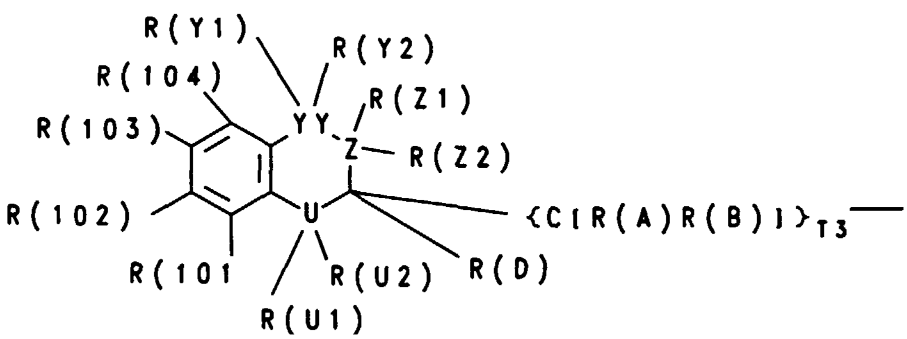

T3 Null, 1 oder 2;

U, YY und Z

unabhängig voneinander C oder N, wobei U, YY, Z folgende Anzahl von Substituenten tragen können:

| U, YY oder Z | im Ring an eine Doppelbindung gebunden | Anzahl der zugelassenen Substituenten |
|---|---|---|
| C | ja | 1 |
| C | nein | 2 |
| N | ja | 0 |
| N | nein | 1 |

R(D) Wasserstoff, $(C_1-C_8)$-Alkyl oder $(C_1-C_8)$-Perfluoralkyl,
R(U1), R(U2), R(Y1), R(Y2), R(Z1), R(Z2)

unabhängig voneinander Wasserstoff, F, Cl, Br, I, CN, OR(114), $(C_1-C_8)$-Alkyl, $(C_3-C_8)$-Cycloalkyl, $O_{zka}(CH_2)_{zla}C_{zma}F_{2zma+1}$, NR(115)R(116), Phenyl oder Benzyl,

wobei die Aromaten nicht substituiert oder substituiert sind mit 1 - 3 Substituenten ausgewählt aus der Gruppe bestehend aus F, Cl, $CF_3$, Methyl, Methoxy, NR(117)R(118),
R(117) und R(118)

Wasserstoff, $(C_1-C_4)$-Alkyl oder $(C_1-C_4)$-Perfluoralkyl,

zka Null oder 1;
zla Null, 1, 2, 3 oder 4;
zma 1, 2, 3, 4, 5, 6, 7 oder 8;
R(114)

Wasserstoff, $(C_1-C_8)$-Alkyl, $(C_1-C_8)$-Perfluoralkyl, $(C_3-C_8)$-Alkenyl, $(C_3-C_8)$-Cycloalkyl, Phenyl oder Benzyl,

wobei die Aromaten nicht substituiert oder substituiert sind mit 1 - 3 Substituenten ausgewählt aus der Gruppe bestehend aus F, Cl, $CF_3$, Methyl, Methoxy und NR(119)R(120);
R(119) und R(120)

Wasserstoff, $(C_1-C_4)$-Alkyl oder $(C_1-C_4)$-Perfluoralkyl;

R(115) und R(116) unabhängig voneinander wie R(114) definiert;
oder
R(115) und R(116)

gemeinsam 4 oder 5 Methylengruppen, von denen eine $CH_2$-Gruppe durch Sauerstoff, S, NH, N-$CH_3$ oder N-Benzyl ersetzt sein kann;

wobei jedoch die Konstitution U gleich Stickstoff (N), YY gleich Stickstoff (N) und Z gleich Kohlenstoff (C) ausgenommen ist,

R(101), R(102), R(103), R(104) und R(105)

unabhängig voneinander Wasserstoff, F, Cl, Br, I, -C≡N, $X_{zoa}$-$(CH_2)_{zpa}$-$(C_{zqa}F_{2zqa+1})$, R(110a)-$SO_{zbm}$, R(110b)R(110c)N-CO, R(111a)-CO- oder R(112a)R(113a)N-$SO_2$-,

wobei die Perfluoralkylgruppe geradkettig oder verzweigt ist,

X Sauerstoff, S oder NR(114a);

R(114a)

H oder ($C_1$-$C_3$)-Alkyl;

zoa Null oder 1;
zbm Null, 1 oder 2;
zpa Null, 1, 2, 3 oder 4;
zqa 1, 2, 3, 4, 5, 6, 7 oder 8;
R(110a), R(110b), R(111a) und R(112a)

unabhängig ($C_1$-$C_8$)-Alkyl, ($C_3$-$C_8$)-Alkenyl, -$C_{zn}H_{2zn}$-R(115a) oder ($C_1$-$C_8$)-Perfluoralkyl;
zn Null, 1, 2, 3 oder 4;
R(115a)

($C_3$-$C_8$)-Cycloalkyl, Phenyl, Biphenylyl oder Naphthyl,

wobei die Aromaten nicht substituiert sind oder substituiert mit 1 - 3 Substituenten ausgewählt aus der Gruppe bestehend aus F, Cl, $CF_3$, Methyl, Methoxy und NR(116a)R(117a);
R(116a) und R(117a)

Wasserstoff, ($C_1$-$C_4$)-Perfluoralkyl oder ($C_1$-$C_4$)-Alkyl;

oder
R(110b), R(111a) und R(112a)

Wasserstoff;

R(110c) und R(113a)

unabhängig Wasserstoff, ($C_1$-$C_4$)-Perfluoralkyl oder ($C_1$-$C_4$)-Alkyl;

oder
R(110b) und R(110c) sowie R(112a) und R(113a)

gemeinsam 4 oder 5 Methylengruppen, von denen eine $CH_2$-Gruppe durch Sauerstoff, Schwefel, NH, N-$CH_3$ oder N-Benzyl ersetzt sein kann;

oder
R(101), R(102), R(103), R(104), R(105)

unabhängig voneinander ($C_1$-$C_8$)-Alkyl, -$C_{zal}H_{2zal}$R(118a) oder ($C_3$-$C_8$)-Alkenyl,
zal Null, 1, 2, 3 oder 4;
R(118a)

($C_3$-$C_8$)-Cycloalkyl, Phenyl, Biphenylyl oder Naphthyl,

wobei die Aromaten nicht substituiert oder substituiert sind mit 1 - 3 Substituenten aus der Gruppe bestehend aus F, Cl, $CF_3$, Methyl, Methoxy oder NR(119a)R(119b);
R(119a) und R(119b)

Wasserstoff, ($C_1$-$C_4$)-Alkyl oder ($C_1$-$C_4$)-Perfluoralkyl;

oder
R(101), R(102), R(103), R(104), R(105)

unabhängig voneinander ($C_1$-$C_9$)-Heteroaryl,

das über C oder N verknüpft ist und das unsubstituiert oder substituiert ist mit 1 - 3 Substituenten aus der Gruppe bestehend aus F, Cl, $CF_3$, $CH_3$, Methoxy, Hydroxy, Amino, Methylamino und Dimethylamino;

oder
R(101), R(102), R(103), R(104), R(105)

unabhängig voneinander -C≡C-R(193);
R(193)

Phenyl, das nicht substituiert oder substituiert ist mit 1 - 3 Substituenten aus der Gruppe bestehend aus F, Cl, $CF_3$, Methyl, Methoxy oder NR(194)R(195);
R(194) und R(195)

Wasserstoff oder $CH_3$;

oder
R(101), R(102), R(103), R(104), R(105)

unabhängig voneinander -Y-para-$C_6H_4$-$(CO)_{zh}$-$(CHOH)_{zi}$-$(CH_2)_{zj}$-$(CHOH)_{zk}$-R(123), -Y-meta-$C_6H_4$-$(CO)_{zad}$-$(CHOH)_{zae}$-$(CH_2)_{zaf}$-$(CHOH)_{zag}$-R(124) oder
-Y-ortho-$C_6H_4$-$(CO)_{zah}$-$(CHOH)_{zao}$-$(CH_2)_{zap}$-$(CHOH)_{zak}$-R(125);
Y Sauerstoff, -S- oder -NR(122d)-;
zh, zad, zah

unabhängig Null oder 1;

zi, zj, zk, zae, zaf, zag, zao, zap und zak

unabhängig Null, 1, 2, 3 oder 4;

wobei jedoch jeweils

zh, zi und zk nicht gleichzeitig Null,
zad, zae und zag nicht gleichzeitig Null sowie
zah, zao und zak nicht gleichzeitig Null sind,

R(123), R(124) R(125) und R(122d)

unabhängig Wasserstoff oder ($C_1$-$C_3$)-Alkyl;

oder
R(101), R(102), R(103), R(104) und R(105)

unabhängig voneinander SR(129), -OR(130), -NR(131)R(132) oder -CR(133)R(134)R(135);
R(129), R(130), R(131) und R(133)

unabhängig -$C_{zab}H_{2zab}$-($C_1$-$C_9$)-Heteroaryl,

das unsubstituiert oder substituiert ist mit 1 - 3 Substituenten aus der Gruppe bestehend aus F, Cl, $CF_3$, $CH_3$, Methoxy, Hydroxy, Amino, Methylamino und Dimethylamino;

zab Null, 1 oder 2;

R(132), R(134) und R(135)

unabhängig voneinander wie R(129) definiert oder Wasserstoff, ($C_1$-$C_4$)-Alkyl oder ($C_1$-$C_4$)-Perfluoralkyl;

oder
R(101), R(102), R(103), R(104) und R(105)

unabhängig voneinander -W-para-($C_6H_4$)-R(196), -W-meta-($C_6H_4$)-R(197) oder -W-ortho-($C_6H_4$)-R(198);
R(196), R(197) und R(198)

unabhängig ($C_1$-$C_9$)-Heteroaryl,

das über C oder N verknüpft ist und das unsubstituiert oder substituiert ist mit 1 bis 3 Substituenten aus der Gruppe bestehend aus F, Cl, $CF_3$, $CH_3$, Methoxy, Hydroxy, Amino, Methylamino, Dimethylamino und Benzyl;

W Sauerstoff, S oder NR(136)-;

R(136)

Wasserstoff oder ($C_1$-$C_4$)-Alkyl;

oder
R(101), R(102), R(103), R(104) und R(105)

unabhängig voneinander R(146)X(1a)-;

X(1a)

Sauerstoff, S, NR(147), (D=O)A-, NR(148)C=MN$^{(*)}$R(149)-;
M Sauerstoff oder Schwefel;
A Sauerstoff oder NR(150);
D C oder SO;

R(146)

($C_1$-$C_8$)-Alkyl, ($C_3$-$C_8$)-Alkenyl, $(CH_2)_{zbz}C_{zdz}F_{2zdz+1}$ oder -$C_{zxa}H_{2zxa}$-R(151);
zbz Null oder 1;
zdz 1, 2, 3, 4, 5, 6 oder 7;
zxa Null, 1, 2, 3 oder 4;
R(151)

($C_3$-$C_8$)-Cycloalkyl, Phenyl, Biphenylyl oder Naphthyl,

wobei die Aromaten nicht substituiert oder substituiert sind mit 1 - 3 Substituenten aus der Gruppe bestehend aus F, Cl, $CF_3$, Methyl, Methoxy und NR(152)R(153);
R(152) und R(153)

Wasserstoff, ($C_1$-$C_4$)-Alkyl oder ($C_1$-$C_4$)-Perfluoralkyl;

R(147), R(148) und R(150)

unabhängig Wasserstoff, ($C_1$-$C_4$)-Alkyl, ($C_1$-$C_4$)-Perfluoralkyl;

R(149) definiert wie R(146),

oder

R(146) und R(147) beziehungsweise R(146) und R(148)

gemeinsam 4 oder 5 Methylengruppen, von denen eine $CH_2$-Gruppe durch Sauerstoff, Schwefel, NH, N-$CH_3$ oder N-Benzyl ersetzt sein kann;

wobei A und N(*) an den Phenylkern des Alkanoyl-Grundkörpers gebunden sind;

oder
R(101), R(102), R(103), R(104) und R(105)

unabhängig voneinander -SR(164), -OR(165), -NHR(166), -NR(167)R(168), -CHR(169)R(170), -CR(154)R(155)OH, -C≡CR(156), -CR(158)=CR(157) oder $-[CR(159)R(160)]_{zu}-(C=O)-[CR(161)R(162)]_{zv}-R(163)$;
R(164), R(165), R(166), R(167), R(169)

gleich oder verschieden $-(CH_2)_{zy}-(CHOH)_{zz}-(CH_2)_{zaa}-(CHOH)_{zt}-R(171)$ oder $-(CH_2)_{zab}-O-(CH_2-CH_2O)_{zac}-R(172)$;
R(171) und R(172)

Wasserstoff oder Methyl;

zu 1, 2, 3 oder 4;
zv Null, 1, 2, 3 oder 4;

zy, zz, zaa, zab, zac

gleich oder verschieden Null, 1, 2, 3 oder 4;

zt 1, 2, 3 oder 4;

R(168), R(170), R(154), R(155)

gleich oder verschieden Wasserstoff oder $(C_1-C_6)$-Alkyl,

oder
R(169) und R(170) beziehungsweise R(154) und R(155)

zusammen mit dem sie tragenden Kohlenstoff-Atom ein $(C_3-C_8)$-Cycloalkyl;

R(163)

Wasserstoff, $(C_1-C_6)$-Alkyl, $(C_3-C_8)$-Cycloalkyl oder $-C_{zeb}H_{2zeb}-R(173)$;
zeb Null, 1, 2, 3 oder 4;
R(156), R(157) und R(173)

unabhängig Phenyl, das unsubstituiert oder substituiert ist mit 1 - 3 Substituenten aus der Gruppe bestehend aus F, Cl, $CF_3$, Methyl, Methoxy und NR(174)R(175);
R(174) und R(175)

Wasserstoff oder $(C_1-C_4)$-Alkyl;

oder
R(156), R(157) und R(173)

unabhängig $(C_1-C_9)$-Heteroaryl,

das unsubstituiert oder wie Phenyl substituiert ist;

R(158), R(159), R(160), R(161) und R(162)

Wasserstoff oder Methyl,

oder

R(101), R(102), R(103), R(104), R(105)

unabhängig voneinander R(176)-NH-$SO_2$-;
R(176)

R(177)R(178)N-(C=Y')-;
Y' Sauerstoff, S oder N-R(179);
R(177) und R(178)

gleich oder verschieden Wasserstoff, ($C_1$-$C_8$)-Alkyl, ($C_3$-$C_6$)-Alkenyl oder -$C_{zfa}H_{2zfa}$-R(180);
zfa Null, 1, 2, 3 oder 4;
R(180)

($C_5$-$C_7$)-Cycloalkyl oder Phenyl,

welches unsubstituiert mit 1 - 3 Substituenten ausgewählt aus der Gruppe bestehend aus F, Cl, $CF_3$, Methoxy oder ($C_1$-$C_4$)-Alkyl;

oder
R(177) und R(178)

gemeinsam 4 oder 5 Methylengruppen, von denen eine $CH_2$-Gruppe durch Sauerstoff, Schwefel, NH, N-$CH_3$ oder N-Benzyl ersetzt sein kann;

R(179)

wie R(177) definiert oder gleich Amidin,

oder
R(101), R(102), R(103), R(104), R(105)

unabhängig voneinander NR(184a)R(185), OR(184b), SR(184c) oder -$C_{znx}H_{2znx}$-R(184d);
znx Null, 1, 2, 3 oder 4;
R(184d)

($C_3$-$C_7$)-Cycloalkyl oder Phenyl,

welches nicht substituiert ist oder substituiert mit 1 - 3 Substituenten aus der Gruppe bestehend aus F, Cl, $CF_3$, Methyl, Methoxy und NR(116k)R(117k);
R(116k) und R(117k)

Wasserstoff oder $C_1$-$C_4$-Alkyl;

R(184a), R(184b), R(184c), R(185)

unabhängig voneinander Wasserstoff, ($C_1$-$C_8$)-Alkyl, ($C_1$-$C_8$)-Perfluoralkyl oder $(CH_2)_{zao}$-R(184g);
zao Null, 1, 2, 3 oder 4;
184g

($C_3$-$C_7$)-Cycloalkyl oder Phenyl,

welches nicht substituiert ist oder substituiert mit 1 - 3 Substituenten aus der Gruppe bestehend aus F, Cl, $CF_3$, Methyl, Methoxy und NR(184u)R(184v);
R(184u) und R(184v)

Wasserstoff oder $C_1$-$C_4$-Alkyl;

oder

R(184a) und R(185)

gemeinsam 4 oder 5 Methylengruppen, von denen eine $CH_2$-Gruppe durch Sauerstoff, Schwefel, NH, N-$CH_3$ oder N-Benzyl ersetzt sein kann;

sowie deren pharmazeutisch verträgliche Salze;

(HOE 93/F 254 - EP-OS 640 588, NZ 264 307)
u) Benzoylguanidine der Formel I

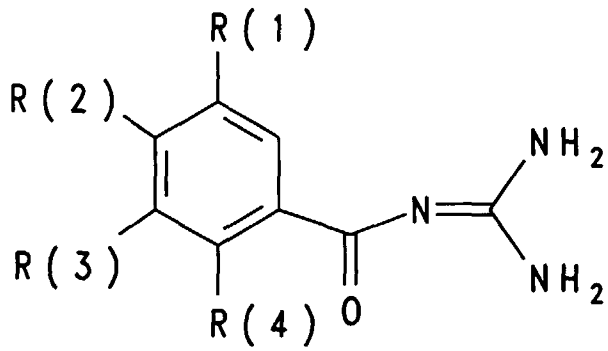

worin bedeuten:

R(1) H, F, Cl, Br, I, CN, $NO_2$, ($C_1$-$C_8$)-Alkyl, ($C_3$-$C_8$)-Cycloalkyl oder $X_a$-$(CH_2)_b$-$(CF_2)_c$-$CF_3$;

X Sauerstoff, S oder NR(5);
a Null oder 1;
b Null, 1 oder 2;
c Null, 1, 2 oder 3;

R(5) H, ($C_1$-$C_4$)-Alkyl oder -$C_dH_{2d}$R(6);

d Null, 1, 2, 3 oder 4;
R(6) ($C_3$-$C_8$)-Cycloalkyl, Phenyl, Biphenylyl oder Naphthyl,

wobei die Aromaten nicht substituiert oder substituiert sind mit 1 bis 3 Substituenten ausgewählt aus der Gruppe bestehend aus F, Cl, $CF_3$, Methyl, Methoxy und NR(7)R(8);
R(7) und R(8)

unabhängig H oder ($C_1$-$C_4$)-Alkyl;

oder
R(1) -SR(10), -OR(10) oder -CR(10)R(11)R(12);

R(10) -$C_fH_{2f}$-($C_3$-$C_8$)-Cycloalkyl, -($C_1$-$C_9$)-Heteroaryl oder Phenyl,

wobei die aromatischen Systeme unsubstituiert oder substituiert sind mit 1 bis 3 Substituenten ausgewählt aus der Gruppe bestehend aus F, Cl, $CF_3$, $CH_3$, Methoxy, Hydroxy, Amino, Methylamino und Dimethylamino;

f Null, 1 oder 2;
R(11) und R(12)

unabhängig voneinander wie R(10) definiert oder Wasserstoff oder ($C_1$-$C_4$)-Alkyl;

oder

R(1) Phenyl, Naphthyl, Biphenylyl oder ($C_1$-$C_9$)-Heteroaryl,

letzteres über C oder N verknüpft, und die unsubstituiert oder substituiert sind mit 1 bis 3 Substituenten ausgewählt aus der Gruppe bestehend aus F, Cl, $CF_3$, $CH_3$, Methoxy, Hydroxy, Amino, Methylamino und Dimethylamino;

oder

R(1) -SR(13), -OR(13), -NHR(13), -NR(13)R(14), -CHR(13)R(15), -C[R(15)R(16)]OH, -C≡CR(18), -C[R(19)]=CR(18), $-[CR(20)R(21)]_k$-(CO)-$[CR(22)R(23)R(24)]_l$

R(13) und R(14)

gleich oder verschieden $-(CH_2)_g-(CHOH)_h-(CH_2)_i-(CHOH)_j-R(17)$,
R(17) Wasserstoff oder Methyl;
$-(CH_2)_g-O-(CH_2-CH_2O)_h-R(24)$,
g, h, i

gleich oder verschieden Null, 1, 2, 3 oder 4;

j 1, 2, 3 oder 4;

R(15) und R(16)

gleich oder verschieden Wasserstoff, ($C_1$-$C_6$)-Alkyl oder zusammen mit dem sie tragenden Kohlenstoff-Atom ein ($C_3$-$C_8$)-Cycloalkyl;

R(18) Phenyl,

das unsubstituiert oder substituiert ist mit 1 bis 3 Substituenten ausgewählt aus der Gruppe bestehend aus F, Cl, $CF_3$, Methyl, Methoxy und NR(25)R(26);
R(25) und R(26)

H oder ($C_1$-$C_4$)-Alkyl;

oder

R(18) ($C_1$-$C_9$)-Heteroaryl,

das unsubstituiert oder wie Phenyl substituiert ist;

oder

R(18) ($C_1$-$C_6$)-Alkyl,

das unsubstituiert oder mit 1 bis 3 OH substituiert ist;

oder

R(18) ($C_3$-$C_8$)-Cycloalkyl;
R(19), R(20), R(21), R(22) und R(23)

Wasserstoff oder Methyl;

k Null, 1, 2, 3 oder 4;
l Null, 1, 2, 3 oder 4;

R(24) H, ($C_1$-$C_6$)-Alkyl, ($C_3$-$C_8$)-Cycloalkyl oder $-C_mH_{2m}-R(18)$;
m 1, 2, 3 oder 4;

R(2) und R(3)

unabhängig voneinander wie R(1) definiert;

R(4) $(C_1-C_3)$-Alkyl, F, Cl, Br, I, CN oder $-(CH_2)_n-(CF_2)_o-CF_3$;

n Null oder 1;
o Null, 1 oder 2;

sowie deren pharmazeutisch verträgliche Salze;

(HOE 93/F 436 - EP-OS 659 748), NZ 270 264)
v) Acylguanidine der Formel I

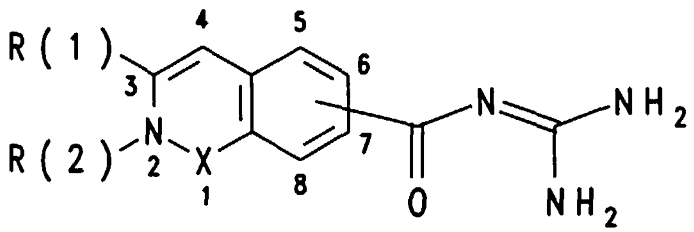

(I)

worin bedeuten:

X Carbonyl, Sulfonyl,

R(1) H, $(C_1-C_8)$-Alkyl,

unsubstituiert oder durch Hydroxy substituiert,

$(C_3-C_8)$-Cycloalkyl, Phenyl,

das unsubstituiert oder substituiert ist mit 1 - 3 Substituenten aus der Gruppe F, Cl, $CF_3$, $CH_3$, Methoxy, Hydroxy, Amino, Methylamino oder Dimethylamino,

R(2) H, $(C_1-C_4)$-Alkyl,
sowie deren pharmazeutisch verträgliche Salze;

(HOE 94/F 014 K - EP-OS 666 252, NZ 270 370)
w) Perfluoralkylgruppen tragende phenylsubstituierte Alkylcarbonsäure-guanidide der Formel I

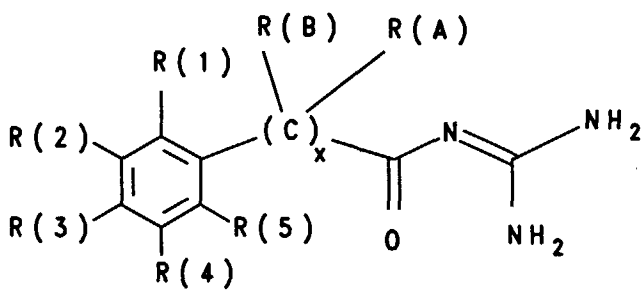

(I)

worin bedeuten:

R(A) Wasserstoff, F, Cl, Br, I, CN, OR(6), $(C_1-C_8)$-Alkyl, $(C_3-C_8)$-Cycloalkyl, $O_r(CH_2)_aC_bF_{2b+1}$ oder NR(7)R(8);

r Null oder 1;

a Null, 1, 2, 3 oder 4;
b 1, 2, 3, 4, 5, 6, 7 oder 8;
R(6) Wasserstoff, $(C_1-C_8)$-Alkyl, $(C_3-C_8)$-Alkenyl, $(C_3-C_8)$-Cycloalkyl, Phenyl oder Benzyl,

wobei die Aromaten nicht substituiert oder substituiert sind mit 1 - 3 Substituenten ausgwählt aus der Gruppe bestehend aus F, Cl, $CF_3$, Methyl, Methoxy und NR(9)R(10);
R(9) und R(10)

H, $(C_1-C_4)$-Alkyl oder $(C_1-C_4)$-Perfluoralkyl;

R(7) und R(8)

unabhängig voneinander wie R(6) definiert;

R(B) unabhängig wie R(A) definiert;
X 1, 2 oder 3;
R(1) Wasserstoff, $(C_1-C_8)$-Alkyl, $(C_3-C_8)$-Cycloalkyl, $-O_t(CH_2)_dC_eF_{2e+1}$, F, Cl, Br, I oder CN;

t Null oder 1;
d Null, 1, 2, 3 oder 4;
e 1, 2, 3, 4, 5, 6, 7 oder 8;

R(2), R(3), R(4) und R(5)

unabhängig voneinander wie R(1) definiert;

jedoch unter der Bedingung,
daß mindestens einer der Substituenten R(1), R(2), R(3), R(4), R(5), R(A) und R(B) eine $-O_t(CH_2)_dC_eF_{2e+1}$ oder eine $O_r(CH_2)_aC_bF_{2b+1}$-Gruppe ist,
sowie deren pharmazeutisch verträgliche Salze;

(HOE 94/F 094 - EP-OS 676 395, NZ 270 894)
x) Heteroaroylguanidine der Formel I

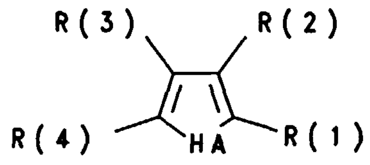

I

worin bedeuten:

HA $SO_m$, O oder NR(5);

m Null, 1 oder 2;
R(5) Wasserstoff, $(C_1-C_8)$-Alkyl oder $-C_{am}H_{2am}R(81)$;

am Null, 1 oder 2;
R(81) $(C_3-C_8)$-Cycloalkyl oder Phenyl,

welches nicht substituiert oder substituiert ist mit 1 - 3 Substituenten ausgewählt aus der Gruppe bestehend aus F, Cl, $CF_3$, Methyl, Methoxy und NR(82)R(83);
R(82) und R(83)

H oder $CH_3$;

oder

R(81) ($C_1$-$C_9$)-Heteroaryl,

das über C oder N verknüpft ist und das unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;

einer der beiden Substituenten R(1) und R(2)

$-CO-N=C(NH_2)_2$;

und der jeweils andere

Wasserstoff, F, Cl, Br, I, ($C_1$-$C_3$)-Alkyl, -OR(6), $C_rF_{2r+1}$, $-CO-N=C(NH_2)_2$ oder -NR(6)R(7);

R(6) und R(7)

unabhängig Wasserstoff oder ($C_1$-$C_3$)-Alkyl;

r 1, 2, 3 oder 4;

R(3) und R(4)

unabhängig voneinander Wasserstoff, F, Cl, Br, I, -C≡N, X-$(CH_2)_p$-($C_q$-$F_{2q+1}$), R(8)-$SO_{bm}$, R(9)R(10)N-CO, R(11)-CO- oder R(12)R(13)N-$SO_2$-,

wobei die Perfluoralkylgruppe geradkettig oder verzweigt ist,

X Sauerstoff, S oder NR(14);

R(14) H oder ($C_1$-$C_3$)-Alkyl;

bm Null, 1 oder 2;

p Null, 1 oder 2;

q Null, 1, 2, 3, 4, 5 oder 6;

R(8), R(9), R(11) und R(12)

unabhängig ($C_1$-$C_8$)-Alkyl, ($C_3$-$C_6$)-Alkenyl, $-C_nH_{2n}$-R(15), $CF_3$;

n Null, 1, 2, 3 oder 4;

R(15) ($C_3$-$C_7$)-Cycloalkyl oder Phenyl;

welches nicht substituiert ist oder substituiert mit 1 - 3 Substituenten ausgewählt aus der Gruppe bestehend aus F, Cl, $CF_3$, Methyl, Methoxy oder NR(16)R(17);

R(16) und R(17)

H oder $C_1$-$C_4$-Alkyl;

oder

R(9), R(11) und R(12)

H;

R(10) und R(13)

unabhängig H oder ($C_1$-$C_4$)-Alkyl;

oder

R(9) und R(10) sowie R(12) und R(13)

gemeinsam 4 oder 5 Methylengruppen, von denen eine $CH_2$-Gruppe durch Sauerstoff, S, NH, N-$CH_3$ oder N-Benzyl ersetzt sein kann,

oder
R(3) und R(4)

unabhängig voneinander ($C_1$-$C_8$)-Alkyl oder -$C_{al}H_{2al}$R(18);
al Null, 1 oder 2;
R(18) ($C_3$-$C_8$)-Cycloalkyl oder Phenyl;

welches nicht substituiert oder substituiert ist mit 1 - 3 Substituenten ausgewählt aus der Gruppe bestehend aus F, Cl, $CF_3$, Methyl, Methoxy und NR(19)R(20);
R(19) und R(20)

H oder $CH_3$;

oder
R(3) und R(4)

unabhängig voneinander ($C_1$-$C_9$)-Heteroaryl,

das über C oder N verknüpft ist und das unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;

oder
R(3) und R(4)

unabhängig voneinander

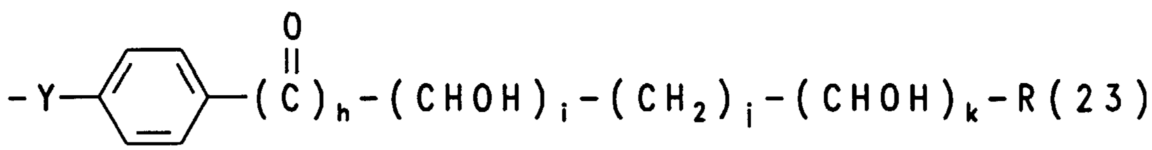

oder 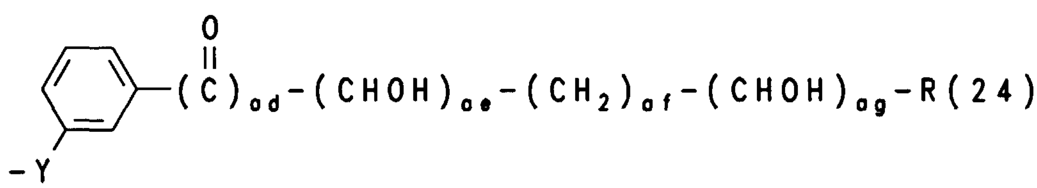

oder 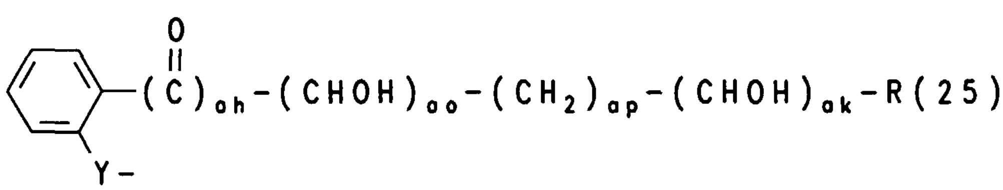

Y Sauerstoff, -S- oder -NR(22)-;
h, ad, ah unabhängig Null oder 1;
i, j, k, ae, af, ag, ao, ap und ak unabhängig Null, 1, 2, 3, 4,
wobei jedoch jeweils

h, i und k nicht gleichzeitig Null,
ad, ae und ag nicht gleichzeitig Null sowie
ah, ao und ak nicht gleichzeitig Null sind,

R(23), R(24) R(25) und R(22)

unabhängig Wasserstoff oder ($C_1$-$C_3$)-Alkyl;

oder
R(3) und R(4)

unabhängig voneinander Wasserstoff, F, Cl, Br, I, CN, ($C_1$-$C_8$)-Alkyl, ($C_1$-$C_8$)-Perfluoralkyl, ($C_3$-$C_8$)-Alkenyl oder -$C_gH_{2g}$R(26);
g Null, 1, 2, 3 oder 4;
R(26) ($C_3$-$C_8$)-Cycloalkyl, Phenyl, Biphenylyl oder Naphthyl,

wobei die Aromaten nicht substituiert oder substituiert sind mit 1 - 3 Substituenten ausgewählt aus der Gruppe bestehend aus F, Cl, $CF_3$, Methyl, Methoxy und NR(27)R(28);
R(27) und R(28)

H, ($C_1$-$C_4$)-Alkyl oder ($C_1$-$C_4$)-Perfluoralkyl;

oder
R(3) und R(4)

unabhängig voneinander SR(29), -OR(30), -NR(31)R(32) oder -CR(33)R(34)R(35);
R(29), R(30), R(31) und R(33)

unabhängig -$C_aH_{2a}$-($C_1$-$C_9$)-Heteroaryl,

das unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;

a Null, 1 oder 2;

R(32), R(34) und R(35)

unabhängig voneinander wie R(29) definiert oder Wasserstoff, ($C_1$-$C_4$)-Alkyl oder ($C_1$-$C_4$)-Perfluoralkyl;

oder
R(3) und R(4)

unabhängig voneinander

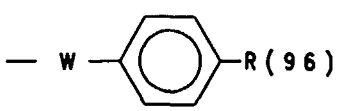 , 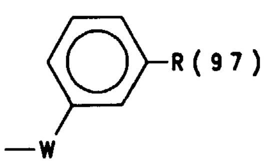 oder 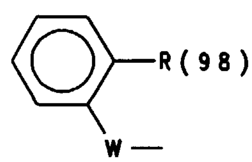

R(96), R(97) und R(98)

unabhängig ($C_1$-$C_9$)-Heteroaryl,

das über C oder N verknüpft ist und das unsubstituiert oder substituiert ist mit 1 bis 3 Substituenten

ausgewählt aus der Gruppe bestehend aus F, Cl, $CF_3$, $CH_3$, Methoxy, Hydroxy, Amino, Methylamino, Dimethylamino oder Benzyl;

W Sauerstoff, S oder NR(36)-;

R(36) H oder ($C_1$-$C_4$)-Alkyl;

oder
R(3) und R(4)

unabhängig voneinander R(37)-$SO_{cm}$ oder R(38)R(39)N-$SO_2$-;
cm 1 oder 2;
R(37) ($C_1$-$C_8$)-Alkyl, ($C_1$-$C_8$)-Perfluoralkyl, ($C_3$-$C_8$)-Alkenyl oder - $C_sH_{2s}$R(40);

s Null, 1, 2, 3 oder 4;
R(40) ($C_3$-$C_8$)-Cycloalkyl, Phenyl, Biphenylyl oder Naphthyl,

wobei die Aromaten nicht substituiert oder substituiert sind mit 1 - 3 Substituenten ausgewählt aus der Gruppe bestehend aus F, Cl, $CF_3$, Methyl, Methoxy und NR(41)R(42);
R(41) und R(42)

H, ($C_1$-$C_4$)-Alkyl oder ($C_1$-$C_4$)-Perfluoralkyl;

R(38) H, ($C_1$-$C_8$)-Alkyl, ($C_1$-$C_8$)-Perfluoralkyl, ($C_3$-$C_8$)-Alkenyl oder - $C_wH_{2w}$-R(43);

w Null, 1, 2, 3 oder 4;
R(43) ($C_3$-$C_8$)-Cycloalkyl, Phenyl, Biphenylyl oder Naphthyl,

wobei die Aromaten nicht substituiert oder substituiert sind mit 1 - 3 Substituenten ausgewählt aus der Gruppe bestehend aus F, Cl, $CF_3$, Methyl, Methoxy und NR(44)R(45);
R(44) und R(45)

H, ($C_1$-$C_4$)-Alkyl oder ($C_1$-$C_4$)-Perfluoralkyl;

R(39) H, ($C_1$-$C_4$)-Alkyl oder ($C_1$-$C_4$)-Perfluoralkyl;
oder
R(38) und R(39)

gemeinsam 4 oder 5 Methylengruppen, von denen eine $CH_2$-Gruppe durch Sauerstoff, S, NH, N-$CH_3$ oder N-Benzyl ersetzt sein kann;

oder
R(3) und R(4)

unabhängig voneinander R(46)X(1)-;
X(1) Sauerstoff, S, NR(47), (D=O)A-, NR(48)C =MN$^{(*)}$R(49)-,

M Sauerstoff oder S;
A Sauerstoff oder NR(50);
D C oder SO;

R(46) ($C_1$-$C_8$)-Alkyl, ($C_3$-$C_8$)-Alkenyl, $(CH_2)_bC_dF_{2d+1}$ oder -$C_xH_{2x}$-R(51);

b Null oder 1;
d 1, 2, 3, 4, 5, 6 oder 7;
x Null, 1, 2, 3 oder 4;
R(51) ($C_3$-$C_8$)-Cycloalkyl, Phenyl, Biphenylyl oder Naphthyl,

wobei die Aromaten nicht substituiert oder substituiert sind mit 1 - 3 Substituenten ausgewählt aus der Gruppe bestehend aus F, Cl, $CF_3$, Methyl, Methoxy und NR(52)R(53);
R(52) und R(53)

H, $(C_1-C_4)$-Alkyl oder $(C_1-C_4)$-Perfluoralkyl;

R(47), R(48) und R(50)

unabhängig Wasserstoff, $(C_1-C_4)$-Alkyl oder $(C_1-C_4)$-Perfluoralkyl;

R(49) definiert wie R(46);
oder
R(46) und R(47) beziehungsweise R(46) und R(48)

gemeinsam 4 oder 5 Methylengruppen, von denen eine $CH_2$-Gruppe durch Sauerstoff, S, NH, N-$CH_3$ oder N-Benzyl ersetzt sein kann,

wobei A und $N^{(*)}$ an den Phenylkern des Benzoylguanidin-Grundkörpers gebunden sind;

oder
R(3) und R(4)

unabhängig voneinander -SR(64), -OR(65), -NHR(66), -NR(67)R(68), -CHR(69)R(70), -C(OH)R(54)R(55), -C≡CR(56), -CR(58)=CHR(57), $-[CR(59)R(60)]_u-(CO)-[CR(61)R(62)]_v-R(63)$;

R(64), R(65), R(66), R(67) und R(69)

gleich oder verschieden $-(CH_2)_y-(CHOH)_z-(CH_2)_{aa}-(CH_2OH)_t-R(71)$ oder $-(CH_2)_{ab}-O-(CH_2-CH_2O)_{ac}-R(72)$,
R(71) und R(72)

Wasserstoff oder Methyl;

u 1, 2, 3 oder 4;

v Null, 1, 2, 3 oder 4;

y, z, aa

gleich oder verschieden Null, 1, 2, 3 oder 4;

t 1, 2, 3 oder 4;

R(68), R(70), R(54) und R(55)

gleich oder verschieden Wasserstoff, $(C_1-C_6)$-Alkyl;

oder

R(69) und R(70) beziehungsweise R(54) und R(55)

zusammen mit dem sie tragenden Kohlenstoff-Atom ein $(C_3-C_8)$-Cycloalkyl;

R(63)

H, $(C_1-C_6)$-Alkyl, $(C_3-C_8)$-Cycloalkyl oder $-C_eH_{2e}-R(73)$;
e Null, 1, 2, 3 oder 4;

R(56), R(57) und R(73)

unabhängig Phenyl,

das unsubstituiert oder substituiert ist mit 1 - 3 Substituenten ausgewählt aus der Gruppe bestehend aus F, Cl, $CF_3$, Methyl, Methoxy und NR(74)R(75);
R(74) und R(75)

H oder ($C_1$-$C_4$)-Alkyl;

oder

R(56), R(57) und R(73)

unabhängig ($C_1$-$C_9$)-Heteroaryl,

das unsubstituiert oder wie Phenyl substituiert ist;

R(58), R(59), R(60), R(61) und R(62)

Wasserstoff oder Methyl,

oder
R(3) und R(4)

unabhängig voneinander R(76)-NH-$SO_2$-;
R(76) R(77)R(78)N-(C=Y')-;
Y' Sauerstoff, S oder N-R(79);
R(77) und R(78)

gleich oder verschieden H, ($C_1$-$C_8$)-Alkyl, ($C_3$-$C_6$)-Alkenyl, -$C_fH_{2f}$-R(80);
f Null, 1, 2, 3 oder 4;
R(80) ($C_5$-$C_7$)-Cycloalkyl oder Phenyl,

welches unsubstituiert oder substituiert mit 1 - 3 Substituenten ausgewählt aus der Gruppe bestehend aus F, Cl, $CF_3$, Methoxy und ($C_1$-$C_4$)-Alkyl;

oder
R(77) und R(78)

gemeinsam 4 oder 5 Methylengruppen, von denen eine $CH_2$-Gruppe durch Sauerstoff, S, NH, N-$CH_3$ oder N-Benzyl ersetzt sein kann, R(79)

wie R(77) definiert oder gleich Amidin;

oder
R(3) und R(4)

unabhängig voneinander NR(84)R(85);
R(84) und R(85)

unabhängig voneinander H, ($C_1$-$C_4$)-Alkyl, oder gemeinsam 4 oder 5 Methylengruppen,

von denen eine $CH_2$-Gruppe durch Sauerstoff, S, NH, N-$CH_3$ oder N-Benzyl ersetzt sein kann; oder von denen eine oder zwei $CH_2$-Gruppen durch CH-$C_{dm}H_{2dm+1}$ ersetzt sein können,

sowie deren pharmazeutisch verträgliche Salze;

(HOE 94/F 123 - EP-OS 682 017, NZ 272 058)
y) Bizyklische Heteroaroylguanidine der Formel I

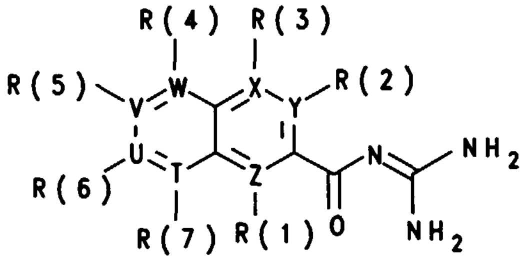

I

worin bedeuten:

T, U, V, W, X, Y und Z

unabhängig voneinander Stickstoff oder Kohlenstoff;
jedoch mit der Einschränkung,
daß X und Z nicht gleichzeitig Stickstoff sind,
und daß T, U, V, W, X, Y und Z keinen Substituenten tragen, wenn sie Stickstoff sind,
und daß nicht mehr als vier von ihnen gleichzeitig Stickstoff sind,

R(1) und R(2)

unabhängig voneinander Wasserstoff, F, Cl, Br, I, ($C_1$-$C_3$)-Alkyl, ($C_1$-$C_3$)-Perfluoralkyl, OR(8), NR(8)R(9) oder $C(=O)N=C(NH_2)_2$;
R(8) und R(9)

unabhängig voneinander Wasserstoff oder ($C_1$-$C_3$)-Alkyl,

oder
R(8) und R(9)

gemeinsam 4 oder 5 Methylengruppen, von denen eine $CH_2$-Gruppe durch Sauerstoff, S, NH, N-$CH_3$ oder N-Benzyl ersetzt sein kann;

R(3), R(4), R(5), R(6) und R(7)

unabhängig voneinander Wasserstoff, F, Cl, Br, I, -C≡N, $X_k$-$(CH_2)_p$-$(C_qF_{2q+1})$, R(10a)-$SO_{bm}$, R(10b)R(10c)N-CO, R(11)-CO- oder R(12)R(13)N-$SO_2$-,

wobei die Perfluoralkylgruppe geradkettig oder verzweigt ist;

X Sauerstoff, S oder NR(14);

R(14) H oder $C_1$-$C_3$)-Alkyl;

bm Null, 1 oder 2;
p Null, 1 oder 2;
k Null oder 1;
q 1, 2, 3, 4, 5 oder 6;
R(10a), R(10b), R(11) und R(12)

unabhängig voneinander($C_1$-$C_8$)-Alkyl, ($C_3$-$C_6$)-Alkenyl, -$C_nH_{2n}$-R(15) oder ($C_1$-$C_8$)-Perfluoralkyl;
n Null, 1, 2, 3 oder 4;
R(15) ($C_3$-$C_7$)-Cycloalkyl oder Phenyl, welches nicht substituiert ist oder substituiert mit 1 - 3 Substituenten ausgewählt aus der Gruppe bestehend aus F, Cl, $CF_3$, Methyl, Methoxy und NR(16)R(17);

R(16) und R(17)

H oder $C_1$-$C_4$-Alkyl;

oder
R(10b), R(11) und R(12)

Wasserstoff;

R(10c) und R(13)

unabhängig Wasserstoff oder ($C_1$-$C_4$)-Alkyl;

oder
R(10b) und R(10c) sowie R(12) und R(13)

gemeinsam 4 oder 5 Methylengruppen, von denen eine $CH_2$-Gruppe durch Sauerstoff, Schwefel, NH, N-$CH_3$ oder N-Benzyl ersetzt sein kann;

oder
R(3), R(4), R(5), R(6) und R(7)

unabhängig voneinander ($C_1$-$C_8$)-Alkyl, -$C_{al}H_{2al}$R(18) oder ($C_3$-$C_8$)-Alkenyl;
al Null, 1 oder 2;
R(18) ($C_3$-$C_8$)-Cycloalkyl, Phenyl, Biphenylyl oder Naphthyl, wobei die Aromaten nicht substituiert oder substituiert sind mit 1 - 3 Substituenten ausgewählt aus der Gruppe bestehend aus F, Cl, $CF_3$, Methyl, Methoxy und NR(19a)R(19b);

R(19a) und R(19b)

Wasserstoff, ($C_1$-$C_4$)-Alkyl oder ($C_1$-$C_4$)-Perfluoralkyl;

oder
R(3), R(4), R(5), R(6) und R(7)

unabhängig voneinander ($C_1$-$C_9$)-Heteroaryl, das über C oder N verknüpft ist und das unsubstituiert oder substituiert ist mit 1 - 3 Substituenten aus der Gruppe bestehend aus F, Cl, $CF_3$, $CH_3$, Methoxy, Hydroxy, Amino, Methylamino oder Dimethylamino;

oder
R(3), R(4), R(5), R(6) und R(7)

unabhängig voneinander

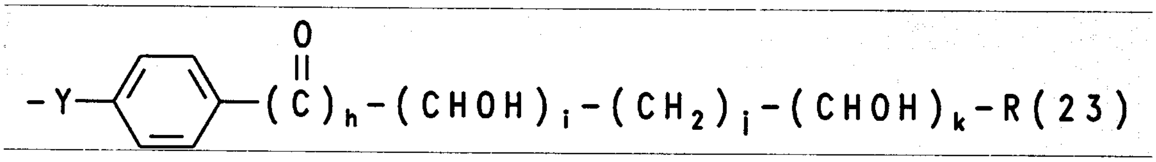

oder 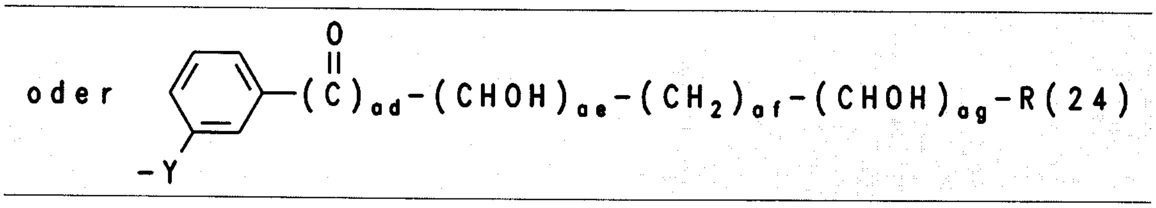

oder 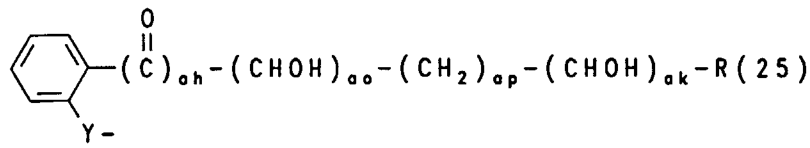

Y Sauerstoff, -S- oder -NR(22)-;

h, ad, ah

unabhängig voneinander Null oder 1;

i, j, k, ae, af, ag, ao, ap und ak

unabhängig voneinander Null, 1, 2, 3 oder 4;

wobei jedoch jeweils

h, i und k nicht gleichzeitig Null,
ad, ae und ag nicht gleichzeitig Null sowie
ah, ao und ak nicht gleichzeitig Null sind,

R(23), R(24) R(25) und R(22)

unabhängig voneinander Wasserstoff oder ($C_1$-$C_3$)-Alkyl;

oder
R(3), R(4), R(5), R(6) und R(7)

unabhängig voneinander SR(29), -OR(30), -NR(31)R(32) oder -CR(33)R(34)R(35);
R(29), R(30), R(31) und R(33)

unabhängig voneinander -$C_aH_{2a}$-($C_1$-$C_9$)-Heteroaryl, das unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;
a Null, 1 oder 2;

R(32), R(34) und R(35)

unabhängig voneinander wie R(29) definiert oder Wasserstoff, $(C_1-C_4)$-Alkyl oder $(C_1-C_4)$-Perfluoralkyl;

oder
R(3), R(4), R(5), R(6) und R(7)

unabhängig voneinander

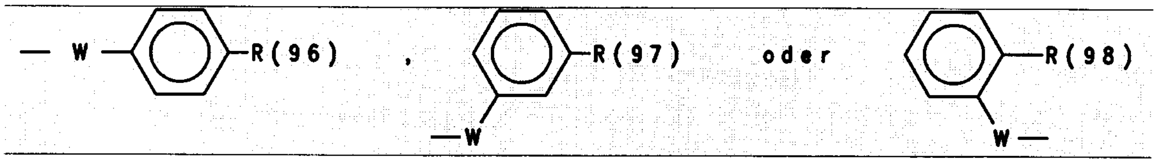

R(96), R(97) und R(98)

unabhängig voneinander $(C_1-C_9)$-Heteroaryl, das über C oder N verknüpft ist und das unsubstituiert oder substituiert ist mit 1 bis 3 Substituenten aus der Gruppe bestehend aus F, Cl, $CF_3$, $CH_3$, Methoxy, Hydroxy, Amino, Methylamino, Dimethylamino oder Benzyl;

W Sauerstoff, S oder NR(36)-;

R(36) H oder $(C_1-C_4)$-Alkyl;

oder
R(3), R(4), R(5), R(6) und R(7)

unabhängig voneinander R(46)X(1)-;
X(1) Sauerstoff, S, NR(47), (D=O)A- oder NR(48)C=MN$^{(*)}$R(49)-;

M Sauerstoff oder Schwefel;
A Sauerstoff oder NR(50);
D C oder SO;

R(46) $(C_1-C_8)$-Alkyl, $(C_3-C_8)$-Alkenyl, $(CH_2)_bC_dF_{2d+1}$ oder $-C_xH_{2x}$-R(51);

b Null oder 1;
d 1, 2, 3, 4, 5, 6 oder 7;
x Null, 1, 2, 3 oder 4;
R(51) $(C_3-C_8)$-Cycloalkyl, Phenyl, Biphenylyl oder Naphthyl, wobei die Aromaten nicht substituiert oder substituiert sind mit 1 - 3 Substituenten ausgewählt aus der Gruppe bestehend aus F, Cl, $CF_3$, Methyl, Methoxy und NR(52)R(53);

R(52) und R(53)

Wasserstoff, $(C_1-C_4)$-Alkyl oder $(C_1-C_4)$-Perfluoralkyl;

R(47), R(48) und R(50) unabhängig

Wasserstoff, $(C_1-C_4)$-Alkyl oder $(C_1-C_4)$-Perfluoralkyl;

R(49) definiert wie R(46);

oder

R(46) und R(47) beziehungsweise R(46) und R(48)

gemeinsam 4 oder 5 Methylengruppen, von denen eine $CH_2$-Gruppe durch Sauerstoff, Schwefel, NH, N-$CH_3$ oder N-Benzyl ersetzt sein kann;

wobei A und N(*) an den Phenylkern des Heteroaroylguanidin-Grundkörpers gebunden sind;

oder
R(3), R(4), R(5), R(6) und R(7)

unabhängig voneinander -SR(64), -OR(65), -NHR(66), -NR(67)R(68), -CHR(69)R(70) oder -CR(54)R(55)OH, -C≡CR(56), -CR(58)=CR(57) oder $-[CR(59)R(60)]_u$-CO-$[CR(61)R(62)]_v$-R(63);

R(64), R(65), R(66), R(67) und R(69)

gleich oder verschieden
$-(CH_2)_y-(CHOH)_z-(CH_2)_{aa}-(CHOH)_t$-R(71) oder $-(CH_2)_{ab}-O-(CH_2-CH_2O)_{ac}$-R(72);
R(71), und R(72)

unabhängig voneinander Wasserstoff oder Methyl;

u 1, 2, 3 oder 4;

v Null, 1, 2, 3 oder 4;

y, z, aa gleich oder verschieden

Null, 1, 2, 3 oder 4;

t 1, 2, 3 oder 4;

R(68), R(70), R(54) und R(55)

gleich oder verschieden Wasserstoff oder $(C_1-C_6)$-Alkyl;

oder

R(69) und R(70) beziehungsweise R(54) und R(55)

zusammen mit dem sie tragenden Kohlenstoff-Atom ein $(C_3-C_8)$-Cycloalkyl;

R(63)

Wasserstoff, $(C_1-C_6)$-Alkyl, $(C_3-C_8)$-Cycloalkyl oder $-C_eH_{2e}$-R(73); e Null, 1, 2, 3 oder 4;

R(56), R(57) und R(73) unabhängig

Phenyl, das unsubstituiert oder substituiert ist mit 1 - 3 Substituenten ausgewählt aus der Gruppe bestehend aus F, Cl, $CF_3$, Methyl, Methoxy und NR(74)R(75);
R(74) und R(75)

Wasserstoff oder $(C_1-C_4)$-Alkyl;

oder

R(56), R(57) und R(73) unabhängig

$(C_1-C_9)$-Heteroaryl, das unsubstituiert oder wie Phenyl substituiert ist;

R(58), R(59), R(60), R(61) und R(62)

Wasserstoff oder Methyl;

oder
R(3), R(4), R(5), R(6) und R(7)

unabhängig voneinander R(76)-NH-$SO_2$-;
R(76) R(77)R(78)N-(C=Y')-;

Y' Sauerstoff, S oder N-R(79);
R(77) und R(78)

gleich oder verschieden Wasserstoff, ($C_1$-$C_8$)-Alkyl, ($C_3$-$C_6$)-Alkenyl oder -$C_fH_{2f}$-R(80);
f Null, 1, 2, 3 oder 4;
R(80) ($C_5$-$C_7$)-Cycloalkyl oder Phenyl, welches unsubstituiert mit 1 - 3 Substituenten ausgewählt aus der Gruppe bestehend aus F, Cl, $CF_3$, Methoxy und ($C_1$-$C_4$)-Alkyl;

oder
R(77) und R(78)

gemeinsam 4 oder 5 Methylengruppen, von denen eine $CH_2$-Gruppe durch Sauerstoff, Schwefel, NH, N-$CH_3$ oder N-Benzyl ersetzt sein kann;

R(79) wie R(77) definiert oder gleich Amidin;

oder
R(3), R(4), R(5), R(6) und R(7)

unabhängig voneinander NR(84a)R(85), OR(84b), SR(84c) oder -$C_nH_{2n}$-R(84d);
n Null, 1, 2, 3 oder 4;
R(84d) ($C_3$-$C_7$)-Cycloalkyl oder Phenyl, welches nicht substituiert ist oder substituiert mit 1 - 3 Substituenten aus der Gruppe bestehend aus F, Cl, $CF_3$, Methyl, Methoxy und NR(16)R(17);
R(16) und R(17)

Wasserstoff, oder $C_1$-$C_4$-Alkyl;

R(84a), R(84b), R(84c) und R(85)

unabhängig voneinander Wasserstoff, ($C_1$-$C_8$)-Alkyl, ($C_1$-$C_8$)-Perfluoralkyl oder $(CH_2)_{ax}$-R(84g);
ax Null, 1, 2, 3 oder 4;
R(84g) ($C_3$-$C_7$)-Cycloalkyl oder Phenyl, welches nicht substituiert ist oder substituiert mit 1 - 3 Substituenten ausgewählt aus der Gruppe bestehend aus F, Cl, $CF_3$, Methyl, Methoxy und NR(84u)R(84v);

R(84u) und R(84v)

Wasserstoff oder $C_1$-$C_4$-Alkyl;

oder
R(84a) und R(85)

gemeinsam 4 oder 5 Methylengruppen, von denen eine $CH_2$-Gruppe durch Sauerstoff, Schwefel, NH, N-$CH_3$ oder N-Benzyl ersetzt sein kann,

sowie deren pharmazeutisch verträgliche Salze;

(HOE 94/F 134 - EP-OS 686 627, NZ 272 103)
z) Benzoylguanidine der Formel I

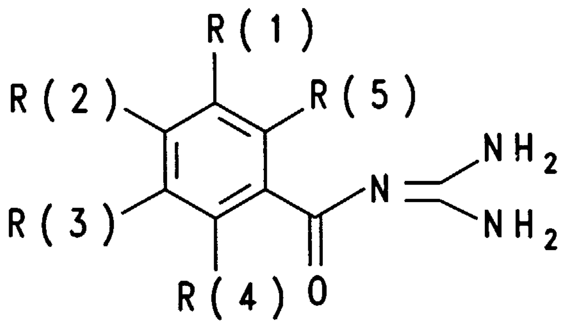

I

worin bedeuten:

R(1) R(6)-$SO_m$;

m Null, 1 oder 2;
R(6) Perfluoralkyl mit 1, 2, 3, 4, 5 oder 6 C-Atomen, das geradkettig oder verzweigt ist;

R(2) und R(3)

unabhängig voneinander Wasserstoff, F, Cl, Br, I, Alkyl mit 1, 2, 3 oder 4 C-Atomen, Alkoxy mit 1, 2, 3 oder 4 C-Atomen oder Phenoxy,

das unsubstituiert ist oder substituiert ist mit 1 - 3 Substituenten ausgewählt aus der Gruppe bestehend aus F, Cl, Methyl und Methoxy;

oder
R(2) und R(3)

unabhängig voneinander Pyrrol-1-yl, Pyrrol-2-yl- oder Pyrrol-3-yl,

welches nicht substituiert ist oder substituiert mit 1 bis 4 Substituenten, ausgewählt aus der Gruppe bestehend aus F, Cl, Br, I, CN, Alkanoyl mit 2, 3, 4, 5, 6, 7 oder 8 C-Atomen, Alkoxycarbonyl mit 2, 3, 4, 5, 6, 7 oder 8 C-Atomen, Formyl, Carboxy, $CF_3$, Methyl und Methoxy;

R(4) und R(5)

unabhängig voneinander Wasserstoff, Alkyl mit 1, 2 oder 3-C-Atomen, F, Cl, Br, I, CN, OR(7), NR(8)R(9) oder $-(CH_2)_n-(CF_2)_o-CF_3$;
R(7), R(8) und R(9)

unabhängig voneinander Wasserstoff oder Alkyl mit 1, 2, 3 oder 4 C-Atomen;

n Null oder 1;
o Null, 1 oder 2;

sowie deren pharmakologisch akzeptable Salze;

(HOE 94/F 168 - EP-OS 690 048, NZ 272 373)
ab) Perfluoralkylgruppen tragende phenylsubstituierte Alkenylcarbonsäureguanidide der Formel I

I

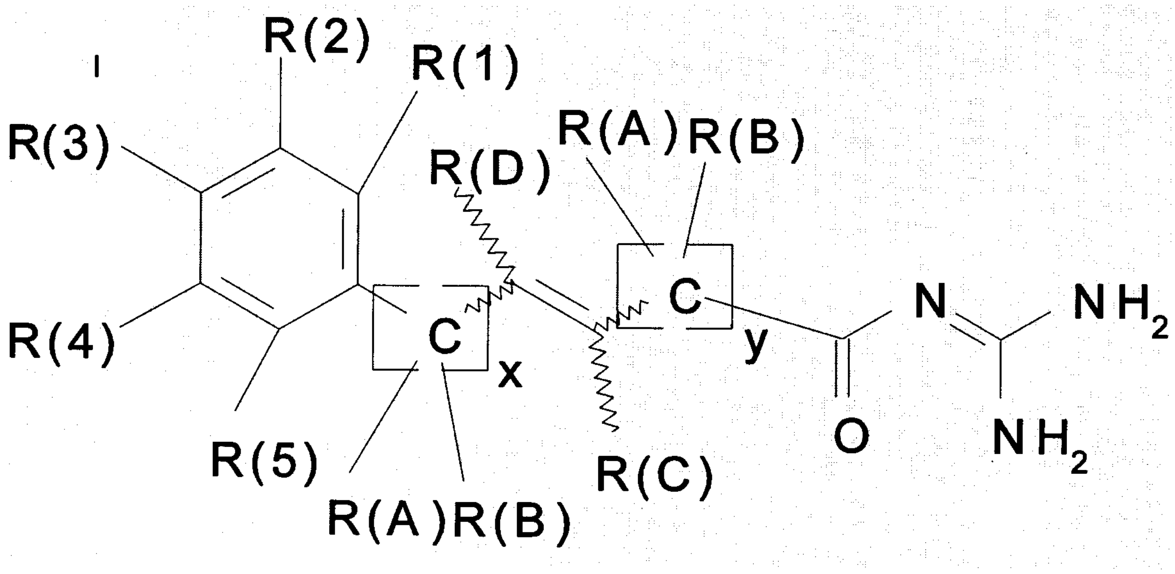

worin bedeuten:

R(A) Wasserstoff, F, Cl, Br, I, CN, OH, OR(6), ($C_1$-$C_8$)-Alkyl, $O_r(CH_2)_aC_bF_{2b+1}$, ($C_3$-$C_8$)-Cycloalkyl oder NR(7)R(8);

r Null oder 1;
a Null, 1, 2, 3 oder 4;
b 1, 2, 3, 4, 5, 6, 7 oder 8;
R(6) ($C_1$-$C_8$)-Alkyl, ($C_1$-$C_4$)-Perfluoralkyl, ($C_3$-$C_8$)-Alkenyl, ($C_3$-$C_8$)-Cycloalkyl, Phenyl oder Benzyl;

wobei die Aromaten nicht substituiert oder substituiert sind mit 1 - 3 Substituenten aus der Gruppe bestehend aus F, Cl, $CF_3$, Methyl, Methoxy und NR(9)R(10);
R(9) und R(10)

H, ($C_1$-$C_4$)-Alkyl oder ($C_1$-$C_4$)-Perfluoralkyl;

R(7) und R(8)

unabhängig voneinander wie R(6) definiert;

oder
R(7) und R(8)

gemeinsam 4 oder 5 Methylengruppen, von denen eine $CH_2$-Gruppe durch Sauerstoff, Schwefel, NH, N-$CH_3$ oder N-Benzyl ersetzt sein kann;

R(B) unabhängig wie R(A) definiert;
X Null, 1 oder 2;
Y Null, 1 oder 2;
R(C) Wasserstoff, F, Cl, Br, I, CN, OR(12), ($C_1$-$C_8$)-Alkyl, $O_p(CH_2)_fC_gF_{2g+1}$ oder ($C_3$-$C_8$)-Cycloalkyl;

p Null oder 1;
f Null, 1, 2, 3 oder 4;
g 1, 2, 3, 4, 5, 6, 7 oder 8;
R(12)

($C_1$-$C_8$)-Alkyl, ($C_1$-$C_4$)-Perfluoralkyl, ($C_3$-$C_8$)-Alkenyl, ($C_3$-$C_8$)-Cycloalkyl, Phenyl oder Benzyl;

wobei die Aromaten Phenyl oder Benzyl nicht substituiert oder substituiert sind mit 1 - 3 Substituenten aus der Gruppe bestehend aus F, Cl, $CF_3$, Methyl, Methoxy und NR(13)R(14);
R(13) und R(14)

unabhängig voneinander H, ($C_1$-$C_4$)-Alkyl oder ($C_1$-$C_4$)-Perfluoralkyl;

R(D) unabhängig wie R(C) definiert,
R(1) Wasserstoff, ($C_1$-$C_8$)-Alkyl, $-O_t(CH_2)_dC_eF_{2e+1}$, ($C_3$-$C_8$)-Cycloalkyl, F, Cl, Br, I oder CN;

t Null oder 1;
d Null, 1, 2, 3 oder 4;
e 1, 2, 3, 4, 5, 6, 7 oder 8;

R(2), R(3), R(4) und R(5)

unabhängig voneinander wie R(1) definiert;

jedoch unter der Bedingung,
daß mindestens einer der Substituenten R(A), R(B), R(C), R(D), R(1), R(2), R(4) oder R(5) eine $O_r(CH_2)_aC_bF_{2b+1}$-, $O_p(CH_2)_fC_gF_{2g+1}$- oder $O_t(CH_2)_dC_eF_{2e+1}$-gruppe und R(3) keine $O_t(CH_2)_dC_eF_{2e+1}$-gruppe ist;
sowie deren pharmazeutisch verträgliche Salze;

(HOE 94/F 182 - EP-OS 690 048, NZ 272 449)
ac) Ortho-amino-substituierte Benzoylguanidine der Formel I

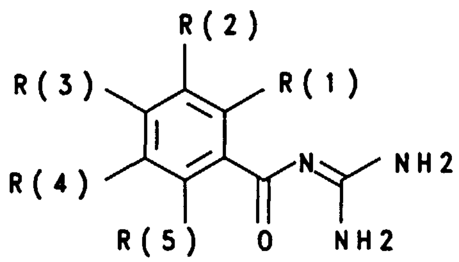

I

worin bedeuten:

R(1) NR(50)R(6),

R(50) und R(6)

unabhängig voneinander Wasserstoff, ($C_1$-$C_8$)-Alkyl oder ($C_1$-$C_8$)-Perfluoralkyl;

R(2), R(3), R(4) und R(5)

unabhängig voneinander R(10)-$SO_a$-, R(11)R(12)N-CO-, R(13)-CO- oder R(14)R(15)N-$SO_2$-;
a Null, 1 oder 2,
R(10), R(11), R(12), R(13), R(14) und R(15)

unabhängig voneinander ($C_1$-$C_8$)-Alkyl, ($C_1$-$C_8$)-Perfluoralkyl, ($C_3$-$C_6$)-Alkenyl oder $-C_{ab}H_{2ab}$-R(16);
ab Null, 1, 2, 3 oder 4;
R(16) ($C_3$-$C_7$)-Cycloalkyl, Phenyl,

welches nicht substituiert ist oder substituiert mit 1 - 3 Substituenten ausgewählt aus der Gruppe bestehend aus F, Cl, $CF_3$, Methyl, Methoxy oder NR(17)R(18);
R(17) und R(18)

unabhängig voneinander H, $CF_3$ oder ($C_1$-$C_4$)-Alkyl;

oder
R(11), R(12), sowie R(14) und R(15)

gemeinsam 4 oder 5 Methylengruppen, von denen eine $CH_2$-Gruppe durch Sauerstoff, S, NH, N-$CH_3$ oder N-Benzyl ersetzt sein kann;

oder
R(11), R(12), R(14) und R(15)

unabhängig voneinander Wasserstoff;

oder
R(2), R(3), R(4) und R(5)

unabhängig voneinander SR(21), -OR(22), -NR(23)R(24) oder -CR(25)R(26)R(27);
R(21), R(22), R(23) und R(25)

unabhängig voneinander -$C_bH_{2b}$-($C_1$-$C_9$)-Heteroaryl, das unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;
b Null, 1 oder 2;

R(24), R(26) und R(27)

unabhängig voneinander Wasserstoff, ($C_1$-$C_4$)-Alkyl oder ($C_1$-$C_4$)-Perfluoralkyl;

oder
R(2), R(3), R(4) und R(5)

unabhängig voneinander Wasserstoff, F, Cl, Br, I, CN, -$(Xa)_{dg}$-$C_{da}H_{2da+1}$,-$(Xb)_{dh}$-$(CH_2)_{db}$-$C_{de}F_{2de+1}$, ($C_3$-$C_8$)-Alkenyl oder -$C_{df}H_{2df}$R(30);
(Xa) O, S oder NR(33);
R(33)

H, ($C_1$-$C_4$)-Alkyl oder ($C_1$-$C_4$)-Perfluoralkyl;

dg Null oder 1;
(Xb) O, S oder NR(34);

R(34)

H, ($C_1$-$C_4$)-Alkyl oder ($C_1$-$C_4$)-Perfluoralkyl;

dh Null oder 1;
da Null, 1, 2, 3, 4, 5, 6, 7, 8;
db Null, 1, 2, 3, 4;
de Null, 1, 2, 3, 4, 5, 6, 7;
df Null, 1, 2, 3, 4;
R(30)

($C_3$-$C_8$)-Cycloalkyl, Phenyl, Biphenylyl oder Naphthyl, wobei die Aromaten Phenyl, Biphenylyl oder Naphthyl nicht substituiert oder substituiert sind mit 1 - 3 Substituenten ausgewählt aus der Gruppe bestehend aus F, Cl, $CF_3$, Methyl, Methoxy und NR(31)R(32);
R(31) und R(32)

H, ($C_1$-$C_4$)-Alkyl oder ($C_1$-$C_4$)-Perfluoralkyl;

oder

R(2), R(3), R(4) und R(5)

unabhängig voneinander NR(40)R(41) oder -(Xe)-$(CH_2)_{eb}$R(45);

R(40) und R(41)

unabhängig voneinander Wasserstoff, $(C_1-C_8)$-Alkyl, $(C_1-C_8)$-Perfluoralkyl oder $(CH_2)_e$-R(42);

e Null, 1, 2, 3 oder 4;

R(42)

$(C_3-C_7)$-Cycloalkyl, Phenyl, welches nicht substituiert ist oder substituiert mit 1 - 3 Substituenten ausgewählt aus der Gruppe bestehend aus F, Cl, $CF_3$, Methyl, Methoxy und NR(43)R(44);

R(43) und R(44)

unabhängig voneinander H, $CF_3$ oder $(C_1-C_4)$-Alkyl;

oder

R(40) und R(41)

gemeinsam 4 oder 5 Methylengruppen, von denen eine $CH_2$-Gruppe durch Sauerstoff, Schwefel, NH, N-$CH_3$ oder N-Benzyl ersetzt sein kann;

(Xe) O, S oder NR(47);

R(47)

H, $(C_1-C_4)$-Alkyl oder $(C_1-C_4)$-Perfluoralkyl;

eb Null, 1, 2, 3 oder 4;

R(45) $(C_3-C_7)$-Cycloalkyl, Phenyl, welches nicht substituiert ist oder substituiert mit 1 - 3 Substituenten ausgewählt aus der Gruppe bestehend aus F, Cl, $CF_3$, Methyl, Methoxy, NR(50)R(51) und -(Xfa)-$(CH_2)_{ed}$-(Xfb)R(46);

Xfa $CH_2$, O, S oder NR(48);

Xfb O, S oder NR(49);

ed 1, 2, 3 oder 4;

R(46)

H, $(C_1-C_4)$-Alkyl oder $(C_1-C_4)$-Perfluoralkyl;

R(48), R(49), R(50) und R(51)

unabhängig voneinander H oder $(C_1-C_4)$-Alkyl oder $(C_1-C_4)$-Perfluoralkyl;

wobei R(3) und R(4) jedoch nicht Wasserstoff sein können,
sowie deren pharmazeutisch verträgliche Salze;

HOE 94/F 265 - NZ 272 946, EP-OS 700 904)
ad) Benzoylguanidine der Formel I

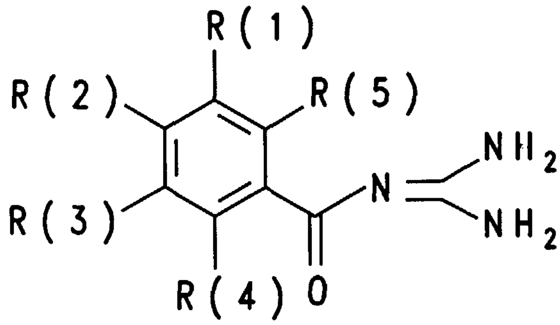

I

worin bedeuten:

einer der drei Substituenten R(1), R(2) und R(3)

($C_1$-$C_9$)-Heteroaryl-N-Oxid,

das über C oder N verknüpft ist und das unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;

oder
einer der drei Substituenten R(1), R(2) und R(3)

-SR(10), -OR(10), -NR(10)R(11) oder -CR(10)R(11)R(12);
R(10)

-$C_aH_{2a}$-($C_1$-$C_9$)-Heteroaryl-N-Oxid,

das unsubstituiert oder substituiert ist mit 1 - 3 Substituenten ausgewählt aus der Gruppe bestehend F, Cl, $CF_3$, $CH_3$, Methoxy, Hydroxy, Amino, Methylamino und Dimethylamino;

a Null, 1 oder 2;
R(11) und R(12)

unabhängig voneinander wie R(10) definiert, Wasserstoff oder ($C_1$-$C_4$)-Alkyl;

und die jeweils anderen Substituenten R(1), R(2) und R(3)

unabhängig voneinander ($C_1$-$C_8$)-Alkyl, ($C_2$-$C_8$)-Alkenyl oder -$C_mH_{2m}$R(14);

m Null, 1 oder 2;
R(14) ($C_3$-$C_8$)-Cycloalkyl oder Phenyl,

welches nicht substituiert oder substituiert ist mit 1 - 3 Substituenten ausgewählt aus der Gruppe bestehend aus F, Cl, $CF_3$, Methyl, Methoxy und NR(15)R(16),
R(15) und R(16)

Wasserstoff oder $CH_3$;

oder
die jeweils anderen Substituenten R(1), R(2) und R(3)

unabhängig voneinander Wasserstoff, F, Cl, Br, I, -C≡N, X-$(CH_2)_p$-$(C_qF_{2q+1})$, R(22)-$SO_u$, R(23)R(24)N-CO, R(25)-CO- oder R(26)R(27)N-$SO_2$-,

wobei die Perfluoralkylgruppe geradkettig oder verzweigt ist;

X eine Bindung, Sauerstoff, S oder NR(28);
u Null, 1 oder 2;
p Null, 1 oder 2;
q Null, 1, 2, 3, 4, 5 oder 6;
R(22), R(23), R(25) und R(26)

unabhängig $(C_1-C_8)$-Alkyl, $(C_2-C_6)$-Alkenyl, $-C_nH_{2n}$-R(29) oder $CF_3$;
n Null, 1, 2, 3 oder 4;
R(28) Wasserstoff oder $(C_1-C_3)$-Alkyl;
R(29) $(C_3-C_7)$-Cycloalkyl oder Phenyl;

welches nicht substituiert ist oder substituiert mit 1 - 3 Substituenten ausgewählt aus der Gruppe bestehend aus F, Cl, $CF_3$, Methyl, Methoxy und NR(30)R(31);
R(30) und R(31)

Wasserstoff oder $C_1$-$C_4$-Alkyl,

oder
R(23), R(25) und R(26)

auch Wasserstoff;

R(24) und R(27)

unabhängig voneinander Wasserstoff oder $(C_1-C_4)$-Alkyl;

oder
R(23) und R(24) sowie R(26) und R(27) gemeinsam 4 oder 5 Methylengruppen, von denen eine $CH_2$-Gruppe durch Sauerstoff, S, NH, N-$CH_3$ oder N-Benzyl ersetzt sein kann;

oder
die jeweils anderen Substituenten R(1), R(2) und R(3)

unabhängig voneinander OR(35) oder NR(35)R(36);
R(35) und R(36)

unabhängig voneinander Wasserstoff oder $(C_1-C_6)$-Alkyl;

oder
R(35) und R(36)

gemeinsam 4 - 7 Methylengruppen, von denen eine $CH_2$-Gruppe durch Sauerstoff, S, NH, N-$CH_3$ oder N-Benzyl ersetzt sein kann,

R(4) und R(5)

unabhängig voneinander Wasserstoff, $(C_1-C_4)$-Alkyl, F, Cl, -OR(32), - NR(33)R(34) oder $C_rF_{2r+1}$;
R(32), R(33) und R(34)

unabhängig voneinander Wasserstoff oder $(C_1-C_3)$-Alkyl;
r 1, 2, 3 oder 4;

sowie deren pharmazeutisch verträgliche Salze;

(HOE 94/F 266 - EP-OS 702 001, NZ 272 948)
ad) Benzoylguanidine der Formel I

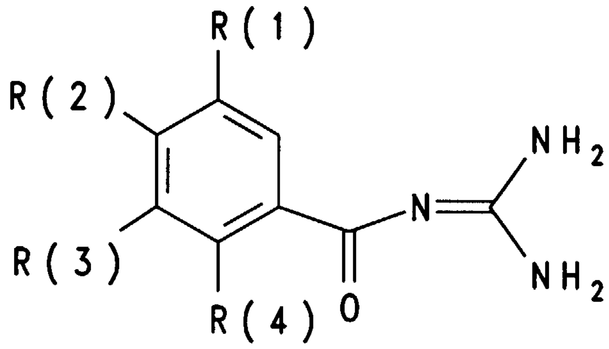

worin bedeuten:

R(1) Wasserstoff, F, Cl, Br, I, CN, $NO_2$, OH, $(C_1$-$C_8)$-Alkyl, $(C_3$-$C_8)$-Cycloalkyl, $O_a$-$(CH_2)_b$-$(CF_2)_c$-$CF_3$;

a Null oder 1;
b Null, 1 oder 2;
c Null, 1, 2 oder 3;

oder
R(1) R(5)-$SO_m$ oder R(6)R(7)N-$SO_2$-;

m Null, 1 oder 2;
R(5) und R(6) unabhängig voneinander

$(C_1$-$C_8)$-Alkyl, $(C_3$-$C_6)$-Alkenyl, $CF_3$ oder -$C_nH_{2n}$-R(8);
n Null, 1, 2, 3 oder 4;

R(7) Wasserstoff oder $(C_1$-$C_4)$-Alkyl;
R(8) $(C_3$-$C_7)$-Cycloalkyl oder Phenyl,

welches nicht substituiert ist oder substituiert mit 1 - 3 Substituenten ausgewählt aus der Gruppe bestehend aus F, Cl, $CF_3$, Methyl, Methoxy und NR(9)R(10);
R(9) und R(10) unabhängig voneinander

Wasserstoff oder $(C_1$-$C_4)$-Alkyl;

oder
R(6) H;
oder R(6) und R(7)

gemeinsam 4 oder 5 Methylengruppen, von denen eine $CH_2$-Gruppe durch Sauerstoff, S, NH, N-$CH_3$ oder N-Benzyl ersetzt sein kann,

oder
R(1) -SR(11), -OR(11) oder-CR(11)R(12)R(13);

R(1 1) -$C_pH_{2p}$-$(C_3$-$C_8)$-Cycloalkyl, -$(C_1$-$C_9)$-Heteroaryl oder Phenyl,

wobei die aromatischen Systeme unsubstituiert oder substituiert sind mit 1 - 3 Substituenten ausgewählt aus der Gruppe bestehend aus F, Cl, $CF_3$, $CH_3$, Methoxy, Hydroxy, Amino, Methylamino und Dimethylamino;
R(12), R(13) unabhängig voneinander

wie R(11) definiert oder Wasserstoff oder $(C_1$-$C_4)$-Alkyl;

p Null, 1 oder 2;

oder

R(1) Phenyl, Naphthyl, Biphenylyl oder ($C_1$-$C_9$)-Heteroaryl, letzteres über C oder N verknüpft,

die unsubstituiert oder substituiert sind mit 1 - 3 Substituenten ausgewählt aus der Gruppe bestehend aus F, Cl, $CF_3$, $CH_3$, Methoxy, Hydroxy, Amino, Methylamino und Dimethylamino;

R(2) $-CF_2R(14)$, -CF[R(15)][R(16)], $-CF[(CF_2)_q-CF_3)][R(15)]$, $-C[(CF_2)_r-CF_3]$ =CR(15)R(16);

R(14) ($C_1$-$C_4$)-Alkyl oder ($C_3$-$C_6$)-Cycloalkyl;

R(15) und R(16) unabhängig voneinander

Wasserstoff oder ($C_1$-$C_4$)-Alkyl;

q Null, 1 oder 2;

r Null, 1 oder 2;

R(3) wie R(1) definiert;

R(4) Wasserstoff, ($C_1$-$C_3$)-Alkyl, F, Cl, Br, I, CN, $-(CH_2)_s-(CF_2)_t-CF_3$;

s Null oder 1;

t Null, 1 oder 2;

sowie deren pharmazeutisch verträgliche Salze;

(HOE 94/F 267 - EP-OS 700 899, NZ 272 947)

ae) Benzoylguanidine der Formel I

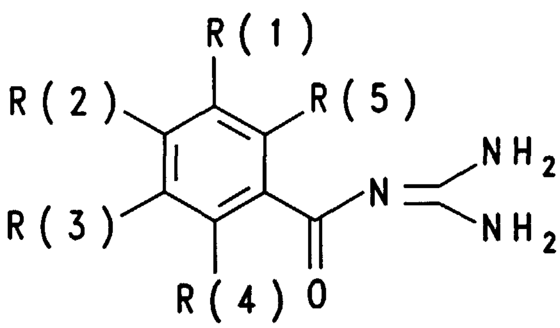

I

worin bedeuten:

einer der drei Substituenten R(1), R(2) und R(3)

$-Y-4-[(CH_2)_k-CHR(7)-(C=O)R(8)]$-Phenyl, $-Y-3-(CH_2)_k-CHR(7)-(C=O)R(8)]$-Phenyl oder $-Y-2-[(CH_2)_k-CHR(7)-(C=O)R(8)]$-Phenyl,

wobei das Phenyl jeweils unsubstituiert oder substituiert ist mit 1 - 2 Substituenten aus der Gruppe F, Cl, $-CF_3$, Methyl, Hydroxy, Methoxy, oder -NR(37)R(38);

R(37) und R(38)

unabhängig voneinander Wasserstoff oder $-CH_3$;

Y eine Bindung, Sauerstoff, -S- oder -NR(9);

R(9) Wasserstoff oder -($C_1$-$C_4$)-Alkyl;

R(7) -OR(10) oder-NR(10)R(11);

R(10) und R(11)

unabhängig voneinander Wasserstoff, $-(C_1-C_8)$-Alkyl, $-(C_1-C_8)$-Alkanoyl, $-(C_1-C_8)$-Alkoxycarbonyl, Benzyl, Benzyloxycarbonyl;

oder
R(10) Trityl;

R(8) -OR(12) oder -NR(12)R(13);

R(12) und R(13)

unabhängig voneinander Wasserstoff, $-(C_1-C_8)$-Alkyl oder Benzyl;

k Null, 1, 2, 3 oder 4;

und die jeweils anderen Reste R(1), R(2) und R(3)

unabhängig voneinander $-(C_1-C_8)$-Alkyl, $-(C_2-C_8)$-Alkenyl oder $-(CH_2)_m$R(14);
m Null, 1 oder 2;
R(14) $-(C_3-C_8)$-Cycloalkyl oder Phenyl,

welches nicht substituiert oder substituiert ist mit 1 - 3 Substituenten ausgewählt aus der Gruppe bestehend aus F, Cl, $-CF_3$, Methyl, Methoxy und -NR(15)R(16);
R(15) und R(16)

Wasserstoff oder $-CH_3$;

oder
die jeweils anderen Reste R(1), R(2) und R(3)

unabhängig voneinander R(18)R(19)N-(C=Y')-NH-$SO_2$-;

Y' Sauerstoff, -S- oder -N-R(20);

R(18) und R(19)

unabhängig voneinander Wasserstoff, $-(C_1-C_8)$-Alkyl, $-(C_3-C_6)$-Alkenyl oder $-(CH_2)_t$-R(21);
t Null, 1, 2, 3 oder 4;
R(21) $-(C_5-C_7)$-Cycloalkyl oder Phenyl,

welches unsubstituiert ist oder substituiert ist mit 1 - 3 Substituenten ausgewählt aus der Gruppe bestehend aus F, Cl, $-CF_3$, Methoxy und $-(C_1-C_4)$-Alkyl;

oder
R(18) und R(19)

gemeinsam 4 oder 5 Methylengruppen, von denen eine $CH_2$-Gruppe durch Sauerstoff, -S-, -NH-, $-N-CH_3$ oder -N-Benzyl ersetzt sein kann;
R(20)

wie R(18) definiert oder Amidin;

oder
die jeweils anderen Reste R(1), R(2) und R(3)

unabhängig voneinander Wasserstoff, F, Cl, Br, I, -C≡N, X-$(CH_2)_p$-$(C_qF_{2q+1})$, R(22)-$SO_u$-, R(23)R(24)N-CO-, R(25)-CO- oder R(26)R(27)N-$SO_2$-, wobei die Perfluoralkylgruppe geradkettig oder verzweigt ist;

X eine Bindung, Sauerstoff, -S- oder -NR(28);

u Null, 1 oder 2;

p Null, 1 oder 2;

q 1, 2, 3, 4, 5 oder 6;

R(22), R(23), R(25) und R(26)

unabhängig voneinander -($C_1$-$C_8$)-Alkyl, -($C_3$-$C_6$)-Alkenyl, -$(CH_2)_n$-R(29) oder -$CF_3$;

n Null, 1, 2, 3 oder 4;

R(28) Wasserstoff oder -($C_1$-$C_3$)-Alkyl;

R(29) -($C_3$-$C_7$)-Cycloalkyl oder Phenyl,

welches nicht substituiert ist oder substituiert mit 1 - 3 Substituenten ausgewählt aus der Gruppe bestehend aus F, Cl, -$CF_3$, Methyl, Methoxy und -NR(30)R(31);

R(30) und R(31)

Wasserstoff oder -($C_1$-$C_4$)-Alkyl;

oder

R(23), R(25) und R(26)

Wasserstoff;

R(24) und R(27)

unabhängig voneinander Wasserstoff oder -($C_1$-$C_4$)-Alkyl;

oder

R(23) und R(24) sowie R(26) und R(27)

gemeinsam 4 oder 5 Methylengruppen, von denen eine $CH_2$-Gruppe durch Sauerstoff, -S-, -NH-, - N-$CH_3$ oder -N-Benzyl ersetzt sein kann;

oder

die jeweils anderen Reste R(1), R(2) und R(3)

unabhängig voneinander -OR(35) oder -NR(35)R(36);

R(35) und R(36)

unabhängig voneinander Wasserstoff oder -($C_1$-$C_6$)-Alkyl;

oder

R(35) und R(36)

gemeinsam 4 - 7 Methylengruppen, von denen eine $CH_2$-Gruppe durch Sauerstoff, -S-, -NH-, -N-$CH_3$ oder -N-Benzyl ersetzt sein kann;

R(4) und R(5)

unabhängig voneinander Wasserstoff, -($C_1$-$C_4$)-Alkyl, F, Cl, -OR(32), - NR(33)R(34) oder -$C_rF_{2r+1}$;

R(32), R(33) und R(34)

unabhängig voneinander Wasserstoff oder -($C_1$-$C_3$)-Alkyl;

r 1, 2, 3 oder 4;

sowie deren pharmazeutisch verträgliche Salze;

(HOE 94/F 352 - EP-OS 713 684, NZ 280 517)
af) Benzoylguanidine der Formel I

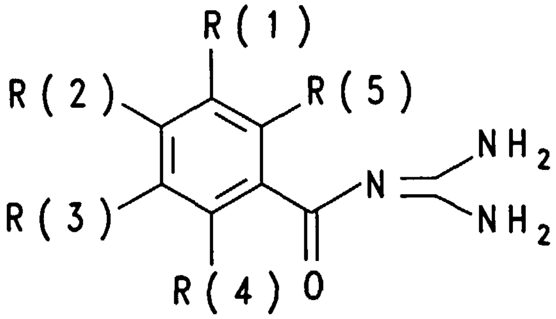

I

worin bedeuten:

R(1) R(6)-CO oder R(7)R(8)N-CO;

R(6) ($C_1$-$C_8$)-Alkyl, ($C_1$-$C_8$)-Perfluoralkyl, ($C_3$-$C_8$)-Alkenyl oder -$C_nH_{2n}$-R(9),

n Null, 1, 2, 3 oder 4;
R(9) ($C_3$-$C_8$)-Cycloalkyl, Phenyl, Biphenylyl oder Naphthyl,

wobei die Aromaten nicht substituiert oder substituiert sind mit 1 - 3 Substituenten ausgewählt aus der Gruppe bestehend aus F, Cl, $CF_3$, Methyl, Methoxy und NR(10)R(11),
R(10) und R(11)

H, ($C_1$-$C_4$)-Alkyl oder ($C_1$-$C_4$)-Perfluoralkyl;

R(7) H, ($C_1$-$C_8$)-Alkyl, ($C_1$-$C_8$)-Perfluoralkyl, ($C_3$-$C_8$)-Alkenyl oder -$C_nH_{2n}$-R(12);

n Null, 1, 2, 3 oder 4;
R(12) ($C_3$-$C_8$)-Cycloalkyl, Phenyl, Biphenylyl oder Naphthyl,

wobei die Aromaten nicht substituiert oder substituiert sind mit 1 - 3 Substituenten ausgewählt aus der Gruppe bestehend aus F, Cl, $CF_3$, Methyl, Methoxy und NR(13)R(14);
R(13) und R(14)

H, ($C_1$-$C_4$)-Alkyl oder ($C_1$-$C_4$)-Perfluoralkyl;

R(8) H, ($C_1$-$C_4$)-Alkyl oder ($C_1$-$C_4$)-Perfluoralkyl;
oder
R(7) und R(8) gemeinsam

4 oder 5 Methylengruppen, von denen eine $CH_2$-Gruppe durch Sauerstoff, S, NH, N-$CH_3$ oder N-Benzyl ersetzt sein kann;

R(2) wie R(1) definiert, oder H, OH, F, Cl, Br, I, CN, $NO_2$, ($C_1$-$C_8$)-Alkyl, ($C_1$-$C_8$)-Perfluoralkyl, ($C_3$-$C_8$)-Alkenyl oder -$C_nH_{2n}$R(15);

n Null, 1, 2, 3 oder 4;
R(15) ($C_3$-$C_8$)-Cycloalkyl, Phenyl, Biphenylyl oder Naphthyl,

wobei die Aromaten nicht substituiert oder substituiert sind mit 1 - 3 Substituenten ausgewählt aus der Gruppe bestehend aus F, Cl, $CF_3$, Methyl, Methoxy und NR(16)R(17);
R(16) und R(17)

H, $(C_1-C_4)$-Alkyl oder $(C_1-C_4)$-Perfluoralkyl;

oder

R(2) $(C_1-C_9)$-Heteroaryl,

das über C oder N verknüpft ist und das unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;

oder

R(2) SR(18), -OR(18), -NR(18)R(19) oder -CR(18)R(19)R(20);

R(18) $-C_aH_{2a}-(C_1-C_9)$-Heteroaryl,

das unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino, Dimethylamino;

a Null, 1 oder 2;

R(19) und R(20)

unabhängig voneinander wie R(18) definiert oder Wasserstoff, $(C_1-C_4)$-Alkyl oder $(C_1-C_4)$-Perfluoralkyl;

oder

R(2) R(21)-$SO_m$ oder R(22)R(23)N-$SO_2$-;

m 1 oder 2;
R(21) $(C_1-C_8)$-Alkyl, $(C_1-C_8)$-Perfluoralkyl, $(C_3-C_8)$-Alkenyl oder - $C_nH_{2n}$-R(24);

n Null, 1, 2, 3 oder 4;
R(24) $(C_3-C_8)$-Cycloalkyl, Phenyl, Biphenylyl oder Naphthyl,

wobei die Aromaten nicht substituiert oder substituiert sind mit 1 - 3 Substituenten ausgewählt aus der Gruppe bestehend aus F, Cl, $CF_3$, Methyl, Methoxy und NR(27)R(28);
R(27) und R(28)

H, $(C_1-C_4)$-Alkyl oder $(C_1-C_4)$-Perfluoralkyl;

R(22) H, $(C_1-C_8)$-Alkyl, $(C_1-C_8)$-Perfluoralkyl, $(C_3-C_8)$-Alkenyl oder - $C_nH_{2n}$-R(29);

n Null, 1, 2, 3 oder 4;
R(29) $(C_3-C_8)$-Cycloalkyl, Phenyl, Biphenylyl oder Naphthyl,

wobei die Aromaten nicht substituiert oder substituiert sind mit 1 - 3 Substituenten ausgewählt aus der Gruppe bestehend aus F, Cl, $CF_3$, Methyl, Methoxy und NR(30)R(31);
R(30) und R(31)

H, $(C_1-C_4)$-Alkyl oder $(C_1-C_4)$-Perfluoralkyl;

R(23) Wasserstoff, $(C_1-C_4)$-Alkyl oder $(C_1-C_4)$-Perfluoralkyl;
oder
R(22) und R(23)

gemeinsam 4 oder 5 Methylengruppen, von denen eine $CH_2$-Gruppe durch Sauerstoff, S, NH, N-$CH_3$ oder N-Benzyl ersetzt sein kann;

oder

R(2) R(33)X-;

X Sauerstoff, S, NR(34), (D = O)A- oder NR(34)C = MN(*)R(35)-;

M Sauerstoff oder S;
A Sauerstoff oder NR(34);
D C oder SO;
R(33) $(C_1-C_8)$-Alkyl, $(C_3-C_8)$-Alkenyl, $(CH_2)_bC_dF_{2d+1}$ oder $-C_nH_{2n}$-R(36);

b Null oder 1;
d 1, 2, 3, 4, 5, 6 oder 7;
n Null, 1, 2 , 3, oder 4;
R(36) $(C_3-C_8)$-Cycloalkyl, Phenyl, Biphenylyl oder Naphthyl,

wobei die Aromaten nicht substituiert oder substituiert sind mit 1 - 3 Substituenten ausgewählt aus der Gruppe bestehend aus F, Cl, $CF_3$, Methyl, Methoxy und NR(37)R(38);
R(37) und R(38)

H, $(C_1-C_4)$-Alkyl oder $(C_1-C_4)$-Perfluoralkyl;

R(34) Wasserstoff, $(C_1-C_4)$-Alkyl oder $(C_1-C_4)$-Perfluoralkyl;
R(35) definiert wie R(33);
oder
R(33) und R(34)

gemeinsam 4 oder 5 Methylengruppen, von denen eine $CH_2$-Gruppe durch Sauerstoff, S, NH, $N-CH_3$ oder N-Benzyl ersetzt sein kann;

wobei A und N(*) an den Phenylkern des Benzoylguanidin-Grundkörpers gebunden sind;

oder

R(2) -SR(40), -OR(40), -NHR(40), -NR(40)R(41), -CHR(40)R(42), -CR(42)R(43)OH, -C≡CR(45), -CR(46)=CR(45) oder $-[CR(47)R(48)]_u$-CO-$[C(R49)R(50)]_v$-R(44);

R(40) und R(41)

unabhängig voneinander $-(CH_2)_p-(CHOH)_q-(CH_2)_r-(CHOH)_t$-R(51) oder $-(CH_2)_p-O-(CH_2-CH_2O)_q$-R(51);
R(51) Wasserstoff oder Methyl;

u 1, 2, 3 oder 4;
v Null, 1, 2, 3 oder 4;

p, q und r

unabhängig voneinander Null, 1, 2, 3 oder 4;

t 1, 2, 3 oder 4;

R(42) und R(43)

unabhängig voneinander Wasserstoff oder $(C_1-C_6)$-Alkyl;

oder
R(42) und R(43)

zusammen mit dem sie tragenden Kohlenstoff-Atom ein ($C_3$-$C_8$)-Cycloalkyl;

R(44) Wasserstoff, ($C_1$-$C_6$)-Alkyl, ($C_3$-$C_8$)-Cycloalkyl, $-C_eH_{2e}$-R(45);

e Null, 1, 2, 3 oder 4;

R(45) Phenyl,

das unsubstituiert oder substituiert ist mit 1 - 3 Substituenten ausgewählt aus der Gruppe bestehend aus F, Cl, $CF_3$, Methyl, Methoxy und NR(52)R(53);
R(52) und R(53)

H oder ($C_1$-$C_4$)-Alkyl;

oder
R(45) ($C_1$-$C_9$)-Heteroaryl,

das unsubstituiert oder wie Phenyl substituiert ist;

oder
R(45) ($C_1$-$C_6$)-Alkyl,

das unsubstituiert oder mit 1 - 3 OH substituiert ist;

R(46), R(47), R(48), R(49) und R(50)

unabhängig voneinander Wasserstoff oder Methyl;

oder

R(2) R(55)-NH-$SO_2$-;

R(55) R(56)R(57)N-(C=Y)-;
Y Sauerstoff, S oder N-R(58);
R(56) und R(57)

unabhängig voneinander Wasserstoff, ($C_1$-$C_8$)-Alkyl, ($C_3$-$C_6$)-Alkenyl oder $-C_fH_{2f}$-R(59);
f Null, 1, 2, 3 oder 4;
R(59) ($C_5$-$C_7$)-Cycloalkyl, Phenyl,

welches unsubstituiert ist oder substituiert mit 1 - 3 Substituenten ausgewählt aus der Gruppe bestehend aus F, Cl, $CF_3$, Methoxy und ($C_1$-$C_4$)-Alkyl;

oder
R(56) und R(57)

gemeinsam 4 oder 5 Methylengruppen, von denen eine $CH_2$-Gruppe durch Sauerstoff, S, NH, N-$CH_3$ oder N-Benzyl ersetzt sein kann;

R(58)

wie R(56) definiert oder gleich Amidin;

R(3), R(4) und R(5) sind unabhängig voneinander wie R(1) oder R(2) definiert,
wobei jedoch mindestens einer der Substituenten R(2), R(3), R(4) und R(5) gleich OH sein muß;
sowie deren pharmazeutisch verträgliche Salze;

(HOE 95/F 007 K - EP-OS 723 956, NZ 280 887)

ag) Benzoylguanidine der Formel I

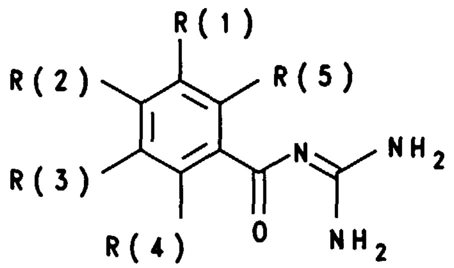

I

worin bedeuten:

einer der drei Substituenten R(1), R(2) und R(3)

R(6)-A-B-D-; R(6) ein basischer protonierbarer Rest, d.h. eine Aminogruppe -NR(7)R(8), eine Amidinogruppe R(7)R(8)N-C[=N-R(9)]- oder eine Guanidinogruppe

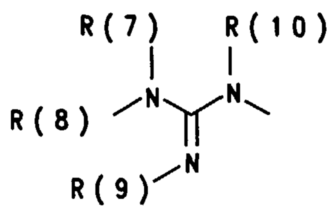

R(7), R(8), R(9) und R(10)

unabhängig voneinander Wasserstoff oder Alkyl mit 1, 2, 3 oder 4 C-Atomen;

oder
R(7) und R(8)

gemeinsam $C_aH_{2a}$;
a 4, 5, 6 oder 7;
wobei im Falle von a = 5, 6 oder 7 eine Methylengruppe der Gruppe $C_aH_{2a}$ durch eine Heteroatomgruppe O, $SO_m$ oder NR(11) ersetzt sein kann,

oder
R(8) und R(9) oder R(9) und R(10) oder R(7) und R(10)

eine Gruppe $C_aH_{2a}$;
a 2, 3, 4 oder 5;

wobei im Falle von a = 3, 4 oder 5 eine Methylengruppe der Gruppe $C_aH_{2a}$ durch eine Heteroatomgruppe O, $SO_m$ oder NR(11) ersetzt sein kann;

m Null, 1 oder 2;
R(11) Wasserstoff oder Methyl;

oder

R(6) ein basisches heteroaromatisches Ringsystem mit 1 - 9 C-Atomen;

A $C_bH_{2b}$;

b 1, 2, 3, 4, 5, 6, 7, 8, 9 oder 10;

wobei in der Gruppe $C_bH_{2b}$ ein oder zwei Methylengruppen durch eine der Gruppierungen ausgewählt aus der Gruppe bestehend aus -O-, -CO-, -CH[OR(20)]-, $-SO_m-$, -NR(20)-, -NR(20)-CO-, -NR(20)-CO-NH-, $-NR(20)-CO-NH-SO_2-$und

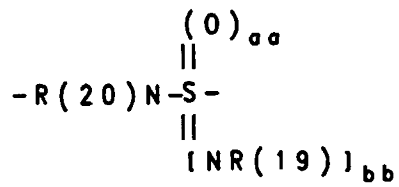

$-SO_{aa}[NR(19)]_{bb}-$ ersetzt sein können;
und wobei in der Gruppe $C_bH_{2b}$ eine Methylengruppe durch -CH-R(99) ersetzt sein kann, wobei R(99) gemeinsam mit R(7) einen Pyrrolidin- oder Piperidin-Ring bildet;
aa 1 oder 2;
bb 0 oder 1;
aa + bb = 2 ;
R(19) Wasserstoff oder Alkyl mit 1, 2, 3 oder 4 C-Atomen
R(20) Wasserstoff oder Methyl;

B einen Phenylen- oder Naphthylenrest,

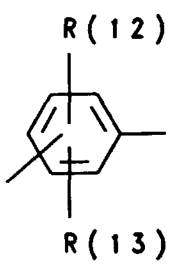

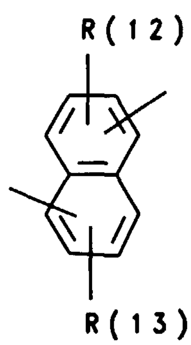

R(12) und R(13)

unabhängig voneinander Wasserstoff, Methyl, F, Cl, Br, J, $CF_3$ oder $-SO_w-R(14)$;
R(14) Methyl oder NR(15)R(16);
R(15) und R(16)

unabhängig voneinander Wasserstoff oder Alkyl mit 1, 2, 3 oder 4 C-Atomen;

w Null, 1 oder 2;

D $-C_dH_{2d}-X_f-$;

d Null, 1, 2, 3 oder 4;
X -O-, -CO-, -CH[OR(21)]-, $-SO_m-$ oder -NR(21)-;
f Null oder 1;

R(21) Wasserstoff oder Methyl;
m Null, 1 oder 2;

und die jeweils anderen Substituenten R(1) und R(2) und R(3)

unabhängig voneinander Wasserstoff, F, Cl, Br, J, -CN, -($C_1$-$C_8$)-Alkyl, -($C_2$-$C_8$)-Alkenyl, -NR(35)R(36) oder R(17)-$C_gH_{2g}$-$Z_h$-;
g Null, 1, 2, 3 oder 4;
h Null oder 1;
R(35) und R(36)

unabhängig voneinander Wasserstoff oder Alkyl mit 1, 2, 3, 4, 5 oder 6 C-Atomen;

oder
R(35) und R(36)

gemeinsam 4 - 7 Methylengruppen, von denen eine $CH_2$-Gruppe durch Sauerstoff, -S-, -NH-, $-NCH_3$ oder -N-Benzyl ersetzt sein kann;

Z -O-, -CO-, -$SO_v$-, -NR(18)-, -NR(18)-CO-, -NR(18)-CO-NH- oder -NR(18)-$SO_2$-;

R(18) Wasserstoff oder Methyl;
v Null, 1 oder 2;

R(17) Wasserstoff, Cycloalkyl mit 3, 5 oder 6 C-Atomen oder $C_kF_{2k+1}$-;

k 1, 2 oder 3,

oder
R(17) Pyrrol-1-yl, Pyrrol-2-yl oder Pyrrol-3-yl,

welches nicht substituiert oder substituiert ist mit 1 - 4 Substituenten ausgewählt aus der Gruppe bestehend aus F, Cl, Br, I, -CN, ($C_2$-$C_8$)-Alkanoyl, ($C_2$-$C_8$)-Alkoxycarbonyl, Formyl, Carboxy, $-CF_3$, Methyl und Methoxy;

oder
R(17) -($C_3$-$C_8$)-Cycloalkyl oder Phenyl,

welches nicht substituiert oder substituiert ist mit 1 - 3 Substituenten ausgewählt aus der Gruppe bestehend aus F und Cl, $-CF_3$, Methyl, Hydroxy, Methoxy, -NR(37)R(38), $CH_3SO_2$- und $H_2NO_2S$-;
R(37) und R(38)

Wasserstoff oder $-CH_3$;

R(4) und R(5)

unabhängig voneinander Wasserstoff, Alkyl mit 1, 2, 3 oder 4 C-Atomen, F, Cl, -OR(32), -NR(33)R(34) oder -$C_rF_{2r+1}$;
R(32), R(33) und R(34)

unabhängig voneinander Wasserstoff oder Alkyl mit 1, 2 oder 3 C-Atomen;

r 1, 2, 3 oder 4;

sowie deren pharmakologisch verträgliche Salze;

(HOE 95/F 072 - EP-OS 738 712, NZ 286 380
ah) Indenoylguanidine der Formel I

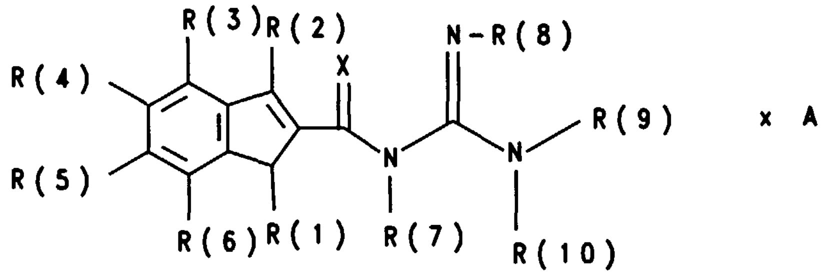

I

worin bedeuten:

R(1) und R(2)

unabhängig voneinander Wasserstoff, Alkyl mit 1, 2, 3, 4, 5, 6, 7, 8, 9 oder 10 C-Atomen, Cycloalkyl mit 3, 4, 5 oder 6 C-Atomen. O-Alkyl mit 1, 2, 3 or 4 C-Atomen, O-C(=O)-Alkyl mit 1, 2, 3 oder 4 C-Atomen oder $C_mH_{2m}$-NR(12)R(13);

R(12) und R(13)

unabhängig voneinander Wasserstoff oder Alkyl mit 1, 2, 3 or 4 C-Atomen;

m Null, 2, 3 oder 4;

NH-C(=O)-$NH_2$, C(=O)-O-Alkyl mit 1, 2, 3 oder 4 C-Atomen, C(=O)-$NH_2$, C(=O)-NH-Alkyl mit 1, 2, 3 oder 4 C-Atomen, C(=O)-N$(Alkyl)_2$ mit 1, 2, 3 oder 4 C-Atomen in jeder Alkylgruppe, Alkenyl mit 2, 3, 4, 5, 6, 7, 8, 9 oder 10 C-Atomen, alkinyl mit 2, 3, 4, 5, 6, 7, 8, 9 oder 10 C-Atomen, Alkylaryl mit 1, 2, 3 or 4 C-Atomen in der Alkylgruppe, Alkenyl-aryl mit 2, 3, 4, 5, 6, 7, 8, 9 oder 10 C-Atomen in der Alkenylgruppe, Alkinyl-aryl mit 2, 3, 4, 5, 6, 7, 8, 9 oder 10 C-Atomen in der Alkinylgruppe, $C_1$-$C_4$-alkyl-substituiertes aryl, $C_1$-$C_4$-Alkyl-heteroaryl, $C_1$-$C_4$-Alkenyl-heteroaryl, Aminoalkyl-aryl mit 1, 2, 3 oder 4 C-Atomen in der Alkylgruppe, substituiertes Aryl, Heteroaryl und substituiertes Heteroaryl;

R(3), R(4), R(5) and R(6)

unabhängig voneinander Wasserstoff, Alkyl mit 1, 2, 3, 4, 5, 6, 7, 8, 9 oder 10 C-Atomen, O-Alkyl mit 1, 2, 3, 4, 5, 6, 7, 8, 9 oder 10 C-Atomen, Halogen, (wie F, Cl, Br, I), OH, Aryl, substituiertes Aryl, Heteroaryl, substituiertes Heteroaryl, O-Niederalkyl, O-Aryl, O-Niederalkylaryl, O-substituiertes Aryl, O-Niederalkyl-substituiertes Aryl, O-C(=O)-$C_1$-$C_4$-Alkyl-aryl, O-C(=O)-NH-$C_1$-$C_4$-Alkyl, O-C(=O)-N$(C_1$-$C_4$-$Alkyl)_2$, $NO_2$, CN, $CF_3$, $NH_2$, NH-C(=O)-$C_1$-$C_4$-Alkyl, NH-C(=O)-$NH_2$, COOH, C(=O)-O-$C_1$-$C_4$-alkyl, C(=O)-$NH_2$, C(=O)-NH-$C_1$-$C_4$-Alkyl, C(=O)-N$(C_1$-$C_4$-$Alkyl)_2$, $C_1$-$C_4$-COOH, $C_1$-$C_4$-Alkyl-C(=O)-O-$C_1$-$C_4$-alkyl, $SO_3H$, $SO_2$-Alkyl, $SO_2$-Alkylaryl, $SO_2$-N-$(Alkyl)_2$, $SO_2$-N(Alkyl)(alkylaryl), C(=O)-R(11), $C_1$-$C_{10}$-Alkyl-C(=O)-R(11), $C_2$-$C_{10}$-Alkenyl-C(=O)-R(11), $C_2$-$C_{10}$-Alkinyl-C(=O)-R(11), NH-C(=O)-$C_1$-$C_{10}$-Alkyl-C(=O)-R(11), O-$C_1$-$C_{11}$-Alkyl-C(=O)-R(11); R(11) $C_1$-$C_4$-Alkyl, $C_1$-$C_4$-Alkynyl, Aryl, substituiertes Aryl, $NH_2$, NH-$C_1$-$C_4$-Alkyl, N-$(C_1$-$C_4$-$Alkyl)_2$, $SO_3H$, $SO_2$-alkyl, $SO_2$-Alkylaryl, $SO_2$-N-$(Alkyl)_2$, $SO_2$-N(Alkyl)(alkylaryl);

X O, S oder NH;

R(7), R(8), R(9) und R(10)

unabhängig voneinander Wasserstoff, Alkyl, Cycloalkyl, Aryl, Alkylaryl;

oder

R(8) und R(9)

gemeinsam Teil eines 5, 6 oder 7-gliedrigen heterocyclischen Rings;

A abwesend oder eine ungiftige organische oder Mineralsäure.

(HOE 95/F 109 - EP 748 795, NZ 286 583)
ai) Benzyloxycarbonylguanidine der Formel I

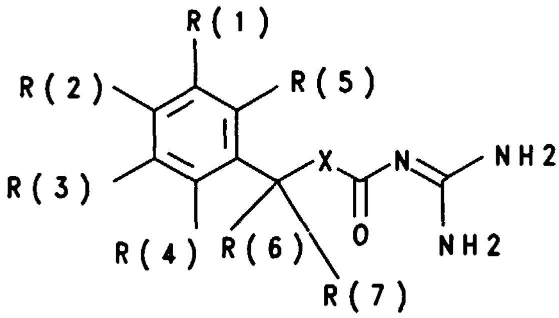

I

worin bedeuten:

R(1), R(2) und R(3)

unabhängig voneinander -Y-[4-R(8)-Phenyl], -Y-[3-R(8)-Phenyl] oder - Y-[2-R(8)-Phenyl],

wobei das Phenyl jeweils unsubstituiert oder substituiert ist mit 1 - 2 Substituenten aus der Gruppe bestehend aus F, Cl, $-CF_3$, Methyl, Hydroxy, Methoxy und -NR(96)R(97);
R(96) und R(97)

unabhängig voneinander Wasserstoff oder $-CH_3$;

Y eine Bindung, $CH_2$, Sauerstoff, -S- oder -NR(9);

R(9) Wasserstoff oder Alkyl mit 1, 2, 3 oder 4 C-Atomen;

R(8) $SO_a[NR(98)]_bNR(99)R(10)$;

a 1 oder 2;
b 0 oder 1;
a + b = 2 ;
R(98), R(99) und R(10)

unabhängig voneinander Wasserstoff, $-(C_1-C_8)$-Alkyl, Benzyl, - $(C_2-C_8)$-Alkylen-NR(11)R(12), $(C_2-C_8)$-Alkylen-NR(13)-$(C_2-C_8)$-Alkylen-NR(37)R(38) oder $(C_0-C_8)$-Alkylen-CR(39)R(40)-CR(41)R(42)$(C_0-C_8)$-Alkylen-NR(43)R(44);
R(11), R(12), R(13), R(37), R(38), R(43) und R(44)

unabhängig voneinander Wasserstoff, $-(C_1-C_8)$-Alkyl oder Benzyl:

R(39), R(40), R(41) und R(42)

unabhängig voneinander Wasserstoff, $-(C_1-C_8)$-Alkyl oder $-(C_0-C_3)$-Alkylen-Phenyl,

wobei das Phenyl nicht substituiert oder substituiert ist mit 1 - 3 Substituenten ausgewählt aus der Gruppe bestehend aus F, Cl,$-CF_3$, Methyl und Methoxy;

oder
R(99) und R(10)

gemeinsam 4 - 6 Methylengruppen, von denen eine $CH_2$-Gruppe durch Sauerstoff, -S-, -NH-, -N-$CH_3$ oder -N-Benzyl ersetzt sein kann;

oder

R(8) $SO_a[NR(98)]_bNR(95)$-C[=N-R(94)]-NR(93)R(92);

R(92), R(93), R(94) und R(95)

unabhängig voneinander Wasserstoff oder Alkyl mit 1, 2, 3 oder 4 C-Atomen;

oder
R(1), R(2) und R(3)

unabhängig voneinander Pyrrol-1-yl, Pyrrol-2-yl oder Pyrrol-3-yl,

welches nicht substituiert oder substituiert ist mit 1 - 4 Substituenten ausgewählt aus der Gruppe bestehend aus F, Cl, Br, I, -CN, ($C_2$-$C_8$)-Alkanoyl, ($C_2$-$C_8$)-Alkoxycarbonyl, Formyl, Carboxy, - $CF_3$, Methyl, Methoxy;

oder
R(1), R(2) und R(3)

unabhängig voneinander Wasserstoff, -($C_1$-$C_8$)-Alkyl, -($C_2$-$C_8$)-Alkenyl oder -$(CH_2)_m$R(14);
m Null, 1 oder 2;
R(14) -($C_3$-$C_8$)-Cycloalkyl oder Phenyl,

welches nicht substituiert oder substituiert ist mit 1 - 3 Substituenten ausgewählt aus der Gruppe bestehend aus F und Cl, -$CF_3$, Methyl, Methoxy und -NR(15)R(16);
R(15) und R(16)

Wasserstoff oder -$CH_3$;

oder
R(1), R(2) und R(3)

unabhängig voneinander -Q-4-[$(CH_2)_k$-CHR(17)-(C=O)R(20)]-Phenyl, -Q-3-$(CH_2)_k$-CHR(17)-(C=O)R(20)]-Phenyl oder -Q-2-[$(CH_2)_k$-CHR(17)-(C=O)R(20)]-Phenyl,

wobei das Phenyl jeweils unsubstituiert oder substituiert ist mit 1 - 2 Substituenten aus der Gruppe F, Cl, -$CF_3$, Methyl, Hydroxy, Methoxy und -NR(35)R(36);
R(35) und R(36)

unabhängig voneinander Wasserstoff oder -$CH_3$;

Q eine Bindung, Sauerstoff, -S- oder -NR(18);

R(18) Wasserstoff oder -($C_1$-$C_4$)-Alkyl;

R(17) -OR(21) oder -NR(21)R(22);

R(21) und R(22)

unabhängig voneinander Wasserstoff, -($C_1$-$C_8$)-Alkyl, -($C_1$-$C_8$)-Alkanoyl, -($C_1$-$C_8$)-Alkoxycarbonyl, Benzyl, Benzyloxycarbonyl;

oder
R(21) Trityl;

R(20) -OR(23) oder -NR(23)R(24);

R(23), R(24) unabhängig voneinander

Wasserstoff, -($C_1$-$C_8$)-Alkyl oder Benzyl;

k Null, 1, 2, 3 oder 4;

oder
R(1), R(2) und R(3)

unabhängig voneinander ($C_1$-$C_9$)-Heteroaryl,

das über C oder N verknüpft ist und das unsubstituiert oder substituiert ist mit 1-3 Substituenten aus der Gruppe F, Cl, $CF_3$, $CH_3$, Methoxy, Hydroxy, Amino, Methylamino und Dimethylamino;

oder
R(1), R(2) und R(3)

-SR(25), -OR(25), -NR(25)R(26), -CR(25)R(26)R(27);
R(25) -$C_fH_{2f}$-($C_1$-$C_9$)-Heteroaryl,

das unsubstituiert oder substituiert ist mit 1-3 Substituenten aus der Gruppe F, Cl, $CF_3$, $CH_3$, Methoxy, Hydroxy, Amino, Methylamino und Dimethylamino;

f Null, 1 oder 2;
R(26) und R(27)

unabhängig voneinander wie R(25) definiert oder Wasserstoff oder ($C_1$-$C_4$)-Alkyl,

oder
R(1), R(2) und R(3)

unabhängig voneinander ($C_1$-$C_9$)-Heteroaryl-N-Oxid,

das über C oder N verknüpft ist und das unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;

oder
R(1), R(2) und R(3)

unabhängig voneinander -SR(28), -OR(28), -NR(28)R(29) oder -CR(28)R(29)R(30);
R(28) -$C_gH_{2g}$-($C_1$-$C_9$)-Heteroaryl-N-Oxid,

das unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;

g Null, 1 oder 2;
R(29), R(30)

unabhängig voneinander wie R(28) definiert, Wasserstoff oder ($C_1$-$C_4$)-Alkyl;

oder
R(1), R(2) und R(3)

unabhängig voneinander Wasserstoff, F, Cl, Br, I, -C≡N, T-$(CH_2)_h$-($C_iF_{2i+1}$), R(31)$SO_l$-, R(32)R(33)N-CO-, R(34)-CO- oder R(45)R(46)N-$SO_2$, wobei die Perfluoralkylgruppe geradkettig oder verzweigt ist;

T eine Bindung, Sauerstoff, -S- oder -NR(47);
I Null, 1 oder 2;
h Null, 1 oder 2;
i 1, 2, 3, 4, 5 oder 6;
R(31), R(32), R(34) und R(45)

unabhängig voneinander -($C_1$-$C_8$)-Alkyl, -($C_3$-$C_6$)Alkenyl, $(CH_2)_n$R(48) oder -$CF_3$;
n Null, 1, 2, 3 oder 4;
R(47) Wasserstoff oder Alkyl mit 1, 2 oder 3 C-Atomen;
R(48) -($C_3$-$C_7$)-Cycloalkyl oder Phenyl,

welches nicht substituiert ist oder substituiert mit 1 - 3 Substituenten ausgewählt aus der Gruppe bestehend aus F, Cl, -$CF_3$, Methyl, Methoxy und -NR(49)R(50);

R(49) und R(50)

Wasserstoff oder Alkyl mit 1, 2, 3 oder 4 C-Atomen;

oder
R(32), R(34) und R(45)

Wasserstoff;

R(33) und R(46)

unabhängig voneinander Wasserstoff oder Alkyl mit 1, 2, 3 oder 4 C-Atomen;

oder
R(32) und R(33) sowie R(45) und R(46)

gemeinsam 5 oder 6 Methylengruppen, von denen eine $CH_2$-Gruppe durch Sauerstoff, -S-, -NH-, -$NCH_3$ oder -N-Benzyl ersetzt sein kann;

oder
R(1), R(2) und R(3)

unabhängig voneinander R(51)-A-G-D-;
R(51) ein basischer protonierbarer Rest, d.h. eine Aminogruppe -NR(52)R(53), eine Amidinogruppe R(52)R(53)N-C[=N-R(54)]- oder eine Guanidinogruppe R(52)R(53)N-C[=N-R(54)]-NR(55)-;
R(52), R(53), R(54) und R(55)

unabhängig voneinander Wasserstoff oder Alkyl mit 1, 2, 3 oder 4 C-Atomen;
oder
R(52) und R(53)

eine Gruppe $C_\alpha H_{2\alpha}$;
$\alpha$ 4, 5, 6 oder 7;
wobei im Falle von $\alpha$ = 5, 6 oder 7 ein C-Atom der Gruppe $C_\alpha H_{2\alpha}$ durch eine Heteroatomgruppe O, $SO_d$ oder NR(56) ersetzt sein kann,

oder
R(53) und R(54) oder R(54) und R(55) oder R(52) und R(55)

eine Gruppe $C_yH_{2y}$;
y 2, 3, 4 oder 5;
wobei im Falle von y = 3, 4 oder 5 ein C-Atom der Gruppe $C_yH_{2y}$ durch eine Heteroatomgruppe O, $SO_d$ oder NR(56) ersetzt sein kann;
d Null, 1 oder 2;

R(56) Wasserstoff oder Methyl;

oder
R(51) ein basisches heteroaromatisches Ringsystem mit 1 - 9 C-Atomen;
A eine Gruppe $C_eH_{2e}$;

e Null, 1, 2, 3, 4, 5, 6, 7, 8, 9 oder 10;
wobei in der Gruppe $C_eH_{2e}$ ein C-Atom durch eine der Gruppierungen -O-,-CO-, -CH[OR(57)]-, -$SO_r$-, -NR(57)-, -NR(57)-CO-, -NR(57)-CO-NH-, -NR(57)-CO-NH-$SO_2$- oder -NR(57)-$SO_2$-ersetzt sein kann;

r Null, 1 oder 2;

G einen Phenylenrest,

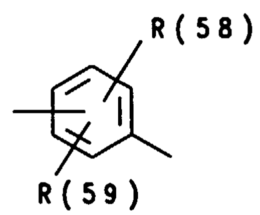

R(58) und R(59)

unabhängig voneinander Wasserstoff, Methyl, Methoxy, F, Cl, Br, J, $CF_3$ oder -$SO_s$-R(60);
R(60) Methyl oder NR(61)R(62);

R(61) und R(62)

unabhängig voneinander Wasserstoff oder Alkyl mit 1, 2, 3 oder 4 C-Atomen;

D -$C_vH_{2v}$-$E_w$-;

v Null, 1, 2, 3 oder 4;
E -O-,-CO-, -CH[OR(63)]-, -$SO_{aa}$- oder -NR(63)-;
w Null oder 1;
aa Null, 1 oder 2

R(63) Wasserstoff oder Methyl,

oder
R(1), R(2) und R(3)

unabhängig voneinander -$CF_2$R(64), -CF[R(65)][R(66)], -CF[$(CF_2)_q$-$CF_3$)][R(65)], -C[$(CF_2)_p$-$CF_3$]=CR(65)R(66);
R(64) Alkyl mit 1,2,3 oder 4 C-Atomen oder Cycloalkyl mit 3, 4, 5, 6 oder 7 C-Atomen;
R(65) und R(66) unabhängig voneinander Wasserstoff oder Alkyl mit 1, 2, 3 oder 4 C-Atomen;
q Null, 1 oder 2;
p Null, 1 oder 2;

oder
R(1), R(2) und R(3)

unabhängig voneinander -OR(67) oder -NR(67)R(68);
R(67) und R(68)

unabhängig voneinander Wasserstoff oder Alkyl mit 1, 2, 3, 4, 5 oder 6 C-Atomen;

oder
R(67) und R(68)

gemeinsam 4, 5, 6 oder 7 Methylengruppen, von denen eine $CH_2$-Gruppe durch Sauerstoff, -S-, $SO_2$, -NH-, -$NCH_3$ oder -N-Benzyl ersetzt sein kann;

R(4) und R(5)

unabhängig voneinander Wasserstoff, Alkyl mit 1, 2, 3 oder 4 C-Atomen, F, Cl, -OR(69), -NR(70)R(71) oder -$C_zF_{2z+1}$;
R(69), R(70) und R(71)

unabhängig voneinander Wasserstoff oder Alkyl mit 1, 2 oder 3 C-Atomen;

z 1, 2, 3 oder 4;

R(6) und R(7)

unabhängig voneinander Wasserstoff oder Alkyl mit 1, 2, 3 oder 4 C-Atomen;

X Sauerstoff oder NR(72);

R(72) Wasserstoff oder Alkyl mit 1, 2, 3 oder 4 C-Atomen;

sowie deren pharmazeutisch verträgliche Salze;

(HOE 95/F 115 - EP 744 397, NZ 286 622)
ak) Fluorphenylgruppen tragende Alkenylcarbonsäure-guanidide der Formel I

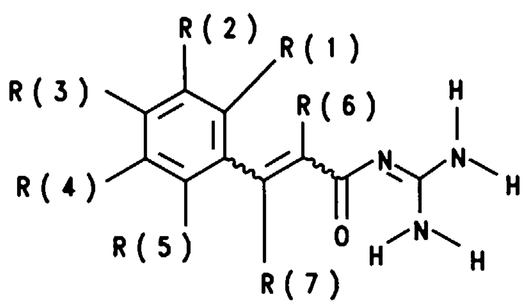

I

worin bedeuten:

R(6) Wasserstoff, ($C_1$-$C_8$)-Alkyl, ($C_3$-$C_8$)-Cycloalkyl oder Phenyl,

wobei die Phenylgruppe nicht substituiert oder substituiert ist mit 1 - 3 Substituenten ausgewählt aus der Gruppe bestehend aus F, Cl, $CF_3$, Methyl, Methoxy und NR(9)R(10);
R(9) und R(10)

Wasserstoff, ($C_1$-$C_4$)-Alkyl oder ($C_1$-$C_4$)-Perfluoralkyl;

R(7) unabhängig wie R(6) definiert;
R(1), R(2), R(3), R(4) und R(5)

unabhängig voneinander Wasserstoff oder F;

wobei jedoch mindestens einer der Reste R(1), R(2), R(3), R(4) und R(5) Fluor sein muß;

sowie deren pharmazeutisch verträgliche Salze;

(HOE 95/F 167 - NZ 299 015)
al) Benzoylguanidine der Formel I

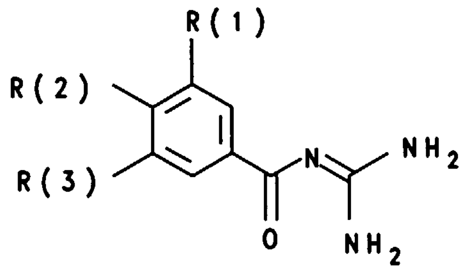

I

worin bedeuten:

R(1) R(4)-$SO_m$ oder R(5)R(6)N-$SO_2$-;

m 1 oder 2;
R(4) und R(5)

unabhängig voneinander Alkyl mit 1, 2, 3, 4, 5, 6, 7 oder 8 C-Atomen, Alkenyl mit 3, 4, 5 oder 6 C-Atomen, $CF_3$ oder -$C_nH_{2n}$-R(7);
n Null, 1, 2, 3 oder 4;
R(6) H oder Alkyl mit 1, 2, 3 oder 4 C-Atomen;
R(7) Cycloalkyl mit 3, 4, 5, 6, 7 oder 8 C-Atomen oder Phenyl,

welches nicht substituiert ist oder substituiert mit 1 - 3 Substituenten ausgewählt aus der Gruppe bestehend aus F, Cl, $CF_3$, Methyl, Methoxy und NR(8)R(9);
R(8) und R(9)

H oder Alkyl mit 1, 2, 3 oder 4 C-Atomen;

oder

R(5) auch Wasserstoff;
oder
R(5) und R(6)

gemeinsam 4 oder 5 Methylengruppen, von denen eine $CH_2$-Gruppe durch Sauerstoff, S, NH, N-$CH_3$ oder N-Benzyl ersetzt sein kann;

oder
R(1) -$O_p$-$(CH_2)_q$-$(CF_2)_r$-$CF_3$;

p Null oder 1;
q Null, 1 oder 2;
r Null, 1, 2 oder 3;

oder
R(1) -SR(10), -OR(10) oder -CR(10)R(11)R(12);

R(10), R(11) und R(12)

unabhängig voneinander Wasserstoff, Alkyl mit 1, 2, 3, 4, 5, 6, 7 oder 8 C-Atomen, $-C_sH_{2s}-(C_3-C_8)$-Cycloalkyl oder ein aromatisches System ausgewählt aus der Gruppe bestehend aus Pyridyl, Pyrrolyl, Chinolyl, Isochinolyl, Imidazolyl oder Phenyl;

s Null, 1 oder 2;

wobei die aromatischen Systeme Pyridyl, Pyrrolyl, Chinolyl, Isochinolyl, Imidazolyl und Phenyl unsubstituiert oder substituiert sind mit 1 - 3 Substituenten ausgewählt aus der Gruppe bestehend aus F, Cl, $CF_3$, $CH_3$, Methoxy, Hydroxy, Amino, Methylamino und Dimethylamino;

R(2) $-(CH_2)_u-(CF_2)_t-CF_3$;

t Null, 1, 2 oder 3;
u Null oder 1;

R(3) Wasserstoff oder unabhängig wie R(1) definiert;
sowie deren pharmazeutisch verträgliche Salze;

(HOE 95/F 173 - NZ 299 052)
am) Substituierte Zimtsäureguanidide der Formel I

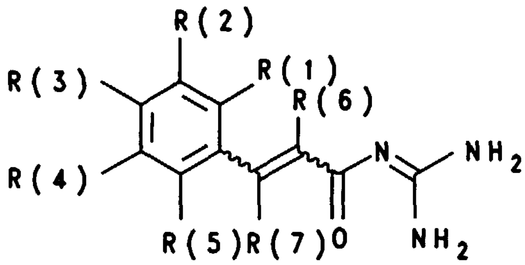

I

worin bedeuten:

mindestens einer der Substituenten R(1), R(2), R(3), R(4) und R(5)

$-X_a-Y_b-L_n-U$;
X CR(16)R(17), O, S oder NR(18);

R(16), R(17) und R(18)

unabhängig voneinander H, Alkyl mit 1, 2, 3 oder 4 C-Atomen oder Perfluoralkyl mit 1, 2, 3 oder 4 C-Atomen;

a Null oder 1;
Y Alkylen mit 1, 2, 3, 4, 5, 6, 7 oder 8 C-Atomen, Alkylen-T mit 1, 2, 3, 4, 5, 6, 7 oder 8 C-Atomen in der Alkylengruppe, T, T-Alkylen mit 1, 2, 3, 4, 5, 6, 7 oder 8 C-Atomen in der Alkylengruppe;

T NR(20), O, S oder Phenylen,

wobei das Phenylen nicht substituiert oder substituiert ist mit 1 - 3 Substituenten ausgewählt aus der Gruppe bestehend aus F, Cl, $CF_3$, Methyl, Methoxy und NR(21)R(22);
R(20), R(21) und R(22)

unabhängig voneinander H, Alkyl mit 1, 2, 3 oder 4 C-Atomen oder Perfluoralkyl mit 1, 2, 3 oder 4 C-Atomen;

b Null oder 1;

L O, S, NR(23) oder $C_kH_{2k}$;

k 1, 2, 3, 4, 5, 6, 7, 8;

n Null oder 1;
U NR(24)R(25) oder einen N-haltigen Heterocyclus mit 1, 2, 3, 4, 5, 6, 7, 8 oder 9 C-Atomen;

R(24) und R(25)

unabhängig voneinander Wasserstoff, Alkyl mit 1, 2, 3, 4, 5, 6, 7 oder 8 C-Atomen, Perfluoralkyl mit 1, 2, 3, 4, 5, 6, 7 oder 8 C-Atomen;

oder
R(24) und R(25)

gemeinsam 4 oder 5 Methylenguppen, von denen eine $CH_2$-Gruppe durch Sauerstoff, S, NH, N-$CH_3$ oder N-Benzyl ersetzt sein kann;

wobei die N-haltigen Heterocyclen N- oder C-verbrückt sind und nicht substituiert oder substituiert sind mit 1 - 3 Substituenten ausgewählt aus der Gruppe bestehend aus F, Cl, $CF_3$, Methyl, Methoxy und NR(27)R(28);
R(23), R(27) und R(28)

unabhängig voneinander H, Alkyl mit 1, 2, 3 oder 4 C-Atomen oder Perfluoralkyl mit 1, 2, 3 oder 4 C-Atomen;

und die jeweils anderen Substituenten R(1), R(2), R(3), R(4) und R(5)

unabhängig voneinander H, F, Cl, Br, I, CN, $-O_n-C_mH_{2m+1}$, $-O_p-(CH_2)_s-C_qF_{2q+1}$ oder $-C_rH_{2r}R(10)$;
n Null oder 1;
m Null 1, 2, 3, 4, 5, 6, 7 oder 8;
p Null oder 1;
q 1, 2, 3, 4, 5, 6, 7 oder 8;
s Null, 1, 2, 3 oder 4;
r Null, 1, 2, 3 oder 4;
R(10)

Cycloalkyl mit 3, 4, 5, 6, 7 oder 8 C-Atomen, oder Phenyl,

wobei das Phenyl nicht substituiert oder substituiert ist mit 1 - 3 Substituenten ausgewählt aus der Gruppe bestehend aus F, Cl, $CF_3$, Methyl, Methoxy und NR(11)R(12);
R(11) und R(12)

unabhängig voneinander H, Alkyl mit 1, 2, 3 oder 4 C-Atomen oder Perfluoralkyl mit 1, 2, 3 oder 4 C-Atomen;

R(6) und R(7)

unabhängig voneinander Wasserstoff, F, Cl, Br, I, CN, Alkyl mit 1, 2, 3, 4, 5, 6, 7 oder 8 C-Atomen, Perfluoralkyl mit 1, 2, 3, 4, 5, 6, 7 oder 8 C-Atomen, Cycloalkyl mit 3, 4, 5, 6, 7 oder 8 C-Atomen, oder Phenyl,

das nicht substituiert oder substituiert ist mit 1 - 3 Substituenten ausgewählt aus der Gruppe bestehend aus F, Cl, $CF_3$, Methyl, Methoxy und NR(14)R(15);
R(14) und R(15)

unabhängig voneinander H, Alkyl mit 1, 2, 3 oder 4 C-Atomen oder Perfluoralkyl mit 1, 2, 3 oder 4 C-Atomen;

sowie deren pharmazeutisch verträgliche Salze;

(HOE 95/F 220 - NZ 299 052)
an) Benzoylguanidine der Formel I

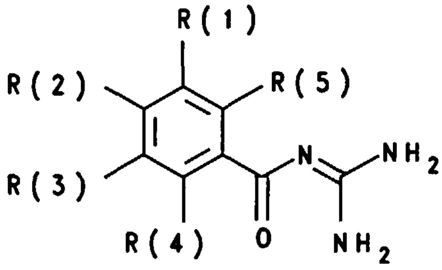

I

worin bedeuten:

mindestens einer der Substituenten R(1), R(2) und R(3)

R(6)-C(OH)$_2$-;
R(6) Perfluoralkyl mit 1, 2 oder 3-C-Atomen, das geradkettig oder verzweigt ist;

und die übrigen Substituenten R(1), R(2) und R(3)

unabhängig voneinander Wasserstoff, OH, F, Cl, Br, I, Alkyl mit 1, 2, 3, 4, 5 oder 6 C-Atomen, Cycloalkyl mit 3, 4, 5 oder 6 C-Atomen, Alkoxy mit 1, 2, 3 oder 4 C-Atomen oder Phenoxy,

das unsubstituiert ist oder substituiert ist mit 1 - 3 Substituenten ausgewählt auf der Gruppe bestehend aus F, Cl, Methyl und Methoxy;

oder
die übrigen Substituenten R(1), R(2) und R(3)

unabhängig voneinander Alkyl-SO$_x$, -CR(7)=CR(8)R(9) oder -C≡CR(9);
x Null, 1 oder 2;
R(7) Wasserstoff oder Methyl;
R(8) und R(9)

unabhängig voneinander Wasserstoff, Alkyl mit 1, 2, 3 oder 4 C-Atomen, Cycloalkyl mit 3, 4, 5, 6, 7 oder 8 C-Atomen oder Phenyl,

das unsubstituiert ist oder substituiert ist mit 1 - 3 Substituenten ausgewählt auf der Gruppe bestehend aus F, Cl, $CF_3$, Methyl und Methoxy;

oder
die übrigen Substituenten R(1), R(2) und R(3)

unabhängig voneinander Phenyl, $C_6H_5$-($C_1$-$C_4$)-Alkyl, Naphthyl, Biphenylyl, Chinolinyl, Isochinolinyl oder Imidazolyl,

wobei Chinolinyl, Isochinolinyl oder Imidazolyl über C oder N gebunden sind, und wobei Phenyl, $C_6H_5$-($C_1$-$C_4$)-Alkyl, Naphthyl, Biphenylyl, Chinolinyl, Isochinolinyl und Imidazolyl unsubstituiert sind oder substituiert sind mit 1 - 3 Substituenten ausgewählt aus der Gruppe bestehend aus F, Cl, $CF_3$, $CH_3$, Methoxy, Hydroxy, Amino, Methylamino und Dimethylamino;

oder
die übrigen Substituenten R(1), R(2) und R(3)

unabhängig voneinander SR(10), -OR(10), -CR(10)R(11)R(12); R(10)

$-C_fH_{2f}-(C_3-C_8)$-Cycloalkyl, Chinolinyl, Isochinolinyl, Pyridinyl, Imidazolyl oder Phenyl,

wobei die aromatischen Systeme Chinolinyl, Isochinolinyl, Pyridinyl, Imidazolyl und Phenyl unsubstituiert sind oder substituiert sind mit 1 - 3 Substituenten ausgewählt aus der gruppe bestehend aus F, Cl, $CF_3$, $CH_3$, Methoxy, Hydroxy, Amino, Methylamino und Dimethylamino;

f Null, 1 oder 2;

R(11) und R(12)

unabhängig voneinander wie R(10) definiert, Wasserstoff oder Alkyl mit 1, 2, 3 oder 4 C-Atomen;

R(4) und R(5)

unabhängig voneinander Wasserstoff, Alkyl mit 1, 2 oder 3-C-Atomen, F, Cl, Br, I, CN, OR(13), NR(14)R(15), $-(CH_2)_n-(CF_2)_o-CF_3$;
R(13), R(14) und R(15)

unabhängig voneinander Wasserstoff oder Alkyl mit 1, 2, 3 oder 4 C-Atomen;

n Null oder 1;
o Null, 1 oder 2;

sowie deren pharmakologisch akzeptable Salze;

(HOE 95/F 253 - NZ 299 682)
ao) Sulfonimidamide der Formel I

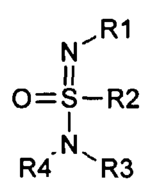

I

worin bedeuten:

mindestens einer der drei Substituenten R(1), R(2) und R(3)

ein Benzoylguanidin,

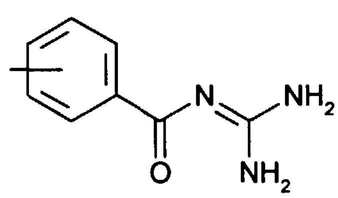

das im Phenylteil unsubstituiert oder substituiert ist mit 1 - 4 Resten ausgewählt aus der Gruppe bestehend aus Alkyl mit 1, 2, 3, 4, 5, 6, 7 oder 8 C-Atomen, Alkenyl mit 2, 3, 4, 5, 6, 7 oder 8 C-Atomen, $-(CH_2)_m-$R(14), F, Cl, Br, I, -C≡N, $CF_3$, R(22)$SO_2$-, R(23)R(24)N-CO-, R(25)-CO-, R(26)R(27)N-$SO_2$, -OR(35), -SR(35) oder -NR(35)R(36);

m Null, 1 oder 2;
R(14)

-($C_3$-$C_8$)-Cycloalkyl oder Phenyl,

welches nicht substituiert oder substituiert ist mit 1 - 3 Substituenten ausgewählt aus der Gruppe bestehend aus F und Cl, -$CF_3$, Methyl, Methoxy und -NR(15)R(16);
R(15) und R(16)

unabhängig voneinander Wasserstoff oder -$CH_3$;

R(22), R(23), R(25) und R(26)

unabhängig voneinander Alkyl mit 1, 2, 3, 4, 5, 6, 7 oder 8 C-Atomen, Alkenyl mit 2, 3, 4, 5, 6, 7 oder 8 C-Atomen, $(CH_2)_n$R(29) oder -$CF_3$;
n Null, 1, 2, 3 oder 4;
R(29) -($C_3$-$C_7$)-Cycloalkyl oder Phenyl,

welches nicht substituiert ist oder substituiert mit 1 - 3 Substituenten ausgewählt aus der Gruppe bestehend aus F, Cl, -$CF_3$, Methyl, Methoxy und -NR(30)R(31);
R(30) und R(31)

Wasserstoff oder Alkyl mit 1, 2, 3 oder 4 C-Atomen;

oder
R(23), R(25) und R(26)

Wasserstoff;

R(24) und R(27)

unabhängig voneinander Wasserstoff oder Alkyl mit 1, 2, 3 oder 4 C-Atomen;

oder
R(23) und R(24) sowie R(26) und R(27)

gemeinsam 5 oder 6 Methylengruppen, von denen eine $CH_2$-Gruppe durch Sauerstoff, -S-, -NH-, -$NCH_3$ oder -N-Benzyl ersetzt sein kann;

R(35) und R(36)

unabhängig voneinander Wasserstoff oder Alkyl mit 1, 2, 3, 4, 5 oder 6 C-Atomen;

oder
R(35) und R(36)

gemeinsam 4 - 7 Methylengruppen, von denen eine $CH_2$-Gruppe durch Sauerstoff, -S-, -NH-, -$NCH_3$ oder -N-Benzyl ersetzt sein kann;

oder
R(35) Phenyl,

welches nicht substituiert ist oder substituiert mit 1 - 3 Substituenten ausgewählt aus der Gruppe bestehend aus F, Cl, -$CF_3$, Methyl, Methoxy, $SO_2$R(5), $SO_2$NR(6)R(7) und -NR(32)R(33);
R(5) Alkyl mit 1, 2, 3, 4, 5 oder 6 C-Atomen
R(6) und R(7)

unabhängig voneinander Wasserstoff oder Alkyl mit 1, 2, 3 oder 4 C-Atomen;

R(32) und R(33)

unabhängig voneinander Wasserstoff oder Alkyl mit 1, 2, 3 oder 4 C-Atomen;

oder
R(35) $C_1$-$C_9$-Heteroaryl,

das unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;

und die jeweils anderen Substituenten R(1), R(2) und R(3)

unabhängig voneinander Alkyl mit 1, 2, 3, 4, 5, 6, 7 oder 8 C-Atomen, $(CH_2)_pR(10)$
p Null, 1, 2, 3 oder 4;
R(10) Phenyl,

welches nicht substituiert ist oder substituiert mit 1 - 3 Substituenten ausgewählt aus der Gruppe bestehend aus F, Cl, $-CF_3$, Methyl, Methoxy, $-SO_2NR(17)R(8)$ und $-SO_2R(9)$;
R(17) und R(8)

unabhängig voneinander Wasserstoff oder Alkyl mit 1, 2, 3 oder 4 C-Atomen;

R(9) Alkyl mit 1, 2, 3 oder 4 C-Atomen;

oder
der jeweils andere Rest R(1) und R(3)

Wasserstoff,

R(4) Wasserstoff oder Alkyl mit 1, 2, 3 oder 4 C-Atomen;
sowie deren pharmazeutisch verträgliche Salze;

(HOE 95/F 265 - NZ 299 739)
ap) Benzoylguanidine der Formel I

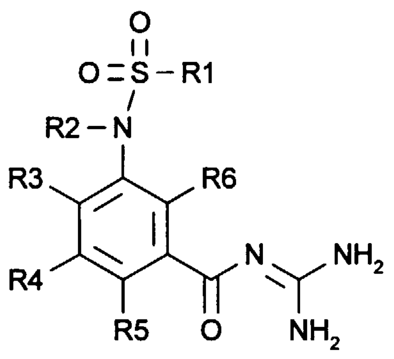

I

worin bedeuten:

R(1) Alkyl mit 1, 2, 3, 4, 5, 6, 7 oder 8 C-Atomen oder NR(7)R(8);

R(7) und R(8)

unabhängig voneinander Wasserstoff oder Alkyl mit 1, 2, 3, 4, 5, 6, 7 oder 8 C-Atomen

R(2) Wasserstoff, Alkyl mit 1, 2, 3, 4, 5, 6, 7 oder 8 C-Atomen oder - $SO_2R(9)$

R(9) unabhängig wie R(1) definiert;

R(3) Wasserstoff, -SR(25), -OR(25), -NR(25)R(26) oder -CR(25)R(26)R(27);

R(25) Wasserstoff, Alkyl mit 1, 2, 3, 4, 5, 6, 7 oder 8 C-Atomen, oder Phenyl,

das unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;

oder
R(25)

-($C_1$-$C_9$)-Heteroaryl,

das unsubstituiert oder substituiert ist mit 1 - 3 Substituenten ausgewählt aus der Gruppe bestehend aus der Gruppe F, Cl, $CF_3$, $CH_3$, Methoxy, Hydroxy, Amino, Methylamino und Dimethylamino;

R(26) und R(27)

unabhängig voneinander wie R(25) definiert oder Wasserstoff oder Alkyl mit 1, 2, 3, 4, 5, 6, 7 oder 8 C-Atomen;

R(4) Wasserstoff, F, Cl, Br, I, OH, -C≡N, $CF_3$, Alkyl mit 1, 2, 3, 4, 5, 6, 7 oder 8 C-Atomen, Alkenyl mit 2, 3, 4, 5, 6, 7 oder 8 C-Atomen oder $-(CH_2)_mR(14)$;

m Null, 1 oder 2;
R(14) -($C_3$-$C_8$)-Cycloalkyl oder Phenyl,

welches nicht substituiert oder substituiert ist mit 1 - 3 Substituenten ausgewählt aus der Gruppe bestehend aus F und Cl, -$CF_3$, Methyl, Methoxy und -NR(15)R(16);
R(15) und R(16)

unabhängig voneinander Wasserstoff oder -$CH_3$;

R(5) und R(6)

unabhängig voneinander Wasserstoff, Alkyl mit 1, 2, 3 oder 4 C-Atomen, F, Cl, -OR(32), -NR(33)R(34) oder $CF_3$;
R(32), R(33) und R(34)

unabhängig voneinander Wasserstoff oder Alkyl mit 1, 2, 3 oder 4 C-Atomen;

sowie deren pharmazeutisch verträgliche Salze;

(HOE 95/F 269 K - EP 774 458)
aq) Benzoldicarbonsäure-diguanidide der Formel I

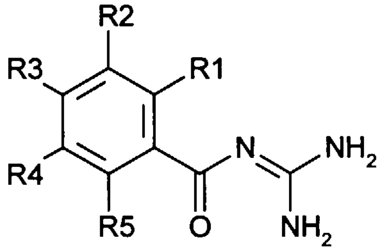

I

worin bedeuten:

einer der Reste R(1), R(2), R(3) und R(4)

-CO-N=C($NH_2$)$_2$;

und die jeweils anderen Reste R(1), R(2), R(3) und R(4):
R(1) Wasserstoff, Alkyl mit 1, 2, 3 oder 4 C-Atomen, F, Cl, Br, I, -OR(32), -NR(33)R(34) oder $CF_3$;

R(32), R(33) und R(34)

unabhängig voneinander Wasserstoff oder Alkyl mit 1, 2, 3 oder 4 C-Atomen;

R(2) und R(4)

unabhängig voneinander Wasserstoff, F, Cl, Br, I, OH, -CN, $CF_3$, -CO-N=C($NH_2$)$_2$, Alkyl mit 1, 2, 3, 4, 5, 6, 7 oder 8 C-Atomen, Alkenyl mit 2, 3, 4, 5, 6, 7 oder 8 C-Atomen oder -($CH_2$)$_m$R(14);
m Null, 1 oder 2;
R(14) -($C_3$-$C_8$)-Cycloalkyl oder Phenyl,

welches nicht substituiert oder substituiert ist mit 1 - 3 Substituenten ausgewählt aus der Gruppe bestehend aus F und Cl, -$CF_3$, Methyl, Methoxy und -NR(15)R(16);
R(15) und R(16)

Wasserstoff oder -$CH_3$;

oder
R(2) und R(4)

unabhängig voneinander Pyrrol-1-yl, Pyrrol-2-yl oder Pyrrol-3-yl,

welches nicht substituiert oder substituiert ist mit 1 - 4 Substituenten ausgewählt aus der Gruppe bestehend aus F, Cl, Br, I, -CN, ($C_2$-$C_8$)-Alkanoyl, ($C_2$-$C_8$)-Alkoxycarbonyl, Formyl, Carboxy, - $CF_3$, Methyl, Methoxy;

oder
R(2) und R(4)

unabhängig voneinander R(22)-$SO_2$-, R(23)R(24)N-CO-, R(28)-CO- oder R(29)R(30)N-$SO_2$;
R(22) und R(28)

unabhängig voneinander Methyl oder -$CF_3$;

R(23), R(24), R(29) und R(30)

unabhängig voneinander Wasserstoff oder Methyl;

oder
R(2) und R(4)

unabhängig voneinander -OR(35) oder -NR(35)R(36);
R(35) und R(36)

unabhängig voneinander Wasserstoff oder Alkyl mit 1, 2, 3, 4, 5 oder 6 C-Atomen;

oder
R(35) und R(36)

gemeinsam 4 - 7 Methylengruppen, von denen eine $CH_2$-Gruppe durch Sauerstoff, -S-, -NH-, -$NCH_3$ oder -N-Benzyl ersetzt sein kann;

R(3) Wasserstoff, -SR(25), -OR(25), -NR(25)R(26), -CR(25)R(26)R(27);

R(25) Wasserstoff, Alkyl mit 1, 2, 3, 4, 5, 6, 7 oder 8 C-Atomen oder Phenyl,

das unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;

oder
R(25) -($C_1$-$C_9$)-Heteroaryl,

das unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;

R(26) und R(27)

unabhängig voneinander wie R(25) definiert oder Wasserstoff oder Alkyl mit 1, 2, 3, 4, 5, 6, 7 oder 8 C-Atomen;

R(5) Alkyl mit 1, 2, 3 oder 4 C-Atomen, F, Cl, Br, I, X-$(CH_2)_y$-$CF_3$ oder Phenyl,

welches nicht substituiert oder substituiert ist mit 1 - 3 Substituenten ausgewählt aus der Gruppe bestehend aus F und Cl, - $CF_3$, Methyl, Methoxy und -NR(6)R(7);
R(6) und R(7)

unabhängig voneinander Wasserstoff oder -$CH_3$;

X eine Bindung oder Sauerstoff;
y Null, 1 oder 2;

sowie deren pharmazeutisch verträgliche Salze;

(HOE 95/F 269 BK - EP 774 457)
ar) Benzoldicarbonsäure-diguanidide der Formel I

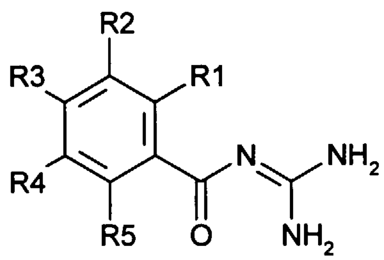

I

worin bedeuten:

einer der Reste R(1), R(2), R(3) und R(5)

-CO-N=C$(NH_2)_2$;

und die jeweils anderen Reste R(1), R(2), R(3) und R(5):

R(1) und R(5)

unabhängig voneinander Wasserstoff, Alkyl mit 1, 2, 3 oder 4 C-Atomen, F, Cl, -OR(32), -NR(33)R(34) oder $CF_3$;
R(32), R(33) und R(34)

unabhängig voneinander Wasserstoff oder Alkyl mit 1, 2, 3 oder 4 C-Atomen;

R(2) Wasserstoff, F, Cl, Br, I, OH, -C°N, $CF_3$, -CO-N=C($NH_2$)$_2$, Alkyl mit 1, 2, 3, 4, 5, 6, 7 oder 8 C-Atomen, Alkenyl mit 2, 3, 4, 5, 6, 7 oder 8 C-Atomen oder -$(CH_2)_m$R(14);

m Null, 1 oder 2;
R(14) -($C_3$-$C_8$)-Cycloalkyl oder Phenyl,

welches nicht substituiert oder substituiert ist mit 1 - 3 Substituenten ausgewählt aus der Gruppe bestehend aus F und Cl, -$CF_3$, Methyl, Methoxy und -NR(15)R(16);
R(15) und R(16)

unabhängig voneinander Wasserstoff oder -$CH_3$;

oder

R(2) R(22)-$SO_2$-, R(23)R(24)N-CO-, R(28)-CO- oder R(29)R(30)N-$SO_2$;

R(22) und R(28)

unabhängig voneinander Methyl oder -$CF_3$;

R(23), R(24), R(29) und R(30)

unabhängig voneinander Wasserstoff oder Methyl;

oder

R(2) -OR(35) oder -NR(35)R(36);

R(35) und R(36)

unabhängig voneinander Wasserstoff oder Alkyl mit 1, 2, 3, 4, 5 oder 6 C-Atomen;

oder
R(35) und R(36)

gemeinsam 4 - 7 Methylengruppen, von denen eine $CH_2$-Gruppe durch Sauerstoff, -S-, -NH-, -$NCH_3$ oder -N-Benzyl ersetzt sein kann;

R(3) Wasserstoff, -SR(25), -OR(25), -NR(25)R(26), -CR(25)R(26)R(27);

R(25) Wasserstoff, Alkyl mit 1, 2, 3, 4, 5, 6, 7 oder 8 C-Atomen oder Phenyl,

das unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;

oder
R(25 -($C_1$-$C_9$)-Heteroaryl,

das unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;

R(26) und R(27)

unabhängig voneinander wie R(25) definiert oder Wasserstoff oder Alkyl mit 1, 2, 3, 4, 5, 6, 7 oder 8 C-Atomen;

R(4) $CF_3$, Alkyl mit 1, 2, 3, 4, 5, 6, 7 oder 8 C-Atomen, Alkenyl mit 2, 3, 4, 5, 6, 7 oder 8 C-Atomen, -($C_3$-$C_8$)-Cycloalkyl oder -$(CH_2)_m$R(14);

m 1 oder 2;
R(14) -($C_3$-$C_8$)-Cycloalkyl oder Phenyl,

welches nicht substituiert oder substituiert ist mit 1 - 3 Substituenten ausgewählt aus der Gruppe bestehend aus F und Cl, -$CF_3$, Methyl, Methoxy und -NR(15)R(16);
R(15) und R(16)

unabhängig voneinander Wasserstoff oder -$CH_3$;

oder

R(4) Phenyl,

welches substituiert ist mit 2, 3, 4 oder fünf Substituenten ausgewählt aus der Gruppe bestehend aus F, Cl, $CF_3$, Methyl, Methoxy und -NR(15)R(16);

R(15) und R(16)

unabhängig voneinander Wasserstoff oder $CH_3$;

sowie deren pharmazeutisch verträgliche Salze;

(HOE 96/F 013 - EP-OS 787 717)
as) Diaryldicarbonsäure-diguanidide der Formel I

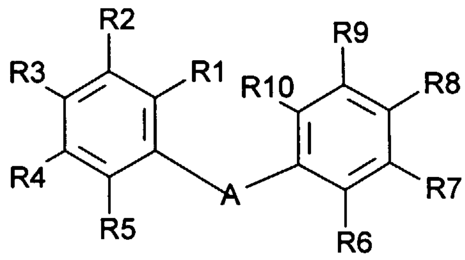

I

worin bedeuten:

einer der Reste R(1), R(2), R(3), R(4) und R(5)

-CO-N=C$(NH_2)_2$;

die jeweils anderen Reste R(1) und R(5)

unabhängig voneinander Wasserstoff, Alkyl mit 1, 2, 3 oder 4 C-Atomen, F, Cl, -OR(32), -NR(33)R(34) oder $CF_3$;
R(32), R(33) und R(34)

unabhängig voneinander Wasserstoff oder Alkyl mit 1, 2, 3 oder 4 C-Atomen;

die jeweils anderen Reste R(2) und R(4)

unabhängig voneinander Wasserstoff, F, Cl, Br, I, OH, -CN, $CF_3$, -CO-N=C$(NH_2)_2$, Alkyl mit 1, 2, 3, 4, 5, 6, 7 oder 8 C-Atomen, Alkenyl mit 2, 3, 4, 5, 6, 7 oder 8 C-Atomen oder -$(CH_2)_m$R(14);
m Null, 1 oder 2;
R(14) -($C_3$-$C_8$)-Cycloalkyl oder Phenyl,

welches nicht substituiert oder substituiert ist mit 1 - 3 Substituenten ausgewählt aus der Gruppe bestehend aus F und Cl, -$CF_3$, Methyl, Methoxy und -NR(15)R(16);
R(15) und R(16)

Wasserstoff oder -$CH_3$;

oder
die jeweils anderen Reste R(2) und R(4)

unabhängig voneinander Pyrrol-1-yl, Pyrrol-2-yl oder Pyrrol-3-yl,

welches nicht substituiert oder substituiert ist mit 1 - 4 Substituenten ausgewählt aus der Gruppe bestehend aus F, Cl, Br, I, -CN, ($C_2$-$C_8$)-Alkanoyl, ($C_2$-$C_8$)-Alkoxycarbonyl, Formyl, Carboxy, - $CF_3$, Methyl, Methoxy;

oder
die jeweils anderen Reste R(2) und R(4)

R(22)-$SO_2$-, R(23)R(24)N-CO-, R(28)-CO- oder R(29)R(30)N-SO2;
R(22) und R(28)

unabhängig voneinander Methyl oder -$CF_3$;

R(23), R(24), R(29) und R(30)

unabhängig voneinander Wasserstoff oder Methyl;

oder
die jeweils anderen Reste R(2) und R(4)

unabhängig voneinander -OR(35) oder -NR(35)R(36);
R(35) und R(36)

unabhängig voneinander Wasserstoff oder Alkyl mit 1, 2, 3, 4, 5 oder 6 C-Atomen;

oder
R(35) und R(36)

gemeinsam 4 - 7 Methylengruppen, von denen eine $CH_2$-Gruppe durch Sauerstoff, -S-, -NH-, -$NCH_3$ oder -N-Benzyl ersetzt sein kann;

der jeweils andere Rest R(3)

Wasserstoff, -SR(25), -OR(25), -NR(25)R(26), -CR(25)R(26)R(27);
R(25) Wasserstoff, Alkyl mit 1, 2, 3, 4, 5, 6, 7 oder 8 C-Atomen oder Phenyl,

das unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;

oder
R(25) -($C_1$-$C_9$)-Heteroaryl,

das unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;

R(26) und R(27)

unabhängig voneinander wie R(25) definiert oder Wasserstoff oder Alkyl mit 1, 2, 3, 4, 5, 6, 7 oder 8 C-Atomen;

einer der Reste R(6), R(7), R(8), R(9) und R(10)

$-CO-N=C(NH_2)_2$;

die jeweils anderen Reste R(6) und R(10)

unabhängig voneinander Wasserstoff, Alkyl mit 1, 2, 3 oder 4 C-Atomen, F, Cl, -OR(132), -NR(133)R(134) oder $CF_3$;
R(132), R(133) und R(134)

unabhängig voneinander Wasserstoff oder Alkyl mit 1, 2, 3 oder 4 C-Atomen;

die jeweils anderen Reste R(7) und R(9)

unabhängig voneinander Wasserstoff, F, Cl, Br, I, OH, -CN, $CF_3$, $-CO-N=C(NH_2)_2$, Alkyl mit 1, 2, 3, 4, 5, 6, 7 oder 8 C-Atomen, Alkenyl mit 2, 3, 4, 5, 6, 7 oder 8 C-Atomen oder $-(CH_2)_{mm}R(114)$;
mm Null, 1 oder 2;
R(114)

$-(C_3-C_8)$-Cycloalkyl oder Phenyl,

welches nicht substituiert oder substituiert ist mit 1 - 3 Substituenten ausgewählt aus der Gruppe bestehend aus F und Cl, $-CF_3$, Methyl, Methoxy und -NR(115)R(116);
R(115) und R(116)

Wasserstoff oder $-CH_3$;

oder
die jeweils anderen Reste R(7) und R(9)

unabhängig voneinander Pyrrol-1-yl, Pyrrol-2-yl oder Pyrrol-3-yl,

welches nicht substituiert oder substituiert ist mit 1 - 4 Substituenten ausgewählt aus der Gruppe bestehend aus F, Cl, Br, I, -CN, $(C_2-C_8)$-Alkanoyl, $(C_2-C_8)$-Alkoxycarbonyl, Formyl, Carboxy, - $CF_3$, Methyl und Methoxy;

oder
die jeweils anderen Reste R(7) und R(9)

$R(122)-SO_2-$, R(123)R(124)N-CO-, R(128)-CO- oder $R(129)R(130)N-SO_2$;
R(122) und R(128)

unabhängig voneinander Methyl oder $-CF_3$;

R(123), R(124), R(129) und R(130)

unabhängig voneinander Wasserstoff oder Methyl;

oder
die jeweils anderen Reste R(7) und R(9)

unabhängig voneinander -OR(135) oder -NR(135)R(136);
R(135) und R(136)

unabhängig voneinander Wasserstoff oder Alkyl mit 1, 2, 3, 4, 5 oder 6 C-Atomen;

oder

R(135) und R(136)

gemeinsam 4 - 7 Methylengruppen, von denen eine $CH_2$-Gruppe durch Sauerstoff, -S-, -NH-, -$NCH_3$ oder -N-Benzyl ersetzt sein kann;

der jeweils andere Rest R(8)

Wasserstoff, -SR(125), -OR(125), -NR(125)R(126) oder -CR(125)R(126)R(127);

R(125)

Wasserstoff, Alkyl mit 1, 2, 3, 4, 5, 6, 7 oder 8 C-Atomen oder Phenyl,

das unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;

oder

R(125)

-($C_1$-$C_9$)-Heteroaryl,

das unsubstituiert oder substituiert ist mit 1 - 3 Substituenten ausgewählt aus der Gruppe bestehend aus der Gruppe F, Cl, $CF_3$, $CH_3$, Methoxy, Hydroxy, Amino, Methylamino und Dimethylamino;

R(126) und R(127)

unabhängig voneinander wie R(125) definiert oder Wasserstoff oder Alkyl mit 1, 2, 3, 4, 5, 6, 7 oder 8 C-Atomen;

A abwesend, -NR(11)-CO-, -NR(12)-CO-NR(13)-, -NR(17)-CO-NR(18)-$SO_2$-, - NR(19)-$SO_2$-, -$SO_2$-NR(19)-$SO_2$, -$SO_2$-NR(19)-CO-, -O-CO-NR(19)-$SO_2$- oder -CR(20)=CR(21)-;

R(11), R(12), R(13), R(17), R(18), R(19), R(20) und R(21)

unabhängig voneinander Wasserstoff oder Alkyl mit 1, 2, 3, 4, 5, 6, 7 oder 8 C-Atomen

sowie deren pharmazeutisch verträgliche Salze;

(HOE 96/F 026 - EP 790 245)

at) Substituierte Thiophenylalkenylcarbonsäureguanidide der Formel I

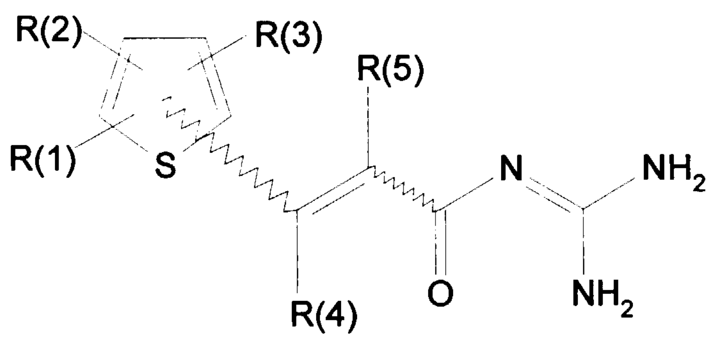

I

worin bedeuten:

mindestens einer der Substituenten R(1), R(2) und R(3)

$-O_p-(CH_2)_s-C_qF_{2q+1}$, R(40)CO- oder $R(31)SO_k$-;
p Null oder 1;
s Null, 1, 2, 3 oder 4;
q 1, 2, 3, 4, 5, 6, 7 oder 8;
k Null, 1 oder 2;
R(40) Alkyl mit 1, 2, 3, 4, 5, 6, 7 oder 8 C-Atomen, Perfluoralkyl mit 1, 2, 3, 4, 5, 6, 7 oder 8 C-Atomen, Cycloalkyl mit 3, 4, 5, 6, 7 oder 8 C-Atomen, oder Phenyl,

das nicht substituiert oder substituiert ist mit 1 - 3 Substituenten ausgewählt aus der Gruppe bestehend aus F, Cl, $CF_3$, Methyl und Methoxy;

R(31) Alkyl mit 1, 2, 3, 4, 5, 6, 7 oder 8 C-Atomen, Perfluoralkyl mit 1, 2, 3, 4, 5, 6, 7 oder 8 C-Atomen, Cycloalkyl mit 3, 4, 5, 6, 7 oder 8 C-Atomen, oder Phenyl,

das nicht substituiert oder substituiert ist mit 1 - 3 Substituenten ausgewählt aus der Gruppe bestehend aus F, Cl, $CF_3$, Methyl oder Methoxy;

oder
R(31) NR(41)R(42);

R(41) und R(42)

unabhängig voneinander Wasserstoff, Alkyl mit 1, 2, 3 oder 4 C-Atomen, Perfluoralkyl mit 1, 2, 3 oder 4 C-Atomen,

oder
R(41) und R(42)

gemeinsam 4 oder 5 Methylengruppen, von denen eine $CH_2$-Gruppe durch Sauerstoff, S, NH, $N-CH_3$ oder N-Benzyl ersetzt sein kann;

und die jeweils anderen Substituenten R(1), R(2) und R(3)

unabhängig voneinander H, F, Cl, Br, I, CN, $-O_{na}-C_{ma}H_{2ma+1}$ oder - $O_{ga}C_{ra}H_{2ra}R(10)$;
na Null oder 1;
ma Null, 1, 2, 3, 4, 5, 6, 7 oder 8;
ga Null oder 1;
ra Null, 1, 2, 3 oder 4;
R(10) Cycloalkyl mit 3, 4, 5, 6, 7 oder 8 C-Atomen oder Phenyl,

wobei das Phenyl nicht substituiert oder substituiert ist mit 1 - 3 Substituenten ausgewählt aus der Gruppe bestehend aus F, Cl, $CF_3$, Methyl und Methoxy;

R(4) und R(5)

unabhängig voneinander Wasserstoff, F, Cl, Br, I, CN, Alkyl mit 1, 2, 3, 4, 5, 6, 7 oder 8 C-Atomen, Perfluoralkyl mit 1, 2, 3, 4, 5, 6, 7 oder 8 C-Atomen, Cycloalkyl mit 3, 4, 5, 6, 7 oder 8 C-Atomen oder Phenyl,

das nicht substituiert oder substituiert ist mit 1 - 3 Substituenten ausgewählt aus der Gruppe bestehend aus F, Cl, $CF_3$, Methyl, Methoxy und NR(14)R(15);
R(14) und R(15)

unabhängig voneinander H, Alkyl mit 1, 2, 3 oder 4 C-Atomen oder Perfluoralkyl mit 1, 2, 3 oder 4 C-Atomen;

sowie deren pharmazeutisch verträgliche Salze;

(HOE 96/F 032 - EP 791 577)

au) Ortho-substituierte Benzoylguanidine der Formel I

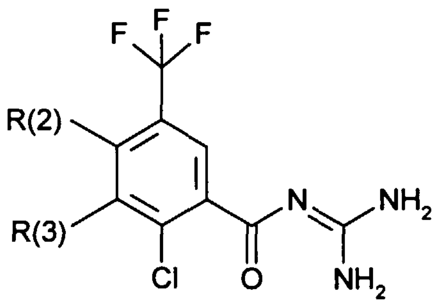

worin bedeuten:

R(2) und R(3)

unabhängig voneinander Wasserstoff, Cl, Br, I, $(C_1-C_8)$-Alkyl, $(C_3-C_8)$-Cycloalkyl oder -OR(5);
R(5) $(C_1-C_8)$-Alkyl oder $-C_dH_{2d}-(C_3-C_8)$-Cycloalkyl;

d Null, 1 oder 2;

wobei stets einer der beiden Substituenten R(2) und R(3) Wasserstoff ist, jedoch nicht beide Substituenten R(2) und R(3) gleichzeitig Wasserstoff sind,
sowie deren pharmazeutisch verträgliche Salze;

(HOE 96/F 042 - EP 794 171)
av) Benzoylguanidine der Formel I

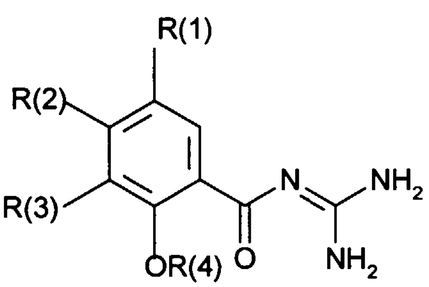

worin bedeuten:

R(1) H, F, Cl, Br, I, CN, $NO_2$, Alkyl mit 1, 2, 3, 4, 5, 6, 7 oder 8 C-Atomen, Alkoxy mit 1, 2, 3, 4, 5, 6, 7 oder 8 C-Atomen, Cycloalkyl mit 3, 4, 5, 6, 7 oder 8 C-Atomen, Cycloalkoxy mit 3, 4, 5, 6, 7 oder 8 C-Atomen oder $X_a-(CH_2)_b-(CF_2)_c-CF_3$;

X Sauerstoff, S, NR(5),
a Null oder 1;
b Null, 1 oder 2;
c Null, 1, 2 oder 3;

R (5) H, Alkyl mit 1, 2, 3 oder 4 C-Atomen oder $-C_dH_{2d}R(6)$;

d Null, 1, 2, 3 oder 4;
R(6) Cycloalkyl mit 3, 4, 5, 6, 7 oder 8 C-Atomen, Phenyl, Biphenylyl oder Naphthyl,

wobei die Aromaten Phenyl, Biphenylyl oder Naphthyl nicht substituiert oder substituiert sind mit 1 - 3 Substituenten ausgewählt aus der Gruppe bestehend aus F, Cl, $CF_3$, Methyl, Methoxy und NR(7)R(8);

R(7) und R(8)

unabhängig H oder Alkyl mit 1, 2, 3 oder 4 C-Atomen;

oder

R(1) -SR(10), -OR(10) oder -CR(10)R(11)R(12);

R(10) -$C_fH_{2f}$-Cycloalkyl mit 3, 4, 5, 6, 7 oder 8 C-Atomen im Cycloalkylring, oder Phenyl,

wobei Phenyl unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;

f Null, 1 oder 2;

R(11) und R(12)

unabhängig voneinander wie R(10) definiert oder Wasserstoff oder Alkyl mit 1, 2, 3 oder 4 C-Atomen;

oder

R(1) Phenyl, Naphthyl, Biphenylyl oder Heteroaryl mit 1, 2, 3, 4, 5, 6, 7, 8 oder 9 C-Atomen, letzteres über ein C- oder ein N-Atom des Rings verknüpft,

die jeweils unsubstituiert oder substituiert sind mit 1 - 3 Substituenten ausgewählt aus der Gruppe bestehend aus F, Cl, $CF_3$, $CH_3$, Methoxy, Hydroxy, Amino, Methylamino und Dimethylamino,

oder

R(1) -SR(13), -OR(13), -NHR(13), -NR(13)R(14), -CHR(13)R(15), -C[R(15)R(16)OH], -C≡CR(18), -C[R(19)]=CHR(18), -C[R(20)R(21)]$_k$-(CO)-[CR(22)R(23)]$_l$-R(24),

k Null, 1, 2, 3 oder 4;

l Null, 1, 2, 3 oder 4;

R(13) und R(14)

gleich oder verschieden -$(CH_2)_g$-$(CHOH)_h$-$(CH_2)_i$-$(CHOH)_j$-R(17) oder -$(CH_2)_g$-O-$(CH_2$-$CH_2O)_h$-R(24);

R(17) Wasserstoff oder Methyl,

g, h und i

gleich oder verschieden Null, 1, 2, 3 oder 4;

j 1, 2, 3 oder 4;

R(15) und R(16)

gleich oder verschieden Wasserstoff, Alkyl mit 1, 2, 3, 4, 5 oder 6 C-Atomen, oder zusammen mit dem sie tragenden Kohlenstoffatom Cycloalkyl mit 3, 4, 5, 6, 7 oder 8 C-Atomen;

R(18)

Phenyl,

das unsubstituiert oder substituiert ist mit 1 - 3 Substituenten ausgewählt aus der Gruppe bestehend aus F, Cl, $CF_3$, Methyl, Methoxy und NR(25)R(26);

R(25) und R(26)

H oder Alkyl mit 1, 2, 3 oder 4 C-Atomen;

oder
R(18) Heteroaryl mit 1, 2, 3, 4, 5, 6, 7, 8 oder 9 Kohlenstoffatomen,

das unsubstituiert oder wie Phenyl substituiert ist;

oder
R(18) Alkyl mit 1, 2, 3, 4, 5 oder 6 C-Atomen,

das unsubstituiert oder mit 1 - 3 OH substituiert ist;

oder
R (18)

Cycloalkyl mit 3, 4, 5, 6, 7 oder 8 C-Atomen;

R(19), R(20), R(21), R(22) und R(23)

gleich oder verschieden Wasserstoff oder Methyl;

R(24) H, Alkyl mit 1, 2, 3, 4, 5 oder 6 C-Atomen, Cycloalkyl mit 3, 4, 5, 6, 7 oder 8 C-Atomen oder $-C_mH_2m-$R(18);

m 1, 2, 3 oder 4;

R(2) und R(3)

wie R(1) definiert;

R(4) Alkyl mit 1, 2, 3 oder 4 C-Atomen;
sowie deren pharmazeutisch verträgliche Salze;

(HOE 96/F 043 - EP 794 172)
aw) Ortho-substituierte Benzoylguanidine der Formel I

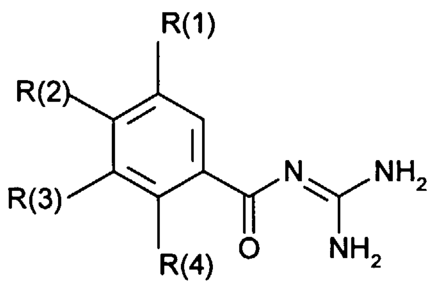

worin bedeuten:

R(1) H, F, Cl, Br, I, CN, $NO_2$, Alkyl mit 1, 2, 3, 4, 5, 6, 7 oder 8 C-Atomen, Alkoxy mit 1, 2, 3, 4, 5, 6, 7 oder 8 C-Atomen, Cycloalkyl mit 3, 4, 5, 6, 7 oder 8 C-Atomen, Cycloalkoxy mit 3, 4, 5, 6, 7 oder 8 C-Atomen oder $X_a-(CH_2)_b-(CF_2)_c-CF_3$;

X Sauerstoff, S, NR(5),
a Null oder 1;
b Null, 1 oder 2;
c Null, 1, 2 oder 3;

R (5) H, Alkyl mit 1, 2, 3 oder 4 C-Atomen oder $-C_dH_{2d}$R(6);

d Null, 1, 2, 3 oder 4;
R(6) Cycloalkyl mit 3, 4, 5, 6, 7 oder 8 C-Atomen, Phenyl, Biphenylyl oder Naphthyl,

wobei die Aromaten Phenyl, Biphenylyl oder Naphthyl nicht substituiert oder substituiert sind mit 1 - 3 Substituenten ausgewählt aus der Gruppe bestehend aus F, Cl, $CF_3$, Methyl, Methoxy und NR(7)R(8);
R(7) und R(8)

unabhängig H oder Alkyl mit 1, 2, 3 oder 4 C-Atomen;

oder
R(1) -SR(10), -OR(10) oder -CR(10)R(11)R(12);

R(10) -$C_fH_{2f}$-Cycloalkyl mit 3, 4, 5, 6, 7 oder 8 C-Atomen im Cycloalkylring, oder Phenyl,

wobei Phenyl unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;

f Null, 1 oder 2;
R(11) und R(12)

unabhängig voneinander wie R(10) definiert oder Wasserstoff oder Alkyl mit 1, 2, 3 oder 4 C-Atomen;

oder
R(1) Phenyl, Naphthyl, Biphenylyl oder Heteroaryl mit 1, 2, 3, 4, 5, 6, 7, 8 oder 9 C-Atomen, letzteres über ein C- oder ein N-Atom des Rings verknüpft,

die jeweils unsubstituiert oder substituiert sind mit 1 - 3 Substituenten ausgewählt aus der Gruppe bestehend aus F, Cl, $CF_3$, $CH_3$, Methoxy, Hydroxy, Amino, Methylamino und Dimethylamino,

oder
R(1) -SR(13), -OR(13), -NHR(13), -NR(13)R(14), -CHR(13)R(15), -C[R(15)R(16)OH], -C≡CR(18), -C[R(19)]=CHR(18), -C[R(20)R(21)]$_k$-(CO)-[CR(22)R(23)]$_l$-R(24),

k Null, 1, 2, 3 oder 4;
l Null, 1, 2, 3 oder 4;
R(13) und R(14)

gleich oder verschieden -$(CH_2)_g$-$(CHOH)_h$-$(CH_2)_i$-$(CHOH)_j$-R(17) oder -$(CH_2)_g$-O-$(CH_2$-$CH_2O)_h$-R(24);
R(17) Wasserstoff oder Methyl,
g, h und i

gleich oder verschieden Null, 1, 2, 3 oder 4;

j 1, 2, 3 oder 4;

R(15) und R(16)

gleich oder verschieden Wasserstoff, Alkyl mit 1, 2, 3, 4, 5 oder 6 C-Atomen, oder zusammen mit dem sie tragenden Kohlenstoffatom Cycloalkyl mit 3, 4, 5, 6, 7 oder 8 C-Atomen;

R(18)

Phenyl,

das unsubstituiert oder substituiert ist mit 1 - 3 Substituenten ausgewählt aus der Gruppe bestehend aus F, Cl, $CF_3$, Methyl, Methoxy und NR(25)R(26);
R(25) und R(26)

H oder Alkyl mit 1, 2, 3 oder 4 C-Atomen;

oder
R(18) Heteroaryl mit 1, 2, 3, 4, 5, 6, 7, 8 oder 9 Kohlenstoffatomen,

das unsubstituiert oder wie Phenyl substituiert ist;

oder
R(18) Alkyl mit 1, 2, 3, 4, 5 oder 6 C-Atomen,

das unsubstituiert oder mit 1 - 3 OH substituiert ist;

oder
R (18)

Cycloalkyl mit 3, 4, 5, 6, 7 oder 8 C-Atomen;

R(19), R(20), R(21), R(22) und R(23)

gleich oder verschieden Wasserstoff oder Methyl;

R(24) H, Alkyl mit 1, 2, 3, 4, 5 oder 6 C-Atomen, Cycloalkyl mit 3, 4, 5, 6, 7 oder 8 C-Atomen oder $-C_mH_{2m}-$ R(18);

m 1, 2, 3 oder 4;

einer der beiden Substituenten R(2) und R(3)

Hydroxyl;

und
der jeweils andere der Substituenten R(2) und R(3)

definiert ist wie R(1);

R(4) Alkyl mit 1, 2, 3 oder 4 C-Atomen; Alkoxy mit 1, 2, 3 oder 4 C-Atomen, F, Cl, Br, I oder $-(CH_2)_n-(CF_2)_o-CF_3$;

n Null oder 1;
o Null oder 1;

sowie deren pharmazeutisch verträgliche Salze.

(HOE 96/F 135 - EP 810 207)
ax) Bis-ortho-substituierte Benzoylguanidine der Formel I

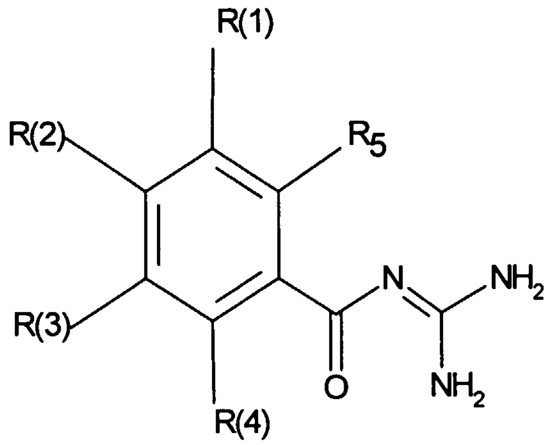

worin bedeuten:

R(1), R(2) und R(3)

unabhängig voneinander R(10)-$SO_a$- oder R(14)R(15)N-$SO_2$-;
a Null, 1 oder 2,
R(10), R(14) und R(15)

unabhängig voneinander Alkyl mit 1, 2, 3, 4, 5, 6, 7 oder 8 C-Atomen, Perfluoralkyl mit 1, 2, 3, 4, 5, 6, 7 oder 8 C-Atomen, Alkenyl mit 3, 4, 5 oder 6 C-Atomen oder -$C_{ab}H_{2ab}$-R(16);
ab Null, 1, 2, 3 oder 4;
R(16) Cycloalkyl mit 3, 4, 5, 6 oder 7 C-Atomen oder Phenyl,

welches nicht substituiert ist oder substituiert mit 1 - 3 Substituenten ausgewählt aus der Gruppe bestehend aus F, Cl, $CF_3$, Methyl, Methoxy und NR(17)R(18);
R(17) und R(18)

unabhängig voneinander Wasserstoff, $CF_3$ oder Alkyl mit 1, 2, 3 oder 4 C-Atomen;

oder
R(14) und R(15)

gemeinsam 4 oder 5 Methylengruppen, von denen eine $CH_2$-Gruppe durch Sauerstoff, Schwefel, NH, N-$CH_3$ oder N-Benzyl ersetzt sein kann;
oder
R(14) und R(15)

Wasserstoff;

oder
R(1), R(2) und R(3)

unabhängig voneinander SR(21), -OR(22), -NR(23)R(24) oder -CR(25)R(26)R(27);
R(21), R(22), R(23) und R(25)

unabhängig voneinander -$C_bH_{2b}$-($C_1$-$C_9$)-Heteroaryl,

das unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;
b Null, 1 oder 2;

R(24), R(26) und R(27)

unabhängig voneinander Wasserstoff, Alkyl mit 1, 2, 3 oder 4 C-Atomen oder Perfluoralkyl mit 1, 2, 3 oder 4 C-Atomen;

oder
R(1), R(2) und R(3)

unabhängig voneinander Wasserstoff, F, Cl, Br, I, CN, $-(Xa)_{dg}-C_{da}H_{2da+1}$, $-(Xb)_{dh}-(CH_2)_{db}-C_{de}F_{2de+1}$, Alkenyl mit 3, 4, 5, 6, 7 oder 8 C-Atomen oder $-C_{df}H_{2df}R(30)$;
(Xa) Sauerstoff, Schwefel oder NR(33);
R(33) Wasserstoff, Alkyl mit 1, 2, 3 oder 4 C-Atomen oder Perfluoralkyl mit 1, 2, 3 oder 4 C-Atomen;
dg Null oder 1;
(Xb) Sauerstoff, Schwefel oder NR(34);

R(34) Wasserstoff, Alkyl mit 1, 2, 3 oder 4 C-Atomen oder Perfluoralkyl mit 1, 2, 3 oder 4 C-Atomen;

dh Null oder 1;
da Null, 1, 2, 3, 4, 5, 6, 7 oder 8;
db Null, 1, 2, 3 oder 4;
de Null, 1, 2, 3, 4, 5, 6 oder 7;
df Null, 1, 2, 3 oder 4;
R(30) Cycloalkyl mit 3, 4, 5, 6, 7 oder 8 C-Atomen, Phenyl, Biphenylyl oder Naphthyl,

wobei die Aromaten Phenyl, Biphenylyl oder Naphthyl nicht substituiert oder substituiert sind mit 1 - 3 Substituenten ausgewählt aus der Gruppe bestehend aus F, Cl, $CF_3$, Methyl, Methoxy und NR(31)R(32);
R(31) und R(32)

Wasserstoff, Alkyl mit 1, 2, 3 oder 4 C-Atomen oder Perfluoralkyl mit 1, 2, 3 oder 4 C-Atomen;

oder
R(1), R(2) und R(3)

unabhängig voneinander NR(40)R(41) oder $-(Xe)-(CH_2)_{eb}R(45)$;
R(40) und R(41)

unabhängig voneinander Wasserstoff, Alkyl mit 1, 2, 3, 4, 5, 6, 7 oder 8 C-Atomen, Perfluoralkyl mit 1, 2, 3, 4, 5, 6, 7 oder 8 C-Atomen oder $(CH_2)_e$-R(42);
e Null, 1, 2, 3 oder 4;
R(42) Cycloalkyl mit 3, 4, 5, 6 oder 7 C-Atomen oder Phenyl,

welches nicht substituiert ist oder substituiert mit 1 - 3 Substituenten ausgewählt aus der Gruppe bestehend aus F, Cl, $CF_3$, Methyl, Methoxy und NR(43)R(44);
R(43) und R(44)

unabhängig voneinander Wasserstoff, $CF_3$ oder Alkyl mit 1, 2, 3 oder 4 C-Atomen;

oder
R(40) und R(41)

gemeinsam 4 oder 5 Methylengruppen, von denen eine $CH_2$-Gruppe durch Sauerstoff, Schwefel, NH, N-$CH_3$ oder N-Benzyl ersetzt sein kann;

(Xe) Sauerstoff, Schwefel oder NR(47);

R(47) Wasserstoff, Alkyl mit 1, 2, 3 oder 4 C-Atomen oder Perfluoralkyl mit 1, 2, 3 oder 4 C-Atomen;

eb Null, 1, 2, 3 oder 4;

R(45) Cycloalkyl mit 3, 4, 5, 6 oder 7 C-Atomen oder Phenyl,

welches nicht substituiert ist oder substituiert mit 1 - 3 Substituenten ausgewählt aus der Gruppe bestehend aus F, Cl, $CF_3$, Methyl, Methoxy, NR(50)R(51) und -(Xfa)-$(CH_2)_{ed}$-(Xfb)R(46);
Xfa $CH_2$, Sauerstoff, Schwefel oder NR(48);
Xfb Sauerstoff, Schwefel oder NR(49);

R(48), R(49), R(50) und R(51)

unabhängig voneinander Wasserstoff, Alkyl mit 1, 2, 3 oder 4 C-Atomen oder Perfluoralkyl mit 1, 2, 3 oder 4 C-Atomen;

ed 1, 2, 3 oder 4;
R(46) Wasserstoff, Alkyl mit 1, 2, 3 oder 4 C-Atomen oder Perfluoralkyl mit 1, 2, 3 oder 4 C-Atomen;

oder
R(1), R(2) und R(3)

unabhängig voneinander -CHR(52)R(53);
R(52) $-(CH_2)_g-(CHOH)_h-(CH)_i-(CHOH)_k-$R(54) oder $-(CH_2)_g-O-(CH_2-CH_2O)_h-$R(54);

R(54) Wasserstoff oder Methyl;
g, h, i

gleich oder verschieden Null, 1, 2, 3 oder 4;

k 1, 2, 3 oder 4;

R(53) Wasserstoff oder Alkyl mit 1, 2, 3 oder 4 C-Atomen;

oder
R(1), R(2) und R(3)

unabhängig voneinander -C(OH)R(55)R(56);
R(55) und R(56)

gleich oder verschieden Wasserstoff oder Alkyl mit 1, 2, 3 oder 4 C-Atomen;

oder
R(55) und R(56)

gemeinsam Cycloalkyl mit 3, 4, 5 oder 6 C-Atomen;

oder
R(55) $-CH_2OH$;

und
R(4) und R(5)

unabhängig voneinander Alkyl mit 1, 2, 3 oder 4 C-Atomen, Alkoxy mit 1, 2, 3 oder 4 C-Atomen, OH, F, Cl, Br, I, CN, $-O_n-(CH_2)_o-(CF_2)_p-CF_3$;
n Null oder 1;
o Null, 1 oder 2;
p Null, 1 oder 2;

sowie deren pharmazeutisch verträgliche Salze.

(HOE 96/F 136 - EP 810 205)

ay) Substituierte 1-Naphthoylguanidine der Formel I

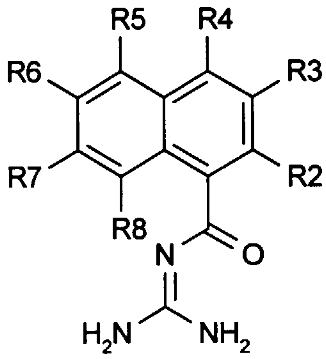

I

worin bedeuten

R2, R3, R4, R5, R6, R7 und R8

unabhängig voneinander H, F, Cl, Br, I, CN, $NO_2$, $CF_3$, $C_2F_5$ oder $X_aY_bZ$;
X O, S, NR(10), CR(11)R(12), C=O, C(=O)NR(10), C(=O)O, SO, $SO_2$, $SO_2NR(10)$, OC=O, NR(10)C=O oder $NR(10)SO_2$,

wobei die Verknüpfung mit dem Naphthalinring jeweils über das links stehende Atom erfolgt;

R(10), R(11) und R(12)

unabhängig voneinander H, Alkyl mit 1, 2, 3, 4, 5 oder 6 C-Atomen, Perfluoralkyl mit 1, 2, 3 oder 4 C-Atomen oder Cycloalkyl mit 3, 4, 5, 6 oder 7 C-Atomen;

a Null oder 1;
Y Alkylen mit 1, 2, 3, 4, 5, 6, 7 oder 8 $CH_2$-Gruppen,

wobei eine dieser $CH_2$-Gruppen durch O, S, NR(13) oder o-, p- oder m-Phenylen ersetzt sein kann;
R(13) H, Alkyl mit 1, 2, 3, 4, 5 oder 6 C-Atomen, Perfluoralkyl mit 1, 2, 3 oder 4 C-Atomen oder Cycloalkyl mit 3, 4, 5 oder 6 C-Atomen;

b Null oder 1;
Z H, Alkyl mit 1, 2, 3, 4, 5, 6 oder 7 C-Atomen, Cycloalkyl mit 3, 4, 5, 6 oder 7 C-Atomen, C(=O)R(15), $SO_2R(15)$, NR(16)R(17) oder Phenyl,

das nicht substituiert oder substituiert ist mit 1 - 3 Substituenten ausgewählt aus der Gruppe bestehend aus F, Cl, Br, $CF_3$, Methyl, Methoxy, NR(21)R(22);

R(21) und R(22)

unabhängig voneinander H oder Alkyl mit 1, 2, 3 oder 4 C-Atomen oder Perfluoralkyl mit 1, 2, 3 oder 4 C-Atomen;

R(15) N =$C(NH_2)_2$, NR(18)R(19), $N(CH_2)_cNR(18)R(19)$ oder OR(20);

c 2 oder 3;
R(18) und R(19)

unabhängig voneinander H, Alkyl mit 1, 2, 3, 4, 5, 6, 7 oder 8 C-Atomen oder Perfluoralkyl mit 1, 2, 3 oder 4 C-Atomen;

oder

R(18) und R(19)

gemeinsam 4 oder 5 Methylengruppen,

von denen eine $CH_2$-Gruppe durch Sauerstoff, S, NH, N-$CH_3$, N-Benzyl oder N-(p-Chlorphenyl) ersetzt sein kann;

R(20) H, Alkyl mit 1, 2, 3, 4, 5 oder 6 C-Atomen, Perfluoralkyl mit 1, 2, 3 oder 4 C-Atomen oder Cycloalkyl mit 3, 4, 5, 6 oder 7 C-Atomen;

R(16) und R(17)

unabhängig voneinander H, Alkyl mit 1, 2, 3, 4, 5, 6, 7 oder 8 C-Atomen oder Perfluoralkyl mit 1, 2, 3 oder 4 C-Atomen;

oder

R(16) und R(17)

gemeinsam 4 oder 5 Methylengruppen, von denen eine $CH_2$-Gruppe durch Sauerstoff, S, NH, N-$CH_3$, N-Benzyl oder N-(p-Chlorphenyl) ersetzt sein kann;

oder

Z ein N-haltiger Heterocyclus mit 1, 2, 3, 4, 5, 6, 7, 8 oder 9 C-Atomen,

wobei der N-haltige Heterocyclus über N oder C verknüpft ist und nicht substituiert oder substituiert ist mit 1 - 3 Substituenten ausgewählt aus der Gruppe bestehend aus F, Cl, Br, $CF_3$, Methyl, Methoxy und NR(21)R(22);

wobei jedoch für den Fall, daß R(4) für einen Alkoxyrest steht, mindestens einer der Substituenten R(2), R(3), R(5), R(6), R(7) und R(8) ungleich Wasserstoff ist;
sowie deren pharmazeutisch verträgliche Salze.

(HOE96/F 137 - EP 810 206)
az) Substituierte 2-Naphthoylguanidine der Formel I

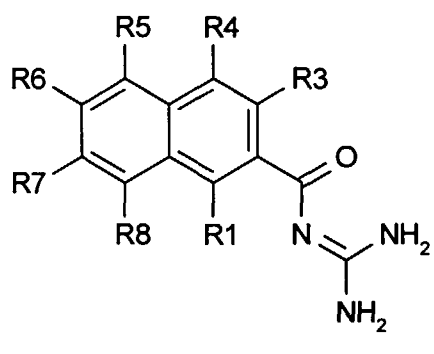

I

worin bedeuten:

mindestens einer der Substituenten R1, R3, R4, R5, R6, R7 und R8

$XY_a$ WZ oder $X'Y_aWZ'$;

X O,S, NR(10) oder CR(11)R(12);

R(10), R(11) und R(12)

unabhängig voneinander H, Alkyl mit 1, 2, 3, 4, 5 oder 6 C-Atomen, Perfluoralkyl mit 1, 2, 3 oder 4 C-Atomen oder Cycloalkyl mit 3, 4, 5, 6 oder 7 C-Atomen;

Y Alkylen mit 1, 2, 3, 4, 5, 6, 7 oder 8 $CH_2$-Gruppen,

wobei eine dieser $CH_2$-Gruppen durch O, S, NR(13) oder o-, p- oder m-Phenylen ersetzt sein kann;

R(13) H, Alkyl mit 1, 2, 3, 4, 5 oder 6 C-Atomen, Perfluoralkyl mit 1, 2, 3 oder 4 C-Atomen oder Cycloalkyl mit 3, 4, 5 oder 6 C-Atomen;

a Null oder 1;

W $CH_2$, $SO_2$, S(=O)(=NH) oder - falls W nicht unmittelbar auf ein Heteroatom der Gruppe $XY_a$ folgt - auch O oder NR(14);

R(14) H, Alkyl mit 1, 2, 3, 4, 5 oder 6 C-Atomen, Perfluoralkyl mit 1, 2, 3 oder 4 C-Atomen oder Cycloalkyl mit 3, 4, 5 oder 6 C-Atomen;

Z C(=O)R(15), $SO_2$R(15) oder - falls W nicht O oder NR(14) bedeutet -auch NR(16)R(17);

R(15)

N=C($NH_2$)$_2$, NR(18)R(19), N($CH_2$)$_b$NR(18)R(19) oder OR(20);
b 2 oder 3;
R(18) und R(19)

unabhängig voneinander H, Alkyl mit 1, 2, 3, 4, 5, 6, 7 oder 8 C-Atomen oder Perfluoralkyl mit 1, 2, 3 oder 4 C-Atomen;

oder
R(18) und R(19)

gemeinsam 4 oder 5 Methylengruppen,

von denen eine $CH_2$-Gruppe durch Sauerstoff, S, NH, N-$CH_3$, N-Benzyl oder N-(p-Chlorphenyl) ersetzt sein kann;

R(20)

H, Alkyl mit 1, 2, 3, 4, 5 oder 6 C-Atomen, Perfluoralkyl mit 1, 2, 3 oder 4 C-Atomen oder Cycloalkyl mit 3, 4, 5, 6 oder 7 C-Atomen;

R(16) und R(17)

unabhängig voneinander H, Alkyl mit 1, 2, 3, 4, 5, 6, 7 oder 8 C-Atomen oder Perfluoralkyl mit 1, 2, 3 oder 4 C-Atomen;

oder
R(16) und R(17)

gemeinsam 4 oder 5 Methylengruppen,

von denen eine $CH_2$-Gruppe durch Sauerstoff, S, NH, N-$CH_3$, N-Benzyl oder N-(p-Chlorphenyl) ersetzt sein kann;

X' C=O, C(=O)NR(30), C(=O)O, SO, $SO_2$, $SO_2$NR(30), OC=O, NR(30)C=O oder NR(30)$SO_2$,

wobei die Verknüpfung mit dem Naphthalinring jeweils über das links stehende Atom erfolgt;

R(30) H, Alkyl mit 1, 2, 3, 4, 5 oder 6 C-Atomen, Perfluoralkyl mit 1, 2, 3 oder 4 C-Atomen oder Cycloalkyl mit 3, 4, 5, 6 oder 7 C-Atomen;

Z' C(=O)R(15), $SO_2$R(15), einen N-haltigen Heterocyclus mit 1, 2, 3, 4, 5, 6, 7, 8 oder 9 C-Atomen,

wobei der N-haltige Heterocyclus über N oder C verknüpft ist und nicht substituiert oder substituiert ist mit 1 - 3 Substituenten ausgewählt aus der Gruppe bestehend aus F, Cl, Br, $CF_3$, Methyl, Methoxy und NR(21)R(22);
R(21) und R(22)

unabhängig voneinander H, Alkyl mit 1, 2, 3 oder 4 C-Atomen oder Perfluoralkyl mit 1, 2, 3 oder 4 C-Atomen;

R(15)

N=C($NH_2$)$_2$, NR(18)R(19), N($CH_2$)$_b$NR(18)R(19) oder OR(20); R(18) und R(19)

unabhängig voneinander H, Alkyl mit 1, 2, 3, 4, 5, 6, 7 oder 8 C-Atomen oder Perfluoralkyl mit 1, 2, 3 oder 4 C-Atomen;

oder
R(18) und R(19)

gemeinsam 4 oder 5 Methylengruppen,

von denen eine $CH_2$-Gruppe durch Sauerstoff, S, NH, N-$CH_3$, N-Benzyl oder N-(p-Chlorphenyl) ersetzt sein kann;

b 2 oder 3;
R(20) H, Alkyl mit 1, 2, 3, 4, 5 oder 6 C-Atomen, Perfluoralkyl mit 1, 2, 3 oder 4 C-Atomen oder Cycloalkyl mit 3, 4, 5, 6 oder 7 C-Atomen;

oder

Z' - falls W nicht O oder NR(14) bedeutet - NR(16)R(17);

R(16) und R(17)

unabhängig voneinander H, Alkyl mit 1, 2, 3, 4, 5, 6, 7 oder 8 C-Atomen oder Perfluoralkyl mit 1, 2, 3 oder 4 C-Atomen;

oder
R(16) und R(17)

gemeinsam 4 oder 5 Methylengruppen,

von denen eine $CH_2$-Gruppe durch Sauerstoff, S, NH, N-$CH_3$, N-Benzyl oder N-(p-Chlorphenyl) ersetzt sein kann;

und die jeweils übrigen Substituenten R1, R3, R4, R5, R6, R7 und R8, die noch nicht durch die zuvor gegebenen Definitionen vergeben sind,

unabhängig voneinander H, F, Cl, Br, I, CN, $NO_2$, $CF_3$, $C_2F_5$ oder $V_pQ_qU$;
V O, S, SO, $SO_2$, NR(60), OC=O, C=O, C(=O)NR(60), C(=O)O oder CR(66)R(67);

R(60), R(66) und R(67)

unabhängig voneinander H, Alkyl mit 1, 2, 3, 4, 5 oder 6 C-Atomen, Perfluoralkyl mit 1, 2, 3 oder 4 C-Atomen oder Cycloalkyl mit 3, 4, 5, 6 oder 7 C-Atomen;

p Null oder 1;
Q Alkylen mit 1, 2, 3, 4, 5, 6, 7 oder 8 $CH_2$-Gruppen,

wobei eine dieser $CH_2$-Gruppen durch O, S, NR(68) oder o-, p- oder m-Phenylen ersetzt sein kann;
R(68)

H, Alkyl mit 1, 2, 3, 4, 5 oder 6 C-Atomen, Perfluoralkyl mit 1, 2, 3 oder 4 C-Atomen oder Cycloalkyl mit 3, 4, 5 oder 6 C-Atomen;

q Null oder 1;
U H, Alkyl mit 1, 2, 3, 4, 5, 6 oder 7 C-Atomen, Cycloalkyl mit 3, 4, 5, 6 oder 7 C-Atomen, C(=O)R(65), $SO_2$R(65), NR(61)R(62) oder Phenyl,

das nicht substituiert oder substituiert ist mit 1 - 3 Substituenten ausgewählt aus der Gruppe bestehend aus F, Cl, Br, $CF_3$, Methyl, Methoxy und NR(63)R(64);
R(63) und R(64)

unabhängig voneinander H, Alkyl mit 1, 2, 3 oder 4 C-Atomen oder Perfluoralkyl mit 1, 2, 3 oder 4 C-Atomen;

R(65) N =C($NH_2$)$_2$, NR(61)R(62) oder OR(60);
R(61) und R(62)

unabhängig voneinander H, Alkyl mit 1, 2, 3, 4, 5, 6, 7 oder 8 C-Atomen oder Perfluoralkyl mit 1, 2, 3 oder 4 C-Atomen;

oder
R(61) und R(62)

gemeinsam 4 oder 5 Methylengruppen,

von denen eine $CH_2$-Gruppe durch Sauerstoff, S, NH, N-$CH_3$, N-Benzyl oder N-(p-Chlorphenyl) ersetzt sein kann;

oder
U einen N-haltigen Heterocyclus mit 1, 2, 3, 4, 5, 6, 7, 8 oder 9 C-Atomen,

wobei der N-haltige Heterocyclus über N oder C verknüpft ist und nicht substituiert oder substituiert ist mit 1 - 3 Substituenten ausgewählt aus der Gruppe bestehend aus F, Cl, Br, $CF_3$, Methyl, Methoxy und NR(63)R(64);

wobei jedoch mindestens einer der Substituenten R5, R6, R7 und R8 ungleich Wasserstoff ist; sowie deren pharmazeutisch verträgliche Salze.

(HOE 96/F 141 - EP 811 610))
ba) Ortho-substituierte Benzoylguanidine der Formel I

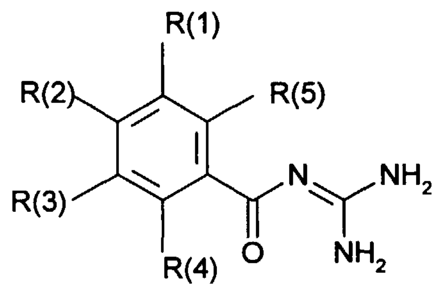

I

worin bedeuten:

R(1) H, F, Cl, Br, I, CN, $NO_2$, Alkyl mit 1, 2, 3, 4, 5, 6, 7 oder 8 C-Atomen, Alkoxy mit 1, 2, 3, 4, 5, 6, 7 oder 8 C-Atomen, Cycloalkyl mit 3, 4, 5, 6, 7 oder 8 C-Atomen, Cycloalkoxy mit 3, 4, 5, 6, 7 oder 8 C-Atomen oder $X_a$-$(CH_2)_b$-$(CF_2)_c$-$CF_3$;

X Sauerstoff, Schwefel oder NR(9),
a Null oder 1;
b Null, 1 oder 2;
c Null, 1, 2 oder 3;

R(9) H, Alkyl mit 1, 2, 3 oder 4 C-Atomen oder -$C_dH_{2d}$R(6);

d Null, 1, 2, 3 oder 4;
R(6) Cycloalkyl mit 3, 4, 5, 6, 7 oder 8 C-Atomen, Phenyl, Biphenylyl oder Naphthyl,

wobei die Aromaten Phenyl, Biphenylyl oder Naphthyl nicht substituiert oder substituiert sind mit 1 - 3 Substituenten ausgewählt aus der Gruppe bestehend aus F, Cl, $CF_3$, Methyl, Methoxy und NR(7)R(8);
R(7) und R(8)

unabhängig H oder Alkyl mit 1, 2, 3 oder 4 C-Atomen;

oder
R(1) -SR(10), -OR(10) oder -CR(10)R(11)R(12);

R(10) -$C_fH_{2f}$-Cycloalkyl mit 3, 4, 5, 6, 7 oder 8 C-Atomen im Cycloalkylring, Heteroaryl mit 1, 2, 3, 4, 5, 6, 7, 8 oder 9 C-Atomen oder Phenyl,

wobei Heteroaryl und Phenyl unsubstituiert oder substituiert sind mit 1 - 3 Substituenten ausgewählt aus der Gruppe bestehend aus F, Cl, $CF_3$, $CH_3$, Methoxy, Hydroxy, Amino, Methylamino und Dimethylamino;

f Null, 1 oder 2;

R(11) und R(12)

unabhängig voneinander wie R(10) definiert oder Wasserstoff oder Alkyl mit 1, 2, 3 oder 4 C-Atomen;

oder
R(1) Phenyl, Naphthyl, Biphenylyl oder Heteroaryl mit 1, 2, 3, 4, 5, 6, 7, 8 oder 9 C-Atomen, letzteres über ein C- oder ein N-Atom des Rings verknüpft,

die jeweils unsubstituiert oder substituiert sind mit 1 - 3 Substituenten ausgewählt aus der Gruppe bestehend aus F, Cl, $CF_3$, $CH_3$, Methoxy, Hydroxy, Amino, Methylamino und Dimethylamino,

oder

R(1) -SR(13), -OR(13), -NHR(13), -NR(13)R(14), -CHR(13)R(15), -C[R(15)R(16)]OH, -C≡CR(18), -C[R(19)]=CHR(18), -C[R(20)R(21)]$_k$-(CO)-[CR(22)R(23)]$_l$-R(24),

k Null, 1, 2, 3 oder 4;
l Null, 1, 2, 3 oder 4;
R(13) und R(14)

gleich oder verschieden $-(CH_2)_g-(CHOH)_h-(CH_2)_i-(CHOH)_{kk}-$R(17) oder $-(CH_2)_g-O-(CH_2-CH_2O)_h-$R(24);
R(17) Wasserstoff oder Methyl,
g, h und i

gleich oder verschieden Null, 1, 2, 3 oder 4;

kk 1, 2, 3 oder 4;

R(15) und R(16)

gleich oder verschieden Wasserstoff, Alkyl mit 1, 2, 3, 4, 5 oder 6 C-Atomen, oder zusammen mit dem sie tragenden Kohlenstoffatom Cycloalkyl mit 3, 4, 5, 6, 7 oder 8 C-Atomen;

R(18)

Phenyl,

das unsubstituiert oder substituiert ist mit 1 - 3 Substituenten ausgewählt aus der Gruppe bestehend aus F, Cl, $CF_3$, Methyl, Methoxy und NR(25)R(26);
R(25) und R(26)

H oder Alkyl mit 1, 2, 3 oder 4 C-Atomen;

oder
R(18) Heteroaryl mit 1, 2, 3, 4, 5, 6, 7, 8 oder 9 Kohlenstoffatomen,

das unsubstituiert oder wie Phenyl substituiert ist;

oder
R(18) Alkyl mit 1, 2, 3, 4, 5 oder 6 C-Atomen,

das unsubstituiert oder mit 1 - 3 OH substituiert ist;

oder
R (18)

Cycloalkyl mit 3, 4, 5, 6, 7 oder 8 C-Atomen;

R(19), R(20), R(21), R(22) und R(23)

gleich oder verschieden Wasserstoff oder Methyl;

R(24) H, Alkyl mit 1, 2, 3, 4, 5 oder 6 C-Atomen, Cycloalkyl mit 3, 4, 5, 6, 7 oder 8 C-Atomen oder $-C_mH_{2m}-$R(18);

m 1, 2, 3 oder 4;

einer der beiden Substituenten R(2) und R(3)

-O-CO-R(27);
R(27) Alkyl mit 1, 2, 3, 4, 5, 6, 7 oder 8 C-Atomen, Cycloalkyl mit 3, 4, 5, 6, 7 oder 8 C-Atomen, Phenyl, Biphenylyl, Naphthyl, Pyridyl oder Chinolinyl,

wobei Phenyl, Biphenylyl, Naphthyl, Pyridyl oder Chinolinyl unsubstituiert oder substituiert sind mit 1 - 3 Substituenten ausgewählt aus der Gruppe bestehend aus F, Cl, $CF_3$, Methyl, Methoxy und NR(7)R(8);
R(7) und R(8)

unabhängig voneinander Wasserstoff oder Alkyl mit 1, 2, 3 oder 4 C-Atomen;

wobei stets einer der Substituenten R(2) und R(3)

definiert ist wie R(1);

R(4) und R(5)

unabhängig voneinander Wasserstoff, Alkyl mit 1, 2, 3 oder 4 C-Atomen; Alkoxy mit 1, 2, 3 oder 4 C-Atomen, F, Cl, Br, I, CN oder $-(CH_2)_n-(CF_2)_o-CF_3$;
n Null oder 1;
o Null oder 1;

sowie deren pharmazeutisch verträgliche Salze.

(HOE 96/F 154 - EP 814 077)
bb) Benzoylguanidine der Formel I

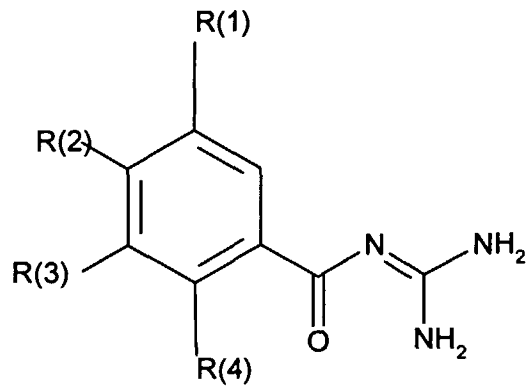

worin bedeuten:

R(1) R(13)-$SO_m$ oder R(14)R(15)N-$SO_2$-;

m 1 oder 2;
R(13) Alkyl mit 1, 2, 3, 4, 5, 6, 7 oder 8 C-Atomen, Perfluoralkyl mit 1, 2, 3, 4, 5, 6, 7 oder 8 C-Atomen, Alkenyl mit 3, 4, 5, 6, 7 oder 8 C-Atomen oder $-C_nH_{2n}$-R(16),

n Null, 1, 2, 3 oder 4;
R(16) Cycloalkyl mit 3, 4, 5, 6, 7 oder 8 C-Atomen, Phenyl, Biphenylyl oder Naphthyl,

wobei Phenyl, Biphenylyl und Naphthyl nicht substituiert oder substituiert sind mit 1 - 3 Substituenten ausgewählt aus der Gruppe bestehend aus F, Cl, $CF_3$, Methyl, Methoxy und NR(25)R(26);
R(25) und R(26)

unabhängig voneinander Wasserstoff, Alkyl mit 1, 2, 3 oder 4 C-Atomen oder Perfluoralkyl mit 1, 2, 3 oder 4 C-Atomen;

R(14) Wasserstoff, Alkyl mit 1, 2, 3, 4, 5, 6, 7 oder 8 C-Atomen, Perfluoralkyl mit 1, 2, 3, 4, 5, 6, 7 oder 8 C-Atomen, Alkenyl mit 3, 4, 5, 6, 7 oder 8 C-Atomen oder $-C_nH_{2n}$-R(27),

n Null, 1, 2, 3 oder 4;
R(27) Cycloalkyl mit 3, 4, 5, 6, 7 oder 8 C-Atomen, Phenyl, Biphenylyl oder Naphthyl,

wobei Phenyl, Biphenylyl und Naphthyl nicht substituiert oder substituiert sind mit 1 - 3 Substituenten ausgewählt aus der Gruppe bestehend aus F, Cl, $CF_3$, Methyl, Methoxy und NR(28)R(29);
R(28) und R(29)

unabhängig voneinander Wasserstoff, Alkyl mit 1, 2, 3 oder 4 C-Atomen oder Perfluoralkyl mit 1, 2, 3 oder 4 C-Atomen;

R(15) Wasserstoff, Alkyl mit 1, 2, 3 oder 4 C-Atomen oder Perfluoralkyl

mit 1, 2, 3 oder 4 C-Atomen;

oder
R(14) und R(15)

gemeinsam 4 oder 5 Methylengruppen, von denen eine $CH_2$-Gruppe durch Sauerstoff, S, NH, N-$CH_3$ oder N-Benzyl ersetzt sein kann;

einer der Substituenten R(2) und R(3)

Wasserstoff;

und der jeweils andere Substituent R(2) und R(3)

-CHR(30)R(31);
R(30)

$-(CH_2)_g-(CHOH)_h-(CH_2)_i-(CHOH)_k$-R(32) oder $-(CH_2)_g-O-(CH_2-CH_2O)_h$-R(24);

R(24 und R(32)

unabhängig voneinander Wasserstoff oder Methyl;

g, h, i

gleich oder verschieden Null, 1, 2, 3 oder 4;

k 1, 2, 3 oder 4;

oder der jeweils andere Substituent R(2) und R(3)

-C(OH)R(33)R(34);
R(31), R(33) und R(34)

gleich oder verschieden Wasserstoff oder Alkyl mit 1, 2, 3 oder 4 C-Atomen

oder

R(33) und R(34)

gemeinsam Cycloalkyl mit 3, 4, 5 oder 6 C-Atomen;

oder
R(33) $-CH_2OH$;

R(4) Alkyl mit 1, 2, 3 oder 4 C-Atomen, Alkoxy mit 1, 2, 3 oder 4 C-Atomen, F, Cl, Br, I, CN, $-(CH_2)_n-(CF_2)_o-CF_3$;

n Null oder 1;
o Null, 1 oder 2;

sowie deren pharmazeutisch verträgliche Salze.

(HOE 96/F 202 - EP 837 055)
bc) Indanylidinacetylguanidine der Formel I

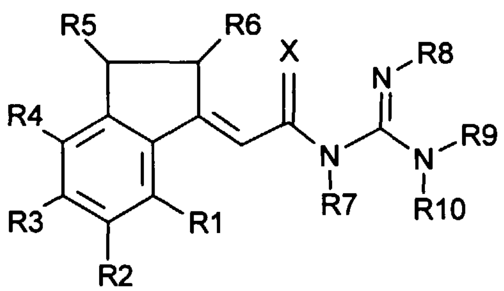
I

worin bedeuten:

R1, R2, R3, R4, R5 und R6

unabhängig voneinander H, $C_1$-$C_{10}$-Alkyl; Haloalkyl mit 1 - 6 Kohlenstoffatomen, O-$C_1$-$C_{10}$-Alkyl, Haloalkoxy mit 1 - 6 C-Atomen, F, Cl, Br, I, Aryl, substituiertes Aryl, Heteroaryl, substituiertes Heteroaryl, OH, O-Niederalkyl, O-Aryl, O-Niederalkylaryl, O-substituiertes Aryl, O-Niederalkyl-substituiertes Aryl, O-C(=O)-$C_1$-$C_4$-Alkyl-Aryl, O-C(=O)-NH-$C_1$-$C_4$-Alkyl, O-C(=O)-N($C_1$-$C_4$-Alkyl)$_2$, $NO_2$, CN, $CF_3$, $NH_2$, NH-C(=O)-$C_1$-$C_4$-Alkyl, NH-C(=O)-$NH_2$, COOH, C(=O)-O-$C_1$-$C_4$-Alkyl, C(=O)-$NH_2$, C(=O)-NH-$C_1$-$C_4$-Alkyl, C(=O)-N($C_1$-$C_4$-Alkyl)$_2$, $C_1$-$C_4$-COOH, $C_1$-$C_4$-Alkyl-C(=O)-O-$C_1$-$C_4$-Alkyl, $SO_3H$, $SO_2$-Alkyl; $SO_2$-Alkylaryl, $SO_2$-N-(Alkyl)$_2$, $SO_2$-N(Alkyl)(Alkylaryl), C(=O)-R11, $C_1$-$C_{10}$-Alkyl-C(=O)-R11, $C_2$-$C_{10}$-Alkenyl-C(= O)-R11, $C_2$-$C_{10}$-Alkinyl-C(=O)-R11, NH-C(=O)-$C_1$-$C_{10}$-Alkyl-C(=O)-R11 oder O-$C_1$-$C_{11}$-Alkyl-C(=O)-R11;
R11 $C_1$-$C_4$-Alkyl, $C_1$-$C_4$-Alkinyl, Aryl, substituiertes Aryl, $NH_2$, NH-$C_1$-$C_4$-Alkyl, N-($C_1$ -$C_4$-Alkyl)$_2$, $SO_3H$, $SO_2$-Alkyl, $SO_2$-Alkylaryl, $SO_2$-N-(Alkyl)$_2$ oder $SO_2$-N(Alkyl)(Alkylaryl);

X O, S oder NH;

R7, R8, R9 und R10

unabhängig voneinander H, Alkyl, Cycloalkyl, Aryl, Alkylaryl,

oder
R8 und R9

zusammen Teil eines a 5-, 6- oder 7-gliedrigen heterocyclischen Rings;

oder ihre pharmazeutisch akzeptablen Salze.

(HOE 96/F 226 - EP 825 178)
bd) Phenylsubstituierte Alkenylcarbonsäure-guanidide der Formel I

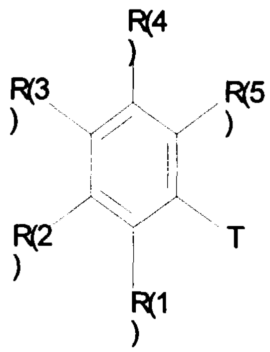

I

worin bedeuten:

T

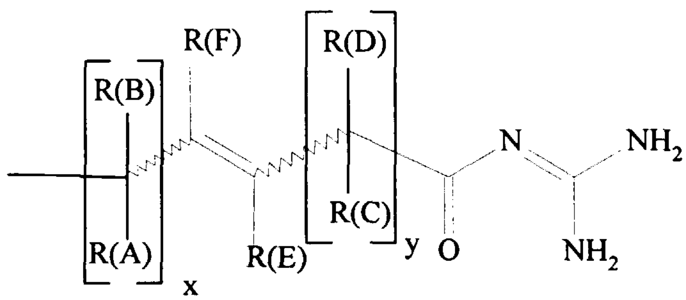

R(A) Wasserstoff, F, Cl, Br, I, CN, OH, OR(6), ($C_1$-$C_4$)-Alkyl, $O_r(CH_2)_aC_bF_{2b+1}$, ($C_3$-$C_8$)-Cycloalkyl oder NR(7)R(8)

r Null oder 1;
a Null, 1, 2, 3 oder 4;
b 1, 2, 3 oder 4;
R(6) ($C_1$-$C_4$)-Alkyl, ($C_1$-$C_4$)-Perfluoralkyl, ($C_3$-$C_6$)-Alkenyl, ($C_3$-$C_8$)-Cycloalkyl, Phenyl oder Benzyl,

wobei der Phenylkern nicht substituiert oder substituiert ist mit 1 - 3 Substituenten ausgewählt aus der Gruppe bestehend aus F, Cl, $CF_3$, Methyl, Methoxy und NR(9)R(10);
R(9) und R(10)

H, ($C_1$-$C_4$)-Alkyl oder ($C_1$-$C_4$)-Perfluoralkyl;

R(7) und R(8)

unabhängig voneinander wie R(6) definiert;

oder
R(7) und R(8)

gemeinsam 4 oder 5 Methylengruppen, von denen eine $CH_2$-Gruppe durch Sauerstoff, Schwefel, NH, N-$CH_3$ oder N-Benzyl ersetzt sein kann;

R(B), R(C) und R(D)

unabhängig wie R(A) definiert;

x Null, 1 oder 2;

y Null, 1 oder 2;

R(F) Wasserstoff, F, Cl, Br, I, CN, OR(12), ($C_1$-$C_8$)-Alkyl, $O_p(CH_2)_fC_gF_{2g+1}$, ($C_3$-$C_8$)-Cycloalkyl oder ($C_1$-$C_9$)-Heteroaryl;

p Null oder 1;
f Null, 1, 2, 3 oder 4;
g 1, 2, 3, 4, 5, 6, 7 oder 8;
R(12) ($C_1$-$C_8$)-Alkyl, ($C_1$-$C_4$)-Perfluoralkyl, ($C_3$-$C_8$)-Alkenyl, ($C_3$-$C_8$)-Cycloalkyl, Phenyl oder Benzyl,

wobei der Phenylkern nicht substituiert ist oder substituiert 1 - 3 Substituenten ausgewählt aus der Gruppe bestehend aus F, Cl, $CF_3$, Methyl, Methoxy und NR(13)R(14);
R(13) und R(14)

H, ($C_1$-$C_4$)-Alkyl oder ($C_1$-$C_4$)-Perfluoralkyl;

R(E) unabhängig wie R(F) definiert;

R(1) unabhängig wie T definiert;
oder
R(1) Wasserstoff, $-O_kC_mH_{2m+1}$, $-O_n(CH_2)_pC_qF_{2q+1}$, F, Cl, Br, I, CN, -(C=O)-N =$C(NH_2)_2$, $-SO_rR(17)$, -$SO_{r2}NR(31)R(32)$; $-O_u(CH_2)_vC_6H_5$, $-O_{u2}$-($C_1$-$C_9$)-Heteroaryl oder $-S_{u2}$-($C_1$-$C_9$)-Heteroaryl;

k Null oder 1;
m Null, 1, 2, 3, 4, 5, 6, 7 oder 8;
n Null oder 1;
p Null, 1, 2, 3 oder 4;
q 1, 2, 3, 4, 5, 6, 7 oder 8;
r Null, 1, 2;
r2 Null, 1, 2;
R(31) und R(32)

unabhängig voneinander Wasserstoff, ($C_1$-$C_8$)-Alkyl oder ($C_1$-$C_8$)-Perfluoralkyl;

oder
R(31) und R(32)

gemeinsam 4 oder 5 Methylengruppen, von denen eine $CH_2$-Gruppe durch Sauerstoff, S, NH, N-$CH_3$ oder N-Benzyl ersetzt sein kann;

R(17) ($C_1$-$C_8$)-Alkyl;
u Null oder 1;
u2 Null oder 1;
v Null, 1, 2, 3 oder 4;

wobei der Phenylkern substituiert ist oder substituiert mit 1 - 3 Substituenten ausgewählt aus der Gruppe bestehend aus F, Cl, $CF_3$, Methyl, Methoxy, $-(CH_2)_wNR(21)R(22)$, NR(18)R(19) und ($C_1$-$C_9$)-Heteroaryl;
R(18), R(19), R(21) und R(22)

unabhängig voneinander ($C_1$-$C_4$)-Alkyl oder ($C_1$-$C_4$)-Perfluoralkyl;

w 1, 2, 3 oder 4;
wobei der Heterocyclus des ($C_1$-$C_9$)-Heteroaryl unsubstituiert oder substituiert ist mit 1 - 3 Substituenten ausgewählt aus der Gruppe bestehend aus F, Cl, $CF_3$, Methyl oder Methoxy;

R(2), R(3), R(4) und R(5)

unabhängig voneinander wie R(1) definiert,

oder
R(1) und R(2) oder R(2) und R(3)

jeweils gemeinsam -CH-CH=CH-CH-,

das nicht substituiert oder substituiert ist mit 1 - 3 Substituenten ausgewählt aus der Gruppe bestehend aus F, Cl, $CF_3$, Methyl, Methoxy, $-(CH_2)_{w2}NR(24)R(25)$ und NR(26)R(27);
R(24), R(25), R(26) und R(27)

H, $(C_1-C_4)$-Alkyl oder $(C_1-C_4)$-Perfluoralkyl;

w2 1, 2, 3 oder 4;

wobei der Rest T im Molekül mindestens zweimal, jedoch nur höchstens dreimal vorhanden ist;
sowie deren pharmazeutisch verträgliche Salze.

(HOE 97/ F 082)
be) Benzoylguanidine der Formel I

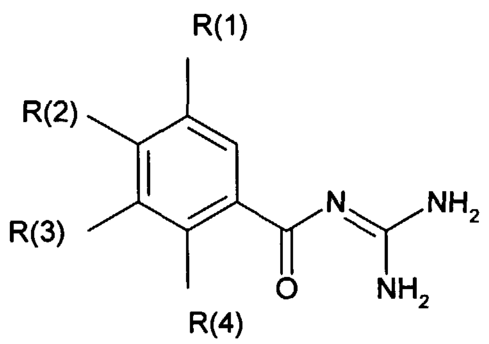

I

worin bedeuten:

R(1) $CF_3$;
einer der Substituenten R(2) und R(3)

Wasserstoff;

und der jeweils andere Substituent R(2) und R(3)

$-C(OH)(CH_3)-CH_2OH$, $-CH(CH_3)-CH_2OH$ oder $-C(OH)(CH_3)_2$;

R(4) Methyl, Methoxy, Cl oder $CF_3$;
sowie deren pharmazeutisch verträgliche Salze.

(DE 195 02 895, DE 44 30 212, EP 667 341, DE 44 04 183, EP 708 088, EP 723 963, EP 0 694 537, DE 44 21 495, EP 699 660, EP 699 663, EP 699 666, DE 43 37 611, EP 0719 766, WO 94/26709, WO 96 04 241, EP 726 254, US 4 251 545, DE 35 02 629, WO 84/00875, Kumamoto et al., Pharm. Bull. [1966], 7 - 13; US 3 780 027, JP 8225513; EP 743 301)
II. Geeignet sind auch Verbindungen der Formel

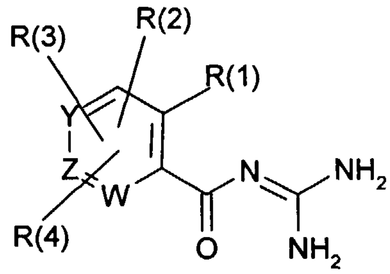

worin bedeuten:

W, Y und Z

ein Stickstoffatom oder ein mit R(2) oder R(3) oder R(4) substituiertes C-atom;

R(1) Wasserstoff, A, Hal, $-CF_3$, $-CH_2F$, $-CHF_2$, $-CH_2CF_3$, $-C_2F_5$, -CN, $-NO_2$, - Ethinyl, oder einen X-R';

A Alkyl mit 1 bis 6 C-Atomen;
Hal F, Cl, Br oder I;
X Sauerstoff, S oder NR";

R" Wasserstoff, A oder eine cyclische Methylenkette mit 3 bis 7 C-Atomen;

R' H, A, HO-A-, HOOC-A-, $(C_3$-$C_7)$-Cycloalkyl, $(C_6$-$C_8)$-Cycloalkyl-alkyl, $CF_3$, $CH_2F$, $CHF_2$, $CH_2$-$CF_3$, Ph, -$CH_2$-Ph oder Het;

Ph unsubstituiertes oder ein-, zwei-, oder dreifach durch A, OA, NR'R", Hal, $CF_3$ substituiertes Phenyl, Naphthyl, oder Biphenylyl;
Het einen ein- oder zweikernigen gesättigten, ungesättigten oder aromatischen Heterocyclus mit 1 bis 4 N-, O-, und/oder S-Atomen,

der unsubstituiert oder ein-, zwei- oder dreifach substitiert ist durch Hal, $CF_3$, A, OH, OA, -X-R', -CN, $-NO_2$, und/oder Carbonylsauerstoff,

wobei Het über N oder oder eine Alkylenkette $C_mH_{2m}$ mit m = Null bis 6 gebunden ist;

oder
R' und R"

zusammen Alkylen mit 4 - 5 C-Atomen, worin eine $CH_2$-Gruppe auch durch Sauerstoff, S, NH, N-A, N-Ph und N-$CH_2$-Ph ersetzt sein kann;

R(2) und R(3)

unabhängig voneinander Wasserstoff, Hal, A, HO-A-, X-R', -C(=N-OH)-A, A-O-CO-$(C_1$-$C_4)$-Alkyl-, CN, $NO_2$, COOH, halogensubstituiertes A, insbesondere $CF_3$, $CH_2F$, $CHF_2$, $C_2F_5$, $CH_2CF_3$, oder $S(O)_nR'''$;
R''' A, Ph oder -Het;
n Null, 1 oder 2;

oder
R(2) und R(3)

unabhängig voneinander $SO_2NR'R''$, Ph oder -O-Ph, -O-$CH_2$-Ph, -CO-A, -CHO, -COOA, -CSNR'R", CONR'R", -CH=CH-COOH, -CH=CH-COOA, Indenyl, Indanyl, Decahydronaphthyl, Cyclopentenyl, Dihydrothienyl, Dihydrofuryl, Heterobicyclyl, Alkylthienyl, Halothienyl, Haloalkylthienyl, Acylthienyl, Halofuryl, Haloalkylfuryl oder Pyrrolyl;

oder
R(2) und R(3)

unabhängig voneinander R(5)-O-;
R(5) Wasserstoff, A, $(C_1-C_6)$-Alkenyl oder $(C_3-C_7)$-Cycloalkyl;

R(4) Ph, Het, -O-Het; $CF_3$, $S(O)_nR'''$, $-SO_2NR'R''$, Alk;

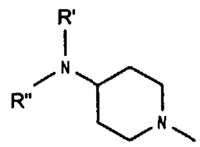 ;

oder
zwei der Substituenten R(1) bis R(4)

miteinander eine Gruppe -O-CR(6)R(7)-CO-NR(8)-,

oder 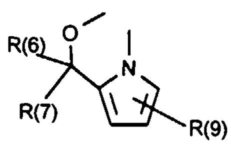 oder 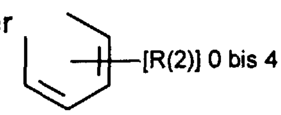 ;

mit R(2) in der angegebenen Bedeutung;

R(6), R(7), R(8) und R(9)

unabhängig voneinander H oder A;

oder
R(8) $(C_5-C_7)$-Cycloalkyl;
oder
R(9) Cyano;

Alk geradkettiges oder verzweigtes $(C_1-C_8)$-Alkyl oder $(C_3-C_8)$-Cycloalkyl,

welches unsubstituiert oder durch A ein-, zwei- oder dreifach substituiert ist;

oder
Alk ein durch H, A, Ph oder Het substituierter Ethenyl oder Ethinylrest;

( EP 708 091, EP 622 356, JP 5-125085)
III. Geeignet sind ebenfalls Indoloylguanidin-Derivate der Formel

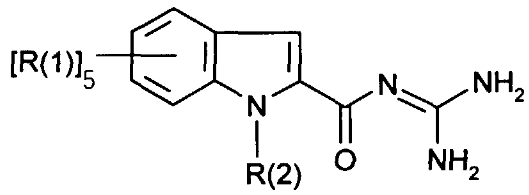

worin bedeuten

R(2) Wasserstoff, unsubstituiertes oder substituiertes ($C_1$-$C_8$)-Alkyl, ($C_3$-$C_7$)-Cycloalkyl, OH, ($C_1$-$C_6$)-Alkyl-O-, einen aromatischen Rest oder eine Gruppe -$CH_2$-R(20);

R(20) ($C_2$-$C_6$)-Alkenyl oder ($C_2$-$C_6$)-Alkinyl;

R(1) 1 bis 5 gleiche oder verschiedene Substituenten, die bedeuten:

Wasserstoff, unsubstituiertes oder substituiertes ($C_1$-$C_8$)-Alkyl, ($C_2$-$C_6$)-Alkenyl, ($C_2$-$C_6$)-Alkinyl, ($C_3$-$C_7$)-Cycloalkyl, Halogen, -$NO_2$, ($C_2$-$C_8$)-Alkanoyl, Arylalkanoyl mit bis zu 10 C-Atomen, Aroyl mit bis zu 11 C-Atomen, -COOH, ($C_2$-$C_6$)-Alkoxycarbonyl, eine aromatische Gruppe, eine der nachfolgend aufgeführten Gruppen: -OR(3), -NR(6)R(7) oder -$S(O)_n$R(40);
R(3) Wasserstoff, ($C_1$-$C_8$)-Alkyl, substituiertes ($C_1$-$C_8$)-Alkyl, ($C_3$-$C_7$)-Cycloalkyl, einen aromatischen Rest oder eine Gruppe -$CH_2$-R(30) R(30) Alkenyl oder Alkinyl;
R(6) und R(7)

unabhängig voneinander Wasserstoff, unsubstituiertes oder substituiertes ($C_1$-$C_8$)-Alkyl, ($C_3$-$C_7$)-Cycloalkyl, ($C_2$-$C_8$)-Alkanoyl, eine Arylalkanoylgruppe mit bis zu 10 C-Atomen, eine Aroylgruppe mit bis zu 11 C-Atomen, eine aromatische Gruppe oder -$CH_2$-R(60);
R(60) ($C_2$-$C_6$)-Alkenyl oder ($C_2$-$C_6$)-Alkinyl;

oder
R(6) und R(7)

gemeinsam mit dem N-Atom ein 5 - 7-gliedriges cyclisches Amin, das noch weitere Heteroatome im Ring enthalten kann;

n Null, 1 oder 2;
R(40) unsubstituiertes oder substituiertes ($C_1$-$C_8$)-Alkyl, oder eine aromatische Gruppe, oder eine Gruppe

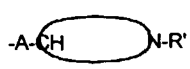

A Sauerstoff, -$S(O)_n$- oder -N(R50)-;

R(50) Wasserstoff oder ($C_1$-$C_8$)-Alkyl;

R' Wasserstoff, unsubstituiertes oder substituiertes ($C_1$-$C_8$)-Alkyl,
worin der Ring einen gesättigten 3 - 8 gliedrigen Heterocyclus mit einem N-atom repräsentiert,

besagtes substituiertes Alkyl eine oder mehrere Gruppen trägt ausgewählt aus der Gruppe bestehend aus Halogen, -OH, ($C_1$-$C_6$)-Alkoxy, -CN, -COOH, ($C_2$-$C_6$)-Alkoxycarbonyl, ($C_2$-$C_8$)-Alkanoyl, Arylalkanoyl mit bis zu 10 C-Atomen, Aroyl mit bis zu 11 C-Atomen, eine aromatische Gruppe, - CONR(4)(R5),
R(4) und R(5)

gleich oder verschieden Wasserstoff oder ($C_1$-$C_8$)-Alkyl;

oder
R(4) und R(5)

miteinander verbunden und bilden zusammen ein 5 - 7-gliedriges cyclisches Amin, das noch weitere Heteroatome im Ring enthalten kann,

oder besagtes substituiertes Alkyl eine Gruppe

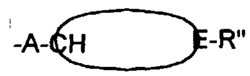

trägt, worin bedeuten:
E ein N-Atom oder eine CH-Gruppe;
R" Wasserstoff, unsubstituiertes oder mit OH substituiertes ($C_1$-$C_8$)-Alkyl oder substituiertes ($C_1$-$C_8$)-Alkyl, ($C_1$-$C_6$)-Alkoxy, -CN, -COOH, ($C_2$-$C_6$)-Alkoxycarbonyl, ($C_2$-$C_8$)-Alkanoyl, Aralkanoyl mit bis zu 10 C-Atomen, Aroyl mit bis zu 11 C-Atomen, eine aromatische Gruppe, -NR(6)R(7), -CONR(4)R(5);

R(4) und R(5)

unabhängig voneinander Wasserstoff oder ($C_1$-$C_8$)-Alkyl;

wobei das cyclische System der Formel

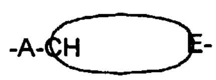

ein 3 - 8 gliedriges gesättigtes aliphatisches oder heterocyclisches Ringsystem mit einem N-Atom bedeutet,

und wobei die erwähnten aromatischen Gruppen einen Arylrest mit bis zu 10 C-Atomen, einen 5- oder 6-gliedrigen Heteroarylrest mit 1-4 N-Atomen, eine 5- oder 6-gliedrige Heteroarylgruppe enthaltend 1 oder 2 N-Atome und ein Heteroatom in der Bedeutung von Sauerstoff oder Schwefel, oder Furyl bedeuten,
und wobei die erwähnten Arylreste unsubstituiert oder substituiert sein können durch unsubstituiertes ($C_1$-$C_8$)-Alkyl oder substituiertes ($C_1$-$C_8$)-Alkyl, Halogen, - $NO_2$, ($C_2$-$C_6$)-Alkoxycarbonyl, COOH, -OR(3), NR(6)R(7), -CONR(4)R(5), -$SO_2$NR(6)R(7) oder $S(O)_n$R(40),
wobei R(1) und der Guanidinocarbonylrest an einer beliebigen Stelle des 5-oder 6-gliedrigen Rings des Indolsystems stehen können,
sowie die zugehörigen pharmazeutisch verträglichen Salze.

(WO 95 04052)
IV. Weiterhin geeignet sind heterocyclische Guanidin-Derivate der Formel

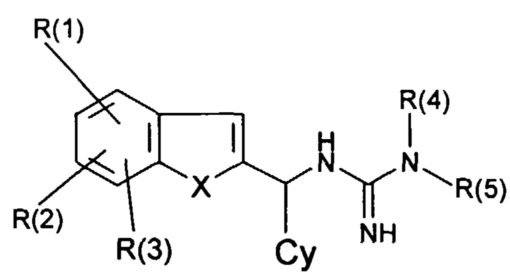

worin bedeuten:

X -O-, -S-, -NH-, -N[($C_1$-$C_4$)-Alkyl]- oder -N(Phenyl)-;
R(1), R(2) und R(3)

Wasserstoff, Halogen, ($C_1$-$C_4$)-Alkyl, ($C_1$-$C_4$)-Alkyl-O-, Phenyl, Benzyl;

oder
zwei der Substituenten R(1), R(2) und R(3)

zusammen mit einer Seite des Benzosystems einen 4 - 6-gliedrigen carbocyclischen Ring;

R(4) und R(5)

unabhängig voneinander Wasserstoff, ($C_1$-$C_{12}$)-Alkyl, Benzhydryl, Aralkyl,

das unsubstituiert oder substituiert ist durch einen oder mehrere Substituenten aus den Gruppen Halogen, ($C_1$-$C_4$)-Alkyl, ($C_1$-$C_4$)-Alkyl-O- oder -$CF_3$, -$(CH_2)_m$-$CH_2$-T,
m Null bis 3;
T -CO-O-T(1);

T(1) Wasserstoff oder (C1-C4)-Alkyl;

Cy einen benzokondensierten ungesättigten oder Dihydro-5-Ringheterocyclus

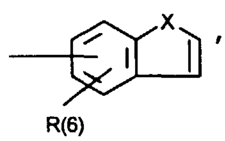

einen Pyrazol- oder Imidazolring der Formel

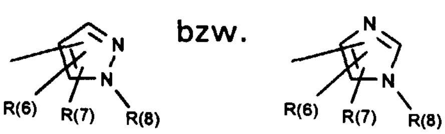

einen Naphthylrest oder ein Dihydro- oder Tetrahydronaphthylrest

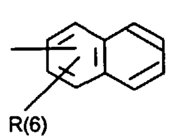

einen 2-, 3- oder 4-Pyridylrest

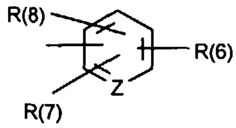

Z N- oder CH;

einen Thienylrest

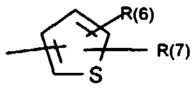

R(6) Wasserstoff, Halogen, Hydroxy, $(C_1$-$C_{10})$-Alkyl, $(C_1$-$C_{10})$-Alkyl-O-, Phenoxy, $(C_1$-$C_{10})$-Alkyloxymethyloxy- oder $-(O)_nS$-R(9);

R(9) $(C_1$-$C_{10})$-Alkyl, Thienyl, Pyridyl-, Thiazolyl, Thiadiazolyl, Imidazolyl, Pyrazolyl oder Phenyl,

die unsubstituiert oder ein- oder zweifach substituiert sind mit Halogen, $(C_1$-$C_4)$-Alkyl oder $(C_1$-$C_4)$-Alkyl-O-;

R(7) und R(8)

Wasserstoff, Halogen, Hydroxy, $(C_1$-$C_{10})$-Alkyl, $(C_1$-$C_{10})$-Alkyl-O-, Phenyl, Phenoxy oder $(C_1$-$C_{10})$-Alkoxy-methyloxy;

oder
Cy Phenyl,

das unsubstituiert ist oder ein- oder zweifach substituiert ist mit Halogen, $(C_1$-$C_4)$-Alkyl oder $(C_1$-$C_4)$-Alkyl-O-;

oder
Cy -Gr-Am;

Gr -R(13)-R(12)-$(CH_2)_q$-C[W][W(1)]-$(CH_2)_{q'}$-; R(13)R(14)- oder -R(15)-;

R(12) eine Einfachbindung, -O-, $-(O)_nS$, -CO- oder -CONH-;
R(13) eine Einfachbindung, Phenyl, Thienyl, Pyridyl, Thiazolyl, Thiadiazolyl, Imidazolyl oder Pyrazolyl;
R(14) eine Einfachbindung $SO_2$-;
R(15) $(C_2$-$C_{10})$-Alkenyl- oder $(C_2$-$C_{10})$-Alkinyl;
W und W(1)

unabhängig voneinander Wasserstoff, $(C_1$-$C_4)$-Alkyl;

oder
W und W(1)

miteinander cyclisch verbunden einen $(C_3$-$C_8)$-Kohlenwasserstoffring;

q und q'

Null bis 9;

Am -NR(10)R(11);

R(10) Wasserstoff, ($C_1$-$C_4$)-Alkyl oder Benzyl,
R(11) ($C_1$-$C_4$)-Alkyl, Phenyl oder Benzyl;
oder
R(10) und R(11)

gemeinsam eine ($C_3$-$C_{10}$)-Alkylengruppe,

die unsubstituiert ist oder substituiert mit -COOH, ($C_1$-$C_5$)-Alkoxycarbonyl, ($C_2$-$C_4$)-Hydroxyalkylen oder Benzyl;

oder

Am Pyrrolyl, Pyridyl, Pyrazolyl, Morpholinyl, Dihydropyridyl, Tetrahydropyridyl, Chinuclidinyl, Imidazolyl, 3-Azabicyclo[3.2.1]octyl,

das unsubstituiert ist oder substituiert mit ($C_1$-$C_4$)-Alkyl,

oder

Am Azabicyclo[3.2.2]nonyl;
oder Am eine Piperazingruppe der Formel

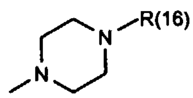

R(16) Wasserstoff, ($C_1$-$C_4$)-Alkyl, ($C_3$-$C_6$)-Cycloalkyl, Phenyl, Tolyl, Methoxyphenyl, Halophenyl, Diphenylmethylen, Benzyl oder Pyridyl;

oder

Am eine Azidogruppe -$(O)_t$-(CH2)q-C[W][W(1)]-(CH2)q'-$N_3$;

t Null oder 1;
wobei W und W(1) die vorher angegebene Bedeutung besitzen;

sowie die optischen Enantiomeren und die pharmakologisch verträglichen Salze.

V. Geeignet sind ferner die Guanidin-Verbindungen, wie sie beschrieben werden in
ER- 743 301 (DE 195 17 848), EP 758 644 (DE 195 29 612), EP 760 365 (DE 195 31 138)

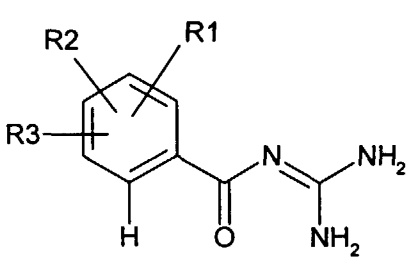

mit R1 = R2 gleich H, Halo, Alkyl, CN, $NO_2$, Perfluoralkyl, $SO_nCF_3$; R3 gleich $CH{=}CH_2$, $CH_2{-}CH{=}CH_2$, $CH_2{-}CH_2{-}CH{=}CH_2$, Cycloalkenyl, Cycloalkenylalkyl; R4 gleich Alkyl, (substituiertes) Phenyl,
oder wie beschrieben in DE 195 48 708, WO 97 25 310, WO 97 27 183, DE 196 01 303, EP 787 728, JP 82 25 513, JP 090 59 245, JP 090 67 332, JP 090 67 340, WO 97 11 055 und EP 743 301.

Besonders geeignet für die Verwendung nach der Erfindung sind die Verbindungen, die ausgewählt ist aus der Gruppe bestehend aus den Verbindungen
I. (HOE 89/F 288 - US 5 292 755)
a) Benzoylguanidine der Formel I

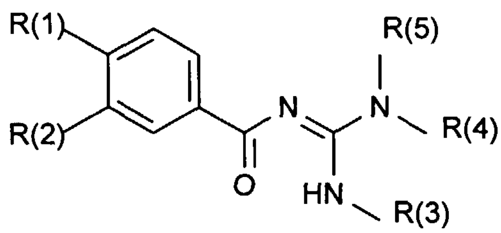

I

worin bedeuten:

R(1) oder R(2)

R(6)-$S(O)_n$- oder R(7)R(8)N-$O_2S$-;

und der jeweils andere Substituent R(1) oder R(2)

H, F, Cl, Br, $(C_1-C_4)$-Alkyl, $(C_1-C_4)$-Alkoxy oder Phenoxy,

das unsubstituiert ist oder substituiert mit 1 - 3 Substituenten ausgewählt aus der Gruppe bestehend aus Fluor, Chlor, Methyl und Methoxy;

oder der jeweils andere Substituent R(1) oder R(2)

R(6)-$S(O)_n$ oder R(7)R(8)N-;
n Null, 1 oder 2;
R(6) $(C_1-C_6)$-Alkyl, $(C_5-C_7)$-Cycloalkyl, Cyclopentylmethyl, Cyclohexylmethyl oder Phenyl,

das unsubstituiert ist oder substituiert mit 1 - 3 Substituenten ausgewählt aus der Gruppe bestehend aus Fluor, Chlor, Methyl und Methoxy;

R(7) und R(8)

gleich oder verschieden H oder $(C_1-C_6)$-Alkyl;

oder
R(7) Phenyl-$(CH_2)_m$;

m 1 - 4;

oder
R(7) Phenyl,

das unsubstituiert ist oder substituiert mit 1 - 2 Substituenten ausgewählt aus der Gruppe bestehend aus Fluor, Chlor, Methyl und Methoxy;

oder
R(7) und R(8)

gemeinsam eine geradkettige oder verzweigte ($C_4$-$C_7$)-Kette, wobei die Kette zusätzlich durch O, S oder NR(9) unterbrochen sein kann;
R(9) H oder Methyl;

oder
R(7) und R(8)

gemeinsam mit dem Stickstoffatom, an das sie gebunden sind, ein Dihydroindol-, Tetrahydrochinolin- oder Tetrahydroisochinolin-System;

R(3), R(4) und R(5)

unabhängig voneinander H oder ($C_1$-$C_2$)-Alkyl,

oder

R(3) und R(4)

gemeinsam eine ($C_2$-$C_4$)-Alkylenkette;

oder

R(4) und R(5)

gemeinsam eine ($C_4$-$C_7$)-Alkylenkette;

sowie deren pharmazeutisch verträgliche Salze;

(HOE 92/F 34 - US 5 373 924)
b) Benzoylguanidine der Formel I

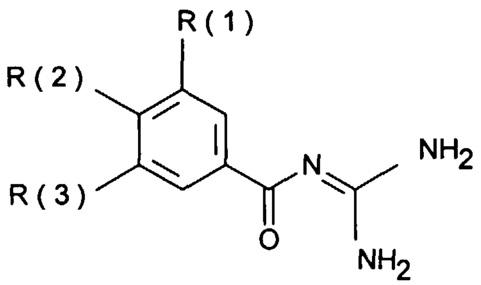

(I)

worin bedeuten:

R(1) R(4)-$SO_m$ oder R(5)R(6)N-$SO_2$-;

m Null, 1 oder 2;
R(4) und R(5)

$C_1$-$C_8$-Alkyl, $C_3$-$C_6$-Alkenyl oder -$C_nH_{2n}$-R(7);
n Null, 1, 2, 3 oder 4;
R(7) $C_5$-$C_7$-Cycloalkyl oder Phenyl,

welches unsubstituiert ist oder substituiert mit 1 - 3 Substituenten ausgewählt aus der Gruppe bestehend aus F, Cl, $CF_3$, Methyl, Methoxy und NR(8)R(9);
R(8) und R(9)

H oder $C_1$-$C_4$-Alkyl;

oder
R(5) H;
R(6) H oder $C_1$-$C_4$-Alkyl,
oder
R(5) und R(6)

gemeinsam 4 oder 5 Methylengruppen, von denen eine $CH_2$-Gruppe durch ein O, S, NH, N-$CH_3$ oder N-Benzyl ersetzt sein kann;

R(2) Wasserstoff, F, Cl, Br, ($C_1$-$C_4$)-Alkyl-, O-$(CH_2)_mC_pF_{2p+1}$ oder -X-R(10);

m Null oder 1;
p 1, 2 oder 3;
X O, S oder NR(11);
R(10) H, $C_1$-$C_6$-Alkyl, $C_5$-$C_7$-Cycloalkyl, Cyclohexylmethyl, Cyclopentylmethyl oder -$C_nH_{2n}$-R(12);

n Null, 1, 2, 3 oder 4;
R(12) Phenyl,

das unsubstituiert oder substituiert ist mit 1 - 3 Substituenten ausgewählt aus der Gruppe bestehend aus F, Cl, $CF_3$, Methyl, Methoxy und NR(8)R(9);
R(8) und R(9)

gleich H oder $C_1$-$C_4$-Alkyl;

R(11) Wasserstoff oder $C_1$-$C_3$-Alkyl;
oder
R(10) und R(11)

gemeinsam 4 oder 5 Methylengruppen, von denen eine $CH_2$-Gruppe durch O, S, NH, N-$CH_3$ oder N-Benzyl ersetzt sein kann;

R(3) wie R(1) definiert, oder $C_1$-$C_6$-Alkyl, Nitro, Cyano, Trifluormethyl, F, Cl, Br, J oder-X-R(10);

X O, S oder NR(11);

R(10) gleich H, $C_1$-$C_6$-Alkyl, $C_5$-$C_7$-Cycloalkyl, Cyclohexylmethyl, Cyclopentylmethyl oder -$C_nH_{2n}$-R(12);
n null bis 4;
R(12) Phenyl,

das unsubstituiert ist oder substituiert mit 1 - 3 Substituenten ausgewählt aus der Gruppe bestehend aus F, Cl, $CF_3$, Methyl, Methoxy und NR(8)R(9);
R(8) und R(9)

H oder $C_1$-$C_4$-Alkyl;

R(11) $C_1$-$C_3$-Alkyl,
oder
R(10) und R(11)

gemeinsam 4 oder 5 Methylengruppen, von denen eine $CH_2$-Gruppe durch O, S, NH, N-$CH_3$ oder N-Benzyl ersetzt sein kann;

sowie deren pharmazeutisch verträgliche Salze;

(HOE 92/F 303 K - EP-OS589 336, NZ 248 703)
f) Benzoylguanidine der Formel I

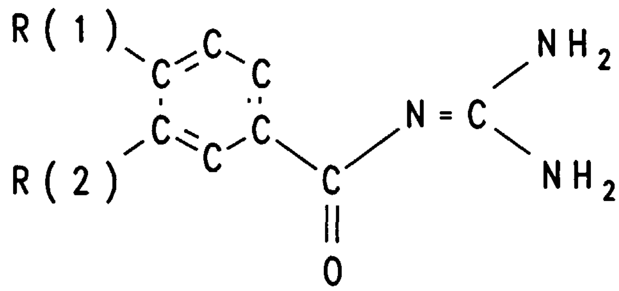

I

worin bedeuten:

R(1) oder R(2)

R(3)-S(O)$_n$- oder R(4)R(5)N-SO$_2$-

der jeweils andere Substituent R(1) oder R(2)

H, OH, F, Cl, Br, J, $C_1$-$C_4$-Alkyl, $C_1$-$C_4$-Alkoxy, Benzyloxy oder Phenoxy,

das unsubstituiert ist oder einen bis drei Substituenten ausgewählt aus der Gruppe bestehend aus Fluor, Chlor, Methyl, Methoxy, Hydroxy oder Benzyloxy trägt,

R(3)-S(O)$_n$, -NR4)R(5) oder 3,4-Dehydropiperidin
R(3) $C_1$-$C_6$-Alkyl, $C_5$-$C_7$-Cycloalkyl, Cyclopentylmethyl, Cyclohexylmethyl oder Phenyl,

das unsubstituiert ist oder einen bis drei Substituenten ausgewählt aus der Gruppe bestehend aus Fluor, Chlor, Methyl und Methoxy trägt;

R(4) und R(5)

gleich oder verschieden, H oder $C_1$-$C_6$-Alkyl;

oder
R(4) Phenyl-$(CH_2)_m$-

m 1, 2, 3 oder 4;

oder
R(4) Phenyl,

das unsubstituiert ist oder einen bis zwei Substituenten ausgewählt aus der Gruppe bestehend aus Fluor, Chlor, Methyl und Methoxy trägt;

oder
R(4) und R(5)
gemeinsam eine geradkettige oder verzweigte $C_4$-$C_7$-Kette, wobei die Kette zusätzlich durch O, S oder NR(6) unterbrochen sein kann,
R(6) H oder Methyl;
oder
R(4) und R(5)

gemeinsam mit dem Stickstoffatom, an das sie gebunden sind, ein Dihydroindol, Tetrahydrochinolin oder Tetrahydroisochinolin-System;
n Null, 1 oder 2;

sowie deren pharmazeutisch verträgliche Salze.

(HOE 92/F 411 - NZ 250 450, EP 603 650)
k) Benzoylguanidine der Formel I

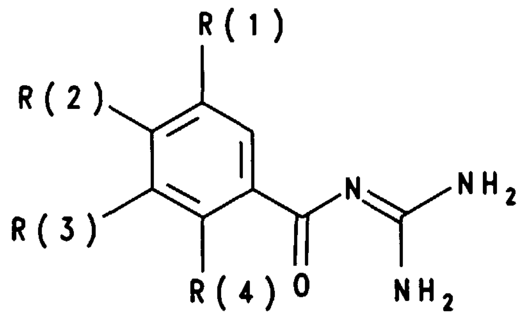

(I)

worin bedeuten:

einer der Substituenten R(1), R(2), R(3) oder R(4):

eine Aminogruppe

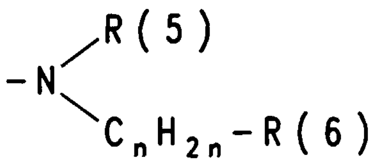

;

R(5) Wasserstoff oder $C_{(1-6)}$-Alkyl;
n Null, 1, 2, 3 oder 4;
R(6) H oder $C_{(1-4)}$-Alkyl;
worin eine $CH_2$-Gruppe durch 1 S-Atom oder eine Gruppe NR(7) ersetzt sein kann;
R(7) Wasserstoff, Methyl oder Ethyl;
oder
R(6) $C_{(3-8)}$-Cycloalkyl oder Phenyl,

das unsubstituiert ist oder 1, 2 oder 3 Substituenten ausgewählt aus der Gruppe bestehend aus F, Cl, Br, Methyl, Methoxy, -NR(8)R(9) trägt;
R(8) und R(9)

H, Methyl oder Ethyl;

oder
R(5) und R(6)

gemeinsam mit dem Stickstoffatom einen 5-, 6- oder 7-gliedrigen Ring, in welchem 1 C-Atom durch Sauerstoff, S oder NR(10) ersetzt sein kann;
R(10) H, $C_{(1-3)}$-Alkyl oder Benzyl;

und die jeweils anderen Substituenten R(1), R(2), R(3), R(4):

Wasserstoff, F, Cl, Br, I, CN, $CF_3$, $NO_2$, $CF_3$-O-, $C_mF_{2m+1}$-$CH_2$-O- oder R(11)-$C_qH_{2q}$-$X_p$-;
m 1, 2 oder 3;
q Null, 1, 2, 3 oder 4;

p Null oder 1;
X Sauerstoff oder NR(12);

R(12) H oder $C_{(1-3)}$-Alkyl;

R(11) Wasserstoff, $C_{(1-6)}$-Alkyl, $C_{(3-8)}$-Cycloalkyl oder Phenyl,

das unsubstituiert oder substituiert ist mit 1, 2 oder 3 Substituenten ausgewählt aus der Gruppe bestehend aus F, Cl, $CH_3$, $CH_3$-O- und NR(13)R(14);
R(13), R(14)

H, Methyl oder Ethyl;

sowie deren pharmazeutisch verträgliche Salze;

(93/F 054 - NZ 250 919, EP-OS 612 723)
m) Benzoylguanidine der Formel I

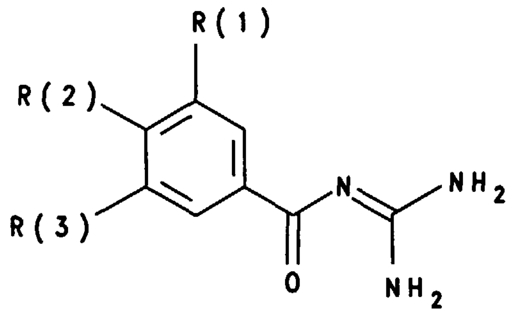

(I)

worin bedeuten:

R(1), R(2), R(3)

Wasserstoff, F, Cl, Br, I oder ($C_1$-$C_{12}$)-Alkyl;

einer der Substituenten R(1), R(2) oder R(3)

$N_3$, CN, OH oder ($C_1$-$C_{10}$)-Alkyloxy, wenn mindestens einer der restlichen Substituenten R(1), R(2) oder R(3) einen hinreichend lipophilen Alkylrest mit 3 bis 12 Kohlenstoff-Atomen bedeutet;

oder
einer der Substituenten R(1), R(2) oder R(3)

R(4)-$C_nH_{2n}$-$O_m$-;
m Null oder 1;
n Null, 1, 2 oder 3;
R(4) $C_pF_{2p+1}$;

p 1, 2 oder 3, sofern n gleich Null oder 1 ist;

oder
R(4) ($C_3$-$C_{12}$)-Cycloalkyl, Phenyl, Pyridyl, Chinolyl oder Isochinolyl, wobei die aromatischen und heteroaromatischen Ringsysteme unsubstituiert sind oder substituiert mit einem Substituenten ausgewählt aus der Gruppe bestehend aus F, Cl, $CF_3$, Methyl, Methoxy und NR(5)R(6);
R(5) und R(6)

Wasserstoff oder ($C_1$-$C_4$)-Alkyl;

oder einer der Substituenten R(1), R(2) oder R(3)

-C≡CR(5) oder -C[R(6)] = CR(5);
R(5) Phenyl,

das unsubstituiert oder substituiert ist mit 1 - 3 Substituenten ausgewählt aus der Gruppe bestehend aus F, Cl, $CF_3$, Methyl, Methoxy, Hydroxy, Amino, Methylamino und Dimethylamino,
($C_1$-$C_9$)-Heteroaryl,

das unsubstituiert oder wie Phenyl substituiert ist,

oder
R(5) ($C_1$-$C_6$)-Alkyl,

das unsubstituiert oder mit 1 - 3 OH substituiert ist;

oder
R(5) ($C_3$-$C_8$)-Cycloalkyl,
R(6) Wasserstoff oder Methyl;

sowie deren pharmakologisch akzeptable Salze;

(93/F 153 - EP-OS 627 413, NZ 260 660)
o) Benzoylguanidine der Formel I

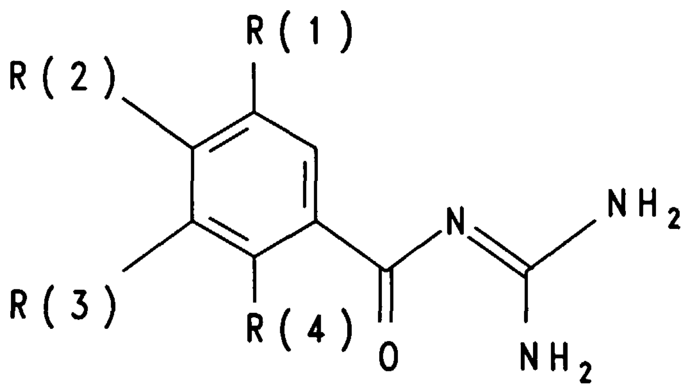

(I)

worin bedeuten:

R(1) Wasserstoff, F, Cl, Br, I, $-NO_2$, -C≡N, $X_o$-$(CH_2)_p$-$(CF_2)_q$-$CF_3$, R(5)-$SO_m$, R(6)-CO- oder R(6)R(7)N-$SO_2$-, mit

X Sauerstoff, S oder NR(14);
m Null, 1 oder 2;
o Null oder 1;
p Null, 1 oder 2;
q Null, 1, 2, 3, 4, 5 oder 6;
R(5) und R(6)

($C_1$-$C_8$)-Alkyl, ($C_3$-$C_6$)-Alkenyl, $-C_nH_{2n}$-R(8) oder $CF_3$;
n Null, 1, 2, 3 oder 4;
R(8) ($C_3$-$C_7$)-Cycloalkyl oder Phenyl,

welches nicht substituiert ist oder substituiert mit 1 - 3 Substituenten ausgewählt aus der Gruppe bestehend aus F, Cl, $CF_3$, Methyl, Methoxy und NR(9)R(10);
R(9) und R(10)

H oder $C_1$-$C_4$-Alkyl;

oder
R(6) H;
R(7) H oder ($C_1$-$C_4$)-Alkyl;
oder
R(6) und R(7)

gemeinsam 4 oder 5 Methylengruppen, von denen eine $CH_2$-Gruppe durch Sauerstoff, S, NH, N-$CH_3$ oder N-Benzyl ersetzt sein kann;

R(2)

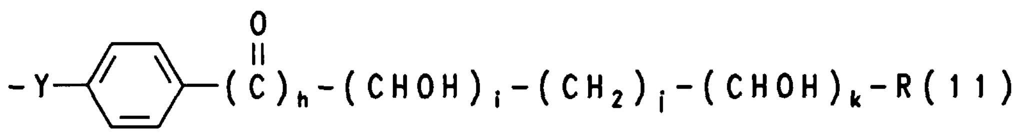

oder 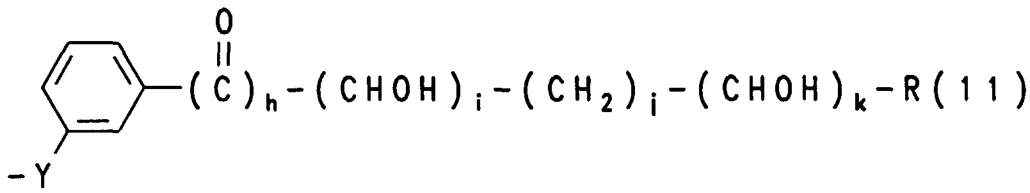

oder 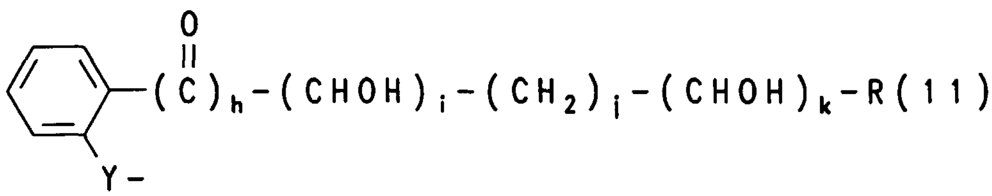

Y Sauerstoff, -S- oder -NR(12)-;
R(11) und R(12)

Wasserstoff oder ($C_1$-$C_3$)-Alkyl;

h Null oder 1;
i, j und k

unabhängig Null, 1, 2, 3 oder 4;

wobei jedoch nicht h, i und k gleichzeitig Null sind,

R(3) wie R(1) definiert, oder ($C_1$-$C_6$)-Alkyl oder -X-R(13);

X Sauerstoff, S oder NR(14);

R(14) H oder ($C_1$-$C_3$)-Alkyl;
R(13) H, ($C_1$-$C_6$)-Alkyl, ($C_3$-$C_8$)-Cycloalkyl oder -$C_bH_{2b}$-R(15);
b Null, 1, 2, 3 oder 4;

oder

R(13) und R(14)

gemeinsam 4 oder 5 Methylengruppen, wobei eine $CH_2$-Gruppe durch Sauerstoff, S, NH, N-$CH_3$ oder N-Benzyl ersetzt sein kann;

R(15) Phenyl,

das unsubstituiert oder substituiert ist mit 1 - 3 Substituenten ausgewählt aus der Gruppe bestehend aus F, Cl, $CF_3$, Methyl, Methoxy und NR(9)R(10);

R(9) und R(10)

H oder ($C_1$-$C_4$)-Alkyl;

R(4) Wasserstoff, -OR(16) oder -NR(16)R(17);

R(16) und R(17)

unabhängig Wasserstoff oder ($C_1$-$C_3$)-Alkyl;

sowie deren pharmazeutisch verträgliche Salze;

(HOE 93/F 223 K - EP 639 573, NZ 264 130)

r) Benzokondensierte 5-Ringheterocyclen der Formel I

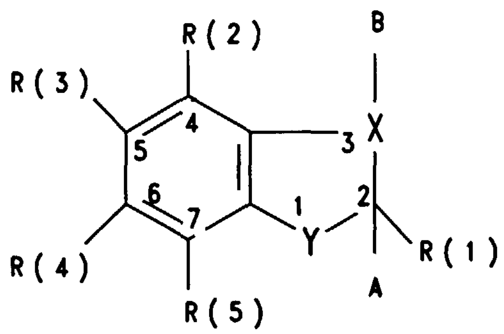

(I),

worin bedeuten:

X N oder CR(6);

Y Sauerstoff, S oder NR(7);

A, B gemeinsam eine Bindung

oder

A, B beide Wasserstoff, sofern gleichzeitig X gleich CR(6) und Y gleich NR(7) sind;

einer der Substituenten R(1) bis R(6) eine -CO-N=C($NH_2$)$_2$-Gruppe;

die jeweils anderen Substituenten R(1) bis R(6)

Wasserstoff, F, Cl, Br, I oder ($C_1$-$C_6$)-Alkyl;

bis zu zwei der anderen Substituenten R(1) bis R(6)

CN, $NO_2$, $N_3$, ($C_1$-$C_4$)-Alkyloxy oder $CF_3$;

bis zu einem der anderen Substituenten

R(8)-$C_nH_{2n}$-Z-;

n Null bis 10;

wobei die Alkylenkette $-C_nH_{2n}-$ geradkettig oder verzweigt ist und wobei ein C-Atom durch ein Sauerstoff- oder S-Atom oder durch ein N-Atom ersetzt sein kann;

R(8) Wasserstoff, $(C_2-C_6)$-Alkenyl oder $(C_3-C_{10})$-Cycloalkyl,

das unsubstituiert oder durch 1 bis 4 Methylgruppen oder eine OH-Gruppe substituiert ist, oder eine Ethylengruppe - CH=CH- enthalten kann, und worin eine Methylengruppe durch ein Sauerstoff- oder S-Atom oder durch ein N-Atom ersetzt sein kann;

oder
R(8) Phenyl,

unsubstituiert oder substituiert durch 1 bis 3 Substituenten ausgewählt aus der Gruppe bestehend aus F, Cl, Br, I, $CF_3$, $CH_3-S(O)_s-$ oder $R(9)-W_y-$;
s Null, 1 oder 2;
R(9) H, Methyl, Ethyl,
W Sauerstoff oder NR(10);

R(10) H oder Methyl;

y Null oder 1;

oder
R(8) $C_mF_{2m+1}$;

m 1 bis 3;

oder
R(8) 1 - oder 2-Naphthyl, Pyridyl, Chinolyl oder Isochinolyl;

Z -CO-, $-CH_2-$ oder $-[CR(11)(OH)]_q-$;

q 1, 2 oder 3;
R(11) H oder Methyl;

oder
Z Sauerstoff oder -NR(12)-;

R(12) H oder Methyl;

oder
Z $-S(O)_s-$;

s Null, 1 oder 2;

oder
Z $-SO_2-NR(13)-$;

R(13) H oder $(C_1-C_4)$-Alkyl;

R(7) Wasserstoff, $(C_1-C_{10})$-Alkyl, $(C_2-C_{10})$-Alkenyl oder $R(8)-C_nH_{2n}-$;

sowie deren pharmazeutisch verträgliche Salze;

(HOE 94/F 134 - EP-OS 686 627, NZ 272 103)
z) Benzoylguanidine der Formel I

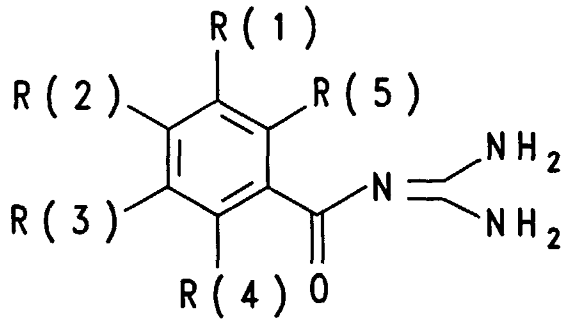

I

worin bedeuten:

R(1) R(6)-$SO_m$;

m Null, 1 oder 2;
R(6) Perfluoralkyl mit 1, 2, 3, 4, 5 oder 6 C-Atomen, das geradkettig oder verzweigt ist;

R(2) und R(3)

unabhängig voneinander Wasserstoff, F, Cl, Br, I, Alkyl mit 1, 2, 3 oder 4 C-Atomen, Alkoxy mit 1, 2, 3 oder 4 C-Atomen oder Phenoxy,

das unsubstituiert ist oder substituiert ist mit 1 - 3 Substituenten ausgewählt aus der Gruppe bestehend aus F, Cl, Methyl und Methoxy;

oder
R(2) und R(3)

unabhängig voneinander Pyrrol-1-yl, Pyrrol-2-yl- oder Pyrrol-3-yl,

welches nicht substituiert ist oder substituiert mit 1 bis 4 Substituenten, ausgewählt aus der Gruppe bestehend aus F, Cl, Br, I, CN, Alkanoyl mit 2, 3, 4, 5, 6, 7 oder 8 C-Atomen, Alkoxycarbonyl mit 2, 3, 4, 5, 6, 7 oder 8 C-Atomen, Formyl, Carboxy, $CF_3$, Methyl und Methoxy;

R(4) und R(5)

unabhängig voneinander Wasserstoff, Alkyl mit 1, 2 oder 3-C-Atomen, F, Cl, Br, I, CN, OR(7), NR(8)R(9) oder $-(CH_2)_n-(CF_2)_o-CF_3$;
R(7), R(8) und R(9)

unabhängig voneinander Wasserstoff oder Alkyl mit 1, 2, 3 oder 4 C-Atomen;

n Null oder 1;
o Null, 1 oder 2;

sowie deren pharmakologisch akzeptable Salze;

(HOE 95/F 007 K - EP-OS 723 956, NZ 280 887)
ag) Benzoylguanidine der Formel I

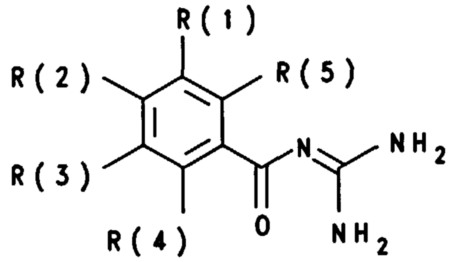

I

worin bedeuten:

einer der drei Substituenten R(1), R(2) und R(3)

R(6)-A-B-D-;

R(6) ein basischer protonierbarer Rest, d.h. eine Aminogruppe -NR(7)R(8), eine Amidinogruppe R(7)R(8)N-C[=N-R(9)]- oder eine Guanidinogruppe

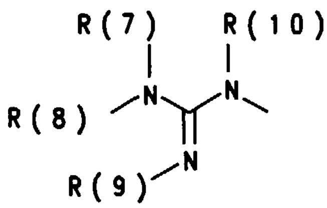

R(7), R(8), R(9) und R(10)

unabhängig voneinander Wasserstoff oder Alkyl mit 1, 2, 3 oder 4 C-Atomen;

oder

R(7) und R(8)

gemeinsam $C_aH_{2a}$;

a 4, 5, 6 oder 7;

wobei im Falle von a = 5, 6 oder 7 eine Methylengruppe der Gruppe $C_aH_{2a}$ durch eine Heteroatomgruppe O, $SO_m$ oder NR(11) ersetzt sein kann,

oder

R(8) und R(9) oder R(9) und R(10) oder R(7) und R(10)

eine Gruppe $C_aH_{2a}$;

a 2, 3, 4 oder 5;

wobei im Falle von a = 3, 4 oder 5 eine Methylengruppe der Gruppe $C_aH_{2a}$ durch eine Heteroatomgruppe O, $SO_m$ oder NR(11) ersetzt sein kann;

m Null, 1 oder 2;

R(11) Wasserstoff oder Methyl;

oder

R(6) ein basisches heteroaromatisches Ringsystem mit 1 - 9 C-Atomen;

A $C_bH_{2b}$;

b 1, 2, 3, 4, 5, 6, 7, 8, 9 oder 10;

wobei in der Gruppe $C_bH_{2b}$ ein oder zwei Methylengruppen durch eine der Gruppierungen ausgewählt aus der Gruppe bestehend aus -O-,-CO-, -CH[OR(20)]-, $-SO_m-$, -NR(20)-, -NR(20)-CO-, -NR(20)-CO-NH-, -NR(20)-CO-NH-$SO_2$-und

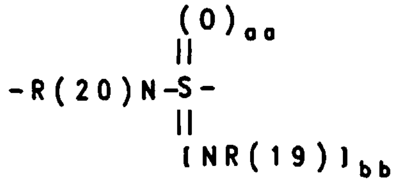

$-SO_{aa}[NR(19)]_{bb}$- ersetzt sein können;
und wobei in der Gruppe $C_bH_{2b}$ eine Methylengruppe durch -CH-R(99) ersetzt sein kann, wobei R(99) gemeinsam mit R(7) einen Pyrrolidin- oder Piperidin-Ring bildet;
aa 1 oder 2;
bb 0 oder 1;
aa + bb = 2;
R(19) Wasserstoff oder Alkyl mit 1, 2, 3 oder 4 C-Atomen
R(20) Wasserstoff oder Methyl;

B einen Phenylen- oder Naphthylenrest,

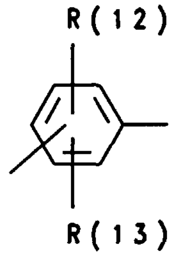

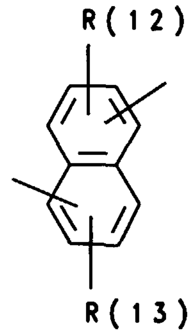

R(12) und R(13)

unabhängig voneinander Wasserstoff, Methyl, F, Cl, Br, J, $CF_3$ oder $-SO_w$-R(14);
R(14) Methyl oder NR(15)R(16);
R(15) und R(16)

unabhängig voneinander Wasserstoff oder Alkyl mit 1, 2, 3 oder 4 C-Atomen;

w Null, 1 oder 2;

D $-C_dH_{2d}-X_f-$;

d Null, 1, 2, 3 oder 4;
X -O-, -CO-, -CH[OR(21)]-, $-SO_m-$ oder -NR(21)-;
f Null oder 1;

R(21) Wasserstoff oder Methyl;
m Null, 1 oder 2;

und die jeweils anderen Substituenten R(1) und R(2) und R(3)

unabhängig voneinander Wasserstoff, F, Cl, Br, J, -CN, $-(C_1-C_8)$-Alkyl, $-(C_2-C_8)$-Alkenyl, -NR(35)R(36) oder R(17)-$C_gH_{2g}$-$Z_h$-;
g Null, 1, 2, 3 oder 4;
h Null oder 1;
R(35) und R(36)

unabhängig voneinander Wasserstoff oder Alkyl mit 1, 2, 3, 4, 5 oder 6 C-Atomen;

oder
R(35) und R(36)

gemeinsam 4 - 7 Methylengruppen, von denen eine $CH_2$-Gruppe durch Sauerstoff, -S-, -NH-, $-NCH_3$ oder -N-Benzyl ersetzt sein kann;

Z -O-,-CO-, $SO_v$-, -NR(18)-, -NR(18)-CO-, -NR(18)-CO-NH- oder -NR(18)-$SO_2$-;

R(18) Wasserstoff oder Methyl;
v Null, 1 oder 2;

R(17) Wasserstoff, Cycloalkyl mit 3, 5 oder 6 C-Atomen oder $C_kF_{2k+1}$-;

k 1, 2 oder 3,

oder
R(17) Pyrrol-1-yl, Pyrrol-2-yl oder Pyrrol-3-yl,

welches nicht substituiert oder substituiert ist mit 1 - 4 Substituenten ausgewählt aus der Gruppe bestehend aus F, Cl, Br, I, -CN, $(C_2-C_8)$-Alkanoyl, $(C_2-C_8)$-Alkoxycarbonyl, Formyl, Carboxy, $-CF_3$, Methyl und Methoxy;

oder
R(17) $-(C_3-C_8)$-Cycloalkyl oder Phenyl,

welches nicht substituiert oder substituiert ist mit 1 - 3 Substituenten ausgewählt aus der Gruppe bestehend aus F und Cl, $-CF_3$, Methyl, Hydroxy, Methoxy, -NR(37)R(38), $CH_3SO_2$- und $H_2NO_2S$-;
R(37) und R(38)

Wasserstoff oder $-CH_3$;

R(4) und R(5)

unabhängig voneinander Wasserstoff, Alkyl mit 1, 2, 3 oder 4 C-Atomen, F, Cl, -OR(32), -NR(33)R(34) oder -$C_rF_{2r+1}$;

R(32), R(33) und R(34)

unabhängig voneinander Wasserstoff oder Alkyl mit 1, 2 oder 3 C-Atomen;

r 1, 2, 3 oder 4;

sowie deren pharmakologisch verträgliche Salze;

(HOE 95/F 173 - NZ 299 052)
am) Substituierte Zimtsäureguanidide der Formel I

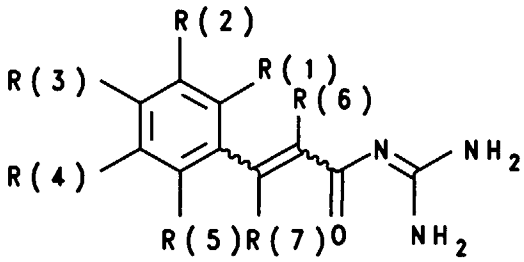

I

worin bedeuten:

mindestens einer der Substituenten R(1), R(2), R(3), R(4) und R(5) $-X_a-Y_b-L_n-U$;

X CR(16)R(17), O, S oder NR(18);

R(16), R(17) und R(18)

unabhängig voneinander H, Alkyl mit 1, 2, 3 oder 4 C-Atomen oder Perfluoralkyl mit 1, 2, 3 oder 4 C-Atomen;

a Null oder 1;

Y Alkylen mit 1, 2, 3, 4, 5, 6, 7 oder 8 C-Atomen, Alkylen-T mit 1, 2, 3, 4, 5, 6, 7 oder 8 C-Atomen in der Alkylengruppe, T, T-Alkylen mit 1, 2, 3, 4, 5, 6, 7 oder 8 C-Atomen in der Alkylengruppe;

T NR(20), O, S oder Phenylen,

wobei das Phenylen nicht substituiert oder substituiert ist mit 1 - 3 Substituenten ausgewählt aus der Gruppe bestehend aus F, Cl, $CF_3$, Methyl, Methoxy und NR(21)R(22);

R(20), R(21) und R(22)

unabhängig voneinander H, Alkyl mit 1, 2, 3 oder 4 C-Atomen oder Perfluoralkyl mit 1, 2, 3 oder 4 C-Atomen;

b Null oder 1;

L O, S, NR(23) oder $C_kH_{2k}$;

k 1, 2, 3, 4, 5, 6, 7, 8;

n Null oder 1;

U NR(24)R(25) oder einen N-haltigen Heterocyclus mit 1, 2, 3, 4, 5, 6, 7, 8 oder 9 C-Atomen;

R(24) und R(25)

unabhängig voneinander Wasserstoff, Alkyl mit 1, 2, 3, 4, 5, 6, 7 oder 8 C-Atomen, Perfluoralkyl mit 1, 2, 3, 4, 5, 6, 7 oder 8 C-Atomen;

oder

R(24) und R(25)

gemeinsam 4 oder 5 Methylenguppen, von denen eine $CH_2$-Gruppe durch Sauerstoff, S, NH, $N-CH_3$ oder N-Benzyl ersetzt sein kann;

wobei die N-haltigen Heterocyclen N- oder C-verbrückt sind und nicht substituiert oder substituiert sind mit 1 - 3 Substituenten ausgewählt aus der Gruppe bestehend aus F, Cl, $CF_3$, Methyl, Methoxy und NR(27)R(28);

R(23), R(27) und R(28)

unabhängig voneinander H, Alkyl mit 1, 2, 3 oder 4 C-Atomen oder Perfluoralkyl mit 1, 2, 3 oder 4 C-Atomen;

und die jeweils anderen Substituenten R(1), R(2), R(3), R(4) und R(5)

unabhängig voneinander H, F, Cl, Br, I, CN, $-O_n-C_mH_{2m+1}$, $-O_p-(CH_2)_s-C_qF_{2q+1}$ oder $-C_rH_{2r}R(10)$;
n Null oder 1;
m Null 1, 2, 3, 4, 5, 6, 7 oder 8;
p Null oder 1;
q 1, 2, 3, 4, 5, 6, 7 oder 8;
s Null, 1, 2, 3 oder 4;
r Null, 1, 2, 3 oder 4;
R(10)

Cycloalkyl mit 3, 4, 5, 6, 7 oder 8 C-Atomen, oder Phenyl,

wobei das Phenyl nicht substituiert oder substituiert ist mit 1 - 3 Substituenten ausgewählt aus der Gruppe bestehend aus F, Cl, $CF_3$, Methyl, Methoxy und NR(11)R(12);
R(11) und R(12)

unabhängig voneinander H, Alkyl mit 1, 2, 3 oder 4 C-Atomen oder Perfluoralkyl mit 1, 2, 3 oder 4 C-Atomen;

R(6) und R(7)

unabhängig voneinander Wasserstoff, F, Cl, Br, I, CN, Alkyl mit 1, 2, 3, 4, 5, 6, 7 oder 8 C-Atomen, Perfluoralkyl mit 1, 2, 3, 4, 5, 6, 7 oder 8 C-Atomen, Cycloalkyl mit 3, 4, 5, 6, 7 oder 8 C-Atomen, oder Phenyl,

das nicht substituiert oder substituiert ist mit 1 - 3 Substituenten ausgewählt aus der Gruppe bestehend aus F, Cl, $CF_3$, Methyl, Methoxy und NR(14)R(15);
R(14) und R(15)

unabhängig voneinander H, Alkyl mit 1, 2, 3 oder 4 C-Atomen oder Perfluoralkyl mit 1, 2, 3 oder 4 C-Atomen;

sowie deren pharmazeutisch verträgliche Salze;

(HOE 96/F 226 - EP 825 178)
bd) Phenylsubstituierte Alkenylcarbonsäure-guanidide der Formel I

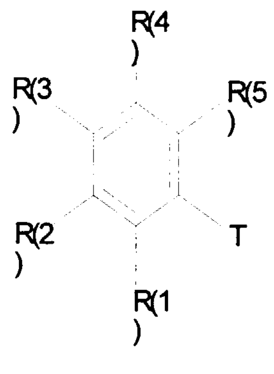

I

worin bedeuten:

T

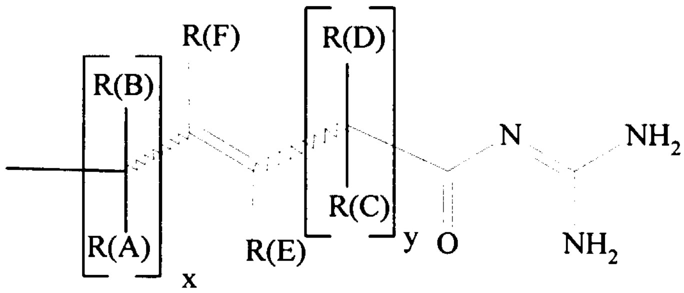

R(A) Wasserstoff, F, Cl, Br, I, CN, OH, OR(6), ($C_1$-$C_4$)-Alkyl, $O_r(CH_2)_aC_bF_{2b+1}$, ($C_3$-$C_8$)-Cycloalkyl oder NR(7)R(8)

r Null oder 1;
a Null, 1, 2, 3 oder 4;
b 1, 2, 3 oder 4;
R(6) ($C_1$-$C_4$)-Alkyl, ($C_1$-$C_4$)-Perfluoralkyl, ($C_3$-$C_6$)-Alkenyl, ($C_3$-$C_8$)-Cycloalkyl, Phenyl oder Benzyl,

wobei der Phenylkern nicht substituiert oder substituiert ist mit 1 - 3 Substituenten ausgewählt aus der Gruppe bestehend aus F, Cl, $CF_3$, Methyl, Methoxy und NR(9)R(10);
R(9) und R(10)

H, ($C_1$-$C_4$)-Alkyl oder ($C_1$-$C_4$)-Perfluoralkyl;

R(7) und R(8)

unabhängig voneinander wie R(6) definiert;

oder
R(7) und R(8)

gemeinsam 4 oder 5 Methylengruppen, von denen eine $CH_2$-Gruppe durch Sauerstoff, Schwefel, NH, N-$CH_3$ oder N-Benzyl ersetzt sein kann;

R(B), R(C) und R(D)

unabhängig wie R(A) definiert;

x Null, 1 oder 2;

y Null, 1 oder 2;

R(F) Wasserstoff, F, Cl, Br, I, CN, OR(12), ($C_1$-$C_8$)-Alkyl, $O_p(CH_2)_fC_gF_{2g+1}$, ($C_3$-$C_8$)-Cycloalkyl oder ($C_1$-$C_9$)-Heteroaryl;

p Null oder 1;
f Null, 1, 2, 3 oder 4;
g 1, 2, 3, 4, 5, 6, 7 oder 8;
R(12) ($C_1$-$C_8$)-Alkyl, ($C_1$-$C_4$)-Perfluoralkyl, ($C_3$-$C_8$)-Alkenyl, ($C_3$-$C_8$)-Cycloalkyl, Phenyl oder Benzyl,

wobei der Phenylkern nicht substituiert ist oder substituiert 1 - 3 Substituenten ausgewählt aus der

Gruppe bestehend aus F, Cl, $CF_3$, Methyl, Methoxy und NR(13)R(14);
R(13) und R(14)

H, $(C_1-C_4)$-Alkyl oder $(C_1-C_4)$-Perfluoralkyl;

R(E) unabhängig wie R(F) definiert;

R(1) unabhängig wie T definiert;
oder
R(1) Wasserstoff, $-O_kC_mH_{2m+1}$, $-O_n(CH_2)_pC_qF_{2q+1}$, F, Cl, Br, I, CN,-(C=O)-N=$C(NH_2)_2$, $-SO_rR(17)$, $-SO_{r2}NR(31)R(32)$; $-O_u(CH_2)_vC_6H_5$, $-O_{u2}-(C_1-C_9)$-Heteroaryl oder $-S_{u2}-(C_1-C_9)$-Heteroaryl;

k Null oder 1;
m Null, 1, 2, 3, 4, 5, 6, 7 oder 8;
n Null oder 1;
p Null, 1, 2, 3 oder 4;
q 1, 2, 3, 4, 5, 6, 7 oder 8;
r Null, 1, 2;
r2 Null, 1, 2;
R(31) und R(32)

unabhängig voneinander Wasserstoff, $(C_1-C_8)$-Alkyl oder $(C_1-C_8)$-Perfluoralkyl;

oder
R(31) und R(32)

gemeinsam 4 oder 5 Methylengruppen, von denen eine $CH_2$-Gruppe durch Sauerstoff, S, NH, $N-CH_3$ oder N-Benzyl ersetzt sein kann;

R(17) $(C_1-C_8)$-Alkyl;
u Null oder 1;
u2 Null oder 1;
v Null, 1, 2, 3 oder 4;

wobei der Phenylkern substituiert ist oder substituiert mit 1 - 3 Substituenten ausgewählt aus der Gruppe bestehend aus F, Cl, $CF_3$, Methyl, Methoxy, $-(CH_2)_wNR(21)R(22)$, NR(18)R(19) und $(C_1-C_9)$-Heteroaryl;
R(18), R(19), R(21) und R(22)

unabhängig voneinander $(C_1-C_4)$-Alkyl oder $(C_1-C_4)$-Perfluoralkyl;

w 1, 2, 3 oder 4;
wobei der Heterocyclus des $(C_1-C_9)$-Heteroaryl unsubstituiert oder substituiert ist mit 1 - 3 Substituenten ausgewählt aus der Gruppe bestehend aus F, Cl, $CF_3$, Methyl oder Methoxy;

R(2), R(3), R(4) und R(5)

unabhängig voneinander wie R(1) definiert,

oder
R(1) und R(2) oder R(2) und R(3)

jeweils gemeinsam -CH-CH=CH-CH-,

das nicht substituiert oder substituiert ist mit 1 - 3 Substituenten ausgewählt aus der Gruppe bestehend aus F, Cl, $CF_3$, Methyl, Methoxy, $-(CH_2)_{w2}NR(24)R(25)$ und NR(26)R(27);
R(24), R(25), R(26) und R(27)

H, $(C_1-C_4)$-Alkyl oder $(C_1-C_4)$-Perfluoralkyl;

w2 1, 2, 3 oder 4;

wobei der Rest T im Molekül mindestens zweimal, jedoch nur höchstens dreimal vorhanden ist;
sowie deren pharmazeutisch verträgliche Salze.

Ganz besonders geeignet sind Verbindungen, die ausgewählt sind aus der Gruppe bestehend aus

3-Dimethylamino-5-trifluormethylbenzoylguanidin,
3-Phenyl-5-trifluormethylbenzoylguanidin,
3-Isopropyl-4-fluor-5-trifluormethylbenzoylguanidin,
3-Chlor-5-(1-pyrrolyl)benzoylguanidin,
3-Chlor-5-trifluormethylbenzoylguanidin,
3-Dimethylamino-5-(1-pyrrolyl)benzoylguanidin,
3-Dimethylamino-4-tert.butylbenzoylguanidin,
4-(3-Chloranilino)-3-methylsulfonylbenzoylguanidin,
4-(4-Aminoethylphenoxy)-3-methylsulfonylbenzoylguanidin,
4-(4-Aminoethylsulfonylmethylphenoxy)-3-trifluormethylbenzoylguanidin,
4-(4-Aminoethylthiophenoxy)-3-trifluormethylbenzoylguanidin,
3,5-Di-tert.butylbenzoylguanidin,
3,5-Di-tert.butyl-4-hydroxybenzoylguanidin,
5-Chlor-2-indoloylguanidin,
3,5-Dichlor-2-indoloylguanidin,
5-Chlor-3-phenylthio-2-indoloylguanidin,
5-Chlor-3-phenylsulfonyl-2-indoloylguanidin,
5-Methoxy-3-isopropyl-2-indoloylguanidin,
4-Benzyloxy-2-indoloylguanidin,
5-Chlor-1-(4-picolyl)-2-indoloylguanidin,
2-(4-Hydroxyphenyl)-4-benzimidazoloylguanidin,
N-{3-[3-(3-Guanidino-2-methyl-3-oxo-propenyl)-phenyl]2-methyl-acryloyl}-guanidin,
und deren pharmakologisch verträglichen Salzen.

**Patentansprüche**

1. Verwendung eines Inhibitors des $Na^+/H^+$-Austauschers zur Herstellung eines Medikaments zur Behandlung oder Prophylaxe von Krankheiten, die durch Protozoen verursacht werden.

2. Verwendung eines Inhibitors des $Na^+/H^+$-Austauschers nach Anspruch 1 zur Herstellung eines Medikaments zur Behandlung oder Prophylaxe der Malaria tropica.

3. Verwendung eines Inhibitors des $Na^+/H^+$-Austauschers nach Anspruch 1 zur Herstellung eines Medikaments zur Behandlung oder Prophylaxe der Malaria tertiana.

4. Verwendung eines Inhibitors des $Na^+/H^+$-Austauschers nach Anspruch 1 zur Herstellung eines Medikaments zur Behandlung oder Prophylaxe der Malaria quartana.

5. Verwendung eines Inhibitors des $Na^+/H^+$-Austauschers nach Anspruch 1 zur Herstellung eines Medikaments zur Behandlung oder Prophylaxe der Toxoplasmose.

6. Verwendung eines Inhibitors des $Na^+/H^+$-Austauschers nach Anspruch 1 zur Herstellung eines Medikaments zur Behandlung oder Prophylaxe der Coccidiose.

7. Verwendung eines Inhibitors des $Na^+/H^+$-Austauschers nach Anspruch 1 zur Herstellung eines Medikaments zur Behandlung oder Prophylaxe der Darmsarcosporidose.

8. Verwendung eines Inhibitors des $Na^+/H^+$-Austauschers nach Anspruch 1 zur Herstellung eines Medikaments zur Behandlung oder Prophylaxe der Cryptosporidose.

9. Verwendung eines Inhibitors des $Na^+/H^+$-Austauschers nach Anspruch 1 zur Herstellung eines Medikaments zur

Behandlung oder Prophylaxe der Chargaskrankheit.

10. Verwendung eines Inhibitors des $Na^+/H^+$-Austauschers nach Anspruch 1 zur Herstellung eines Medikaments zur Behandlung oder Prophylaxe der kutanen und visceralen sowie anderen Formen von Leishmaniosen.

11. Verwendung eines Inhibitors des $Na^+/H^+$-Austauschers nach Anspruch 1 zur Herstellung eines Medikaments zur Behandlung oder Prophylaxe von Krankheiten bei Tieren, die durch veterinärpathogene Protozoen infiziert werden.

12. Verwendung nach Ansprüchen 1-11, dadurch gekennzeichnet, daß eine Verbindung benutzt wird, die ausgewählt ist aus der Gruppe bestehend aus den Verbindungen
I. (HOE 89/F 288 - US 5 292 755)
a) Benzoylguanidine der Formel I

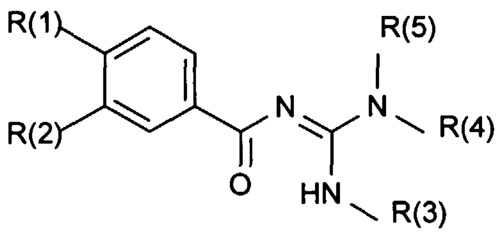

I

worin bedeuten:

R(1) oder R(2)

R(6)-$S(O)_n$- oder R(7)R(8)N-$O_2S$-;

und der jeweils andere Substituent R(1) oder R(2)

H, F, Cl, Br, $(C_1$-$C_4)$-Alkyl, $(C_1$-$C_4)$-Alkoxy oder Phenoxy,

das unsubstituiert ist oder substituiert mit 1 - 3 Substituenten ausgewählt aus der Gruppe bestehend aus Fluor, Chlor, Methyl und Methoxy;

oder der jeweils andere Substituent R(1) oder R(2)

R(6)-$S(O)_n$ oder R(7)R(8)N-;
n Null, 1 oder 2;
R(6) $(C_1$-$C_6)$-Alkyl, $(C_5$-$C_7)$-Cycloalkyl, Cyclopentylmethyl, Cyclohexylmethyl oder Phenyl,

das unsubstituiert ist oder substituiert mit 1 - 3 Substituenten ausgewählt aus der Gruppe bestehend aus Fluor, Chlor, Methyl und Methoxy;

R(7) und R(8)

gleich oder verschieden H oder $(C_1$-$C_6)$-Alkyl;

oder
R(7) Phenyl-$(CH_2)_m$;

m 1 - 4;

oder
R(7) Phenyl,

das unsubstituiert ist oder substituiert mit 1 - 2 Substituenten ausgewählt aus der Gruppe bestehend

aus Fluor, Chlor, Methyl und Methoxy;

oder
R(7) und R(8)

gemeinsam eine geradkettige oder verzweigte ($C_4$-$C_7$)-Kette, wobei die Kette zusätzlich durch O, S oder NR(9) unterbrochen sein kann;
R(9) H oder Methyl;

oder
R(7) und R(8)

gemeinsam mit dem Stickstoffatom, an das sie gebunden sind, ein Dihydroindol-, Tetrahydrochinolin- oder Tetrahydroisochinolin-System;

R(3), R(4) und R(5)

unabhängig voneinander H oder ($C_1$-$C_2$)-Alkyl,

oder
R(3) und R(4)

gemeinsam eine ($C_2$-$C_4$)-Alkylenkette;

oder
R(4) und R(5)

gemeinsam eine ($C_4$-$C_7$)-Alkylenkette;

sowie deren pharmazeutisch verträgliche Salze;

(HOE 92/F 34 - US 5 373 924)
b) Benzoylguanidine der Formel I

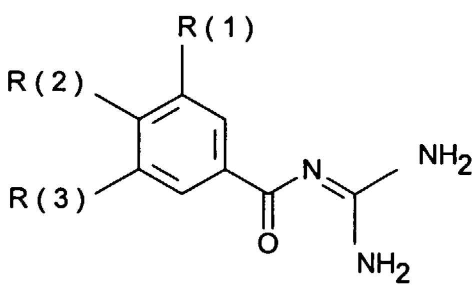

(I)

worin bedeuten:

R(1) R(4)-$SO_m$ oder R(5)R(6)N-$SO_2$-;

m Null, 1 oder 2;
R(4) und R(5)

$C_1$-$C_8$-Alkyl, $C_3$-$C_6$-Alkenyl oder -$C_nH_{2n}$-R(7);
n Null, 1, 2, 3 oder 4;
R(7) $C_5$-$C_7$-Cycloalkyl oder Phenyl,

welches unsubstituiert ist oder substituiert mit 1 - 3 Substituenten ausgewählt aus der Gruppe bestehend aus F, Cl, $CF_3$, Methyl, Methoxy und NR(8)R(9);

R(8) und R(9)

H oder $C_1$-$C_4$-Alkyl;

oder
R(5) H;
R(6) H oder $C_1$-$C_4$-Alkyl,
oder
R(5) und R(6)

gemeinsam 4 oder 5 Methylengruppen, von denen eine $CH_2$-Gruppe durch ein O, S, NH, N-$CH_3$ oder N-Benzyl ersetzt sein kann;

R(2) Wasserstoff, F, Cl, Br, ($C_1$-$C_4$)-Alkyl-, O-$(CH_2)_mC_pF_{2p+1}$ oder-X-R(10);

m Null oder 1;
p 1, 2 oder 3;
X O, S oder NR(11);
R(10) H, $C_1$-$C_6$-Alkyl, $C_5$-$C_7$-Cycloalkyl, Cyclohexylmethyl, Cyclopentylmethyl oder -$C_nH_{2n}$-R(12);
n Null, 1, 2, 3 oder 4;
R(12) Phenyl,

das unsubstituiert oder substituiert ist mit 1 - 3 Substituenten ausgewählt aus der Gruppe bestehend aus F, Cl, $CF_3$, Methyl, Methoxy und NR(8)R(9);
R(8) und R(9)

gleich H oder $C_1$-$C_4$-Alkyl;

R(11) Wasserstoff oder $C_1$-$C_3$-Alkyl;
oder
R(10) und R(11)

gemeinsam 4 oder 5 Methylengruppen, von denen eine $CH_2$-Gruppe durch O, S, NH, N-$CH_3$ oder N-Benzyl ersetzt sein kann;

R(3) wie R(1) definiert, oder $C_1$-$C_6$-Alkyl, Nitro, Cyano, Trifluormethyl, F, Cl, Br, J oder -X-R(10);

X O, S oder NR(11);

R(10) gleich H, $C_1$-$C_6$-Alkyl, $C_5$-$C_7$-Cycloalkyl, Cyclohexylmethyl, Cyclopentylmethyl oder -$C_nH_{2n}$-R(12);
n null bis 4;
R(12) Phenyl,

das unsubstituiert ist oder substituiert mit 1 - 3 Substituenten ausgewählt aus der Gruppe bestehend aus F, Cl, $CF_3$, Methyl, Methoxy und NR(8)R(9);
R(8) und R(9)

H oder $C_1$-$C_4$-Alkyl;

R(11) $C_1$-$C_3$-Alkyl,
oder
R(10) und R(11)

gemeinsam 4 oder 5 Methylengruppen, von denen eine $CH_2$-Gruppe durch O, S, NH, N-$CH_3$ oder N-Benzyl ersetzt sein kann;

sowie deren pharmazeutisch verträgliche Salze;

(HOE 92/F 035 EP-OS 556 673)
c) Ortho-substituierte Benzoylguanidine der Formel I

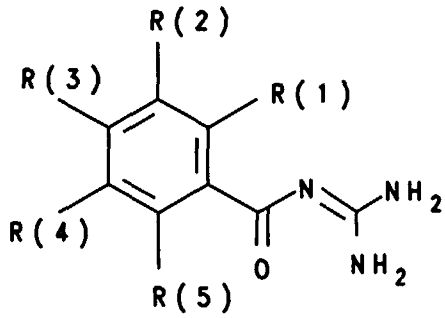

worin bedeuten:

R(1) F, Cl, Br, J, $C_1$-$C_6$-Alkyl oder -X-R(6);

X O, S, NR(7) oder Y-ZO;

Y O oder NR(7);
Z C oder SO;

R(6) H, $C_1$-$C_6$-Alkyl, $C_5$-$C_7$-Cycloalkyl, Cyclohexylmethyl, Cyclopentylmethyl, $-(CH_2)_mC_pF_{2p+1}$ oder $-C_nH_{2n}$-R(8);
m Null oder 1;
p 1 - 3;
n Null bis 4;
R(8) Phenyl,

das unsubstituiert ist oder substituiert mit 1 - 3 Substituenten ausgewählt aus der Gruppe bestehend aus den Gruppen F, Cl, $CF_3$, Methyl, Methoxy und NR(9)R(10);
R(9) und R(10)

H oder $C_1$-$C_4$-Alkyl;

R(7) H oder $C_1$-$C_3$-Alkyl; oder
R(6) und R(7)

gemeinsam 4 oder 5 Methylengruppen, von denen eine $CH_2$-Gruppe durch ein O, S, NH, N-$CH_3$ oder N-Benzyl ersetzt sein kann;

R(3) H oder -X-R(6);

X O, S, NR(7) oder Y-ZO;

R(7) H oder $C_1$-$C_3$-Alkyl;
Y O oder NR(7);

wobei Y am Phenylrest der Formel I gebunden ist,

Z C oder SO;

R(6) H, $C_1$-$C_6$-Alkyl, $C_5$-$C_7$-Cycloalkyl, Cyclohexylmethyl, Cyclopentylmethyl, $-(CH_2)_mC_pF_{2p+1}$ oder $-C_nH_{2n}$-R(8);
m null oder 1;

p 1 - 3;
n null bis 4;
R(8) Phenyl,

das unsubstituiert ist oder substituiert mit 1 - 3 Substituenten ausgewählt aus der Gruppe bestehend aus F, Cl, $CF_3$, Methyl, Methoxy und NR(9)R(10);
R(9) und R(10)

H oder $C_1$-$C_4$-Alkyl;

oder
R(6) und R(7)

gemeinsam 4 oder 5 Methylengruppen, von denen eine $CH_2$-Gruppe durch ein O, S, NH, N-$CH_3$ oder N-Benzyl ersetzt sein kann;

R(2) und R(4)

gleich oder verschieden R(11)-$SO_q$- oder R(12)R(13)N-$SO_2$-;
q null - 2;
R(11) $C_1$-$C_4$-Alkyl,

das unsubstituiert ist oder Phenyl als Substituenten trägt, wobei Phenyl unsubstituiert ist oder substituiert mit 1 - 3 Substituenten ausgewählt aus der Gruppe bestehend aus F, Cl, $CF_3$, Methyl, Methoxy und NR(9)R(10);
R(9) und R(10)

H oder $C_1$-$C_4$-Alkyl;

R(12) und R(13)

wie R(6) und R(7) definiert;

oder
einer der beiden Reste R(2) oder R(4)

Wasserstoff oder wie R(1) definiert;

R(5) H, Methyl, F, Cl oder Methoxy,
sowie deren pharmazeutisch verträgliche Salze.

(HOE 92/F 036 - US 5 364 868)
d) Benzoylguanidine der Formel I

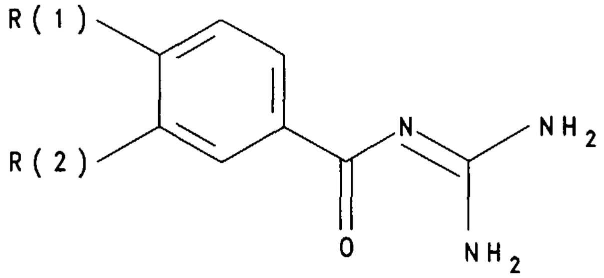

(I)

worin bedeuten:

R(1) oder R(2)

eine Aminogruppe -NR(3)R(4);
R(3) und R(4)

gleich oder verschieden H, $C_1$-$C_6$-Alkyl oder $C_3$-$C_7$-Cycloalkyl;

oder
R(3) Phenyl-$(CH_2)_p$-;

p 0, 1, 2, 3 oder 4;

oder
R(3) Phenyl,
wobei das Phenyl jeweils unsubstituiert ist oder einen bis zwei Substituenten ausgewählt aus der Gruppe bestehend aus Fluor, Chlor, Methyl und Methoxy trägt;
oder
R(3) und R(4)

gemeinsam eine geradkettige oder verzweigte $C_4$-$C_7$-Methylenkette sein können, wobei ein $-CH_2$-Glied der Methylenkette durch Sauerstoff, S oder NR(5) ersetzt sein kann;
R(5) H oder niedriges Alkyl;

der jeweils andere Substituent R(1) oder R(2)

H, F, Cl, $C_1$-$C_4$-Alkyl, $C_1$-$C_4$-Alkoxy, $CF_3$, $C_mF_{2m+1}$-$CH_2$-, Benzyl oder Phenoxy,

wobei der jeweilige Phenylrest unsubstituiert ist oder einen bis zwei Substituenten ausgewählt aus der Gruppe bestehend aus Methyl, Methoxy, Fluor und Chlor trägt;

m 1, 2 oder 3;

sowie deren pharmazeutisch verträgliche Salze;

(92/F 197 K - NZ 248 013)
e) Benzoylguanidine der Formel I

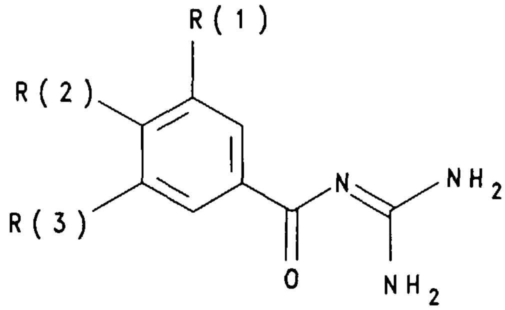

( I )

worin bedeuten:

R(1) R(4)-$SO_m$ oder R(5)R(6)N-$SO_2$-;

m Null, 1 oder 2;
R(4) und R(5)

$C_1$-$C_8$-Alkyl, $C_3$-$C_6$-Alkenyl oder $-C_nH_{2n}$-R(7);
n Null, 1, 2, 3 oder 4;

R(7) $C_5$-$C_7$-Cycloalkyl oder Phenyl,

welches unsubstituiert ist oder substituiert mit 1 - 3 Substituenten ausgewählt aus der Gruppe bestehend aus F, Cl, $CF_3$, Methyl, Methoxy und NR(8)R(9);
R(8) und R(9)

H oder $C_1$-$C_4$-Alkyl;

oder
R(5) H;
R(6) H oder $C_1$-$C_4$-Alkyl;
oder
R(5) und R(6)

gemeinsam 4 oder 5 Methylengruppen, von denen eine $CH_2$-Gruppe durch ein O, S, NH, N-$CH_3$ oder N-Benzyl ersetzt sein kann;

R(2) Wasserstoff, geradkettiges oder verzweigtes ($C_5$-$C_8$)-Alkyl,

-CR(13)=CHR(12) oder -C≡CR(12);
R(12) Phenyl,

das unsubstituiert oder substituiert ist mit 1 - 3 Substituenten ausgewählt aus der Gruppe bestehend aus F, Cl, $CF_3$, Methyl, Methoxy und NR(14)R(15);
R(14) und R(15)

H oder ($C_1$-$C_4$)-Alkyl;

oder
R(12) ($C_1$-$C_9$)-Heteroaryl,

das unsubstituiert oder wie Phenyl substituiert ist,

oder
R(12) ($C_1$-$C_6$)-Alkyl,

das unsubstituiert oder mit 1 - 3 OH substituiert ist,

oder
R(12) ($C_3$-$C_8$)-Cycloalkyl;
R(13) Wasserstoff oder Methyl, oder
R(12) ($C_3$-$C_8$)-Cycloalkyl, ($C_3$-$C_8$)-Cycloalkyl-($C_1$-$C_4$)-alkyl, Phenyl, $C_6H_5$-($C_1$-$C_4$)-Alkyl, Naphthyl, Biphenylyl, 1,1-Diphenyl-($C_1$-$C_4$)-Alkyl, Cyclopentadienyl, Pyridyl, Pyrrolyl, Furanyl, Thienyl, Thiazolyl, Oxazolyl, Indenyl, Chinolyl, Indolyl, Benzofuranyl, Benzothienyl, Benzothiazolyl, Benzoxazolyl, Imidazolyl, Pyrazolyl, Triazolyl, Tetrazolyl, Isoxazolyl, Isothiazolyl, Pyrazinyl, Pyrimidinyl, Pyridazinyl, Indazolyl, Isochinolyl, Phthalazinyl, Chinoxalinyl, Chinazolinyl oder Cinnolinyl;

R(3) wie R(2) definiert;
und wobei die aromatischen Substituenten R(2) bzw. R(3) unsubstituiert oder mit 1-3 Substituenten aus den Gruppen F, Cl, $CF_3$, ($C_1$-$C_4$)-Alkyl, -Alkoxy, oder NR(10)R(11) mit R(10) und R(11) in der Bedeutung von H oder ($C_1$-$C_4$)-Alkyl substituiert sind;
sowie deren pharmazeutisch verträgliche Salze.

(HOE 92/F 303 K - EP-OS589 336, NZ 248 703)
f) Benzoylguanidine der Formel I

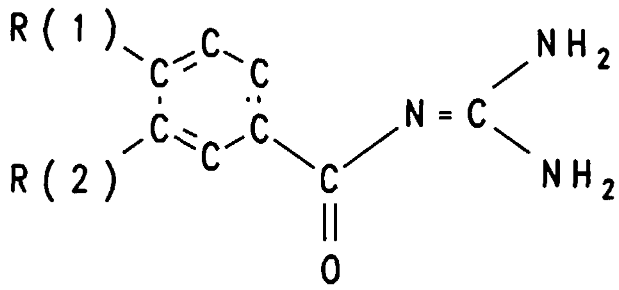

I

worin bedeuten:

R(1) oder R(2)

R(3)-S(O)$_n$- oder R(4)R(5)N-$SO_2$-

der jeweils andere Substituent R(1) oder R(2)

H, OH, F, Cl, Br, J, $C_1$-$C_4$-Alkyl, $C_1$-$C_4$-Alkoxy, Benzyloxy oder Phenoxy,

das unsubstituiert ist oder einen bis drei Substituenten ausgewählt aus der Gruppe bestehend aus Fluor, Chlor, Methyl, Methoxy, Hydroxy oder Benzyloxy trägt,

R(3)-S(O)$_n$, -NR4)R(5) oder 3,4-Dehydropiperidin
R(3) $C_1$-$C_6$-Alkyl, $C_5$-$C_7$-Cycloalkyl, Cyclopentylmethyl, Cyclohexylmethyl oder Phenyl,

das unsubstituiert ist oder einen bis drei Substituenten ausgewählt aus der Gruppe bestehend aus Fluor, Chlor, Methyl und Methoxy trägt;

R(4) und R(5)

gleich oder verschieden, H oder $C_1$-$C_6$-Alkyl;

oder
R(4) Phenyl-($CH_2$)$_m$-;

m 1, 2, 3 oder 4;

oder
R(4) Phenyl,

das unsubstituiert ist oder einen bis zwei Substituenten ausgewählt aus der Gruppe bestehend aus Fluor, Chlor, Methyl und Methoxy trägt;

oder
R(4) und R(5)
gemeinsam eine geradkettige oder verzweigte $C_4$-$C_7$-Kette, wobei die Kette zusätzlich durch O, S oder NR(6) unterbrochen sein kann,
R(6) H oder Methyl;
oder
R(4) und R(5)

gemeinsam mit dem Stickstoffatom, an das sie gebunden sind, ein Dihydroindol, Tetrahydrochinolin oder Tetrahydroisochinolin-System;

n Null, 1 oder 2;

sowie deren pharmazeutisch verträgliche Salze.

(92/F 304 - US 5 416 094)
g) Isochinoline der Formel I

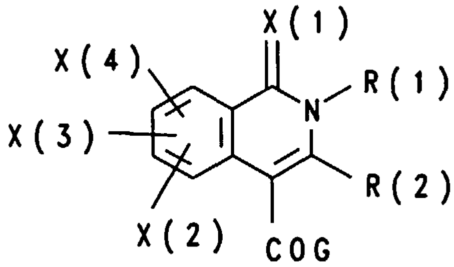

I

worin bedeuten:

R(1) Wasserstoff, Alkyl, Cycloalkyl, Arylalkyl, Alkenyl, substituiertes Aminoalkyl oder ein Aryl- oder Heteroarylring;

wobei die ringe unsubstituiert oder substituiert sind mit 1 - 3 Gruppen ausgewählt aus der Gruppe bestehend aus Halogen, Nitro, Amino, Mono(niederalkyl)amino, Di(niederalkyl)amino, Nieder alkyl, Niederalkoxy, Benzyloxy, Phenoxy, Hydroxy, Trifluoromethyl,

R(2) Wasserstoff, Halogen, Alkyl, oder Aryl;

welches unsubstituiert ist oder substituiert mit 1 - 3 Gruppen ausgewählt aus der Gruppe bestehend aus Halogen, Nitro, Amino, Mono(niederalkyl)amino, Di(niederalkyl)amino, Niederalkyl, Niederalkoxy, Benzyloxy, Phenoxy, Hydroxy,

G

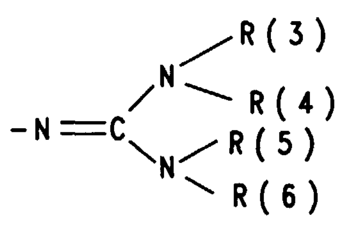

VII

X(2), X(3) and X(4)

unabhängig voneinander Wasserstoff, Halogen, Nitro, Amino, Alkyl, Sulfonamid, Mono(niederalkyl)amino, Di(niederalkyl)amino, Niederalkyl, Benzyloxy, Hydroxy;

X(1) Wasserstoff, Sauerstoff, Schwefel oder NR(7);

R(7) Wasserstoff, Alkyl, Cycloalkyl, Arylalkyl, Alkenyl, substituiertes Aminoalkyl oder ein aryl oder ein Heteroarylring;

weiche ringe unsubstituiert oder substituiert sind mit 1 - 3 Gruppen ausgewählt aus der Gruppe bestehend aus Halogen, Nitro, Amino, Mono(niederalkyl)amino, Di(niederalkyl)amino, Niederalkyl, Niederalkoxy, Benzyloxy, Phenoxy, Hydroxy und Trifluoromethyl;

in welchen Substituenten jede Alkylkette oder Alkenylkette durch Sauerstoff, Schwefel oder NR(8) unterbrochen sein kann;

R(8) Wasserstoff, Alkyl, Cycloalkyl, Arylalkyl, Alkenyl, substituiertes Aminoalkyl oder ein Aryl- oder Heteroarylring,

welche Ringe unsubstituiert oder substituiert sind mit 1 - 3 Gruppen ausgewählt aus der Gruppe bestehend aus Halogen, Nitro, Amino, Mono(niederalkyl)amino, Di(niederalkyl)amino, Niederalkyl, Niederalkoxy, Benzyloxy, Phenoxy, Hydroxy und Trifluoromethyl;

und ihre pharmazeutisch akzeptablen Salze;

92/F 404 - EP 602 522, NZ 250 438)

h) Verbindungen der Formel I

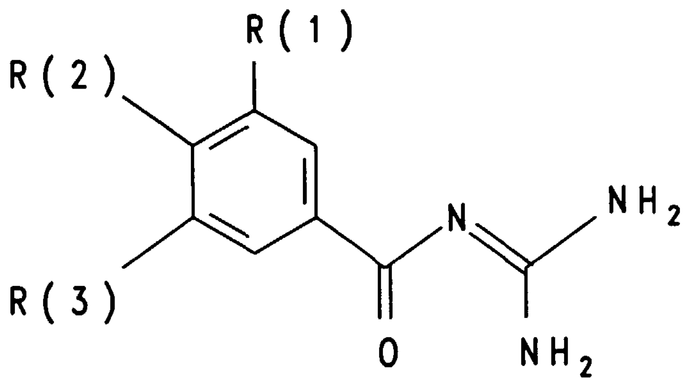

(I)

worin bedeuten:

R(1) Wasserstoff, F, Cl, Br, I, $-NO_2$, -C≡N, $-CF_3$, R(4)-$SO_m$ oder R(5)R(6)N-$SO_2$-;

m Null, 1 oder 2;

R(4) und R(5)

($C_1$-$C_8$)-Alkyl, ($C_3$-$C_6$)-Alkenyl, $-C_nH_{2n}$-R(7) oder $CF_3$;

n Null, 1, 2, 3 oder 4;

R(7) ($C_3$-$C_7$)-Cycloalkyl oder Phenyl,

welches nicht substituiert ist oder substituiert mit 1 - 3 Substituenten ausgewählt aus der Gruppe bestehend aus F, Cl, $CF_3$, Methyl, Methoxy und NR(8)R(9);

R(8) und R(9)

H oder $C_1$-$C_4$-Alkyl;

oder

R(5) H;

R(6) H oder ($C_1$-$C_4$)-Alkyl;

oder

R(5) und R(6)

gemeinsam 4 oder 5 Methylengruppen, von denen eine $CH_2$-Gruppe durch Sauerstoff, S, NH, N-$CH_3$ oder N-Benzyl ersetzt sein kann;

R(2) -SR(10), -OR(10), -NHR(10), -NR(10)R(11), -CHR(10)R(12), -[CR(12)R(13)OR(13')], -{C-[$CH_2$-OR(13')]R(12)(R(13)} oder -[CR(18)R(17)]$_p$-(CO)-[CR(19)R(20)]$_q$-R(14);

R(10) und R(11)

gleich oder verschieden
$-[CHR(16)]_s-(CH_2)_p-(CHOH)_q-(CH_2)_r-(CHOH)_t-R(21)$ oder $-(CH_2)_p-O-(CH_2-CH_2O)_q-R(21)$,
R(21) Wasserstoff, Methyl,
p, q, r gleich oder verschieden

Null, 1, 2, 3 oder 4;

s Null oder 1;
t 1, 2, 3 oder 4;

R(12) und R(13)

gleich oder verschieden Wasserstoff, $(C_1-C_6)$-Alkyl oder zusammen mit dem sie tragenden Kohlenstoff-Atom ein $(C_3-C_8)$-Cycloalkyl,

R(13') Wasserstoff oder $(C_1-C_4)$-Alkyl;
R(14) H, $(C_1-C_6)$-Alkyl, $(C_3-C_8)$-Cycloalkyl oder $-C_aH_{2a}-R(15)$;

a Null, 1, 2, 3 oder 4;
R(15) Phenyl,

das unsubstituiert oder substituiert ist mit 1 - 3 Substituenten ausgewählt aus der Gruppe bestehend aus F, Cl, $CF_3$, Methyl, Methoxy und NR(8)R(9);
R(8) und R(9)

H oder $(C_1-C_4)$-Alkyl;

oder
R(15) $(C_1-C_9)$-Heteroaryl,

das unsubstituiert oder wie Phenyl substituiert ist,

oder
R(15) $(C_1-C_6)$-Alkyl,

das unsubstituiert oder mit 1 - 3 OH substituiert ist;

R(16), R(17), R(18), R(19) und R(20)

Wasserstoff oder $(C_1-C_3)$-Alkyl;

R(3) wie R(1) definiert,
oder
R(3) $(C_1-C_6)$-Alkyl oder -X-R(22);

X Sauerstoff, S oder NR(16);

R(16) H oder $(C_1-C_3)$-Alkyl;
oder
R(22) und R(16)

gemeinsam 4 oder 5 Methylengruppen, von denen eine $CH_2$-Gruppe durch Sauerstoff, S, NH, N-$CH_3$ oder N-Benzyl ersetzt sein kann;

R(22) wie R(14) definiert;

sowie deren pharmazeutisch verträgliche Salze.

(HOE 92/F 405 - EP 602 523, NZ 250 437)
i) Benzoylguanidine der Formel I

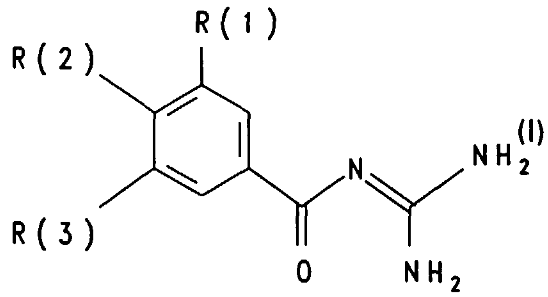

worin bedeuten:

R(1) Wasserstoff, F, Cl, Br, I, $-NO_2$, -C≡N, R(16)-$C_pH_{2p}$-$O_q$, R(4)-$SO_m$ oder R(5)R(6)N-$SO_2$-;

m Null, 1 oder 2;
p Null oder 1;
q Null, 1, 2 oder 3;
R(16) $C_rF_{2r+1}$;

r 1, 2 oder 3;

R(4) und R(5)

($C_1$-$C_8$)-Alkyl, ($C_3$-$C_6$)-Alkenyl, -$C_nH_{2n}$-R(7) oder $CF_3$;
n Null, 1, 2, 3 oder 4;
R(7) ($C_3$-$C_7$)-Cycloalkyl oder Phenyl,

welches nicht substituiert ist oder substituiert mit 1 - 3 Substituenten ausgewählt aus der Gruppe bestehend aus F, Cl, $CF_3$, Methyl, Methoxy und NR(8)R(9);
R(8) und R(9)

H oder $C_1$-$C_4$-Alkyl;

oder
R(5) H;
R(6) H oder ($C_1$-$C_4$)-Alkyl;
oder
R(5) und R(6)

gemeinsam 4 oder 5 Methylengruppen, von denen eine $CH_2$-Gruppe durch Sauerstoff, S, NH, N-$CH_3$ oder N-Benzyl ersetzt sein kann,

R(2) ($C_1$-$C_9$)-Heteroaryl,

das über C oder N verknüpft ist und das unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;

oder

R(2) -SR(10), -OR(10), -NR(10)R(11), -CR(10)R(11)R(12);

R(10) $-C_aH_{2a}-(C_1-C_9)$-Heteroaryl,

das unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;

a Null, 1 oder 2;
R(11) und R(12)

unabhängig voneinander wie R(10) definiert oder Wasserstoff oder $(C_1-C_4)$-Alkyl;

R(3) wie R(1) definiert, oder $(C_1-C_6)$-Alkyl oder -X-R(13);

X Sauerstoff, S, oder NR(14);

R(14) H oder $(C_1-C_3)$-Alkyl;

R(13) H, $(C_1-C_6)$-Alkyl, $(C_3-C_8)$-Cycloalkyl oder $-C_bH_{2b}$-R(15);
b Null, 1, 2, 3 oder 4;
oder
R(13) und R(14)

gemeinsam 4 oder 5 Methylengruppen, von denen $CH_2$-Gruppe durch Sauerstoff, S, NH, N-$CH_3$ oder N-Benzyl ersetzt sein kann;
R(15) Phenyl,

das unsubstituiert oder substituiert ist mit 1 - 3 Substituenten ausgewählt aus der Gruppe bestehend aus F, Cl, $CF_3$, Methyl, Methoxy und NR(8)R(9);
R(8) und R(9)

H oder $(C_1-C_4)$-Alkyl;

sowie deren pharmazeutisch verträgliche Salze;

(HOE 92/F 411 - NZ 250 450, EP 603 650)
k) Benzoylguanidine der Formel I

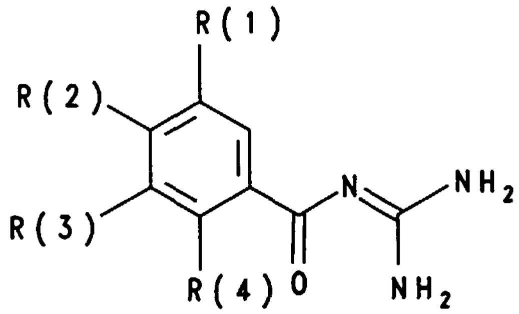

(I)

worin bedeuten:

einer der Substituenten R(1), R(2), R(3) oder R(4):

eine Aminogruppe

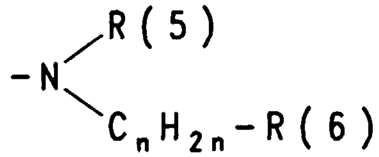

R(5) Wasserstoff oder $C_{(1-6)}$-Alkyl;
n Null, 1, 2, 3 oder 4;
R(6) H oder $C_{(1-4)}$-Alkyl;
worin eine $CH_2$-Gruppe durch 1 S-Atom oder eine Gruppe NR(7) ersetzt sein kann;
R(7) Wasserstoff, Methyl oder Ethyl;
oder
R(6) $C_{(3-8)}$-Cycloalkyl oder Phenyl,

das unsubstituiert ist oder 1, 2 oder 3 Substituenten ausgewählt aus der Gruppe bestehend aus F, Cl, Br, Methyl, Methoxy, -NR(8)R(9) trägt;
R(8) und R(9)

H, Methyl oder Ethyl;

oder
R(5) und R(6)

gemeinsam mit dem Stickstoffatom einen 5-, 6- oder 7-gliedrigen Ring, in welchem 1 C-Atom durch Sauerstoff, S oder NR(10) ersetzt sein kann;
R(10) H, $C_{(1-3)}$-Alkyl oder Benzyl;

und die jeweils anderen Substituenten R(1), R(2), R(3), R(4):

Wasserstoff, F, Cl, Br, I, CN, $CF_3$, $NO_2$, $CF_3$-O-, $C_mF_{2m+1}$-$CH_2$-O- oder R(11)-$C_qH_{2q}$-$X_p$-;
m 1, 2 oder 3;
q Null, 1, 2, 3 oder 4;
p Null oder 1;
X Sauerstoff oder NR(12);

R(12) H oder $C_{(1-3)}$-Alkyl;

R(11) Wasserstoff, $C_{(1-6)}$-Alkyl, $C_{(3-8)}$-Cycloalkyl oder Phenyl, das unsubstituiert oder substituiert ist mit 1, 2 oder 3 Substituenten ausgewählt aus der Gruppe bestehend aus F, Cl, $CH_3$, $CH_3$-O- und NR(13)R(14);

R(13), R(14)

H, Methyl oder Ethyl;

sowie deren pharmazeutisch verträgliche Salze.

(HOE 92/F 422 - EP 604 852)
I) Benzoylguanidine der Formel I

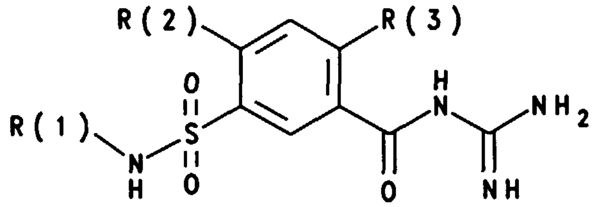

I

worin bedeuten

R(1) R(4)R(5)N-C(X)-;

X Sauerstoff, S oder N-R(6);

R(4) und R(5)

gleich oder verschieden, H, $(C_1-C_8)$-Alkyl, $(C_3-C_6)$-Alkenyl oder $-C_nH_{2n}$-R(7);

n Null, 1, 2, 3 oder 4;

R(7) $(C_5-C_7)$-Cycloalkyl oder Phenyl,

welches unsubstituiert ist oder substituiert mit 1 - 3 Substituenten ausgewählt aus der Gruppe bestehend aus F, Cl, $CF_3$, Methoxy und $(C_1-C_4)$-Alkyl;

oder

R(4) und R(5)

gemeinsam 4 oder 5 Methylengruppen, von denen eine $CH_2$-Gruppe durch Sauerstoff, S, NH, N-$CH_3$ oder N-Benzyl ersetzt sein kann;

R(6) wie R(4) definiert oder gleich Amidin;

R(2) H, F, Cl, Br, I, $(C_1-C_8)$-Alkyl, 1-Alkenyl oder 1-Alkinyl, $(C_3-C_8)$-Cycloalkyl, $(C_3-C_8)$-Cycloalkyl-$(C_1-C_4)$-Alkyl, Phenyl, $C_6H_5$-$(C_1-C_4)$-Alkyl, Naphthyl, Biphenylyl, 1,1-Diphenyl-$(C_1-C_4)$-Alkyl, Cyclopentadienyl, Pyridyl, Thiopyridyl, Pyrrolyl, Furanyl, Thienyl, Thiazolyl, Oxazolyl, Indenyl, Chinolyl, Indolyl, Benzofuranyl, Benzothienyl, Benzothiazolyl, Benzoxazolyl oder -W-R(8);

W Sauerstoff, S oder NR(9);

R(8) H, $(C_1-C_6)$-Alkyl, $(C_5-C_7)$-Cycloalkyl, Cyclohexylmethyl, Cyclopentylmethyl, $-(CH_2)_mC_pF_{2p+1}$ oder $-C_qH_{2q}$-R(10);

m Null oder 1;

p 1, 2 oder 3;

q Null, 1, 2, 3 oder 4;

R(10) Phenyl,

das unsubstituiert oder substituiert ist mit 1 - 3 Substituenten ausgewählt aus der Gruppe bestehend aus F, Cl, $CF_3$, Methyl, Methoxy und NR(11)R(12);

R(11) und R(12)

H oder $(C_1-C_4)$-Alkyl;

R(9) H oder $(C_1-C_3)$-Alkyl;

oder

R(8) und R(9)

gemeinsam 4 oder 5 Methylengruppen, von denen eine $CH_2$-Gruppe durch Sauerstoff, S, NH, N-$CH_3$ oder N-Benzyl ersetzt sein kann;

R(3) H, F, Cl, Br, I, ($C_1$-$C_6$)-Alkyl oder -W-R(8) wie für R(2) definiert, sowie deren pharmazeutisch akzeptable Salze;

(93/F 054 - NZ 250 919, EP-OS 612 723)
m) Benzoylguanidine der Formel I

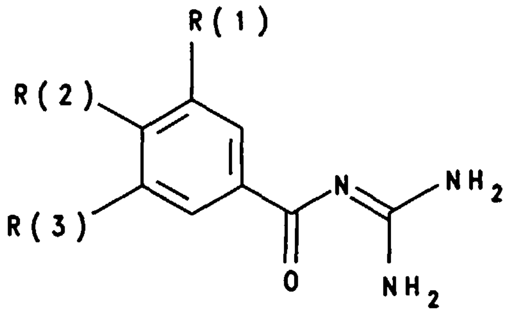

(I)

worin bedeuten:

R(1), R(2), R(3)

Wasserstoff, F, Cl, Br, I oder ($C_1$-$C_{12}$)-Alkyl;

einer der Substituenten R(1), R(2) oder R(3)

$N_3$, CN, OH oder ($C_1$-$C_{10}$)-Alkyloxy, wenn mindestens einer der restlichen Substituenten R(1), R(2) oder R(3) einen hinreichend lipophilen Alkylrest mit 3 bis 12 Kohlenstoff-Atomen bedeutet;

oder
einer der Substituenten R(1), R(2) oder R(3)

R(4)-$C_nH_{2n}$-$O_m$-;
m Null oder 1;
n Null, 1, 2 oder 3;
R(4) $C_pF_{2p+1}$;

p 1, 2 oder 3, sofern n gleich Null oder 1 ist;

oder
R(4) ($C_3$-$C_{12}$)-Cycloalkyl, Phenyl, Pyridyl, Chinolyl oder Isochinolyl, wobei die aromatischen und heteroaromatischen Ringsysteme unsubstituiert sind oder substituiert mit einem Substituenten ausgewählt aus der Gruppe bestehend aus F, Cl, $CF_3$, Methyl, Methoxy und NR(5)R(6);
R(5) und R(6)

Wasserstoff oder ($C_1$-$C_4$)-Alkyl;

oder einer der Substituenten R(1), R(2) oder R(3)

-C≡CR(5) oder -C[R(6)] = CR(5);
R(5) Phenyl,

das unsubstituiert oder substituiert ist mit 1 - 3 Substituenten ausgewählt aus der Gruppe bestehend aus F, Cl $CF_3$, Methyl, Methoxy, Hydroxy, Amino, Methylamino und Dimethylamino, ($C_1$-$C_9$)-Heteroaryl,

das unsubstituiert oder wie Phenyl substituiert ist,

oder
R(5) ($C_1$-$C_6$)-Alkyl,

das unsubstituiert oder mit 1 - 3 OH substituiert ist;

oder
R(5) ($C_3$-$C_8$)-Cycloalkyl,
R(6) Wasserstoff oder Methyl;

sowie deren pharmakologisch akzeptable Salze;

(93/F 153 - EP-OS 627 413, NZ 260 660)
o) Benzoylguanidine der Formel I

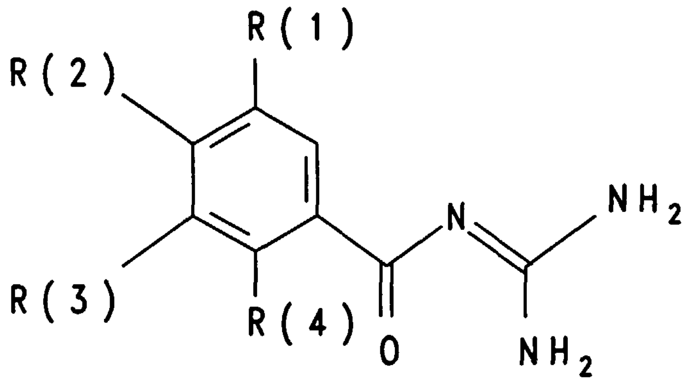

(I)

worin bedeuten:

R(1) Wasserstoff, F, Cl, Br, I, $-NO_2$, -C≡N, $X_o$-$(CH_2)_p$-$(CF_2)_q$-$CF_3$, R(5)-$SO_m$, R(6)-CO- oder R(6)R(7)N-$SO_2$-, mit

X Sauerstoff, S oder NR(14);
m Null 1 oder 2;
o Null oder 1;
p Null, 1 oder 2;
q Null 1, 2, 3, 4, 5 oder 6;
R(5) und R(6)

($C_1$-$C_8$)-Alkyl, ($C_3$-$C_6$)-Alkenyl, $-C_nH_{2n}$-R(8) oder $CF_3$;
n Null, 1, 2, 3 oder 4;
R(8) ($C_3$-$C_7$)-Cycloalkyl oder Phenyl,

welches nicht substituiert ist oder substituiert mit 1 - 3 Substituenten ausgewählt aus der Gruppe bestehend aus F, Cl, $CF_3$, Methyl, Methoxy und NR(9)R(10);
R(9) und R(10)

H oder $C_1$-$C_4$-Alkyl;

oder
R(6) H;
R(7) H oder ($C_1$-$C_4$)-Alkyl;
oder
R(6) und R(7)

gemeinsam 4 oder 5 Methylengruppen, von denen eine $CH_2$-Gruppe durch Sauerstoff, S, NH, $N-CH_3$ oder N-Benzyl ersetzt sein kann;

R(2)

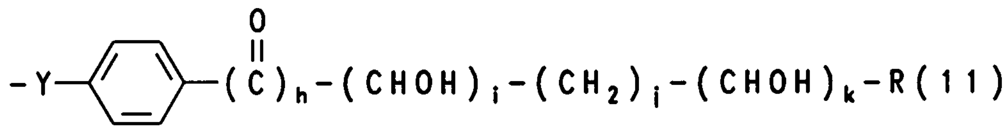

oder 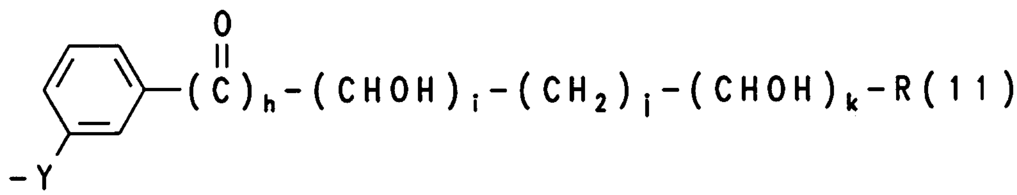

oder 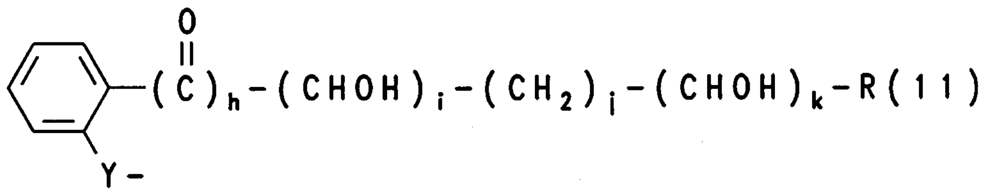

Y Sauerstoff, -S- oder -NR(12)-;
R(11) und R(12)

Wasserstoff oder ($C_1$-$C_3$)-Alkyl;

h Null oder 1;
i, j und k

unabhängig Null, 1, 2, 3 oder 4;

wobei jedoch nicht h, i und k gleichzeitig Null sind,

R(3) wie R(1) definiert, oder ($C_1$-$C_6$)-Alkyl oder -X-R(13);

X Sauerstoff, S oder NR(14);

R(14) H oder ($C_1$-$C_3$)-Alkyl;
R(13) H, ($C_1$-$C_6$)-Alkyl, ($C_3$-$C_8$)-Cycloalkyl oder -$C_bH_{2b}$-R(15);
b Null, 1, 2, 3 oder 4;
oder
R(13) und R(14)

gemeinsam 4 oder 5 Methylengruppen, wobei eine $CH_2$-Gruppe durch Sauerstoff, S, NH, N-$CH_3$ oder N-Benzyl ersetzt sein kann; R(15) Phenyl,

das unsubstituiert oder substituiert ist mit 1 - 3 Substituenten ausgewählt aus der Gruppe bestehend aus F, Cl, $CF_3$, Methyl, Methoxy und NR(9)R(10);
R(9) und R(10)

H oder ($C_1$-$C_4$)-Alkyl;

R(4) Wasserstoff, -OR(16) oder -NR(16)R(17);

R(16) und R(17)

unabhängig Wasserstoff oder ($C_1$-$C_3$)-Alkyl;

sowie deren pharmazeutisch verträgliche Salze;

(HOE 93/F 154 - EP-OS 628 543, NZ 260 681)
p) Benzoylguanidine der Formel I

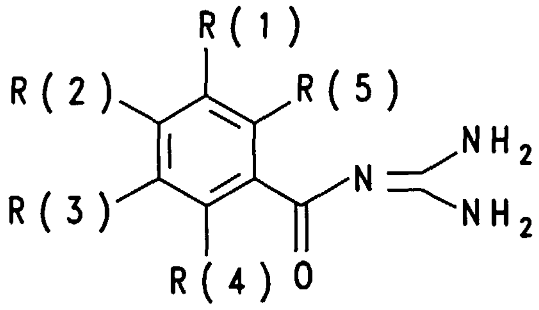

I

worin bedeuten:

R(1) R(6)-CO oder R(7)R(8)N-CO;

R(6) ($C_1$-$C_8$)-Alkyl, ($C_1$-$C_8$)-Perfluoralkyl, ($C_3$-$C_8$)-Alkenyl oder - $C_nH_{2n}$-R(9);
n Null, 1, 2, 3 oder 4;
R(9) ($C_3$-$C_8$)-Cycloalkyl, Phenyl, Biphenylyl oder Naphthyl,

wobei die Aromaten nicht substituiert oder substituiert sind mit 1 - 3 Substituenten ausgewählt aus der Gruppe bestehend aus F, Cl, $CF_3$, Methyl, Methoxy und NR(10)R(11);
R(10) und R(11)

H, ($C_1$-$C_4$)-Alkyl oder ($C_1$-$C_4$)-Perfuoralkyl;

R(7) H, ($C_1$-$C_8$)-Alkyl, ($C_1$-$C_8$)-Perfluoralkyl, ($C_3$-$C_8$)-Alkenyl oder -$C_nH_{2n}$-R(12);

n Null, 1, 2, 3 oder 4;
R(12) ($C_3$-$C_8$)-Cycloalkyl, Phenyl, Biphenylyl oder Naphthyl,

wobei die Aromaten nicht substituiert oder substituiert sind mit 1 - 3 Substituenten ausgewählt aus der Gruppe bestehend aus F, Cl, $CF_3$, Methyl, Methoxy und NR(13)R(14);
R(13) und R(14)

H, ($C_1$-$C_4$)-Alkyl oder ($C_1$-$C_4$)-Perfuoralkyl;

R(8) H, ($C_1$-$C_4$)-Alkyl oder ($C_1$-$C_4$)-Perfluoralkyl;
oder
R(7) und R(8)
gemeinsam 4 oder 5 Methylengruppen, von denen eine $CH_2$-Gruppe durch Sauerstoff, S, NH, N-$CH_3$ oder N-Benzyl ersetzt sein kann;

R(2) R(1) definiert, oder H, F, Cl, Br, I, CN, $NO_2$, ($C_1$-$C_8$)-Alkyl, ($C_1$-$C_8$)-Perfluoralkyl, ($C_3$-$C_8$)-Alkenyl oder -$C_nH_{2n}$R(15);

n Null, 1, 2, 3, 4;
R(15) ($C_3$-$C_8$)-Cycloalkyl, Phenyl, Biphenylyl oder Naphthyl,

wobei die Aromaten nicht substituiert oder substituiert sind mit 1 - 3 Substituenten ausgewählt aus der Gruppe bestehend aus F, Cl, $CF_3$, Methyl, Methoxy und NR(16)R(17);

R(16) und R(17)

H, ($C_1$-$C_4$)-Alkyl oder ($C_1$-$C_4$)-Perfuoralkyl;

oder
R(2) ($C_1$-$C_9$)-Heteroaryl,

das über C oder N verknüpft ist und das unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;

oder
R(2) SR(18), -OR(18), -NR(18)R(19), -CR(18)R(19)R(20);

R(18) -$C_aH_{2a}$-($C_1$-$C_9$)-Heteroaryl,

das unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;

a Null, 1 oder 2;
R(19) und R(20)

unabhängig voneinander wie R(18) definiert oder Wasserstoff, ($C_1$-$C_4$)-Alkyl oder ($C_1$-$C_4$)-Perfluoralkyl;

oder
R(2) R(21)-$SO_m$ oder R(22)R(23)N-$SO_2$-;

m 1 oder 2;
R(21) ($C_1$-$C_8$)-Alkyl, ($C_1$-$C_8$)-Perfluoralkyl, ($C_3$-$C_8$)-Alkenyl, - $C_nH_{2n}$-R(24),

n Null, 1, 2, 3 oder 4;
R(24) ($C_3$-$C_8$)-Cycloalkyl, Phenyl, Biphenylyl oder Naphthyl,

wobei die Aromaten nicht substituiert oder substituiert sind mit 1 - 3 Substituenten ausgewählt aus der Gruppe bestehend aus F, Cl, $CF_3$, Methyl, Methoxy und NR(27)R(28);
R(27) und R(28)

H, ($C_1$-$C_4$)-Alkyl oder ($C_1$-$C_4$)-Perfuoralkyl;

R(22) H, ($C_1$-$C_8$)-Alkyl, ($C_1$-$C_8$)-Perfluoralkyl, ($C_3$-$C_8$)-Alkenyl, -$C_nH_{2n}$-R(29);

n Null, 1, 2, 3 oder 4;
R(29) ($C_3$-$C_8$)-Cycloalkyl, Phenyl, Biphenylyl oder Naphthyl,

wobei die Aromaten nicht substituiert oder substituiert sind mit 1 - 3 Substituenten ausgewählt aus der Gruppe bestehend aus F, Cl, $CF_3$, Methyl, Methoxy und NR(30)R(31);
R(30) und R(31)

H, ($C_1$-$C_4$)-Alkyl oder ($C_1$-$C_4$)-Perfuoralkyl;

R(23) H, ($C_1$-$C_4$)-Alkyl oder ($C_1$-$C_4$)-Perfluoralkyl;
oder
R(22) und R(23)

gemeinsam 4 oder 5 Methylengruppen, von denen eine $CH_2$-Gruppe durch Sauerstoff, S, NH, N-$CH_3$ oder N-Benzyl ersetzt sein kann;

oder
R(2) R(33)X-;

X Sauerstoff, S, NR(34), (D=O)A-, NR(34)C=MN$^{(*)}$R(35)-;

M Sauerstoff oder S;
A Sauerstoff oder NR(34);
D C oder SO;

R(33) ($C_1$-$C_8$)-Alkyl, ($C_3$-$C_8$)-Alkenyl, $(CH_2)_bC_dF_{2d+1}$, -$C_nH_{2n}$-R(36),

b Null oder 1;
d 1, 2, 3, 4, 5, 6 oder 7;
n Null, 1, 2, 3, oder 4;
R(36) ($C_3$-$C_8$)-Cycloalkyl, Phenyl, Biphenylyl oder Naphthyl,

wobei die Aromaten nicht substituiert oder substituiert sind mit 1 - 3 Substituenten ausgewählt aus der Gruppe bestehend aus F, Cl, $CF_3$, Methyl, Methoxy und NR(37)R(38);
R(37) und R(38)

H, ($C_1$-$C_4$)-Alkyl oder ($C_1$-$C_4$)-Perfuoralkyl;

R(34) H, ($C_1$-$C_4$)-Alkyl oder ($C_1$-$C_4$)-Perfuoralkyl;
R(35) definiert wie R(33);
oder
R(33) und R(34)

gemeinsam 4 oder 5 Methylengruppen, von denen eine $CH_2$-Gruppe durch Sauerstoff, S, NH, N-$CH_3$ oder N-Benzyl ersetzt sein kann;

wobei A und N$^{(*)}$ an den Phenylkern des Benzoylguanidin-Grundkörpers gebunden sind:

oder
R(2) -SR(40), -OR(40), -NHR(40), -NR(40)R(41), -CHR(40)R(42), -C[R(42)R(43)OH], -C≡CR(45), -CR(46)=CHR(45), -$[CR(47)R(48)]_u$-(CO)-$[CR49)R(50)]_v$-R(44);

R(40), R(41)

gleich oder verschieden -$(CH_2)_p$-$(CHOH)_q$-$(CH_2)_r$-$(CHOH)_t$-R(51) oder - $(CH_2)_p$-O-$(CH_2$-$CH_2O)_q$-R(51);
R(51) Wasserstoff oder Methyl;

u 1, 2, 3 oder 4;

v Null, 1, 2, 3 oder 4;

p, q, r

gleich oder verschieden Null, 1, 2, 3 oder 4;

t 1, 2, 3 oder 4;

R(42) und R(43)

gleich oder verschieden Wasserstoff oder ($C_1$-$C_6$)-Alkyl;

oder

R(42) und R(43)

zusammen mit dem sie tragenden Kohlenstoff-Atom ein $(C_3-C_8)$-Cycloalkyl bilden;

R(44) H, $(C_1-C_6)$-Alkyl, $(C_3-C_8)$-Cycloalkyl oder $-C_eH_{2e}$-R(45);

e Null, 1, 2, 3 oder 4;
R(45) gleich Phenyl,

das unsubstituiert oder substituiert ist mit 1 - 3 Substituenten aus der Gruppe bestehend aus F, Cl, $CF_3$, Methyl, Methoxy und NR(52)R(53) mit

R(52) und R(53) gleich H oder $(C_1-C_4)$-Alkyl, oder

R(45) $(C_1-C_9)$-Heteroaryl,

das unsubstituiert oder wie Phenyl substituiert ist;

oder

R(45) $(C_1-C_6)$-Alkyl,

das unsubstituiert oder mit 1 - 3 OH substituiert ist;

R(46), R(47), R(48), R(49) und R(50)

Wasserstoff oder Methyl;

oder
R(2) R(55)-NH-$SO_2$-;

R(55) R(56)R(57)N-(C=Y)-;

Y Sauerstoff, S oder N-R(58);
R(56) und R(57)

gleich oder verschieden H, $(C_1-C_8)$-Alkyl, $(C_3-C_6)$-Alkenyl oder $-C_fH_{2f}$-R(59);
f Null, 1, 2, 3 oder 4;
R(59) $(C_5-C_7)$-Cycloalkyl oder Phenyl,

welches unsubstituiert ist oder substituiert mit 1 - 3 Substituenten ausgewählt aus der Gruppe bestehend aus F, Cl, $CF_3$, Methoxy und $(C_1-C_4)$-Alkyl;

oder
R(56) und R(57)

gemeinsam 4 oder 5 Methylengruppen, von denen eine $CH_2$-Gruppe durch Sauerstoff, S, NH, N-$CH_3$ oder N-Benzyl ersetzt sein kann;
R(58) wie R(56) definiert oder Amidin;

R(3), R(4) und R(5)

unabhängig voneinander wie R(1) oder R(2) definiert;

sowie deren pharmazeutisch verträgliche Salze;

(HOE 93/F 220 - EP-OS 640 593, NZ 264 117)
q) Benzoylguanidine der Formel I

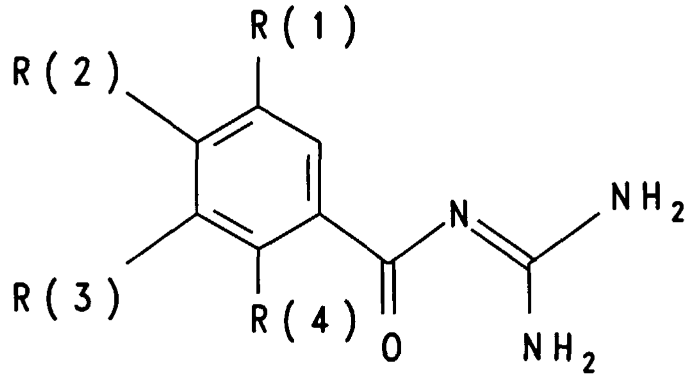 (I)

worin bedeuten:

R(1) Wasserstoff, F, Cl, Br, I, $-NO_2$, -C≡N, $-X_o-(CH_2)_p-(CF_2)_q-CF_3$, $R(5)-SO_m-$, R(6)-CO-, R(6)R(7)N-CO- oder $R(6)R(7)N-SO_2-$;

X Sauerstoff, -S- oder NR(14);
m Null, 1 oder 2;
o Null oder 1;
p Null, 1 oder 2;
q Null, 1, 2, 3, 4, 5 oder 6;
R(5) und R(6)

$(C_1-C_8)$-Alkyl, $(C_3-C_6)$-Alkenyl, $-C_nH_{2n}-R(8)$ oder $CF_3$;
n Null, 1, 2, 3 oder 4;
R(8) $(C_3-C_7)$-Cycloalkyl, Phenyl,

welches nicht substituiert ist oder substituiert mit 1 bis 3 Substituenten ausgewählt aus der Gruppe bestehend aus F, Cl, $CF_3$, Methyl, Methoxy und NR(9)R(10);
R(9) und R(10)

H oder $(C_1-C_4)$-Alkyl;

oder
R(6) Wasserstoff;
R(7) Wasserstoff oder $(C_1-C_4)$-Alkyl;
oder
R(6) und R(7)

gemeinsam 4 oder 5 Methylengruppen, von denen eine $CH_2$-Gruppe durch Sauerstoff, S, NH, $N-CH_3$ oder N-Benzyl ersetzt sein kann;

R(2)

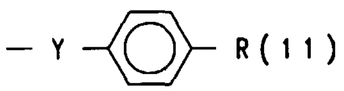 , 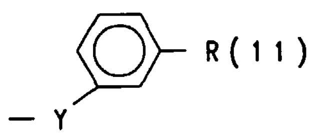 oder 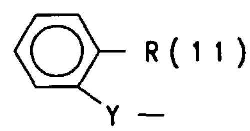

R(11) ($C_1$-$C_9$)-Heteroaryl,

das über C oder N verknüpft ist und das unsubstituiert oder substituiert ist mit 1 bis 3 Substituenten ausgewählt aus der Gruppe bestehend aus F, Cl, $CF_3$, $CH_3$, Methoxy, Hydroxy, Amino, Methylamino, Dimethylamino und Benzyl;

Y Sauerstoff, -S- oder NR(12);
R(12) H oder ($C_1$-$C_4$)-Alkyl;

R(3) wie R(1) definiert;
oder
R(3) ($C_1$-$C_6$)-Alkyl oder -X-R(13);

X Sauerstoff, -S- oder NR(14);

R(14) H oder ($C_1$-$C_3$)-Alkyl;
R(13) H, ($C_1$-$C_6$)-Alkyl, ($C_3$-$C_8$)-Cycloalkyl oder -$C_bH_{2b}$-R(15);
b Null, 1, 2, 3 oder 4;
oder
R(13) und R(14)

gemeinsam 4 oder 5 Methylengruppen, von denen eine $CH_2$-Gruppe durch Sauerstoff, S, NH, N-$CH_3$ oder N-Benzyl ersetzt sein kann;

R(15) Phenyl,

das unsubstituiert oder substituiert ist mit 1 - 3 Substituenten ausgewählt aus der Gruppe bestehend aus F, Cl, $CF_3$, Methyl, Methoxy und NR(9)R(10);
R(9) und R(10)

H oder ($C_1$-$C_4$)-Alkyl;

R(4) Wasserstoff, -OR(16), -NR(16)R(17) oder $C_rF_{2r+1}$;

R(16) und R(17)

unabhängig Wasserstoff oder ($C_1$-$C_3$)-Alkyl;

r 1, 2, 3 oder 4;

sowie deren pharmazeutisch verträgliche Salze;

(HOE 93/F 223 K - EP 639 573, NZ 264 130)
r) Benzokondensierte 5-Ringheterocyclen der Formel I

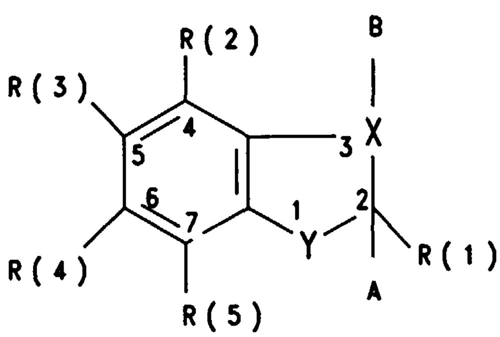

(I),

worin bedeuten:

X N oder CR(6);
Y Sauerstoff, S oder NR(7);
A, B gemeinsam eine Bindung
oder
A, B beide Wasserstoff, sofern gleichzeitig X gleich CR(6) und Y gleich NR(7) sind;
einer der Substituenten R(1) bis R(6) eine $-CO-N=C(NH_2)_2$-Gruppe;
die jeweils anderen Substituenten R(1) bis R(6)

Wasserstoff, F, Cl, Br, I oder $(C_1-C_6)$-Alkyl;

bis zu zwei der anderen Substituenten R(1) bis R(6)

CN, $NO_2$, $N_3$, $(C_1-C_4)$-Alkyloxy oder $CF_3$;

bis zu einem der anderen Substituenten

$R(8)-C_nH_{2n}-Z-$;
n Null bis 10;

wobei die Alkylenkette $-C_nH_{2n}-$ geradkettig oder verzweigt ist und wobei ein C-Atom durch ein Sauerstoff- oder S-Atom oder durch ein N-Atom ersetzt sein kann;

R(8) Wasserstoff, $(C_2-C_6)$-Alkenyl oder $(C_3-C_{10})$-Cycloalkyl,

das unsubstituiert oder durch 1 bis 4 Methylgruppen oder eine OH-Gruppe substituiert ist, oder eine Ethylengruppe -CH=CH- enthalten kann, und worin eine Methylengruppe durch ein Sauerstoff- oder S-Atom oder durch ein N-Atom ersetzt sein kann;

oder
R(8) Phenyl,

unsubstituiert oder substituiert durch 1 bis 3 Substituenten ausgewählt aus der Gruppe bestehend aus F, Cl, Br, I $CF_3$, $CH_3-S(O)_s-$ oder $R(9)-W_y-$;
s Null, 1 oder 2;
R(9) H, Methyl, Ethyl,
W Sauerstoff oder NR(10);

R(10) H oder Methyl;

y Null oder 1;

oder
R(8) $C_mF_{2m+1}$;

m 1 bis 3;

oder
R(8) 1- oder 2- Naphthyl, Pyridyl, Chinolyl oder Isochinolyl;

Z -CO-, $-CH_2-$ oder $-[CR(11)(OH)]_q-$;

q 1, 2 oder 3;
R(11) H oder Methyl;

oder
Z Sauerstoff oder -NR(12)-;

R(12) H oder Methyl;

oder
Z -S(O)$_s$-;

s Null, 1 oder 2;

oder
Z -$SO_2$-NR(13)-;

R(13) H oder ($C_1$-$C_4$)-Alkyl;

R(7) Wasserstoff, ($C_1$-$C_{10}$)-Alkyl, ($C_2$-$C_{10}$)-Alkenyl oder R(8)-$C_nH_{2n}$-;

sowie deren pharmazeutisch verträgliche Salze;

(HOE 93/F 236 - EP-OS 638 548, NZ 264 216)
s) Benzoylguanidine der Formel I

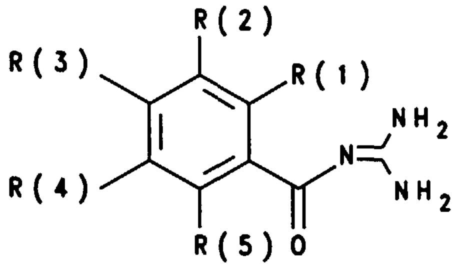

(I)

worin bedeuten:

R(1), R(3) oder R(4)

-NR(6) C=X NR(7)R(8);

X Sauerstoff oder S;
R(6) Wasserstoff, ($C_1$-$C_8$)-Alkyl, ($C_1$-$C_8$)-Perfluoralkyl, ($C_3$-$C_8$)-Alkenyl oder -$C_nH_{2n}$-R(9);

n Null, 1, 2, 3 oder 4;
R(9) ($C_3$-$C_8$)-Cycloalkyl, Phenyl, Biphenylyl oder Naphthyl,

wobei die Aromaten nicht substituiert oder substituiert sind mit 1 - 3 Substituenten ausgewählt aus der Gruppe bestehend aus F, Cl, $CF_3$, Methyl, Methoxy und NR(10)R(11);
R(10) und R(11)

H, ($C_1$-$C_4$)-Alkyl oder ($C_1$-$C_4$)-Perfluoralkyl;

R(7) Wasserstoff, ($C_1$-$C_8$)-Alkyl, ($C_1$-$C_8$)-Perfluoralkyl, ($C_3$-$C_8$)-Alkenyl oder -$C_oH_{2o}$-R(12);

o Null, 1, 2, 3 oder 4;
R(12) ($C_3$-$C_8$)-Cycloalkyl, Phenyl, Biphenylyl oder Naphthyl,

wobei die Aromaten nicht substituiert oder substituiert sind mit 1 - 3 Substituenten ausgewählt aus der Gruppe bestehend aus F, Cl, $CF_3$, Methyl, Methoxy und NR(13)R(14);
R(13) und R(14)

H, ($C_1$-$C_4$)-Alkyl oder ($C_1$-$C_4$)-Perfluoralkyl;

R(8) definiert wie R(7);
oder

R(7) und R(8)

gemeinsam 4 oder 5 Methylengruppen, von denen eine $CH_2$-Gruppe durch Sauerstoff, S, NH, N-$CH_3$ oder N-Benzyl ersetzt sein kann;

die jeweils verbleibenden Substituenten R(2), R(3), R(4), R(5) oder R(1), R(2), R(4), R(5) oder R(1), R(2), R(3), R(5)

unabhängig voneinander Wasserstoff, F, Cl, Br, I, -$O_{ta}$($C_1$-$C_8$)-Alkyl, -$O_{tb}$($C_3$-$C_8$)-Alkenyl, -$O_{tc}(CH_2)_bC_dF_{2d+1}$, -$O_{td}C_pH_{2p}$R(18),
oder bis zu 2 Gruppen CN, $NO_2$, NR(16)R(17),
b Null oder 1;
d 1, 2, 3, 4, 5, 6 oder 7;
ta Null oder 1;
tb Null oder 1;
tc Null oder 1;
td Null oder 1;
p Null, 1, 2, 3 oder 4;
R(18) ($C_3$-$C_8$)-Cycloalkyl, Phenyl, Biphenylyl oder Naphthyl,

wobei die Aromaten nicht substituiert oder substituiert sind mit 1 - 3 Substituenten ausgewählt aus der Gruppe bestehend aus F, Cl, $CF_3$, Methyl, Methoxy und NR(19)R(20);
R(19) und R(20)

Wasserstoff oder ($C_1$-$C_4$)-Alkyl oder ($C_1$-$C_4$)-Perfluoralkyl;

R(16) Wasserstoff, ($C_1$-$C_8$)-Alkyl, ($C_1$-$C_8$)-Perfluoralkyl, ($C_3$-$C_8$)-Alkenyl, - $C_qH_{2q}$-R(21),

q Null, 1, 2, 3 oder 4;
R(21) ($C_3$-$C_8$)-Cycloalkyl, Phenyl, Biphenylyl oder Naphthyl,

wobei die Aromaten nicht substituiert oder substituiert sind mit 1 - 3 Substituenten aus der Gruppe F, Cl, $CF_3$, Methyl, Methoxy oder NR(22)R(23),
R(22) und R(23) Wasserstoff, ($C_1$-$C_4$)-Alkyl oder ($C_1$-$C_4$)-Perfluoralkyl;

R(17) Wasserstoff, ($C_1$-$C_8$)-Alkyl, ($C_1$-$C_8$)-Perfluoralkyl, ($C_3$-$C_8$)-Alkenyl, - $C_rH_{2r}$-R(24);

r Null, 1, 2, 3 oder 4;
R(24) ($C_3$-$C_8$)-Cycloalkyl, Phenyl, Biphenylyl oder Naphthyl,

wobei die Aromaten nicht substituiert oder substituiert sind mit 1 - 3 Substituenten ausgewählt aus der Gruppe bestehend aus F, Cl, $CF_3$, Methyl, Methoxy und NR(25)R(26);
R(25) und R(26)

Wasserstoff, ($C_1$-$C_4$)-Alkyl oder ($C_1$-$C_4$)-Perfluoralkyl;

oder
R(16) und R(17)

gemeinsam 4 oder 5 Methylengruppen, von denen eine $CH_2$-Gruppe durch Sauerstoff, S, NH, N-$CH_3$ oder N-Benzyl ersetzt sein kann;

sowie deren pharmazeutisch verträgliche Salze;

(HOE 93/F 249 - EP-OS 640 587, NZ 264 282)
t) Diacylsubstituierte Guanidine der Formel I

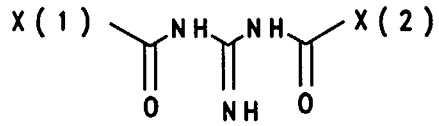

I

worin bedeuten:

X(1) und X(2)

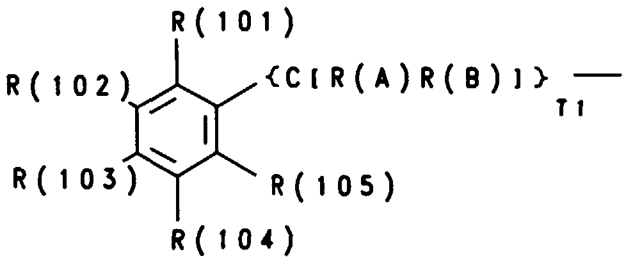

T1 Null, 1, 2, 3 oder 4;
R(A) und R(B)

unabhängig Wasserstoff, F, Cl, Br, I, CN, OR(106), ($C_1$-$C_8$)-Alkyl, ($C_3$-$C_8$)-Cycloalkyl, $O_{zk}(CH_2)_{zl}C_{zm}F_{2zm+1}$, NR(107)R(108), Phenyl oder Benzyl,

wobei die Aromaten nicht substituiert oder substituiert sind mit 1 - 3 Substituenten ausgewählt aus der Gruppe bestehend aus F, Cl, $CF_3$, Methyl, Methoxy und NR(109)R(110);
R(109) und R(110)

Wasserstoff, ($C_1$-$C_4$)-Alkyl oder ($C_1$-$C_4$)-Perfluoralkyl;

zl Null, 1, 2, 3 oder 4;
zk Null oder 1;
zm 1, 2, 3, 4, 5, 6, 7 oder 8;
R(106)

Wasserstoff, ($C_1$-$C_8$)-Alkyl, ($C_1$-$C_8$)-Perfluoralkyl, ($C_3$-$C_8$)-Alkenyl, ($C_3$-$C_8$)-Cycloalkyl, Phenyl oder Benzyl,

wobei die Aromaten nicht substituiert oder substituiert sind mit 1 - 3 Substituenten ausgewählt aus der Gruppe bestehend aus F, Cl, $CF_3$, Methyl, Methoxy und NR(111)R(112);
R(111) und R(112)

Wasserstoff, ($C_1$-$C_4$)-Alkyl oder ($C_1$-$C_4$)-Perfluoralkyl;

R(107) und R(108)

unabhängig voneinander wie R(106) definiert,

oder
R(107) und R(108)

gemeinsam 4 oder 5 Methylengruppen, von denen eine $CH_2$-Gruppe durch Sauerstoff, S, NH, N-$CH_3$

oder N-Benzyl ersetzt sein kann;

oder
X(1) und X(2)

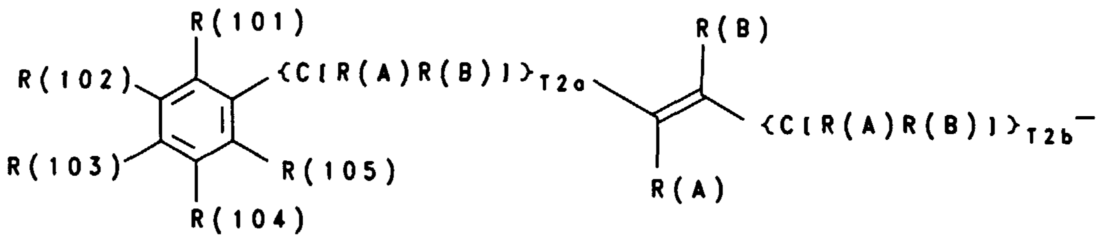

T2a und T2b

unabhängig voneinander Null, 1 oder 2; wobei die Doppelbindung (E)- oder (Z)-konfiguriert sein kann;

oder
X(1) und X(2)

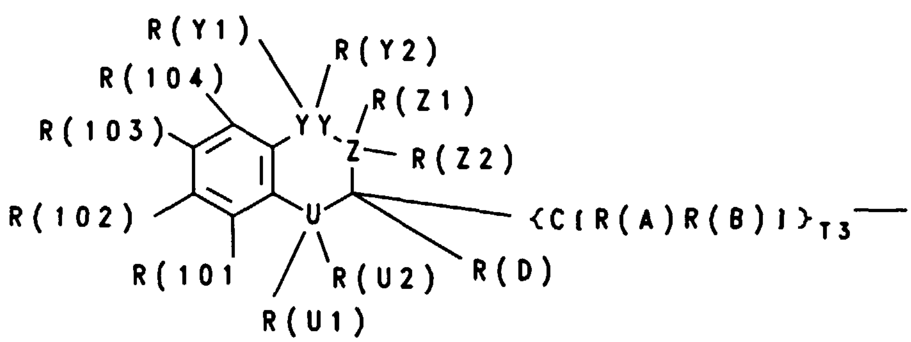

T3 Null, 1 oder 2;
U, YY und Z

unabhängig voneinander C oder N,
wobei U, YY, Z folgende Anzahl von Substituenten tragen können:

| U, YY oder Z | im Ring an eine Doppelbindung gebunden | Anzahl der zugelassenen Substituenten |
|---|---|---|
| C | ja | 1 |
| C | nein | 2 |
| N | ja | 0 |
| N | nein | 1 |

R(D) Wasserstoff, $(C_1-C_8)$-Alkyl oder $(C_1-C_8)$-Perfluoralkyl,
R(U1), R(U2), R(Y1), R(Y2), R(Z1), R(Z2)

unabhängig voneinander Wasserstoff, F, Cl, Br, I, CN, OR(114), ($C_1$-$C_8$)-Alkyl, ($C_3$-$C_8$)-Cycloalkyl, $O_{zka}(CH_2)_{zla}C_{zma}F_{2zma+1}$, NR(115)R(116), Phenyl oder Benzyl,

wobei die Aromaten nicht substituiert oder substituiert sind mit 1 - 3 Substituenten ausgewählt aus der Gruppe bestehend aus F, Cl, $CF_3$, Methyl, Methoxy, NR(117)R(118),
R(117) und R(118)

Wasserstoff, ($C_1$-$C_4$)-Alkyl oder ($C_1$-$C_4$)-Perfluoralkyl,

zka Null oder 1;
zla Null, 1, 2, 3 oder 4;
zma 1, 2, 3, 4, 5, 6, 7 oder 8;
R(114)

Wasserstoff, ($C_1$-$C_8$)-Alkyl, ($C_1$-$C_8$)-Perfluoralkyl, ($C_3$-$C_8$)-Alkenyl, ($C_3$-$C_8$)-Cycloalkyl, Phenyl oder Benzyl,

wobei die Aromaten nicht substituiert oder substituiert sind mit 1 - 3 Substituenten ausgewählt aus der Gruppe bestehend aus F, Cl, $CF_3$, Methyl, Methoxy und NR(119)R(120);
R(119) und R(120)

Wasserstoff, ($C_1$-$C_4$)-Alkyl oder ($C_1$-$C_4$)-Perfluoralkyl;

R(115) und R(116) unabhängig voneinander wie R(114) definiert;
oder
R(115) und R(116)

gemeinsam 4 oder 5 Methylengruppen, von denen eine $CH_2$-Gruppe durch Sauerstoff, S, NH, N-$CH_3$ oder N-Benzyl ersetzt sein kann;

wobei jedoch die Konstitution U gleich Stickstoff (N), YY gleich Stickstoff (N) und Z gleich Kohlenstoff (C) ausgenommen ist,

R(101), R(102), R(103), R(104) und R(105)

unabhängig voneinander Wasserstoff, F, Cl, Br, I, -C≡N, $X_{zoa}$-$(CH_2)_{zpa}$-$(C_{zqa}F_{2zqa+1})$, R(110a)-$SO_{zbm}$, R(110b)R(110c)N-CO, R(111a)-CO- oder R(112a)R(113a)N-$SO_2$-,

wobei die Perfluoralkylgruppe geradkettig oder verzweigt ist,

X Sauerstoff, S oder NR(114a);

R(114a)

H oder ($C_1$-$C_3$)-Alkyl;

zoa Null oder 1;
zbm Null, 1 oder 2;
zpa Null, 1, 2, 3 oder 4;
zqa 1, 2, 3, 4, 5, 6, 7 oder 8;
R(110a), R(110b), R(111a) und R(112a)

unabhängig ($C_1$-$C_8$)-Alkyl, ($C_3$-$C_8$)-Alkenyl, -$C_{zn}H_{2zn}$-R(115a) oder ($C_1$-$C_8$)-Perfluoralkyl;
zn Null, 1, 2, 3 oder 4;
R(115a)

($C_3$-$C_8$)-Cycloalkyl, Phenyl, Biphenylyl oder Naphthyl,

wobei die Aromaten nicht substituiert sind oder substituiert mit 1 - 3 Substituenten ausgewählt aus der Gruppe bestehend aus F, Cl, $CF_3$, Methyl, Methoxy und NR(116a)R(117a);
R(116a) und R(117a)

Wasserstoff, ($C_1$-$C_4$)-Perfluoralkyl oder ($C_1$-$C_4$)-Alkyl;

oder
R(110b), R(111a) und R(112a)

Wasserstoff;

R(110c) und R(113a)

unabhängig Wasserstoff, ($C_1$-$C_4$)-Perfluoralkyl oder ($C_1$-$C_4$)-Alkyl;

oder
R(110b) und R(110c) sowie R(112a) und R(113a)

gemeinsam 4 oder 5 Methylengruppen, von denen eine $CH_2$-Gruppe durch Sauerstoff, Schwefel, NH, N-$CH_3$ oder N-Benzyl ersetzt sein kann;

oder
R(101), R(102), R(103), R(104), R(105)

unabhängig voneinander ($C_1$-$C_8$)-Alkyl, -$C_{zal}H_{2zal}$R(118a) oder ($C_3$-$C_8$)-Alkenyl,
zal Null, 1, 2, 3 oder 4;
R(118a)

($C_3$-$C_8$)-Cycloalkyl, Phenyl, Biphenylyl oder Naphthyl,

wobei die Aromaten nicht substituiert oder substituiert sind mit 1 - 3 Substituenten aus der Gruppe bestehend aus F, Cl, $CF_3$, Methyl, Methoxy oder NR(119a)R(119b);
R(119a) und R(119b)

Wasserstoff, ($C_1$-$C_4$)-Alkyl oder ($C_1$-$C_4$)-Perfluoralkyl;

oder
R(101), R(102), R(103), R(104), R(105)

unabhängig voneinander ($C_1$-$C_9$)-Heteroaryl,

das über C oder N verknüpft ist und das unsubstituiert oder substituiert ist mit 1 - 3 Substituenten aus der Gruppe bestehend aus F, Cl, $CF_3$, $CH_3$, Methoxy, Hydroxy, Amino, Methylamino und Dimethylamino;

oder
R(101), R(102), R(103), R(104), R(105)

unabhängig voneinander -C≡C-R(193);
R(193)

Phenyl, das nicht substituiert oder substituiert ist mit 1 - 3 Substituenten aus der Gruppe bestehend aus F, Cl, $CF_3$, Methyl, Methoxy oder NR(194)R(195);
R(194) und R(195)

Wasserstoff oder $CH_3$;

oder

R(101), R(102), R(103), R(104), R(105)

unabhängig voneinander -Y-para-$C_6H_4$-$(CO)_{zh}$-$(CHOH)_{zi}$-$(CH_2)_{zj}$-$(CHOH)_{zk}$-R(123), -Y-meta-$C_6H_4$-$(CO)_{zad}$-$(CHOH)_{zae}$-$(CH_2)_{zaf}$-$(CHOH)_{zag}$-R(124) oder -Y-ortho-$C_6H_4$-$(CO)_{zah}$-$(CHOH)_{zao}$-$(CH_2)_{zap}$-$(CHOH)_{zak}$-R(125);
Y Sauerstoff, -S- oder -NR(122d)-;
zh, zad, zah

unabhängig Null oder 1;

zi, zj, zk, zae, zaf, zag, zao, zap und zak

unabhängig Null, 1, 2, 3 oder 4;

wobei jedoch jeweils

zh, zi und zk nicht gleichzeitig Null,
zad, zae und zag nicht gleichzeitig Null sowie
zah, zao und zak nicht gleichzeitig Null sind,

R(123), R(124) R(125) und R(122d)

unabhängig Wasserstoff oder $(C_1$-$C_3)$-Alkyl;

oder
R(101), R(102), R(103), R(104) und R(105)

unabhängig voneinander SR(129), -OR(130), -NR(131)R(132) oder -CR(133)R(134)R(135);
R(129), R(130), R(131) und R(133)

unabhängig -$C_{zab}H_{2zab}$-$(C_1$-$C_9)$-Heteroaryl,

das unsubstituiert oder substituiert ist mit 1 - 3 Substituenten aus der Gruppe bestehend aus F, Cl, $CF_3$, $CH_3$, Methoxy, Hydroxy, Amino, Methylamino und Dimethylamino;

zab Null, 1 oder 2;

R(132), R(134) und R(135)

unabhängig voneinander wie R(129) definiert oder Wasserstoff, $(C_1$-$C_4)$-Alkyl oder $(C_1$-$C_4)$-Perfluoralkyl;

oder
R(101), R(102), R(103), R(104) und R(105)

unabhängig voneinander -W-para-$(C_6H_4)$-R(196), -W-meta-$(C_6H_4)$-R(197) oder -W-ortho-$(C_6H_4)$-R(198);
R(196), R(197) und R(198)

unabhängig $(C_1$-$C_9)$-Heteroaryl,

das über C oder N verknüpft ist und das unsubstituiert oder substituiert ist mit 1 bis 3 Substituenten aus der Gruppe bestehend aus F, Cl, $CF_3$, $CH_3$, Methoxy, Hydroxy, Amino, Methylamino, Dimethylamino und Benzyl;

W Sauerstoff, S oder NR(136)-;

R(136)

Wasserstoff oder $(C_1-C_4)$-Alkyl;

oder
R(101), R(102), R(103), R(104) und R(105)

unabhängig voneinander R(146)X(1a)-;
X(1a)

Sauerstoff, S, NR(147), (D=O)A-, NR(148)C=MN$^{(*)}$R(149)-;
M Sauerstoff oder Schwefel;
A Sauerstoff oder NR(150);
D C oder SO;

R(146)

$(C_1-C_8)$-Alkyl, $(C_3-C_8)$-Alkenyl, $(CH_2)_{zbz}C_{zdz}F_{2zdz+1}$ oder - $C_{zxa}H_{2zxa}$-R(151);
zbz Null oder 1;
zdz 1, 2, 3, 4, 5, 6 oder 7;
zxa Null, 1, 2, 3 oder 4;
R(151)

$(C_3-C_8)$-Cycloalkyl, Phenyl, Biphenylyl oder Naphthyl,

wobei die Aromaten nicht substituiert oder substituiert sind mit 1 - 3 Substituenten aus der Gruppe bestehend aus F, Cl, $CF_3$, Methyl, Methoxy und NR(152)R(153);
R(152) und R(153)

Wasserstoff, $(C_1-C_4)$-Alkyl oder $(C_1-C_4)$-Perfluoralkyl;

R(147), R(148) und R(150)

unabhängig Wasserstoff, $(C_1-C_4)$-Alkyl, $(C_1-C_4)$-Perfluoralkyl;

R(149) definiert wie R(146),

oder
R(146) und R(147) beziehungsweise R(146) und R(148)

gemeinsam 4 oder 5 Methylengruppen, von denen eine $CH_2$-Gruppe durch Sauerstoff, Schwefel, NH, N-$CH_3$ oder N-Benzyl ersetzt sein kann;

wobei A und N$^{(*)}$ an den Phenylkern des Alkanoyl-Grundkörpers gebunden sind;

oder
R(101), R(102), R(103), R(104) und R(105)

unabhängig voneinander -SR(164), -OR(165), -NHR(166), -NR(167)R(168), - CHR(169)R(170), -CR(154)R(155)OH, -C≡CR(156), -CR(158)=CR(157) oder -$[CR(159)R(160)]_{zu}$-(C=O)-$[CR(161)R(162)]_{zv}$-R(163);
R(164), R(165), R(166), R(167), R(169)

gleich oder verschieden -$(CH_2)_{zy}$-$(CHOH)_{zz}$-$(CH_2)_{zaa}$-$(CHOH)_{zt}$-R(171) oder - $(CH_2)_{zab}$-O-$(CH_2$-$CH_2O)_{zac}$-R(172);
R(171) und R(172)

Wasserstoff oder Methyl;

zu 1, 2, 3 oder 4;

zv Null, 1, 2, 3 oder 4;

zy, zz, zaa, zab, zac

gleich oder verschieden Null, 1, 2, 3 oder 4;

zt 1, 2, 3 oder 4;

R(168), R(170), R(154), R(155)

gleich oder verschieden Wasserstoff oder $(C_1-C_6)$-Alkyl,

oder
R(169) und R(170) beziehungsweise R(154) und R(155)

zusammen mit dem sie tragenden Kohlenstoff-Atom ein $(C_3-C_8)$-Cycloalkyl;

R(163)

Wasserstoff, $(C_1-C_6)$-Alkyl, $(C_3-C_8)$-Cycloalkyl oder $-C_{zeb}H_{2zeb}$-R(173); zeb Null, 1, 2, 3 oder 4;
R(156), R(157) und R(173)

unabhängig Phenyl, das unsubstituiert oder substituiert ist mit 1 - 3 Substituenten aus der Gruppe bestehend aus F, Cl, $CF_3$, Methyl, Methoxy und NR(174)R(175);
R(174) und R(175)

Wasserstoff oder $(C_1-C_4)$-Alkyl;

oder
R(156), R(157) und R(173)

unabhängig $(C_1-C_9)$-Heteroaryl,

das unsubstituiert oder wie Phenyl substituiert ist;

R(158), R(159), R(160), R(161) und R(162)

Wasserstoff oder Methyl,

oder
R(101), R(102), R(103), R(104), R(105)

unabhängig voneinander R(176)-NH-$SO_2$-;
R(176)

R(177)R(178)N-(C=Y')-;
Y' Sauerstoff, S oder N-R(179);
R(177) und R(178)

gleich oder verschieden Wasserstoff, $(C_1-C_8)$-Alkyl, $(C_3-C_6)$-Alkenyl oder $-C_{zfa}H_{2zfa}$-R(180);
zfa Null, 1, 2, 3 oder 4;
R(180)

$(C_5-C_7)$-Cycloalkyl oder Phenyl,

welches unsubstituiert mit 1 - 3 Substituenten ausgewählt aus der Gruppe bestehend aus F, Cl, $CF_3$, Methoxy oder $(C_1-C_4)$-Alkyl;

oder
R(177) und R(178)

gemeinsam 4 oder 5 Methylengruppen, von denen eine $CH_2$-Gruppe durch Sauerstoff, Schwefel, NH, N-$CH_3$ oder N-Benzyl ersetzt sein kann;

R(179)

wie R(177) definiert oder gleich Amidin,

oder
R(101), R(102), R(103), R(104), R(105)

unabhängig voneinander NR(184a)R(185), OR(184b), SR(184c) oder -$C_{znx}H_{2znx}$-R(184d);
znx Null, 1, 2, 3 oder 4;
R(184d)

($C_3$-$C_7$)-Cycloalkyl oder Phenyl,

welches nicht substituiert ist oder substituiert mit 1 - 3 Substituenten aus der Gruppe bestehend aus F, Cl, $CF_3$, Methyl, Methoxy und NR(116k)R(117k);
R(116k) und R(117k)

Wasserstoff oder $C_1$-$C_4$-Alkyl;

R(184a), R(184b), R(184c), R(185)

unabhängig voneinander Wasserstoff, ($C_1$-$C_8$)-Alkyl, ($C_1$-$C_8$)-Perfluoralkyl oder $(CH_2)_{zao}$-R(184g);
zao Null, 1, 2, 3 oder 4;
184g

($C_3$-$C_7$)-Cycloalkyl oder Phenyl,

welches nicht substituiert ist oder substituiert mit 1 - 3 Substituenten aus der Gruppe bestehend aus F, Cl, $CF_3$, Methyl, Methoxy und NR(184u)R(184v);
R(184u) und R(184v)

Wasserstoff oder $C_1$-$C_4$-Alkyl;

oder
R(184a) und R(185)

gemeinsam 4 oder 5 Methylengruppen, von denen eine $CH_2$-Gruppe durch Sauerstoff, Schwefel, NH, N-$CH_3$ oder N-Benzyl ersetzt sein kann;

sowie deren pharmazeutisch verträgliche Salze;

(HOE 93/F 254 - EP-OS 640 588, NZ 264 307)
u) Benzoylguanidine der Formel I

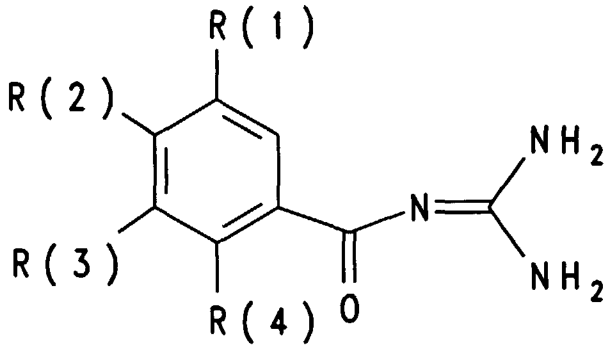

worin bedeuten:

R(1) H, F, Cl, Br, I, CN, $NO_2$, $(C_1$-$C_8)$-Alkyl, $(C_3$-$C_8)$-Cycloalkyl oder $X_a$-$(CH_2)_b$-$(CF_2)_c$-$CF_3$;

X Sauerstoff, S oder NR(5);
a Null oder 1;
b Null, 1 oder 2;
c Null, 1, 2 oder 3;

R(5) H, $(C_1$-$C_4)$-Alkyl oder $-C_dH_{2d}R(6)$;

d Null, 1, 2, 3 oder 4;
R(6) $(C_3$-$C_8)$-Cycloalkyl, Phenyl, Biphenylyl oder Naphthyl,

wobei die Aromaten nicht substituiert oder substituiert sind mit 1 bis 3 Substituenten ausgewählt aus der Gruppe bestehend aus F, Cl, $CF_3$, Methyl, Methoxy und NR(7)R(8);
R(7) und R(8)

unabhängig H oder $(C_1$-$C_4)$-Alkyl;

oder
R(1) -SR(10), -OR(10) oder -CR(10)R(11)R(12);

R(10) $-C_fH_{2f}$-$(C_3$-$C_8)$-Cycloalkyl, -$(C_1$-$C_9)$-Heteroaryl oder Phenyl,

wobei die aromatischen Systeme unsubstituiert oder substituiert sind mit 1 bis 3 Substituenten ausgewählt aus der Gruppe bestehend aus F, Cl, $CF_3$, $CH_3$, Methoxy, Hydroxy, Amino, Methylamino und Dimethylamino;

f Null, 1 oder 2;
R(11) und R(12)

unabhängig voneinander wie R(10) definiert oder Wasserstoff oder $(C_1$-$C_4)$-Alkyl;

oder
R(1) Phenyl, Naphthyl, Biphenylyl oder $(C_1$-$C_9)$-Heteroaryl,

letzteres über C oder N verknüpft,
und die unsubstituiert oder substituiert sind mit 1 bis 3 Substituenten ausgewählt aus der Gruppe bestehend aus F, Cl, $CF_3$, $CH_3$, Methoxy, Hydroxy, Amino, Methylamino und Dimethylamino;

oder
R(1) -SR(13), -OR(13), -NHR(13), -NR(13)R(14), -CHR(13)R(15), -C[R(15)R(16)]OH, $-C{\equiv}CR(18)$, -

C[R(19)]=CR(18), -[CR(20)R(21)]$_k$-(CO)-[CR(22)R(23)R(24)]$_l$

R(13) und R(14)

gleich oder verschieden $-(CH_2)_g-(CHOH)_h-(CH_2)_i-(CHOH)_j-R(17)$,
R(17) Wasserstoff oder Methyl;
$-(CH_2)_g-O-(CH_2-CH_2O)_h-R(24)$,
g, h, i

gleich oder verschieden Null, 1, 2, 3 oder 4;

j 1, 2, 3 oder 4;

R(15) und R(16)

gleich oder verschieden Wasserstoff, $(C_1-C_6)$-Alkyl oder zusammen mit dem sie tragenden Kohlenstoff-Atom ein $(C_3-C_8)$-Cycloalkyl;

R(18) Phenyl,

das unsubstituiert oder substituiert ist mit 1 bis 3 Substituenten ausgewählt aus der Gruppe bestehend aus F, Cl, $CF_3$, Methyl, Methoxy und NR(25)R(26);
R(25) und R(26)

H oder $(C_1-C_4)$-Alkyl;

oder
R(18) $(C_1-C_9)$-Heteroaryl,

das unsubstituiert oder wie Phenyl substituiert ist;

oder
R(18) $(C_1-C_6)$-Alkyl,

das unsubstituiert oder mit 1 bis 3 OH substituiert ist;

oder
R(18) $(C_3-C_8)$-Cycloalkyl;
R(19), R(20), R(21), R(22) und R(23)

Wasserstoff oder Methyl;

k Null, 1, 2, 3 oder 4;
l Null, 1, 2, 3 oder 4;

R(24) H, $(C_1-C_6)$-Alkyl, $(C_3-C_8)$-Cycloalkyl oder $-C_mH_{2m}-R(18)$;
m 1, 2, 3 oder 4;

R(2) und R(3)

unabhängig voneinander wie R(1) definiert;

R(4) $(C_1-C_3)$-Alkyl, F, Cl, Br, I, CN oder $-(CH_2)_n-(CF_2)_o-CF_3$;

n Null oder 1;
o Null, 1 oder 2;

sowie deren pharmazeutisch verträgliche Salze;

(HOE 93/F 436 - EP-OS 659 748), NZ 270 264)
v) Acylguanidine der Formel I

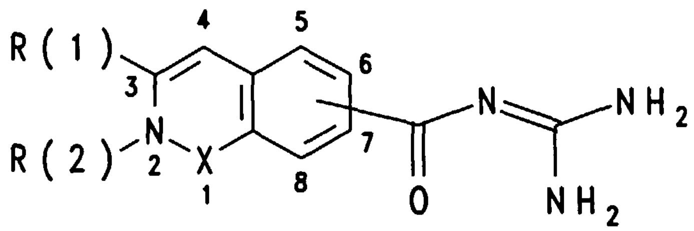

(I)

worin bedeuten:

X Carbonyl, Sulfonyl,
R(1) H, ($C_1$-$C_8$)-Alkyl,

unsubstituiert oder durch Hydroxy substituiert,

($C_3$-$C_8$)-Cycloalkyl, Phenyl,

das unsubstituiert oder substituiert ist mit 1 - 3 Substituenten aus der Gruppe F, Cl, $CF_3$, $CH_3$, Methoxy, Hydroxy, Amino, Methylamino oder Dimethylamino,

R(2) H, ($C_1$-$C_4$)-Alkyl,
sowie deren pharmazeutisch verträgliche Salze;

(HOE 94/F 014 K - EP-OS 666 252, NZ 270 370)
w) Perfluoralkylgruppen tragende phenylsubstituierte Alkylcarbonsäure-guanidide der Formel I

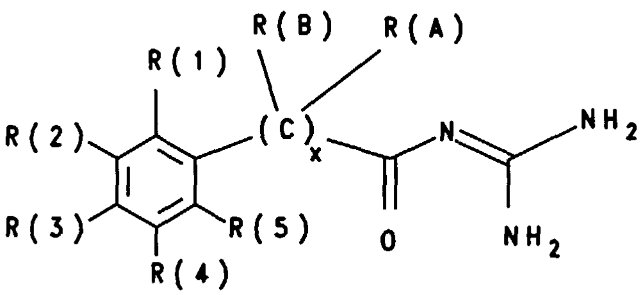

(I)

worin bedeuten:

R(A) Wasserstoff, F, Cl, Br, I, CN, OR(6), ($C_1$-$C_8$)-Alkyl, ($C_3$-$C_8$)-Cycloalkyl, $O_r(CH_2)_aC_bF_{2b+1}$ oder NR(7)R(8);

r Null oder 1;
a Null, 1, 2, 3 oder 4;
b 1, 2, 3, 4, 5, 6, 7 oder 8;
R(6) Wasserstoff, ($C_1$-$C_8$)-Alkyl, ($C_3$-$C_8$)-Alkenyl, ($C_3$-$C_8$)-Cycloalkyl, Phenyl oder Benzyl,

wobei die Aromaten nicht substituiert oder substituiert sind mit 1 - 3 Substituenten ausgwählt aus der Gruppe bestehend aus F, Cl, $CF_3$, Methyl, Methoxy und NR(9)R(10);
R(9) und R(10)

H, ($C_1$-$C_4$)-Alkyl oder ($C_1$-$C_4$)-Perfluoralkyl;

R(7) und R(8)

unabhängig voneinander wie R(6) definiert;

R(B) unabhängig wie R(A) definiert;
X 1, 2 oder 3;
R(1) Wasserstoff, $(C_1\text{-}C_8)$-Alkyl, $(C_3\text{-}C_8)$-Cycloalkyl, $-O_t(CH_2)_dC_eF_{2e+1}$, F, Cl, Br, I oder CN;

t Null oder 1;
d Null, 1, 2, 3 oder 4;
e 1, 2, 3, 4, 5, 6, 7 oder 8;

R(2), R(3), R(4) und R(5)

unabhängig voneinander wie R(1) definiert;

jedoch unter der Bedingung,
daß mindestens einer der Substituenten R(1), R(2), R(3), R(4), R(5), R(A) und R(B) eine $-O_t(CH_2)_dC_eF_{2e+1}$ oder eine $O_r(CH_2)_aC_bF_{2b+1}$-Gruppe ist,
sowie deren pharmazeutisch verträgliche Salze;

(HOE 94/F 094 - EP-OS 676 395, NZ 270 894)
x) Heteroaroylguanidine der Formel I

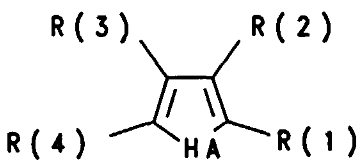

I

worin bedeuten:

HA $SO_m$, O oder NR(5);

m Null, 1 oder 2;
R(5) Wasserstoff, $(C_1\text{-}C_8)$-Alkyl oder $-C_{am}H_{2am}R(81)$;

am Null, 1 oder 2;
R(81) $(C_3\text{-}C_8)$-Cycloalkyl oder Phenyl,

welches nicht substituiert oder substituiert ist mit 1 - 3 Substituenten ausgewählt aus der Gruppe bestehend aus F, Cl, $CF_3$, Methyl, Methoxy und NR(82)R(83);
R(82) und R(83)

H oder $CH_3$;

oder
R(81) $(C_1\text{-}C_9)$-Heteroaryl,

das über C oder N verknüpft ist und das unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;

einer der beiden Substituenten R(1) und R(2)

$-CO-N=C(NH_2)_2$;

und der jeweils andere

Wasserstoff, F, Cl, Br, I, $(C_1-C_3)$-Alkyl, -OR(6), $C_rF_{2r+1}$, $-CO-N=C(NH_2)_2$ oder -NR(6)R(7);
R(6) und R(7)

unabhängig Wasserstoff oder $(C_1-C_3)$-Alkyl;

r 1, 2, 3 oder 4;
R(3) und R(4)

unabhängig voneinander Wasserstoff, F, Cl, Br, I, -C≡N, $X-(CH_2)_p-(C_q-F_{2q+1})$, $R(8)-SO_{bm}$, R(9)R(10)N-CO, R(11)-CO- oder $R(12)R(13)N-SO_2$-,

wobei die Perfluoralkylgruppe geradkettig oder verzweigt ist,

X Sauerstoff, S oder NR(14);

R(14) H oder $(C_1-C_3)$-Alkyl;

bm Null, 1 oder 2;
p Null, 1 oder 2;
q Null, 1, 2, 3, 4, 5 oder 6;
R(8), R(9), R(11) und R(12)

unabhängig $(C_1-C_8)$-Alkyl, $(C_3-C_6)$-Alkenyl, $-C_nH_{2n}$-R(15), $CF_3$;
n Null, 1, 2, 3 oder 4;
R(15) $(C_3-C_7)$-Cycloalkyl oder Phenyl;

welches nicht substituiert ist oder substituiert mit 1 - 3 Substituenten ausgewählt aus der Gruppe bestehend aus F, Cl, $CF_3$, Methyl, Methoxy oder NR(16)R(17);
R(16) und R(17)

H oder $C_1-C_4$-Alkyl;

oder
R(9), R(11) und R(12)

H;

R(10) und R(13)

unabhängig H oder $(C_1-C_4)$-Alkyl;

oder
R(9) und R(10) sowie R(12) und R(13)

gemeinsam 4 oder 5 Methylengruppen, von denen eine $CH_2$-Gruppe durch Sauerstoff, S, NH, N-$CH_3$ oder N-Benzyl ersetzt sein kann,

oder
R(3) und R(4)

unabhängig voneinander $(C_1-C_8)$-Alkyl oder $-C_{al}H_{2al}$R(18);
al Null, 1 oder 2;
R(18) $(C_3-C_8)$-Cycloalkyl oder Phenyl;

welches nicht substituiert oder substituiert ist mit 1 - 3 Substituenten ausgewählt aus der Gruppe bestehend aus F, Cl, $CF_3$, Methyl, Methoxy und NR(19)R(20);
R(19) und R(20)

H oder $CH_3$;

oder
R(3) und R(4)

unabhängig voneinander ($C_1$-$C_9$)-Heteroaryl,

das über C oder N verknüpft ist und das unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;

oder
R(3) und R(4)

unabhängig voneinander

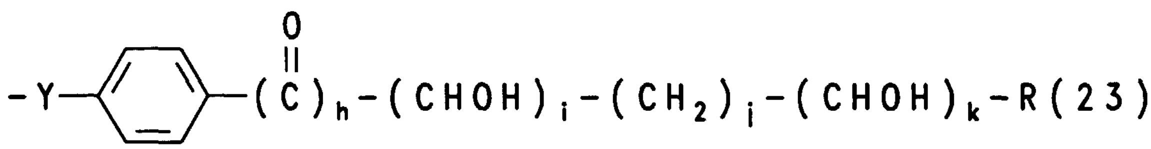

oder 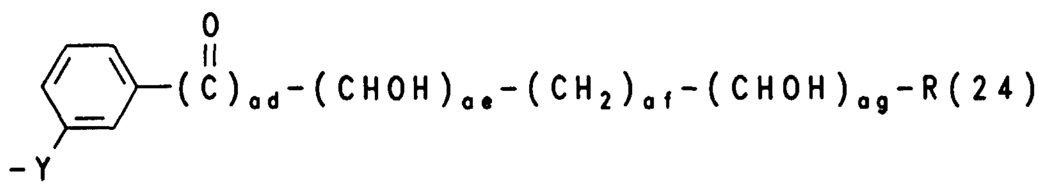

oder 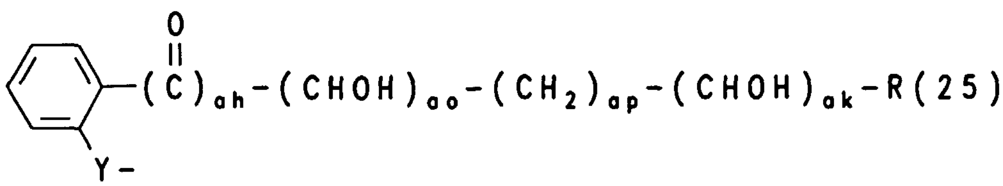

Y Sauerstoff, -S- oder -NR(22)-;

h, ad, ah unabhängig Null oder 1;

i, j, k, ae, af, ag, ao, ap und ak unabhängig Null, 1, 2, 3, 4,
wobei jedoch jeweils

h, i und k nicht gleichzeitig Null,
ad, ae und ag nicht gleichzeitig Null sowie
ah, ao und ak nicht gleichzeitig Null sind,

R(23), R(24) R(25) und R(22)

unabhängig Wasserstoff oder ($C_1$-$C_3$)-Alkyl;

oder
R(3) und R(4)

unabhängig voneinander Wasserstoff, F, Cl, Br, I, CN, $(C_1-C_8)$-Alkyl, $(C_1-C_8)$-Perfluoralkyl, $(C_3-C_8)$-Alkenyl oder $-C_gH_{2g}R(26)$;
g Null, 1, 2, 3 oder 4;
R(26) $(C_3-C_8)$-Cycloalkyl, Phenyl, Biphenylyl oder Naphthyl,

wobei die Aromaten nicht substituiert oder substituiert sind mit 1 - 3 Substituenten ausgewählt aus der Gruppe bestehend aus F, Cl, $CF_3$, Methyl, Methoxy und NR(27)R(28);
R(27) und R(28)

H, $(C_1-C_4)$-Alkyl oder $(C_1-C_4)$-Perfluoralkyl;

oder
R(3) und R(4)

unabhängig voneinander SR(29) -OR(30), -NR(31)R(32) oder -CR(33)R(34)R(35);
R(29), R(30), R(31) und R(33)

unabhängig $-C_aH_{2a}-(C_1-C_9)$-Heteroaryl,

das unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;

a Null, 1 oder 2;

R(32), R(34) und R(35)

unabhängig voneinander wie R(29) definiert oder Wasserstoff, $(C_1-C_4)$-Alkyl oder $(C_1-C_4)$-Perfluoralkyl;

oder
R(3) und R(4)

unabhängig voneinander

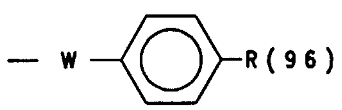 , 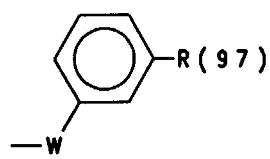 oder 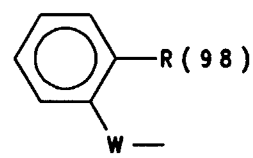

R(96), R(97) und R(98)

unabhängig $(C_1-C_9)$-Heteroaryl,

das über C oder N verknüpft ist und das unsubstituiert oder substituiert ist mit 1 bis 3 Substituenten ausgewählt aus der Gruppe bestehend aus F, Cl, $CF_3$, $CH_3$, Methoxy, Hydroxy, Amino, Methylamino, Dimethylamino oder Benzyl;

W Sauerstoff, S oder NR(36)-;

R(36) H oder $(C_1-C_4)$-Alkyl;

oder
R(3) und R(4)

unabhängig voneinander $R(37)-SO_{cm}$ oder $R(38)R(39)N-SO_2-$;

cm 1 oder 2;

R(37) $(C_1-C_8)$-Alkyl, $(C_1-C_8)$-Perfluoralkyl, $(C_3-C_8)$-Alkenyl oder - $C_sH_{2s}R(40)$;

s Null, 1, 2, 3 oder 4;

R(40) $(C_3-C_8)$-Cycloalkyl, Phenyl, Biphenylyl oder Naphthyl,

wobei die Aromaten nicht substituiert oder substituiert sind mit 1 - 3 Substituenten ausgewählt aus der Gruppe bestehend aus F, Cl, $CF_3$, Methyl, Methoxy und NR(41)R(42);

R(41) und R(42)

H, $(C_1-C_4)$-Alkyl oder $(C_1-C_4)$-Perfluoralkyl;

R(38) H, $(C_1-C_8)$-Alkyl, $(C_1-C_8)$-Perfluoralkyl, $(C_3-C_8)$-Alkenyl oder $-C_wH_{2w}$-R(43);

w Null, 1, 2, 3 oder 4;

R(43) $(C_3-C_8)$-Cycloalkyl, Phenyl, Biphenylyl oder Naphthyl,

wobei die Aromaten nicht substituiert oder substituiert sind mit 1 - 3 Substituenten ausgewählt aus der Gruppe bestehend aus F, Cl, $CF_3$, Methyl, Methoxy und NR(44)R(45);

R(44) und R(45)

H, $(C_1-C_4)$-Alkyl oder $(C_1-C_4)$-Perfluoralkyl;

R(39) H, $(C_1-C_4)$-Alkyl oder $(C_1-C_4)$-Perfluoralkyl;

oder

R(38) und R(39)

gemeinsam 4 oder 5 Methylengruppen, von denen eine $CH_2$-Gruppe durch Sauerstoff, S, NH, N-$CH_3$ oder N-Benzyl ersetzt sein kann;

oder

R(3) und R(4)

unabhängig voneinander R(46)X(1)-;

X(1) Sauerstoff, S, NR(47), (D=O)A-, NR(48)C=MN$^{(*)}$R(49)-,

M Sauerstoff oder S;

A Sauerstoff oder NR(50);

D C oder SO;

R(46) $(C_1-C_8)$-Alkyl, $(C_3-C_8)$-Alkenyl, $(CH_2)_bC_dF_{2d+1}$ oder $-C_xH_{2x}$-R(51);

b Null oder 1;

d 1, 2, 3, 4, 5, 6 oder 7;

x Null, 1, 2, 3 oder 4;

R(51) $(C_3-C_8)$-Cycloalkyl, Phenyl, Biphenylyl oder Naphthyl,

wobei die Aromaten nicht substituiert oder substituiert sind mit 1 - 3 Substituenten ausgewählt aus der Gruppe bestehend aus F, Cl, $CF_3$, Methyl, Methoxy und NR(52)R(53);

R(52) und R(53)

H, $(C_1-C_4)$-Alkyl oder $(C_1-C_4)$-Perfluoralkyl;

R(47), R(48) und R(50)

unabhängig Wasserstoff, $(C_1-C_4)$-Alkyl oder $(C_1-C_4)$-Perfluoralkyl;

R(49) definiert wie R(46);

oder

R(46) und R(47) beziehungsweise R(46) und R(48)

gemeinsam 4 oder 5 Methylengruppen, von denen eine $CH_2$-Gruppe durch Sauerstoff, S, NH, N-$CH_3$ oder N-Benzyl ersetzt sein kann,

wobei A und $N^{(*)}$ an den Phenylkern des Benzoylguanidin-Grundkörpers gebunden sind;

oder

R(3) und R(4)

unabhängig voneinander -SR(64), -OR(65), -NHR(66), -NR(67)R(68), -CHR(69)R(70), -C(OH)R(54)R(55), -C≡CR(56), -CR(58)=CHR(57), $-[CR(59)R(60)]_u-(CO)-[CR(61)R(62)]_v-R(63)$;

R(64), R(65), R(66), R(67) und R(69)

gleich oder verschieden $-(CH_2)_y-(CHOH)_z-(CH_2)_{aa}-(CH_2OH)_t-R(71)$ oder - $(CH_2)_{ab}-O-(CH_2-CH_2O)_{ac}-R(72)$,

R(71) und R(72)

Wasserstoff oder Methyl;

u 1, 2, 3 oder 4;

v Null, 1, 2, 3 oder 4;

y, z, aa

gleich oder verschieden Null, 1, 2, 3 oder 4;

t 1, 2, 3 oder 4;

R(68), R(70), R(54) und R(55)

gleich oder verschieden Wasserstoff, $(C_1-C_6)$-Alkyl;

oder

R(69) und R(70) beziehungsweise R(54) und R(55)

zusammen mit dem sie tragenden Kohlenstoff-Atom ein $(C_3-C_8)$-Cycloalkyl;

R(63)

H, $(C_1-C_6)$-Alkyl, $(C_3-C_8)$-Cycloalkyl oder $-C_eH_{2e}-R(73)$;

e Null, 1, 2, 3 oder 4;

R(56), R(57) und R(73)

unabhängig Phenyl,

das unsubstituiert oder substituiert ist mit 1 - 3 Substituenten ausgewählt aus der Gruppe bestehend aus F, Cl, $CF_3$, Methyl, Methoxy und NR(74)R(75);

R(74) und R(75)

H oder $(C_1-C_4)$-Alkyl;

oder

R(56), R(57) und R(73)

unabhängig $(C_1-C_9)$-Heteroaryl,

das unsubstituiert oder wie Phenyl substituiert ist;

R(58), R(59), R(60), R(61) und R(62)

Wasserstoff oder Methyl,

oder
R(3) und R(4)

unabhängig voneinander R(76)-NH-$SO_2$-;
R(76) R(77)R(78)N-(C=Y')-;
Y' Sauerstoff, S oder N-R(79);
R(77) und R(78)

gleich oder verschieden H, ($C_1$-$C_8$)-Alkyl, ($C_3$-$C_6$)-Alkenyl, -$C_fH_{2f}$-R(80);
f Null, 1, 2, 3 oder 4;
R(80) ($C_5$-$C_7$)-Cycloalkyl oder Phenyl,

welches unsubstituiert oder substituiert mit 1 - 3 Substituenten ausgewählt aus der Gruppe bestehend aus F, Cl, $CF_3$, Methoxy und ($C_1$-$C_4$)-Alkyl;

oder
R(77) und R(78)

gemeinsam 4 oder 5 Methylengruppen, von denen eine $CH_2$-Gruppe durch Sauerstoff, S, NH, N-$CH_3$ oder N-Benzyl ersetzt sein kann,
R(79)

wie R(77) definiert oder gleich Amidin;

oder
R(3) und R(4)

unabhängig voneinander NR(84)R(85);
R(84) und R(85)

unabhängig voneinander H, ($C_1$-$C_4$)-Alkyl, oder gemeinsam 4 oder 5 Methylengruppen,

von denen eine $CH_2$-Gruppe durch Sauerstoff, S, NH, N-$CH_3$ oder N-Benzyl ersetzt sein kann; oder von denen eine oder zwei $CH_2$-Gruppen durch CH-$C_{dm}H_{2dm+1}$ ersetzt sein können,

sowie deren pharmazeutisch verträgliche Salze;

(HOE 94/F 123 - EP-OS 682 017, NZ 272 058)
y) Bizyklische Heteroaroylguanidine der Formel I

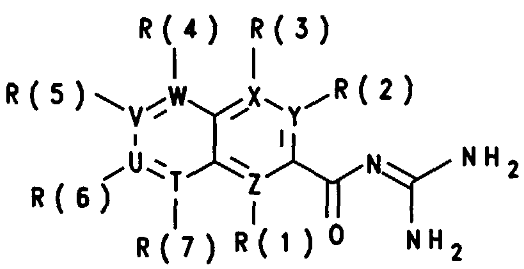
I

worin bedeuten:

T, U, V, W, X, Y und Z

unabhängig voneinander Stickstoff oder Kohlenstoff;
jedoch mit der Einschränkung,
daß X und Z nicht gleichzeitig Stickstoff sind,
und daß T, U, V, W, X, Y und Z keinen Substituenten tragen, wenn sie Stickstoff sind,
und daß nicht mehr als vier von ihnen gleichzeitig Stickstoff sind,

R(1) und R(2)

unabhängig voneinander Wasserstoff, F, Cl, Br, I, ($C_1$-$C_3$)-Alkyl, ($C_1$-$C_3$)-Perfluoralkyl, OR(8), NR(8)R(9) oder C(=O)N=C($NH_2$)$_2$;
R(8) und R(9)

unabhängig voneinander Wasserstoff oder ($C_1$-$C_3$)-Alkyl,

oder
R(8) und R(9)

gemeinsam 4 oder 5 Methylengruppen, von denen eine $CH_2$-Gruppe durch Sauerstoff, S, NH, N-$CH_3$ oder N-Benzyl ersetzt sein kann;

R(3), R(4), R(5), R(6) und R(7)

unabhängig voneinander Wasserstoff, F, Cl, Br, I, -C≡N, $X_k$-($CH_2$)$_p$-($C_qF_{2q+1}$), R(10a)-$SO_{bm}$, R(10b)R(10c)N-CO, R(11)-CO-oder R(12)R(13)N-$SO_2$-,

wobei die Perfluoralkylgruppe geradkettig oder verzweigt ist;

X Sauerstoff, S oder NR(14);

R(14) H oder $C_1$-$C_3$)-Alkyl;

bm Null, 1 oder 2;
p Null, 1 oder 2;
k Null oder 1;
q 1, 2, 3, 4, 5 oder 6;
R(10a), R(10b), R(11) und R(12)

unabhängig voneinander($C_1$-$C_8$)-Alkyl, ($C_3$-$C_6$)-Alkenyl, -$C_nH_{2n}$-R(15) oder ($C_1$-$C_8$)-Perfluoralkyl;
n Null, 1, 2, 3 oder 4;
R(15) ($C_3$-$C_7$)-Cycloalkyl oder Phenyl, welches nicht substituiert ist oder

substituiert mit 1 - 3 Substituenten ausgewählt aus der Gruppe bestehend aus F, Cl, $CF_3$, Methyl, Methoxy und NR(16)R(17);
R(16) und R(17)

H oder $C_1$-$C_4$-Alkyl;

oder
R(10b), R(11) und R(12)

Wasserstoff;

R(10c) und R(13)

unabhängig Wasserstoff oder ($C_1$-$C_4$)-Alkyl;

oder

R(10b) und R(10c) sowie R(12) und R(13)

gemeinsam 4 oder 5 Methylengruppen, von denen eine $CH_2$-Gruppe durch Sauerstoff, Schwefel, NH, N-$CH_3$ oder N-Benzyl ersetzt sein kann;

oder

R(3), R(4), R(5), R(6) und R(7)

unabhängig voneinander ($C_1$-$C_8$)-Alkyl, -$C_{al}H_{2al}$R(18) oder ($C_3$-$C_8$)-Alkenyl;

al Null, 1 oder 2;

R(18) ($C_3$-$C_8$)-Cycloalkyl, Phenyl, Biphenylyl oder Naphthyl, wobei die Aromaten nicht substituiert oder substituiert sind mit 1 - 3 Substituenten ausgewählt aus der Gruppe bestehend aus F, Cl, $CF_3$, Methyl, Methoxy und NR(19a)R(19b);

R(19a) und R(19b)

Wasserstoff, ($C_1$-$C_4$)-Alkyl oder ($C_1$-$C_4$)-Perfluoralkyl;

oder

R(3), R(4), R(5), R(6) und R(7)

unabhängig voneinander ($C_1$-$C_9$)-Heteroaryl, das über C oder N verknüpft ist und das unsubstituiert oder substituiert ist mit 1 - 3 Substituenten aus der Gruppe bestehend aus F, Cl, $CF_3$, $CH_3$, Methoxy, Hydroxy, Amino, Methylamino oder Dimethylamino;

oder

R(3), R(4), R(5), R(6) und R(7)

unabhängig voneinander

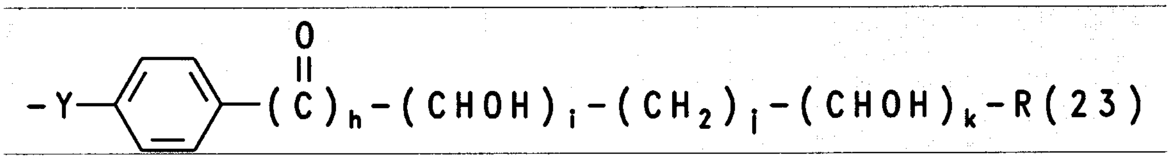

oder

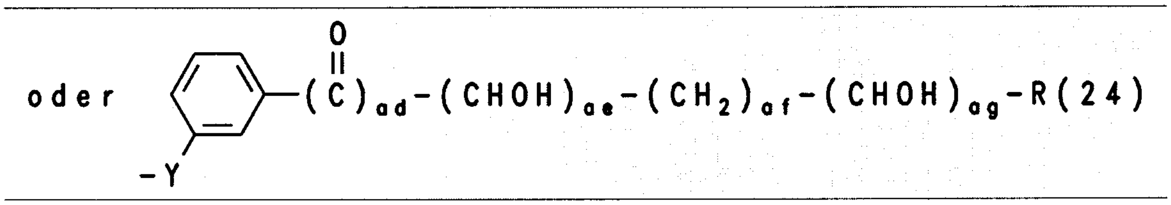

oder

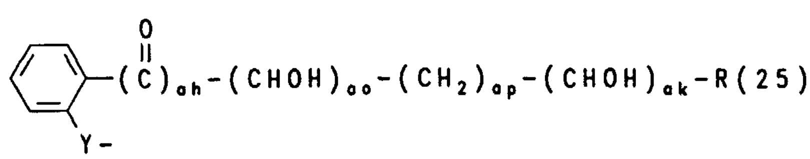

Y Sauerstoff, -S- oder -NR(22)-;

h, ad, ah

unabhängig voneinander Null oder 1;

i, j, k, ae, af, ag, ao, ap und ak

unabhängig voneinander Null, 1, 2, 3 oder 4;

wobei jedoch jeweils

h, i und k nicht gleichzeitig Null,
ad, ae und ag nicht gleichzeitig Null sowie
ah, ao und ak nicht gleichzeitig Null sind,

R(23), R(24) R(25) und R(22)

unabhängig voneinander Wasserstoff oder ($C_1$-$C_3$)-Alkyl;

oder
R(3), R(4), R(5), R(6) und R(7)

unabhängig voneinander SR(29), -OR(30), -NR(31)R(32) oder -CR(33)R(34)R(35);
R(29), R(30), R(31) und R(33)

unabhängig voneinander -$C_aH_{2a}$-($C_1$-$C_9$)-Heteroaryl, das unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;
a Null, 1 oder 2;

R(32), R(34) und R(35)

unabhängig voneinander wie R(29) definiert oder Wasserstoff, ($C_1$-$C_4$)-Alkyl oder ($C_1$-$C_4$)-Perfluoralkyl;

oder
R(3), R(4), R(5), R(6) und R(7)

unabhängig voneinander

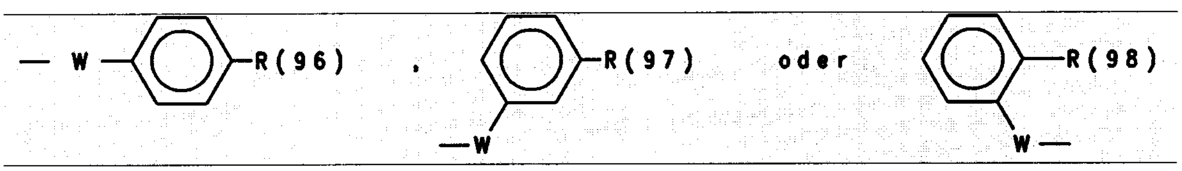 , oder

R(96), R(97) und R(98)

unabhängig voneinander ($C_1$-$C_9$)-Heteroaryl, das über C oder N verknüpft ist und das unsubstituiert oder substituiert ist mit 1 bis 3 Substituenten aus der Gruppe bestehend aus F, Cl, $CF_3$, $CH_3$, Methoxy, Hydroxy, Amino, Methylamino, Dimethylamino oder Benzyl;

W Sauerstoff, S oder NR(36)-;

R(36) H oder ($C_1$-$C_4$)-Alkyl;

oder
R(3), R(4), R(5), R(6) und R(7)

unabhängig voneinander R(46)X(1)-;
X(1) Sauerstoff, S, NR(47), (D=O)A- oder NR(48)C=MN$^{(*)}$R(49)-;

M Sauerstoff oder Schwefel;
A Sauerstoff oder NR(50);
D C oder SO;

R(46) ($C_1$-$C_8$)-Alkyl, ($C_3$-$C_8$)-Alkenyl, $(CH_2)_bC_dF_{2d+1}$ oder $-C_xH_{2x}$-R(51);

b Null oder 1;
d 1, 2, 3, 4, 5, 6 oder 7;
x Null, 1, 2, 3 oder 4;
R(51) ($C_3$-$C_8$)-Cycloalkyl, Phenyl, Biphenylyl oder Naphthyl, wobei die Aromaten nicht substituiert oder substituiert sind mit 1 - 3 Substituenten ausgewählt aus der Gruppe bestehend aus F, Cl, $CF_3$, Methyl, Methoxy und NR(52)R(53);

R(52) und R(53)

Wasserstoff, ($C_1$-$C_4$)-Alkyl oder ($C_1$-$C_4$)-Perfluoralkyl;

R(47), R(48) und R(50) unabhängig

Wasserstoff, ($C_1$-$C_4$)-Alkyl oder ($C_1$-$C_4$)-Perfluoralkyl;

R(49) definiert wie R(46);

oder
R(46) und R(47) beziehungsweise R(46) und R(48)

gemeinsam 4 oder 5 Methylengruppen, von denen eine $CH_2$-Gruppe durch Sauerstoff, Schwefel, NH, N-$CH_3$ oder N-Benzyl ersetzt sein kann;

wobei A und N$^{(*)}$ an den Phenylkern des Heteroaroylguanidin-Grundkörpers gebunden sind;

oder
R(3), R(4), R(5), R(6) und R(7)

unabhängig voneinander -SR(64), -OR(65), -NHR(66), -NR(67)R(68), -CHR(69)R(70) oder -CR(54)R(55)OH, -C≡CR(56), -CR(58)=CR(57) oder $-[CR(59)R(60)]_u$-CO-$[CR(61)R(62)]_v$-R(63);
R(64), R(65), R(66), R(67) und R(69)

gleich oder verschieden $-(CH_2)_y-(CHOH)_z-(CH_2)_{aa}-(CHOH)_t$-R(71) oder $-(CH_2)_{ab}-O-(CH_2-CH_2O)_{ac}$-R(72);
R(71), und R(72)

unabhängig voneinander Wasserstoff oder Methyl;

u 1, 2, 3 oder 4;
v Null, 1, 2, 3 oder 4;

y, z, aa gleich oder verschieden

Null, 1, 2, 3 oder 4;

t 1, 2, 3 oder 4;

R(68), R(70), R(54) und R(55)

gleich oder verschieden Wasserstoff oder ($C_1$-$C_6$)-Alkyl;

oder
R(69) und R(70) beziehungsweise R(54) und R(55)

zusammen mit dem sie tragenden Kohlenstoff-Atom ein ($C_3$-$C_8$)-Cycloalkyl;

R(63)

Wasserstoff, ($C_1$-$C_6$)-Alkyl, ($C_3$-$C_8$)-Cycloalkyl oder -$C_eH_{2e}$-R(73);
e Null, 1, 2, 3 oder 4;

R(56), R(57) und R(73) unabhängig

Phenyl, das unsubstituiert oder substituiert ist mit 1 - 3 Substituenten ausgewählt aus der Gruppe bestehend aus F, Cl, $CF_3$, Methyl, Methoxy und NR(74)R(75);
R(74) und R(75)

Wasserstoff oder ($C_1$-$C_4$)-Alkyl;

oder
R(56), R(57) und R(73) unabhängig

($C_1$-$C_9$)-Heteroaryl, das unsubstituiert oder wie Phenyl substituiert ist;

R(58), R(59), R(60), R(61) und R(62)

Wasserstoff oder Methyl;

oder
R(3), R(4), R(5), R(6) und R(7)

unabhängig voneinander R(76)-NH-$SO_2$-;
R(76) R(77)R(78)N-(C=Y')-;

Y' Sauerstoff, S oder N-R(79);
R(77) und R(78)

gleich oder verschieden Wasserstoff, ($C_1$-$C_8$)-Alkyl, ($C_3$-$C_6$)-Alkenyl oder -$C_fH_{2f}$-R(80);
f Null, 1, 2, 3 oder 4;
R(80) ($C_5$-$C_7$)-Cycloalkyl oder Phenyl, welches unsubstituiert mit 1 - 3 Substituenten ausgewählt aus der Gruppe bestehend aus F, Cl, $CF_3$, Methoxy und ($C_1$-$C_4$)-Alkyl;

oder
R(77) und R(78)

gemeinsam 4 oder 5 Methylengruppen, von denen eine $CH_2$-Gruppe durch Sauerstoff, Schwefel, NH, N-$CH_3$ oder N-Benzyl ersetzt sein kann;

R(79) wie R(77) definiert oder gleich Amidin;

oder
R(3), R(4), R(5), R(6) und R(7)

unabhängig voneinander NR(84a)R(85), OR(84b), SR(84c) oder -$C_nH_{2n}$-R(84d);
n Null, 1, 2, 3 oder 4;

R(84d) ($C_3$-$C_7$)-Cycloalkyl oder Phenyl, welches nicht substituiert ist oder substituiert mit 1 - 3 Substituenten aus der Gruppe bestehend aus F, Cl, $CF_3$, Methyl, Methoxy und NR(16)R(17);
R(16) und R(17)

Wasserstoff, oder $C_1$-$C_4$-Alkyl;

R(84a), R(84b), R(84c) und R(85)

unabhängig voneinander Wasserstoff, ($C_1$-$C_8$)-Alkyl, ($C_1$-$C_8$)-Perfluoralkyl oder $(CH_2)_{ax}$-R(84g);
ax Null, 1, 2, 3 oder 4;
R(84g) ($C_3$-$C_7$)-Cycloalkyl oder Phenyl, welches nicht substituiert ist oder substituiert mit 1 - 3 Substituenten ausgewählt aus der Gruppe bestehend aus F, Cl, $CF_3$, Methyl, Methoxy und NR(84u)R(84v);
R(84u) und R(84v)

Wasserstoff oder $C_1$-$C_4$-Alkyl;

oder
R(84a) und R(85)

gemeinsam 4 oder 5 Methylengruppen, von denen eine $CH_2$-Gruppe durch Sauerstoff, Schwefel, NH, N-$CH_3$ oder N-Benzyl ersetzt sein kann,

sowie deren pharmazeutisch verträgliche Salze;

(HOE 94/F 134 - EP-OS 686 627, NZ 272 103)
z) Benzoylguanidine der Formel I

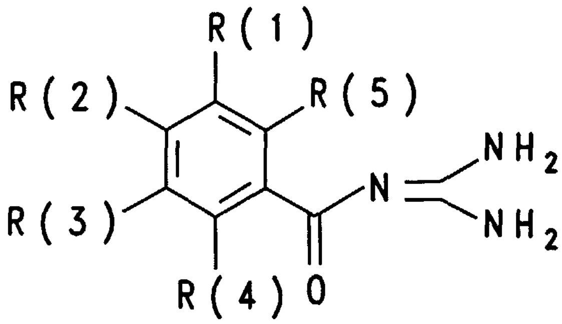

I

worin bedeuten:

R(1) R(6)-$SO_m$;

m Null, 1 oder 2;
R(6) Perfluoralkyl mit 1, 2, 3, 4, 5 oder 6 C-Atomen, das geradkettig oder verzweigt ist;

R(2) und R(3)

unabhängig voneinander Wasserstoff, F, Cl, Br, I Alkyl mit 1, 2, 3 oder 4 C-Atomen, Alkoxy mit 1, 2, 3 oder 4 C-Atomen oder Phenoxy,

das unsubstituiert ist oder substituiert ist mit 1 - 3 Substituenten ausgewählt aus der Gruppe bestehend aus F, Cl Methyl und Methoxy;

oder
R(2) und R(3)

unabhängig voneinander Pyrrol-1-yl, Pyrrol-2-yl- oder Pyrrol-3-yl,

welches nicht substituiert ist oder substituiert mit 1 bis 4 Substituenten, ausgewählt aus der Gruppe bestehend aus F, Cl, Br, I, CN, Alkanoyl mit 2, 3, 4, 5, 6, 7 oder 8 C-Atomen, Alkoxycarbonyl mit 2, 3, 4, 5, 6, 7 oder 8 C-Atomen, Formyl, Carboxy, $CF_3$, Methyl und Methoxy;

R(4) und R(5)

unabhängig voneinander Wasserstoff, Alkyl mit 1, 2 oder 3-C-Atomen, F, Cl, Br, I, CN, OR(7), NR(8)R(9) oder $-(CH_2)_n-(CF_2)_o-CF_3$;
R(7), R(8) und R(9)

unabhängig voneinander Wasserstoff oder Alkyl mit 1, 2, 3 oder 4 C-Atomen;

n Null oder 1;
o Null, 1 oder 2;

sowie deren pharmakologisch akzeptable Salze;

(HOE 94/F 168 - EP-OS 690 048, NZ 272 373)
ab) Perfluoralkylgruppen tragende phenylsubstituierte Alkenylcarbonsäureguanidide der Formel I

I

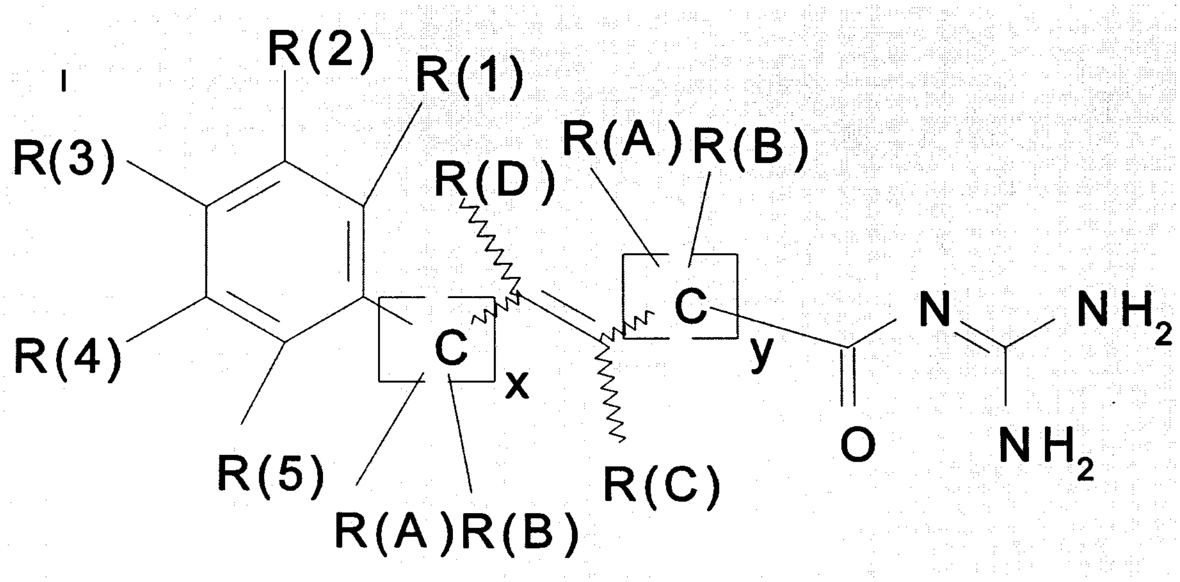

worin bedeuten:

R(A) Wasserstoff, F, Cl, Br, I, CN, OH, OR(6), $(C_1-C_8)$-Alkyl, $O_r(CH_2)_aC_bF_{2b+1}$, $(C_3-C_8)$-Cycloalkyl oder NR(7)R(8);

r Null oder 1;
a Null, 1, 2, 3 oder 4;
b 1, 2, 3, 4, 5, 6, 7 oder 8;
R(6) $(C_1-C_8)$-Alkyl, $(C_1-C_4)$-Perfluoralkyl, $(C_3-C_8)$-Alkenyl, $(C_3-C_8)$-Cycloalkyl, Phenyl oder Benzyl;
wobei die Aromaten nicht substituiert oder substituiert sind mit 1 - 3 Substituenten aus der Gruppe bestehend aus F, Cl, $CF_3$, Methyl, Methoxy und NR(9)R(10);

R(9) und R(10)

H, $(C_1-C_4)$-Alkyl oder $(C_1-C_4)$-Perfluoralkyl;

R(7) und R(8)

unabhängig voneinander wie R(6) definiert;

oder
R(7) und R(8)

gemeinsam 4 oder 5 Methylengruppen, von denen eine $CH_2$-Gruppe durch Sauerstoff, Schwefel, NH, N-$CH_3$ oder N-Benzyl ersetzt sein kann;

R(B) unabhängig wie R(A) definiert;
X Null, 1 oder 2;
Y Null, 1 oder 2;
R(C) Wasserstoff, F, Cl, Br, I, CN, OR(12), ($C_1$-$C_8$)-Alkyl, $O_p(CH_2)_fC_gF_{2g+1}$ oder ($C_3$-$C_8$)-Cycloalkyl;

p Null oder 1;
f Null, 1, 2, 3 oder 4;
g 1, 2, 3, 4, 5, 6, 7 oder 8;
R(12)

($C_1$-$C_8$)-Alkyl, ($C_1$-$C_4$)-Perfluoralkyl, ($C_3$-$C_8$)-Alkenyl, ($C_3$-$C_8$)-Cycloalkyl, Phenyl oder Benzyl;

wobei die Aromaten Phenyl oder Benzyl nicht substituiert oder substituiert sind mit 1 - 3 Substituenten aus der Gruppe bestehend aus F, Cl, $CF_3$, Methyl, Methoxy und NR(13)R(14);
R(13) und R(14)

unabhängig voneinander H, ($C_1$-$C_4$)-Alkyl oder ($C_1$-$C_4$)-Perfluoralkyl;

R(D) unabhängig wie R(C) definiert,
R(1) Wasserstoff, ($C_1$-$C_8$)-Alkyl, -$O_t(CH_2)_dC_eF_{2e+1}$, ($C_3$-$C_8$)-Cycloalkyl, F, Cl, Br, I oder CN;

t Null oder 1;
d Null, 1, 2, 3 oder 4;
e 1, 2, 3, 4, 5, 6, 7 oder 8;

R(2), R(3), R(4) und R(5)

unabhängig voneinander wie R(1) definiert;

jedoch unter der Bedingung,
daß mindestens einer der Substituenten R(A), R(B), R(C), R(D), R(1), R(2), R(4) oder R(5) eine $O_r(CH_2)_aC_bF_{2b+1}$-, $O_p(CH_2)_fC_gF_{2g+1}$- oder $O_t(CH_2)_dC_eF_{2e+1}$-gruppe und R(3) keine $O_t(CH_2)_dC_eF_{2e+1}$-gruppe ist;
sowie deren pharmazeutisch verträgliche Salze;

(HOE 94/F 182 - EP-OS 690 048, NZ 272 449)
ac) Ortho-amino-substituierte Benzoylguanidine der Formel I

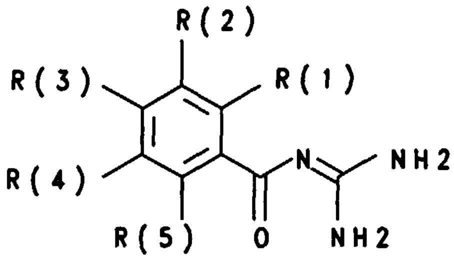

I

worin bedeuten:

R(1) NR(50)R(6),

R(50) und R(6)

unabhängig voneinander Wasserstoff, ($C_1$-$C_8$)-Alkyl oder ($C_1$-$C_8$)-Perfluoralkyl;

R(2), R(3), R(4) und R(5)

unabhängig voneinander R(10)-$SO_a$-, R(11)R(12)N-CO-, R(13)-CO-oder R(14)R(15)N-$SO_2$-;
a Null, 1 oder 2,
R(10), R(11), R(12), R(13), R(14) und R(15)

unabhängig voneinander ($C_1$-$C_8$)-Alkyl, ($C_1$-$C_8$)-Perfluoralkyl, ($C_3$-$C_6$)-Alkenyl oder -$C_{ab}H_{2ab}$-R(16);
ab Null, 1, 2, 3 oder 4;
R(16) ($C_3$-$C_7$)-Cycloalkyl, Phenyl,

welches nicht substituiert ist oder substituiert mit 1 - 3 Substituenten ausgewählt aus der Gruppe bestehend aus F, Cl, $CF_3$, Methyl, Methoxy oder NR(17)R(18);
R(17) und R(18)

unabhängig voneinander H, $CF_3$ oder ($C_1$-$C_4$)-Alkyl;

oder
R(11), R(12), sowie R(14) und R(15)

gemeinsam 4 oder 5 Methylengruppen, von denen eine $CH_2$-Gruppe durch Sauerstoff, S, NH, N-$CH_3$ oder N-Benzyl ersetzt sein kann;

oder
R(11), R(12), R(14) und R(15)

unabhängig voneinander Wasserstoff;

oder
R(2), R(3), R(4) und R(5)

unabhängig voneinander SR(21), -OR(22), -NR(23)R(24) oder -CR(25)R(26)R(27);
R(21), R(22), R(23) und R(25)

unabhängig voneinander -$C_bH_{2b}$-($C_1$-$C_9$)-Heteroaryl, das unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;
b Null, 1 oder 2;

R(24), R(26) und R(27)

unabhängig voneinander Wasserstoff, ($C_1$-$C_4$)-Alkyl oder ($C_1$-$C_4$)-Perfluoralkyl;

oder
R(2), R(3), R(4) und R(5)

unabhängig voneinander Wasserstoff, F, Cl, Br, I, CN, -$(Xa)_{dg}$-$C_{da}H_{2da+1}$, - $(Xb)_{dh}$-$(CH_2)_{db}$-$C_{de}F_{2de+1}$, ($C_3$-$C_8$)-Alkenyl oder -$C_{df}H_{2df}$R(30);
(Xa) O, S oder NR(33);
R(33)

H, ($C_1$-$C_4$)-Alkyl oder ($C_1$-$C_4$)-Perfluoralkyl;

dg Null oder 1;
(Xb) O, S oder NR(34);

R(34)

H, ($C_1$-$C_4$)-Alkyl oder ($C_1$-$C_4$)-Perfluoralkyl;

dh Null oder 1;
da Null, 1, 2, 3, 4, 5, 6, 7, 8;
db Null, 1, 2, 3, 4;
de Null, 1, 2, 3, 4, 5, 6, 7;
df Null, 1, 2, 3, 4;
R(30)

($C_3$-$C_8$)-Cycloalkyl, Phenyl, Biphenylyl oder Naphthyl, wobei die Aromaten Phenyl, Biphenylyl oder Naphthyl nicht substituiert oder substituiert sind mit 1 - 3 Substituenten ausgewählt aus der Gruppe bestehend aus F, Cl, $CF_3$, Methyl, Methoxy und NR(31)R(32);
R(31) und R(32)

H, ($C_1$-$C_4$)-Alkyl oder ($C_1$-$C_4$)-Perfluoralkyl;

oder
R(2), R(3), R(4) und R(5)

unabhängig voneinander NR(40)R(41) oder -(Xe)-$(CH_2)_{eb}$R(45);
R(40) und R(41)

unabhängig voneinander Wasserstoff, ($C_1$-$C_8$)-Alkyl, ($C_1$-$C_8$)-Perfluoralkyl oder $(CH_2)_e$-R(42);
e Null, 1, 2, 3 oder 4;
R(42)

($C_3$-$C_7$)-Cycloalkyl, Phenyl, welches nicht substituiert ist oder substituiert mit 1 - 3 Substituenten ausgewählt aus der Gruppe bestehend aus F, Cl, $CF_3$, Methyl, Methoxy und NR(43)R(44);
R(43) und R(44)

unabhängig voneinander H, $CF_3$ oder ($C_1$-$C_4$)-Alkyl;

oder
R(40) und R(41)

gemeinsam 4 oder 5 Methylengruppen, von denen eine $CH_2$-Gruppe durch Sauerstoff, Schwefel, NH, N-$CH_3$ oder N-Benzyl ersetzt sein kann;
(Xe) O, S oder NR(47);

R(47)

H, $(C_1-C_4)$-Alkyl oder $(C_1-C_4)$-Perfluoralkyl;

eb Null, 1, 2, 3 oder 4;
R(45) $(C_3-C_7)$-Cycloalkyl, Phenyl, welches nicht substituiert ist oder substituiert mit 1 - 3 Substituenten ausgewählt aus der Gruppe bestehend aus F, Cl, $CF_3$, Methyl, Methoxy, NR(50)R(51) und - (Xfa)-$(CH_2)_{ed}$-(Xfb)R(46);

Xfa $CH_2$, O, S oder NR(48);
Xfb O, S oder NR(49);
ed 1, 2, 3 oder 4;
R(46)

H, $(C_1-C_4)$-Alkyl oder $(C_1-C_4)$-Perfluoralkyl;

R(48), R(49), R(50) und R(51)

unabhängig voneinander H oder $(C_1-C_4)$-Alkyl oder $(C_1-C_4)$-Perfluoralkyl;

wobei R(3) und R(4) jedoch nicht Wasserstoff sein können,
sowie deren pharmazeutisch verträgliche Salze;

HOE 94/F 265 - NZ 272 946, EP-OS 700 904)
ad) Benzoylguanidine der Formel I

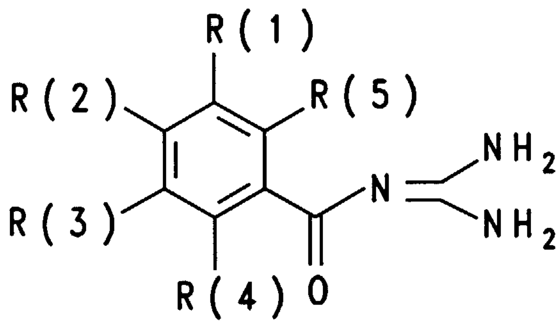
I

worin bedeuten:

einer der drei Substituenten R(1), R(2) und R(3)

$(C_1-C_9)$-Heteroaryl-N-Oxid,

das über C oder N verknüpft ist und das unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;

oder

einer der drei Substituenten R(1), R(2) und R(3)

-SR(10), -OR(10), -NR(10)R(11) oder-CR(10)R(11)R(12);
R(10)

$-C_aH_{2a}-(C_1-C_9)$-Heteroaryl-N-Oxid,

das unsubstituiert oder substituiert ist mit 1 - 3 Substituenten ausgewählt aus der Gruppe beste-

hend F, Cl, $CF_3$, $CH_3$, Methoxy, Hydroxy, Amino, Methylamino und Dimethylamino;

a Null, 1 oder 2;
R(11) und R(12)

unabhängig voneinander wie R(10) definiert, Wasserstoff oder ($C_1$-$C_4$)-Alkyl;

und die jeweils anderen Substituenten R(1), R(2) und R(3)

unabhängig voneinander ($C_1$-$C_8$)-Alkyl, ($C_2$-$C_8$)-Alkenyl oder -$C_mH_{2m}$R(14);

m Null, 1 oder 2;
R(14) ($C_3$-$C_8$)-Cycloalkyl oder Phenyl,

welches nicht substituiert oder substituiert ist mit 1 - 3 Substituenten ausgewählt aus der Gruppe bestehend aus F, Cl, $CF_3$, Methyl, Methoxy und NR(15)R(16),
R(15) und R(16)

Wasserstoff oder $CH_3$;

oder
die jeweils anderen Substituenten R(1), R(2) und R(3)

unabhängig voneinander Wasserstoff, F, Cl, Br, I, -C≡N, X-$(CH_2)_p$-$(C_qF_{2q+1})$, R(22)-$SO_u$, R(23)R(24)N-CO, R(25)-CO- oder R(26)R(27)N-$SO_2$-,

wobei die Perfluoralkylgruppe geradkettig oder verzweigt ist;

X eine Bindung, Sauerstoff, S oder NR(28);
u Null, 1 oder 2;
p Null, 1 oder 2;
q Null, 1, 2, 3, 4, 5 oder 6;
R(22), R(23), R(25) und R(26)

unabhängig ($C_1$-$C_8$)-Alkyl, ($C_2$-$C_6$)-Alkenyl, -$C_nH_{2n}$-R(29) oder $CF_3$;
n Null, 1, 2, 3 oder 4;
R(28) Wasserstoff oder ($C_1$-$C_3$)-Alkyl;
R(29) ($C_3$-$C_7$)-Cycloalkyl oder Phenyl;

welches nicht substituiert ist oder substituiert mit 1 - 3 Substituenten ausgewählt aus der Gruppe bestehend aus F, Cl, $CF_3$, Methyl, Methoxy und NR(30)R(31);
R(30) und R(31)

Wasserstoff oder $C_1$-$C_4$-Alkyl,

oder
R(23), R(25) und R(26)

auch Wasserstoff;

R(24) und R(27)

unabhängig voneinander Wasserstoff oder ($C_1$-$C_4$)-Alkyl;

oder
R(23) und R(24) sowie R(26) und R(27) gemeinsam 4 oder 5 Methylengruppen, von denen eine $CH_2$-Gruppe durch Sauerstoff, S, NH, N-$CH_3$ oder N-Benzyl ersetzt sein kann;

oder
die jeweils anderen Substituenten R(1), R(2) und R(3)

unabhängig voneinander OR(35) oder NR(35)R(36);
R(35) und R(36)

unabhängig voneinander Wasserstoff oder ($C_1$-$C_6$)-Alkyl;

oder
R(35) und R(36)

gemeinsam 4 - 7 Methylengruppen, von denen eine $CH_2$-Gruppe durch Sauerstoff, S, NH, N-$CH_3$ oder N-Benzyl ersetzt sein kann,

R(4) und R(5)

unabhängig voneinander Wasserstoff, ($C_1$-$C_4$)-Alkyl, F, Cl, -OR(32), - NR(33)R(34) oder $C_rF_{2r+1}$;
R(32), R(33) und R(34)

unabhängig voneinander Wasserstoff oder ($C_1$-$C_3$)-Alkyl;
r 1, 2, 3 oder 4;

sowie deren pharmazeutisch verträgliche Salze;

(HOE 94/F 266 - EP-OS 702 001, NZ 272 948)
ad) Benzoylguanidine der Formel I

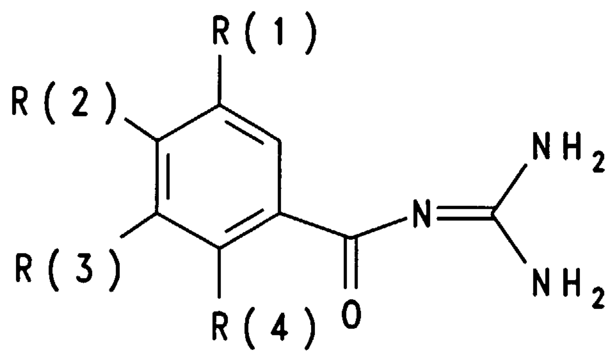

worin bedeuten:

R(1) Wasserstoff, F, Cl, Br, I, CN, $NO_2$, OH, ($C_1$-$C_8$)-Alkyl, ($C_3$-$C_8$)-Cycloalkyl, $O_a$-$(CH_2)_b$-$(CF_2)_c$-$CF_3$;

a Null oder 1;
b Null, 1 oder 2;
c Null, 1, 2 oder 3;

oder
R(1) R(5)-$SO_m$ oder R(6)R(7)N-$SO_2$-;

m Null, 1 oder 2;
R(5) und R(6) unabhängig voneinander

($C_1$-$C_8$)-Alkyl, ($C_3$-$C_6$)-Alkenyl, $CF_3$ oder -$C_nH_{2n}$-R(8);
n Null, 1, 2, 3 oder 4;

R(7) Wasserstoff oder ($C_1$-$C_4$)-Alkyl;

R(8) $(C_3-C_7)$-Cycloalkyl oder Phenyl,

welches nicht substituiert ist oder substituiert mit 1 - 3 Substituenten ausgewählt aus der Gruppe bestehend aus F, Cl, $CF_3$, Methyl, Methoxy und NR(9)R(10);
R(9) und R(10) unabhängig voneinander

Wasserstoff oder $(C_1-C_4)$-Alkyl;

oder
R(6) H;
oder R(6) und R(7)

gemeinsam 4 oder 5 Methylengruppen, von denen eine $CH_2$-Gruppe durch Sauerstoff, S, NH, N-$CH_3$ oder N-Benzyl ersetzt sein kann,

oder
R(1) -SR(11), -OR(11) oder -CR(11)R(12)R(13);

R(11) $-C_pH_{2p}-(C_3-C_8)$-Cycloalkyl, $-(C_1-C_9)$-Heteroaryl oder Phenyl,

wobei die aromatischen Systeme unsubstituiert oder substituiert sind mit 1 - 3 Substituenten ausgewählt aus der Gruppe bestehend aus F, Cl, $CF_3$, $CH_3$, Methoxy, Hydroxy, Amino, Methylamino und Dimethylamino;
R(12), R(13) unabhängig voneinander

wie R(11) definiert oder Wasserstoff oder $(C_1-C_4)$-Alkyl;

p Null 1 oder 2;

oder
R(1) Phenyl, Naphthyl, Biphenylyl oder $(C_1-C_9)$-Heteroaryl, letzteres über C oder N verknüpft,

die unsubstituiert oder substituiert sind mit 1 - 3 Substituenten ausgewählt aus der Gruppe bestehend aus F, Cl, $CF_3$, $CH_3$, Methoxy, Hydroxy, Amino, Methylamino und Dimethylamino;

R(2) $-CF_2R(14)$, -CF[R(15)][R(16)], $-CF[(CF_2)_q-CF_3)][R(15)]$, $-C[(CF_2)_r-CF_3]=CR(15)R(16)$;

R(14) $(C_1-C_4)$-Alkyl oder $(C_3-C_6)$-Cycloalkyl;
R(15) und R(16) unabhängig voneinander

Wasserstoff oder $(C_1-C_4)$-Alkyl;

q Null, 1 oder 2;
r Null, 1 oder 2;

R(3) wie R(1) definiert;
R(4) Wasserstoff, $(C_1-C_3)$-Alkyl, F, Cl, Br, I, CN, $-(CH_2)_s-(CF_2)_t-CF_3$;

s Null oder 1;
t Null, 1 oder 2;

sowie deren pharmazeutisch verträgliche Salze;

(HOE 94/F 267 - EP-OS 700 899, NZ 272 947)
ae) Benzoylguanidine der Formel I

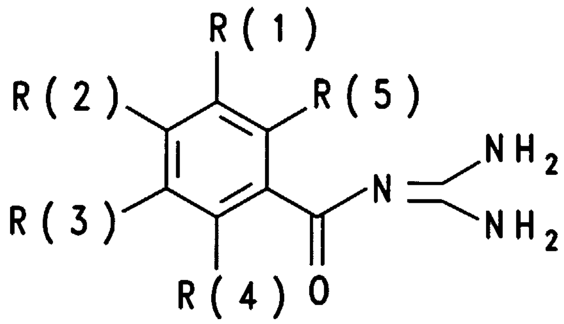

I

worin bedeuten:

einer der drei Substituenten R(1), R(2) und R(3)

-Y-4-[$(CH_2]_k$-CHR(7)-(C=O)R(8)]-Phenyl, -Y-3-$(CH_2)_k$-CHR(7)-(C=O)R(8)]-Phenyl oder -Y-2-[$(CH_2)_k$-CHR(7)-(C=O)R(8)]-Phenyl,

wobei das Phenyl jeweils unsubstituiert oder substituiert ist mit 1 - 2 Substituenten aus der Gruppe F, Cl, $-CF_3$, Methyl, Hydroxy, Methoxy, oder -NR(37)R(38);
R(37) und R(38)

unabhängig voneinander Wasserstoff oder $-CH_3$;

Y eine Bindung, Sauerstoff, -S- oder -NR(9);

R(9) Wasserstoff oder $-(C_1-C_4)$-Alkyl;

R(7) -OR(10) oder-NR(10)R(11);

R(10) und R(11)

unabhängig voneinander Wasserstoff, $-(C_1-C_8)$-Alkyl, $-(C_1-C_8)$-Alkanoyl, $-(C_1-C_8)$-Alkoxycarbonyl, Benzyl, Benzyloxycarbonyl;

oder
R(10) Trityl;

R(8) -OR(12) oder-NR(12)R(13);

R(12) und R(13)

unabhängig voneinander Wasserstoff, $-(C_1-C_8)$-Alkyl oder Benzyl;

k Null, 1, 2, 3 oder 4;

und die jeweils anderen Reste R(1), R(2) und R(3)

unabhängig voneinander $-(C_1-C_8)$-Alkyl, $-(C_2-C_8)$-Alkenyl oder - $(CH_2)_m$R(14);
m Null, 1 oder 2;
R(14) $-(C_3-C_8)$-Cycloalkyl oder Phenyl,

welches nicht substituiert oder substituiert ist mit 1 - 3 Substituenten ausgewählt aus der Gruppe bestehend aus F, Cl, $-CF_3$, Methyl, Methoxy und -NR(15)R(16);
R(15) und R(16)

Wasserstoff oder $-CH_3$;

oder
die jeweils anderen Reste R(1), R(2) und R(3)

unabhängig voneinander R(18)R(19)N-(C=Y')-NH-$SO_2$-;

Y' Sauerstoff, -S- oder -N-R(20);

R(18) und R(19)

unabhängig voneinander Wasserstoff, -($C_1$-$C_8$)-Alkyl, -($C_3$-$C_6$)-Alkenyl oder -$(CH_2)_t$-R(21);
t Null, 1, 2, 3 oder 4;
R(21) -($C_5$-$C_7$)-Cycloalkyl oder Phenyl,

welches unsubstituiert ist oder substituiert ist mit 1 - 3 Substituenten ausgewählt aus der Gruppe bestehend aus F, Cl, - $CF_3$, Methoxy und -($C_1$-$C_4$)-Alkyl;

oder
R(18) und R(19)

gemeinsam 4 oder 5 Methylengruppen, von denen eine $CH_2$-Gruppe durch Sauerstoff, -S-, -NH-, -N-$CH_3$ oder -N-Benzyl ersetzt sein kann;
R(20)

wie R(18) definiert oder Amidin;

oder
die jeweils anderen Reste R(1), R(2) und R(3)

unabhängig voneinander Wasserstoff, F, Cl, Br, I, -C≡N, X-$(CH_2)_p$-($C_qF_{2q+1}$), R(22)-$SO_u$-, R(23)R(24)N-CO-, R(25)-CO- oder R(26)R(27)N-$SO_2$-, wobei die Perfluoralkylgruppe geradkettig oder verzweigt ist;
X eine Bindung, Sauerstoff, -S- oder -NR(28);
u Null, 1 oder 2;
p Null, 1 oder 2;
q 1, 2, 3, 4, 5 oder 6;
R(22), R(23), R(25) und R(26)

unabhängig voneinander -($C_1$-$C_8$)-Alkyl, -($C_3$-$C_6$)-Alkenyl, - $(CH_2)_n$-R(29) oder -$CF_3$;
n Null, 1, 2, 3 oder 4;
R(28) Wasserstoff oder -($C_1$-$C_3$)-Alkyl;
R(29) -($C_3$-$C_7$)-Cycloalkyl oder Phenyl,

welches nicht substituiert ist oder substituiert mit 1 - 3 Substituenten ausgewählt aus der Gruppe bestehend aus F, Cl, - $CF_3$, Methyl, Methoxy und -NR(30)R(31);
R(30) und R(31)

Wasserstoff oder -($C_1$-$C_4$)-Alkyl;

oder
R(23), R(25) und R(26)

Wasserstoff;

R(24) und R(27)

unabhängig voneinander Wasserstoff oder -($C_1$-$C_4$)-Alkyl;

oder

R(23) und R(24) sowie R(26) und R(27)

gemeinsam 4 oder 5 Methylengruppen, von denen eine $CH_2$-Gruppe durch Sauerstoff, -S-, -NH-, - N-$CH_3$ oder -N-Benzyl ersetzt sein kann;

oder

die jeweils anderen Reste R(1), R(2) und R(3)

unabhängig voneinander -OR(35) oder -NR(35)R(36);

R(35) und R(36)

unabhängig voneinander Wasserstoff oder -($C_1$-$C_6$)-Alkyl;

oder

R(35) und R(36)

gemeinsam 4 - 7 Methylengruppen, von denen eine $CH_2$-Gruppe durch Sauerstoff, -S-, -NH-, -N-$CH_3$ oder -N-Benzyl ersetzt sein kann;

R(4) und R(5)

unabhängig voneinander Wasserstoff, -($C_1$-$C_4$)-Alkyl, F, Cl, -OR(32), - NR(33)R(34) oder -$C_rF_{2r+1}$;

R(32), R(33) und R(34)

unabhängig voneinander Wasserstoff oder -($C_1$-$C_3$)-Alkyl;

r 1, 2, 3 oder 4;

sowie deren pharmazeutisch verträgliche Salze;

(HOE 94/F 352 - EP-OS 713 684, NZ 280 517)

af) Benzoylguanidine der Formel I

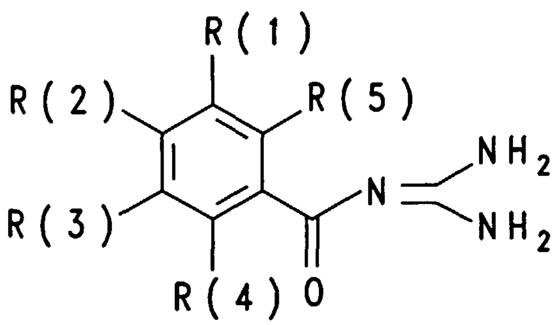

I

worin bedeuten:

R(1) R(6)-CO oder R(7)R(8)N-CO;

R(6) ($C_1$-$C_8$)-Alkyl, ($C_1$-$C_8$)-Perfluoralkyl, ($C_3$-$C_8$)-Alkenyl oder - $C_nH_{2n}$-R(9),

n Null, 1, 2, 3 oder 4;

R(9) ($C_3$-$C_8$)-Cycloalkyl, Phenyl, Biphenylyl oder Naphthyl,

wobei die Aromaten nicht substituiert oder substituiert sind mit 1 - 3 Substituenten ausgewählt aus der Gruppe bestehend aus F, Cl, $CF_3$, Methyl, Methoxy und NR(10)R(11),

R(10) und R(11)

H, $(C_1-C_4)$-Alkyl oder $(C_1-C_4)$-Perfluoralkyl;

R(7) H, $(C_1-C_8)$-Alkyl, $(C_1-C_8)$-Perfluoralkyl, $(C_3-C_8)$-Alkenyl oder $-C_nH_{2n}$-R(12);

n Null, 1, 2, 3 oder 4;
R(12) $(C_3-C_8)$-Cycloalkyl, Phenyl, Biphenylyl oder Naphthyl,

wobei die Aromaten nicht substituiert oder substituiert sind mit 1 - 3 Substituenten ausgewählt aus der Gruppe bestehend aus F, Cl, $CF_3$, Methyl, Methoxy und NR(13)R(14);
R(13) und R(14)

H, $(C_1-C_4)$-Alkyl oder $(C_1-C_4)$-Perfluoralkyl;

R(8) H, $(C_1-C_4)$-Alkyl oder $(C_1-C_4)$-Perfluoralkyl;
oder

R(7) und R(8) gemeinsam

4 oder 5 Methylengruppen, von denen eine $CH_2$-Gruppe durch Sauerstoff, S, NH, N-$CH_3$ oder N-Benzyl ersetzt sein kann;

R(2) wie R(1) definiert, oder H, OH, F, Cl, Br, I, CN, $NO_2$, $(C_1-C_8)$-Alkyl, $(C_1-C_8)$-Perfluoralkyl, $(C_3-C_8)$-Alkenyl oder $-C_nH_{2n}$R(15);

n Null, 1, 2, 3 oder 4;
R(15) $(C_3-C_8)$-Cycloalkyl, Phenyl, Biphenylyl oder Naphthyl,

wobei die Aromaten nicht substituiert oder substituiert sind mit 1 - 3 Substituenten ausgewählt aus der Gruppe bestehend aus F, Cl, $CF_3$, Methyl, Methoxy und NR(16)R(17);
R(16) und R(17)

H, $(C_1-C_4)$-Alkyl oder $(C_1-C_4)$-Perfluoralkyl;

oder
R(2) $(C_1-C_9)$-Heteroaryl,

das über C oder N verknüpft ist und das unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;

oder
R(2) SR(18), -OR(18), -NR(18)R(19) oder -CR(18)R(19)R(20);

R(18) $-C_aH_{2a}-(C_1-C_9)$-Heteroaryl,

das unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino, Dimethylamino;

a Null, 1 oder 2;
R(19) und R(20)

unabhängig voneinander wie R(18) definiert oder Wasserstoff, $(C_1-C_4)$-Alkyl oder $(C_1-C_4)$-Perfluoralkyl;

oder
R(2) R(21)-$SO_m$ oder R(22)R(23)N-$SO_2$-;

m 1 oder 2;

R(21) ($C_1$-$C_8$)-Alkyl, ($C_1$-$C_8$)-Perfluoralkyl, ($C_3$-$C_8$)-Alkenyl oder - $C_nH_{2n}$-R(24);

n Null, 1, 2, 3 oder 4;
R(24) ($C_3$-$C_8$)-Cycloalkyl, Phenyl, Biphenylyl oder Naphthyl,

wobei die Aromaten nicht substituiert oder substituiert sind mit 1 - 3 Substituenten ausgewählt aus der Gruppe bestehend aus F, Cl, $CF_3$, Methyl, Methoxy und NR(27)R(28);
R(27) und R(28)

H, ($C_1$-$C_4$)-Alkyl oder ($C_1$-$C_4$)-Perfluoralkyl;

R(22) H, ($C_1$-$C_8$)-Alkyl, ($C_1$-$C_8$)-Perfluoralkyl, ($C_3$-$C_8$)-Alkenyl oder -$C_nH_{2n}$-R(29);

n Null, 1, 2, 3 oder 4;
R(29) ($C_3$-$C_8$)-Cycloalkyl, Phenyl, Biphenylyl oder Naphthyl,

wobei die Aromaten nicht substituiert oder substituiert sind mit 1 - 3 Substituenten ausgewählt aus der Gruppe bestehend aus F, Cl, $CF_3$, Methyl, Methoxy und NR(30)R(31);
R(30) und R(31)

H, ($C_1$-$C_4$)-Alkyl oder ($C_1$-$C_4$)-Perfluoralkyl;

R(23) Wasserstoff, ($C_1$-$C_4$)-Alkyl oder ($C_1$-$C_4$)-Perfluoralkyl;
oder

R(22) und R(23)

gemeinsam 4 oder 5 Methylengruppen, von denen eine $CH_2$-Gruppe durch Sauerstoff, S, NH, N-$CH_3$ oder N-Benzyl ersetzt sein kann;

oder
R(2) R(33)X-;

X Sauerstoff, S, NR(34), (D =O)A- oder NR(34)C =MN$^{(*)}$R(35)-;

M Sauerstoff oder S;
A Sauerstoff oder NR(34);
D C oder SO;
R(33) ($C_1$-$C_8$)-Alkyl, ($C_3$-$C_8$)-Alkenyl, $(CH_2)_bC_dF_{2d+1}$ oder - $C_nH_{2n}$-R(36);

b Null oder 1;
d 1, 2, 3, 4, 5, 6 oder 7;
n Null, 1, 2, 3, oder 4;
R(36) ($C_3$-$C_8$)-Cycloalkyl, Phenyl, Biphenylyl oder Naphthyl,

wobei die Aromaten nicht substituiert oder substituiert sind mit 1 - 3 Substituenten ausgewählt aus der Gruppe bestehend aus F, Cl, $CF_3$, Methyl, Methoxy und NR(37)R(38);
R(37) und R(38)

H, ($C_1$-$C_4$)-Alkyl oder ($C_1$-$C_4$)-Perfluoralkyl;

R(34) Wasserstoff, ($C_1$-$C_4$)-Alkyl oder ($C_1$-$C_4$)-Perfluoralkyl;
R(35) definiert wie R(33);
oder
R(33) und R(34)

gemeinsam 4 oder 5 Methylengruppen, von denen eine $CH_2$-Gruppe durch Sauerstoff, S, NH, N-$CH_3$ oder N-Benzyl ersetzt sein kann;

wobei A und N(*) an den Phenylkern des Benzoylguanidin-Grundkörpers gebunden sind;

oder
R(2) -SR(40), -OR(40), -NHR(40), -NR(40)R(41), -CHR(40)R(42), -CR(42)R(43)OH, -C≡CR(45), -CR(46)=CR(45) oder $-[CR(47)R(48)]_u$-CO-$[C(R49)R(50)]_v$-R(44);

R(40) und R(41)

unabhängig voneinander $-(CH_2)_p-(CHOH)_q-(CH_2)_r-(CHOH)_t$-R(51) oder - $(CH_2)_p-O-(CH_2-CH_2O)_q$-R(51);
R(51) Wasserstoff oder Methyl;

u 1, 2, 3 oder 4;
v Null, 1, 2, 3 oder 4;

p, q und r

unabhängig voneinander Null, 1, 2, 3 oder 4;

t 1, 2, 3 oder 4;

R(42) und R(43)

unabhängig voneinander Wasserstoff oder $(C_1-C_6)$-Alkyl;

oder
R(42) und R(43)

zusammen mit dem sie tragenden Kohlenstoff-Atom ein $(C_3-C_8)$-Cycloalkyl;

R(44) Wasserstoff, $(C_1-C_6)$-Alkyl, $(C_3-C_8)$-Cycloalkyl, $-C_eH_{2e}$-R(45);

e Null, 1, 2, 3 oder 4;

R(45) Phenyl,

das unsubstituiert oder substituiert ist mit 1 - 3 Substituenten ausgewählt aus der Gruppe bestehend aus F, Cl, $CF_3$, Methyl, Methoxy und NR(52)R(53);
R(52) und R(53)

H oder $(C_1-C_4)$-Alkyl;

oder
R(45) $(C_1-C_9)$-Heteroaryl,

das unsubstituiert oder wie Phenyl substituiert ist;

oder
R(45) $(C_1-C_6)$-Alkyl,

das unsubstituiert oder mit 1 - 3 OH substituiert ist;

R(46), R(47), R(48), R(49) und R(50)

unabhängig voneinander Wasserstoff oder Methyl;

oder

R(2) R(55)-NH-$SO_2$-;

R(55) R(56)R(57)N-(C=Y)-;
Y Sauerstoff, S oder N-R(58);
R(56) und R(57)

unabhängig voneinander Wasserstoff, ($C_1$-$C_8$)-Alkyl, ($C_3$-$C_6$)-Alkenyl oder - $C_fH_{2f}$-R(59);
f Null, 1, 2, 3 oder 4;
R(59) ($C_5$-$C_7$)-Cycloalkyl, Phenyl,

welches unsubstituiert ist oder substituiert mit 1 - 3 Substituenten ausgewählt aus der Gruppe bestehend aus F, Cl, $CF_3$, Methoxy und ($C_1$-$C_4$)-Alkyl;

oder

R(56) und R(57)

gemeinsam 4 oder 5 Methylengruppen, von denen eine $CH_2$-Gruppe durch Sauerstoff, S, NH, N-$CH_3$ oder N-Benzyl ersetzt sein kann;

R(58)

wie R(56) definiert oder gleich Amidin;

R(3), R(4) und R(5) sind unabhängig voneinander wie R(1) oder R(2) definiert,
wobei jedoch mindestens einer der Substituenten R(2), R(3), R(4) und R(5) gleich OH sein muß;
sowie deren pharmazeutisch verträgliche Salze;

(HOE 95/F 007 K - EP-OS 723 956, NZ 280 887)
ag) Benzoylguanidine der Formel I

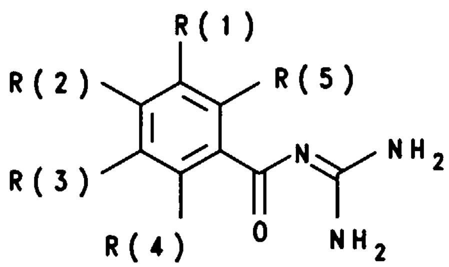

I

worin bedeuten:

einer der drei Substituenten R(1), R(2) und R(3)

R(6)-A-B-D-;
R(6) ein basischer protonierbarer Rest, d.h. eine Aminogruppe -NR(7)R(8), eine Amidinogruppe R(7)R(8)N-C[=N-R(9)]- oder eine Guanidinogruppe

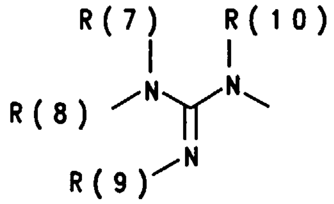

R(7), R(8), R(9) und R(10)

unabhängig voneinander Wasserstoff oder Alkyl mit 1, 2, 3 oder 4 C-Atomen;

oder
R(7) und R(8)

gemeinsam $C_aH_{2a}$;
a 4, 5, 6 oder 7;
wobei im Falle von a = 5, 6 oder 7 eine Methylengruppe der Gruppe $C_aH_{2a}$ durch eine Heteroatomgruppe O, $SO_m$ oder NR(11) ersetzt sein kann,

oder
R(8) und R(9) oder R(9) und R(10) oder R(7) und R(10)

eine Gruppe $C_aH_{2a}$;
a 2, 3, 4 oder 5;

wobei im Falle von a = 3, 4 oder 5 eine Methylengruppe der Gruppe $C_aH_{2a}$ durch eine Heteroatomgruppe O, $SO_m$ oder NR(11) ersetzt sein kann;

m Null, 1 oder 2;
R(11) Wasserstoff oder Methyl;

oder
R(6) ein basisches heteroaromatisches Ringsystem mit 1 - 9 C-Atomen;
A $C_bH_{2b}$;

b 1, 2, 3, 4, 5, 6, 7, 8, 9 oder 10;

wobei in der Gruppe $C_bH_{2b}$ ein oder zwei Methylengruppen durch eine der Gruppierungen ausgewählt aus der Gruppe bestehend aus -O-,-CO-, CH[OR(20)]-, $-SO_m-$, -NR(20)-, -NR(20)-CO-, -NR(20)-CO-NH-, $-NR(20)-CO-NH-SO_2-$und

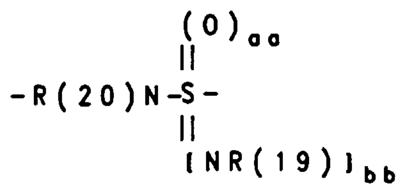

$-SO_{aa}[NR(19)]_{bb}-$ ersetzt sein können;
und wobei in der Gruppe $C_bH_{2b}$ eine Methylengruppe durch -CH-R(99) ersetzt sein kann, wobei R(99) gemeinsam mit R(7) einen Pyrrolidin- oder Piperidin-Ring bildet;
aa 1 oder 2;
bb 0 oder 1;
aa + bb = 2 ;
R(19) Wasserstoff oder Alkyl mit 1, 2, 3 oder 4 C-Atomen
R(20) Wasserstoff oder Methyl;

B einen Phenylen- oder Naphthylenrest,

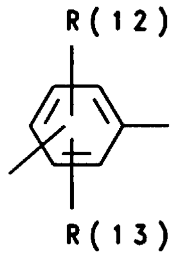

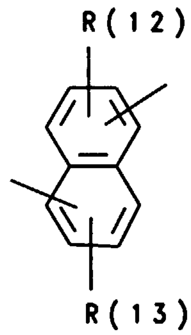

R(12) und R(13)

unabhängig voneinander Wasserstoff, Methyl, F, Cl, Br, J, $CF_3$ oder $-SO_w$-R(14);
R(14) Methyl oder NR(15)R(16);
R(15) und R(16)

unabhängig voneinander Wasserstoff oder Alkyl mit 1, 2, 3 oder 4 C-Atomen;

w Null, 1 oder 2;

D $-C_dH_{2d}-X_f-$;

d Null, 1, 2, 3 oder 4;
X -O-,-CO-, -CH[OR(21)]-, $-SO_m-$ oder -NR(21)-;
f Null oder 1;

R(21) Wasserstoff oder Methyl;
m Null, 1 oder 2;

und die jeweils anderen Substituenten R(1) und R(2) und R(3)

unabhängig voneinander Wasserstoff, F, Cl, Br, J, -CN, $-(C_1-C_8)$-Alkyl, $-(C_2-C_8)$-Alkenyl, -NR(35)R(36) oder R(17)-$C_gH_{2g}-Z_h-$;
g Null, 1, 2, 3 oder 4;
h Null oder 1;
R(35) und R(36)

unabhängig voneinander Wasserstoff oder Alkyl mit 1, 2, 3, 4, 5 oder 6 C-Atomen;

oder
R(35) und R(36)

gemeinsam 4 - 7 Methylengruppen, von denen eine $CH_2$-Gruppe durch Sauerstoff, -S-, -NH-, $-NCH_3$ oder -N-Benzyl ersetzt sein kann;

Z -O-,-CO-, $-SO_v-$, -NR(18)-, -NR(18)-CO-, -NR(18)-CO-NH- oder -NR(18)-$SO_2$-;

R(18) Wasserstoff oder Methyl;
v Null, 1 oder 2;

R(17) Wasserstoff, Cycloalkyl mit 3, 5 oder 6 C-Atomen oder $C_kF_{2k+1}$-;

k 1, 2 oder 3,

oder
R(17) Pyrrol-1-yl, Pyrrol-2-yl oder Pyrrol-3-yl,

welches nicht substituiert oder substituiert ist mit 1 - 4 Substituenten ausgewählt aus der Gruppe bestehend aus F, Cl, Br, I, -CN, ($C_2$-$C_8$)-Alkanoyl, ($C_2$-$C_8$)-Alkoxycarbonyl, Formyl, Carboxy, - $CF_3$, Methyl und Methoxy;

oder
R(17) -($C_3$-$C_8$)-Cycloalkyl oder Phenyl,

welches nicht substituiert oder substituiert ist mit 1 - 3 Substituenten ausgewählt aus der Gruppe bestehend aus F und Cl, - $CF_3$, Methyl, Hydroxy, Methoxy, -NR(37)R(38), $CH_3SO_2$- und $H_2NO_2S$-;
R(37) und R(38)

Wasserstoff oder -$CH_3$;

R(4) und R(5)

unabhängig voneinander Wasserstoff, Alkyl mit 1, 2, 3 oder 4 C-Atomen, F, Cl, -OR(32), -NR(33)R(34) oder -$C_rF_{2r+1}$;
R(32), R(33) und R(34)

unabhängig voneinander Wasserstoff oder Alkyl mit 1, 2 oder 3 C-Atomen;

r 1, 2, 3 oder 4;

sowie deren pharmakologisch verträgliche Salze;

(HOE 95/F 072 - EP-OS 738 712, NZ 286 380
ah) Indenoylguanidine der Formel I

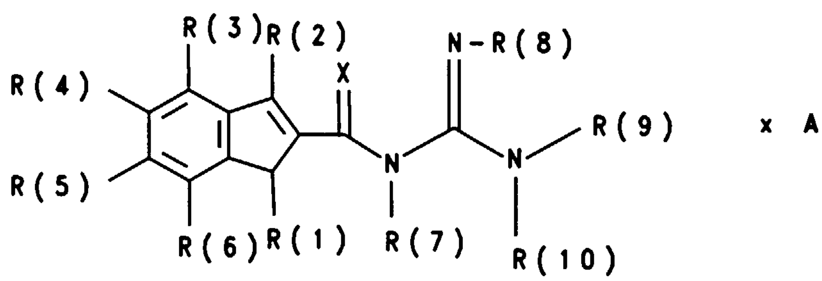

I

worin bedeuten:

R(1) und R(2)

unabhängig voneinander Wasserstoff, Alkyl mit 1, 2, 3, 4, 5, 6, 7, 8, 9 oder 10 C-Atomen, Cycloalkyl mit 3, 4, 5 oder 6 C-Atomen. O-Alkyl mit 1, 2, 3 or 4 C-Atomen, O-C(=O)-Alkyl mit 1, 2, 3 oder 4 C-Atomen oder $C_mH_{2m}$-NR(12)R(13);
R(12) und R(13)

unabhängig voneinander Wasserstoff oder Alkyl mit 1, 2, 3 or 4 C-Atomen;

m Null, 2, 3 oder 4;

NH-C(=O)-$NH_2$, C(=O)-O-Alkyl mit 1, 2, 3 oder 4 C-Atomen, C(=O)-$NH_2$, C(=O)-NH-Alkyl mit 1, 2, 3 oder 4 C-Atomen, C(=O)-N$(Alkyl)_2$ mit 1, 2, 3 oder 4 C-Atomen in jeder Alkylgruppe, Alkenyl mit 2, 3, 4, 5, 6, 7, 8, 9 oder 10 C-Atomen, alkinyl mit 2, 3, 4, 5, 6, 7, 8, 9 oder 10 C-Atomen, Alkylaryl mit 1, 2, 3 or 4 C-Atomen in der Alkylgruppe, Alkenyl-aryl mit 2, 3, 4, 5, 6, 7, 8, 9 oder 10 C-Atomen in der Alkenylgruppe, Alkinyl-aryl mit 2, 3, 4, 5, 6, 7, 8, 9 oder 10 C-Atomen in der Alkinylgruppe, $C_1$-$C_4$-alkyl-substituiertes aryl, $C_1$-$C_4$-Alkyl-heteroaryl, $C_1$-$C_4$-Alkenyl-heteroaryl, Aminoalkyl-aryl mit 1, 2, 3 oder 4 C-Atomen in der Alkylgruppe, substituiertes Aryl, Heteroaryl und substituiertes Heteroaryl;

R(3), R(4), R(5) and R(6)

unabhängig voneinander Wasserstoff, Alkyl mit 1, 2, 3, 4, 5, 6, 7, 8, 9 oder 10 C-Atomen, O-Alkyl mit 1, 2, 3, 4, 5, 6, 7, 8, 9 oder 10 C-Atomen, Halogen, (wie F, Cl, Br, I), OH, Aryl, substituiertes Aryl, Heteroaryl, substituiertes Heteroaryl, O-Niederalkyl, O-Aryl, O-Niederalkyl-aryl, O-substituiertes Aryl, O-Niederalkyl-substituiertes Aryl, O-C(=O)-$C_1$-$C_4$-Alkyl-aryl, O-C(=O)-NH-$C_1$-$C_4$-Alkyl, O-C(=O)-N($C_1$-$C_4$-$Alkyl)_2$, $NO_2$, CN, $CF_3$, $NH_2$, NH-C(=O)-$C_1$-$C_4$-Alkyl, NH-C(=O)-$NH_2$, COOH, C(=O)-O-$C_1$-$C_4$-alkyl, C(=O)-$NH_2$, C(=O)-NH-$C_1$-$C_4$-Alkyl, C(=O)-N($C_1$-$C_4$-$Alkyl)_2$, $C_1$-$C_4$-COOH, $C_1$-$C_4$-Alkyl-C(=O)-O-$C_1$-$C_4$-alkyl, $SO_3H$, $SO_2$-Alkyl, $SO_2$-Alkylaryl, $SO_2$-N-$(Alkyl)_2$, $SO_2$-N(Alkyl)(alkylaryl), C(=O)-R(11), $C_1$-$C_{10}$-Alkyl-C(=O)-R(11), $C_2$-$C_{10}$-Alkenyl-C(=O)-R(11), $C_2$-$C_{10}$-Alkinyl-C(=O)-R(11), NH-C(=O)-$C_1$-$C_{10}$-Alkyl-C(=O)-R(11), O-$C_1$-$C_{11}$-Alkyl-C(=O)-R(11);

R(11) $C_1$-$C_4$-Alkyl, $C_1$-$C_4$-Alkynyl, Aryl, substituiertes Aryl, $NH_2$, NH-$C_1$-$C_4$-Alkyl, N-($C_1$-$C_4$-$Alkyl)_2$, $SO_3H$, $SO_2$-alkyl, $SO_2$-Alkylaryl, $SO_2$-N-$(Alkyl)_2$, $SO_2$-N(Alkyl)(alkylaryl);

X O, S oder NH;

R(7), R(8), R(9) und R(10)

unabhängig voneinander Wasserstoff, Alkyl, Cycloalkyl, Aryl, Alkylaryl;

oder

R(8) und R(9)

gemeinsam Teil eines 5, 6 oder 7-gliedrigen heterocyclischen Rings;

A abwesend oder eine ungiftige organische oder Mineralsäure.

(HOE 95/F 109 - EP 748 795, NZ 286 583)

ai) Benzyloxycarbonylguanidine der Formel I

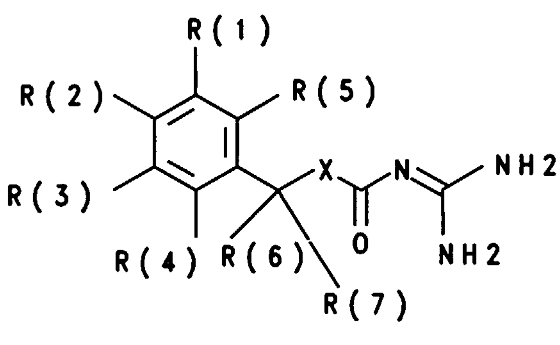

I

worin bedeuten:

R(1), R(2) und R(3)

unabhängig voneinander -Y-[4-R(8)-Phenyl], -Y-[3-R(8)-Phenyl] oder -Y-[2-R(8)-Phenyl],

wobei das Phenyl jeweils unsubstituiert oder substituiert ist mit 1 - 2 Substituenten aus der Gruppe bestehend aus F, Cl, $-CF_3$, Methyl, Hydroxy, Methoxy und -NR(96)R(97);

R(96) und R(97)

unabhängig voneinander Wasserstoff oder $-CH_3$;

Y eine Bindung, $CH_2$, Sauerstoff, -S- oder -NR(9);

R(9) Wasserstoff oder Alkyl mit 1, 2, 3 oder 4 C-Atomen;

R(8) $SO_a[NR(98)]_bNR(99)R(10)$;

a 1 oder 2;
b 0 oder 1;
a + b = 2 ;
R(98), R(99) und R(10)

unabhängig voneinander Wasserstoff, $-(C_1-C_8)$-Alkyl, Benzyl, - $(C_2-C_8)$-Alkylen-NR(11)R(12), $(C_2-C_8)$-Alkylen-NR(13)-$(C_2-C_8)$-Alkylen-NR(37)R(38) oder $(C_0-C_8)$-Alkylen-CR(39)R(40)-CR(41)R(42)$(C_0-C_8)$-Alkylen-NR(43)R(44);
R(11), R(12), R(13), R(37), R(38), R(43) und R(44)

unabhängig voneinander Wasserstoff, $-(C_1-C_8)$-Alkyl oder Benzyl:

R(39), R(40), R(41) und R(42)

unabhängig voneinander Wasserstoff, $-(C_1-C_8)$-Alkyl oder - $(C_0-C_3)$-Alkylen-Phenyl,

wobei das Phenyl nicht substituiert oder substituiert ist mit 1 - 3 Substituenten ausgewählt aus der Gruppe bestehend aus F, Cl,$-CF_3$, Methyl und Methoxy;

oder
R(99) und R(10)

gemeinsam 4 - 6 Methylengruppen, von denen eine $CH_2$-Gruppe durch Sauerstoff, -S-, -NH-, -N-$CH_3$ oder -N-Benzyl ersetzt sein kann;

oder

R(8) $SO_a[NR(98)]_bNR(95)$-C[=N-R(94)]-NR(93)R(92);

R(92), R(93), R(94) und R(95)

unabhängig voneinander Wasserstoff oder Alkyl mit 1, 2, 3 oder 4 C-Atomen;

oder
R(1), R(2) und R(3)

unabhängig voneinander Pyrrol-1-yl, Pyrrol-2-yl oder Pyrrol-3-yl,

welches nicht substituiert oder substituiert ist mit 1 - 4 Substituenten ausgewählt aus der Gruppe bestehend aus F, Cl, Br, I, -CN, $(C_2-C_8)$-Alkanoyl, $(C_2-C_8)$-Alkoxycarbonyl, Formyl, Carboxy, $-CF_3$, Methyl, Methoxy;

oder
R(1), R(2) und R(3)

unabhängig voneinander Wasserstoff, $-(C_1-C_8)$-Alkyl, $-(C_2-C_8)$-Alkenyl oder $-(CH_2)_mR(14)$;
m Null, 1 oder 2;
R(14) $-(C_3-C_8)$-Cycloalkyl oder Phenyl,

welches nicht substituiert oder substituiert ist mit 1 - 3 Substituenten ausgewählt aus der Gruppe bestehend aus F und Cl, - $CF_3$, Methyl, Methoxy und -NR(15)R(16);
R(15) und R(16)

Wasserstoff oder -$CH_3$;

oder
R(1), R(2) und R(3)

unabhängig voneinander -Q-4-[$(CH_2)_k$-CHR(17)-(C=O)R(20)]-Phenyl, -Q-3-$(CH_2)_k$-CHR(17)-(C=O)R(20)]-Phenyl oder -Q-2-[$(CH_2)_k$-CHR(17)-(C=O)R(20)]-Phenyl,

wobei das Phenyl jeweils unsubstituiert oder substituiert ist mit 1 - 2 Substituenten aus der Gruppe F, Cl, -$CF_3$, Methyl, Hydroxy, Methoxy und -NR(35)R(36);
R(35) und R(36)

unabhängig voneinander Wasserstoff oder -$CH_3$;

Q eine Bindung, Sauerstoff, -S- oder -NR(18);

R(18) Wasserstoff oder -($C_1$-$C_4$)-Alkyl;

R(17) -OR(21) oder -NR(21)R(22);

R(21) und R(22)

unabhängig voneinander Wasserstoff, -($C_1$-$C_8$)-Alkyl, -($C_1$-$C_8$)-Alkanoyl, -($C_1$-$C_8$)-Alkoxycarbonyl, Benzyl, Benzyloxycarbonyl;

oder
R(21) Trityl;

R(20) -OR(23) oder -NR(23)R(24);

R(23), R(24) unabhängig voneinander

Wasserstoff, -($C_1$-$C_8$)-Alkyl oder Benzyl;

k Null, 1, 2, 3 oder 4;

oder
R(1), R(2) und R(3)

unabhängig voneinander ($C_1$-$C_9$)-Heteroaryl,

das über C oder N verknüpft ist und das unsubstituiert oder substituiert ist mit 1-3 Substituenten aus der Gruppe F, Cl, $CF_3$, $CH_3$, Methoxy, Hydroxy, Amino, Methylamino und Dimethylamino;

oder
R(1), R(2) und R(3)

-SR(25), -OR(25), -NR(25)R(26), -CR(25)R(26)R(27);
R(25) -$C_fH_{2f}$-($C_1$-$C_9$)-Heteroaryl,

das unsubstituiert oder substituiert ist mit 1-3 Substituenten aus der Gruppe F, Cl, $CF_3$, $CH_3$, Methoxy, Hydroxy, Amino, Methylamino und Dimethylamino;

f Null, 1 oder 2;

R(26) und R(27)

unabhängig voneinander wie R(25) definiert oder Wasserstoff oder ($C_1$-$C_4$)-Alkyl,

oder
R(1), R(2) und R(3)

unabhängig voneinander ($C_1$-$C_9$)-Heteroaryl-N-Oxid,

das über C oder N verknüpft ist und das unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;

oder
R(1), R(2) und R(3)

unabhängig voneinander -SR(28), -OR(28), -NR(28)R(29) oder -CR(28)R(29)R(30);
R(28) -$C_gH_{2g}$-($C_1$-$C_9$)-Heteroaryl-N-Oxid,

das unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;

g Null, 1 oder 2;
R(29), R(30)

unabhängig voneinander wie R(28) definiert, Wasserstoff oder ($C_1$-$C_4$)-Alkyl;

oder
R(1), R(2) und R(3)

unabhängig voneinander Wasserstoff, F, Cl, Br, I, -C≡N, T-$(CH_2)_h$-$(C_iF_{2i+1})$, R(31)$SO_l$-, R(32)R(33)N-CO-, R(34)-CO- oder R(45)R(46)N-$SO_2$, wobei die Perfluoralkylgruppe geradkettig oder verzweigt ist;
T eine Bindung, Sauerstoff, -S- oder -NR(47);
l Null, 1 oder 2;
h Null, 1 oder 2;
i 1, 2, 3, 4, 5 oder 6;
R(31), R(32), R(34) und R(45)

unabhängig voneinander -($C_1$-$C_8$)-Alkyl, -($C_3$-$C_6$)Alkenyl, $(CH_2)_n$R(48) oder - $CF_3$;
n Null, 1, 2, 3 oder 4;
R(47) Wasserstoff oder Alkyl mit 1, 2 oder 3 C-Atomen;
R(48) -($C_3$-$C_7$)-Cycloalkyl oder Phenyl,

welches nicht substituiert ist oder substituiert mit 1 - 3 Substituenten ausgewählt aus der Gruppe bestehend aus F, Cl, -$CF_3$, Methyl, Methoxy und -NR(49)R(50);

R(49) und R(50)

Wasserstoff oder Alkyl mit 1, 2, 3 oder 4 C-Atomen;

oder
R(32), R(34) und R(45)

Wasserstoff;

R(33) und R(46)

unabhängig voneinander Wasserstoff oder Alkyl mit 1, 2, 3 oder 4 C-Atomen;

oder

R(32) und R(33) sowie R(45) und R(46)

gemeinsam 5 oder 6 Methylengruppen, von denen eine $CH_2$-Gruppe durch Sauerstoff, -S-, -NH-, -$NCH_3$ oder -N-Benzyl ersetzt sein kann;

oder

R(1), R(2) und R(3)

unabhängig voneinander R(51)-A-G-D-;

R(51) ein basischer protonierbarer Rest, d.h. eine Aminogruppe -NR(52)R(53), eine Amidinogruppe R(52)R(53)N-C[=N-R(54)]- oder eine Guanidinogruppe R(52)R(53)N-C[=N-R(54)]-NR(55)-;

R(52), R(53), R(54) und R(55)

unabhängig voneinander Wasserstoff oder Alkyl mit 1, 2, 3 oder 4 C-Atomen;

oder

R(52) und R(53)

eine Gruppe $C_\alpha H_{2\alpha}$;

$\alpha$ 4, 5, 6 oder 7;

wobei im Falle von $\alpha$ = 5, 6 oder 7 ein C-Atom der Gruppe $C_\alpha H_{2\alpha}$ durch eine Heteroatomgruppe O, $SO_d$ oder NR(56) ersetzt sein kann,

oder

R(53) und R(54) oder R(54) und R(55) oder R(52) und R(55)

eine Gruppe $C_yH_{2y}$;

y 2, 3, 4 oder 5;

wobei im Falle von y = 3, 4 oder 5 ein C-Atom der Gruppe $C_yH_{2y}$ durch eine Heteroatomgruppe O, $SO_d$ oder NR(56) ersetzt sein kann;

d Null, 1 oder 2;

R(56) Wasserstoff oder Methyl;

oder

R(51) ein basisches heteroaromatisches Ringsystem mit 1 - 9 C-Atomen;

A eine Gruppe $C_eH_{2e}$;

e Null, 1, 2, 3, 4, 5, 6, 7, 8, 9 oder 10;

wobei in der Gruppe $C_eH_{2e}$ ein C-Atom durch eine der Gruppierungen - O-, - CO-, -CH[OR(57)]-, -$SO_r$-, -NR(57)-, -NR(57)-CO-, -NR(57)-CO-NH-, - NR(57)-CO-NH-$SO_2$- oder -NR(57)-$SO_2$- ersetzt sein kann;

r Null, 1 oder 2;

G einen Phenylenrest,

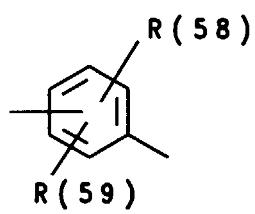

R(58) und R(59)

unabhängig voneinander Wasserstoff, Methyl, Methoxy, F, Cl, Br, J, $CF_3$ oder $-SO_s$-R(60);
R(60) Methyl oder NR(61)R(62);

R(61) und R(62)

unabhängig voneinander Wasserstoff oder Alkyl mit 1, 2, 3 oder 4 C-Atomen;

D $-C_vH_{2v}-E_w-$;

v Null, 1, 2, 3 oder 4;
E -O-,-CO-, -CH[OR(63)]-, $-SO_{aa}-$ oder -NR(63)-;
w Null oder 1;
aa Null, 1 oder 2

R(63) Wasserstoff oder Methyl,

oder
R(1), R(2) und R(3)

unabhängig voneinander $-CF_2$R(64), -CF[R(65)][R(66)], $-CF[(CF_2)_q-CF_3)]$[R(65)], $-C[(CF_2)_p-CF_3]$=CR(65)R(66);
R(64) Alkyl mit 1,2,3 oder 4 C-Atomen oder Cycloalkyl mit 3, 4, 5, 6 oder 7 C-Atomen;
R(65) und R(66) unabhängig voneinander Wasserstoff oder Alkyl mit 1, 2, 3 oder 4 C-Atomen;
q Null, 1 oder 2;
p Null, 1 oder 2;

oder
R(1), R(2) und R(3)

unabhängig voneinander -OR(67) oder -NR(67)R(68);
R(67) und R(68)

unabhängig voneinander Wasserstoff oder Alkyl mit 1, 2, 3, 4, 5 oder 6 C-Atomen;

oder
R(67) und R(68)

gemeinsam 4, 5, 6 oder 7 Methylengruppen, von denen eine $CH_2$-Gruppe durch Sauerstoff, -S-, $SO_2$, -NH-, $-NCH_3$ oder -N-Benzyl ersetzt sein kann;

R(4) und R(5)

unabhängig voneinander Wasserstoff, Alkyl mit 1, 2, 3 oder 4 C-Atomen, F, Cl, -OR(69), -NR(70)R(71) oder $-C_zF_{2z+1}$;
R(69), R(70) und R(71)

unabhängig voneinander Wasserstoff oder Alkyl mit 1, 2 oder 3 C-Atomen;

z 1, 2, 3 oder 4;

R(6) und R(7)

unabhängig voneinander Wasserstoff oder Alkyl mit 1, 2, 3 oder 4 C-Atomen;

X Sauerstoff oder NR(72);

R(72) Wasserstoff oder Alkyl mit 1, 2, 3 oder 4 C-Atomen;

sowie deren pharmazeutisch verträgliche Salze;

(HOE 95/F 115 - EP 744 397, NZ 286 622)
ak) Fluorphenylgruppen tragende Alkenylcarbonsäure-guanidide der Formel I

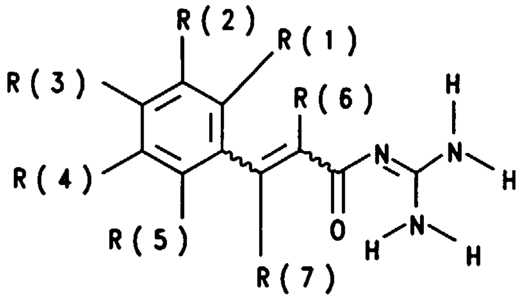

I

worin bedeuten:

R(6) Wasserstoff, ($C_1$-$C_8$)-Alkyl, ($C_3$-$C_8$)-Cycloalkyl oder Phenyl,

wobei die Phenylgruppe nicht substituiert oder substituiert ist mit 1 - 3 Substituenten ausgewählt aus der Gruppe bestehend aus F, Cl, $CF_3$, Methyl, Methoxy und NR(9)R(10);
R(9) und R(10)

Wasserstoff, ($C_1$-$C_4$)-Alkyl oder ($C_1$-$C_4$)-Perfluoralkyl;

R(7) unabhängig wie R(6) definiert;
R(1), R(2), R(3), R(4) und R(5)

unabhängig voneinander Wasserstoff oder F;
wobei jedoch mindestens einer der Reste R(1), R(2), R(3), R(4) und R(5) Fluor sein muß;

sowie deren pharmazeutisch verträgliche Salze;

(HOE 95/F 167 - NZ 299 015)
al) Benzoylguanidine der Formel I

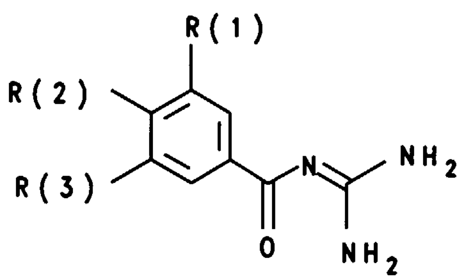

I

worin bedeuten:

R(1) R(4)-$SO_m$ oder R(5)R(6)N-$SO_2$-;

m 1 oder 2;
R(4) und R(5)

unabhängig voneinander Alkyl mit 1, 2, 3, 4, 5, 6, 7 oder 8 C-Atomen, Alkenyl mit 3, 4, 5 oder 6 C-Atomen, $CF_3$ oder $-C_nH_{2n}$-R(7);
n Null, 1, 2, 3 oder 4;
R(6) H oder Alkyl mit 1, 2, 3 oder 4 C-Atomen;
R(7) Cycloalkyl mit 3, 4, 5, 6, 7 oder 8 C-Atomen oder Phenyl,

welches nicht substituiert ist oder substituiert mit 1 - 3 Substituenten ausgewählt aus der Gruppe bestehend aus F, Cl, $CF_3$, Methyl, Methoxy und NR(8)R(9);
R(8) und R(9)

H oder Alkyl mit 1, 2, 3 oder 4 C-Atomen;

oder

R(5) auch Wasserstoff;
oder
R(5) und R(6)

gemeinsam 4 oder 5 Methylengruppen, von denen eine $CH_2$-Gruppe durch Sauerstoff, S, NH, N-$CH_3$ oder N-Benzyl ersetzt sein kann;

oder
R(1) $-O_p-(CH_2)_q-(CF_2)_r-CF_3$;

p Null oder 1;
q Null, 1 oder 2;
r Null, 1, 2 oder 3;

oder
R(1) -SR(10), -OR(10) oder-CR(10)R(11)R(12);

R(10), R(11) und R(12)

unabhängig voneinander Wasserstoff, Alkyl mit 1, 2, 3, 4, 5, 6, 7 oder 8 C-Atomen, $-C_sH_{2s}-(C_3-C_8)$-Cycloalkyl oder ein aromatisches System ausgewählt aus der Gruppe bestehend aus Pyridyl, Pyrrolyl, Chinolyl, Isochinolyl, Imidazolyl oder Phenyl;

s Null, 1 oder 2;

wobei die aromatischen Systeme Pyridyl, Pyrrolyl, Chinolyl, Isochinolyl, Imidazolyl und Phenyl unsubstituiert oder substituiert sind mit 1 - 3 Substituenten ausgewählt aus der Gruppe bestehend aus F, Cl, $CF_3$, $CH_3$, Methoxy, Hydroxy, Amino, Methylamino und Dimethylamino;

R(2) $-(CH_2)_u-(CF_2)_t-CF_3$;

t Null, 1, 2 oder 3;
u Null oder 1;

R(3) Wasserstoff oder unabhängig wie R(1) definiert;
sowie deren pharmazeutisch verträgliche Salze;

(HOE 95/F 173 - NZ 299 052)
am) Substituierte Zimtsäureguanidide der Formel I

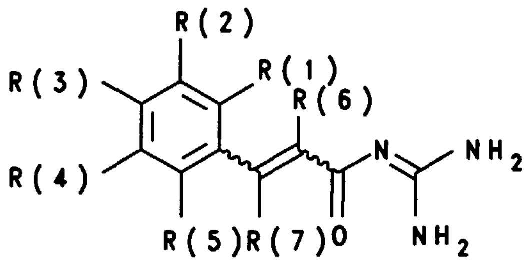

I

worin bedeuten:

mindestens einer der Substituenten R(1), R(2), R(3), R(4) und R(5)

$-X_a-Y_b-L_n-U$;
X CR(16)R(17), O, S oder NR(18);

R(16), R(17) und R(18)

unabhängig voneinander H, Alkyl mit 1, 2, 3 oder 4 C-Atomen oder Perfluoralkyl mit 1, 2, 3 oder 4 C-Atomen;

a Null oder 1;
Y Alkylen mit 1, 2, 3, 4, 5, 6, 7 oder 8 C-Atomen, Alkylen-T mit 1, 2, 3, 4, 5, 6, 7 oder 8 C-Atomen in der Alkylengruppe, T, T-Alkylen mit 1, 2, 3, 4, 5, 6, 7 oder 8 C-Atomen in der Alkylengruppe;

T NR(20), O, S oder Phenylen,

wobei das Phenylen nicht substituiert oder substituiert ist mit 1 - 3 Substituenten ausgewählt aus der Gruppe bestehend aus F, Cl, $CF_3$, Methyl, Methoxy und NR(21)R(22);
R(20), R(21) und R(22)

unabhängig voneinander H, Alkyl mit 1, 2, 3 oder 4 C-Atomen oder Perfluoralkyl mit 1, 2, 3 oder 4 C-Atomen;

b Null oder 1;
L O, S, NR(23) oder $C_kH_{2k}$;

k 1, 2, 3, 4, 5, 6, 7, 8;

n Null oder 1;
U NR(24)R(25) oder einen N-haltigen Heterocyclus mit 1, 2, 3, 4, 5, 6, 7, 8 oder 9 C-Atomen;

R(24) und R(25)

unabhängig voneinander Wasserstoff, Alkyl mit 1, 2, 3, 4, 5, 6, 7 oder 8 C-Atomen, Perfluoralkyl mit 1, 2, 3, 4, 5, 6, 7 oder 8 C-Atomen;

oder
R(24) und R(25)

gemeinsam 4 oder 5 Methylenguppen, von denen eine $CH_2$-Gruppe durch Sauerstoff, S, NH, N-$CH_3$ oder N-Benzyl ersetzt sein kann;

wobei die N-haltigen Heterocyclen N- oder C-verbrückt sind und nicht substituiert oder substituiert sind mit 1 - 3 Substituenten ausgewählt aus der Gruppe bestehend aus F, Cl, $CF_3$, Methyl, Methoxy und NR(27)R(28);

R(23), R(27) und R(28)

unabhängig voneinander H, Alkyl mit 1, 2, 3 oder 4 C-Atomen oder Perfluoralkyl mit 1, 2, 3 oder 4 C-Atomen;

und die jeweils anderen Substituenten R(1), R(2), R(3), R(4) und R(5)

unabhängig voneinander H, F, Cl, Br, I, CN, $-O_n-C_mH_{2m+1}$, $-O_p-(CH_2)_s-C_qF_{2q+1}$ oder $-C_rH_{2r}R(10)$;
n Null oder 1;
m Null 1, 2, 3, 4, 5, 6, 7 oder 8;
p Null oder 1;
q 1, 2, 3, 4, 5, 6, 7 oder 8;
s Null, 1, 2, 3 oder 4;
r Null, 1, 2, 3 oder 4;
R(10)

Cycloalkyl mit 3, 4, 5, 6, 7 oder 8 C-Atomen, oder Phenyl,

wobei das Phenyl nicht substituiert oder substituiert ist mit 1 - 3 Substituenten ausgewählt aus der Gruppe bestehend aus F, Cl, $CF_3$, Methyl, Methoxy und NR(11)R(12);
R(11) und R(12)

unabhängig voneinander H, Alkyl mit 1, 2, 3 oder 4 C-Atomen oder Perfluoralkyl mit 1, 2, 3 oder 4 C-Atomen;

R(6) und R(7)

unabhängig voneinander Wasserstoff, F, Cl, Br, I, CN, Alkyl mit 1, 2, 3, 4, 5, 6, 7 oder 8 C-Atomen, Perfluoralkyl mit 1, 2, 3, 4, 5, 6, 7 oder 8 C-Atomen, Cycloalkyl mit 3, 4, 5, 6, 7 oder 8 C-Atomen, oder Phenyl,

das nicht substituiert oder substituiert ist mit 1 - 3 Substituenten ausgewählt aus der Gruppe bestehend aus F, Cl, $CF_3$, Methyl, Methoxy und NR(14)R(15);
R(14) und R(15)

unabhängig voneinander H, Alkyl mit 1, 2, 3 oder 4 C-Atomen oder Perfluoralkyl mit 1, 2, 3 oder 4 C-Atomen;

sowie deren pharmazeutisch verträgliche Salze;

(HOE 95/F 220 - NZ 299 052)
an) Benzoylguanidine der Formel I

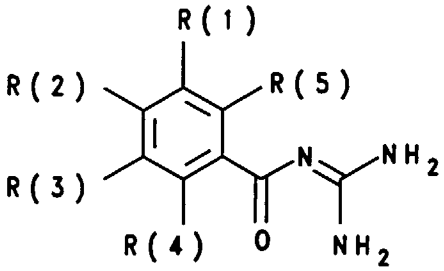

I

worin bedeuten:

mindestens einer der Substituenten R(1), R(2) und R(3)

$R(6)-C(OH)_2-$;

R(6) Perfluoralkyl mit 1, 2 oder 3-C-Atomen, das geradkettig oder verzweigt ist;

und die übrigen Substituenten R(1), R(2) und R(3)

unabhängig voneinander Wasserstoff, OH, F, Cl, Br, I, Alkyl mit 1, 2, 3, 4, 5 oder 6 C-Atomen, Cycloalkyl mit 3, 4, 5 oder 6 C-Atomen, Alkoxy mit 1, 2, 3 oder 4 C-Atomen oder Phenoxy,

das unsubstituiert ist oder substituiert ist mit 1 - 3 Substituenten ausgewählt auf der Gruppe bestehend aus F, Cl, Methyl und Methoxy;

oder
die übrigen Substituenten R(1), R(2) und R(3)

unabhängig voneinander Alkyl-$SO_x$, -CR(7)=CR(8)R(9) oder -C≡CR(9);
x Null, 1 oder 2;
R(7) Wasserstoff oder Methyl;
R(8) und R(9)

unabhängig voneinander Wasserstoff, Alkyl mit 1, 2, 3 oder 4 C-Atomen, Cycloalkyl mit 3, 4, 5, 6, 7 oder 8 C-Atomen oder Phenyl,

das unsubstituiert ist oder substituiert ist mit 1 - 3 Substituenten ausgewählt auf der Gruppe bestehend aus F, Cl, $CF_3$, Methyl und Methoxy;

oder
die übrigen Substituenten R(1), R(2) und R(3)

unabhängig voneinander Phenyl, $C_6H_5$-($C_1$-$C_4$)-Alkyl, Naphthyl, Biphenylyl, Chinolinyl, Isochinolinyl oder Imidazolyl,

wobei Chinolinyl, Isochinolinyl oder Imidazolyl über C oder N gebunden sind, und wobei Phenyl, $C_6H_5$-($C_1$-$C_4$)-Alkyl, Naphthyl, Biphenylyl, Chinolinyl, Isochinolinyl und Imidazolyl unsubstituiert sind oder substituiert sind mit 1 - 3 Substituenten ausgewählt aus der Gruppe bestehend aus F, Cl, $CF_3$, $CH_3$, Methoxy, Hydroxy, Amino, Methylamino und Dimethylamino;

oder
die übrigen Substituenten R(1), R(2) und R(3)

unabhängig voneinander SR(10), -OR(10), -CR(10)R(11)R(12);
R(10)

-$C_fH_{2f}$-($C_3$-$C_8$)-Cycloalkyl, Chinolinyl, Isochinolinyl, Pyridinyl, Imidazolyl oder Phenyl,

wobei die aromatischen Systeme Chinolinyl, Isochinolinyl, Pyridinyl, Imidazolyl und Phenyl unsubstituiert sind oder substituiert sind mit 1 - 3 Substituenten ausgewählt aus der gruppe bestehend aus F, Cl, $CF_3$, $CH_3$, Methoxy, Hydroxy, Amino, Methylamino und Dimethylamino;

f Null, 1 oder 2;

R(11) und R(12)

unabhängig voneinander wie R(10) definiert, Wasserstoff oder Alkyl mit 1, 2, 3 oder 4 C-Atomen;

R(4) und R(5)

unabhängig voneinander Wasserstoff, Alkyl mit 1, 2 oder 3-C-Atomen, F, Cl, Br, I, CN, OR(13), NR(14)R(15), -$(CH_2)_n$-$(CF_2)_o$-$CF_3$;
R(13), R(14) und R(15)

unabhängig voneinander Wasserstoff oder Alkyl mit 1, 2, 3 oder 4 C-Atomen;

n Null oder 1;
o Null, 1 oder 2;

sowie deren pharmakologisch akzeptable Salze;

(HOE 95/F 253 - NZ 299 682)
ao) Sulfonimidamide der Formel I

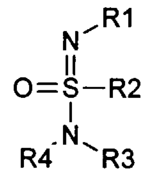

I

worin bedeuten:

mindestens einer der drei Substituenten R(1), R(2) und R(3)

ein Benzoylguanidin,

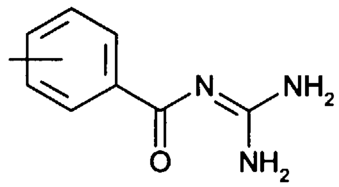

das im Phenylteil unsubstituiert oder substituiert ist mit 1 - 4 Resten ausgewählt aus der Gruppe bestehend aus Alkyl mit 1, 2, 3, 4, 5, 6, 7 oder 8 C-Atomen, Alkenyl mit 2, 3, 4, 5, 6, 7 oder 8 C-Atomen, $-(CH_2)_m$-R(14), F, Cl, Br, I, -C≡N, $CF_3$, R(22)$SO_2$-, R(23)R(24)N-CO-, R(25)-CO-, R(26)R(27)N-$SO_2$, -OR(35), -SR(35) oder -NR(35)R(36);
m Null, 1 oder 2;
R(14)

-($C_3$-$C_8$)-Cycloalkyl oder Phenyl,

welches nicht substituiert oder substituiert ist mit 1 - 3 Substituenten ausgewählt aus der Gruppe bestehend aus F und Cl, $-CF_3$, Methyl, Methoxy und -NR(15)R(16);
R(15) und R(16)

unabhängig voneinander Wasserstoff oder $-CH_3$;

R(22), R(23), R(25) und R(26)

unabhängig voneinander Alkyl mit 1, 2, 3, 4, 5, 6, 7 oder 8 C-Atomen, Alkenyl mit 2, 3, 4, 5, 6, 7 oder 8 C-Atomen, $(CH_2)_n$R(29) oder $-CF_3$;
n Null, 1, 2, 3 oder 4;
R(29) -($C_3$-$C_7$)-Cycloalkyl oder Phenyl,

welches nicht substituiert ist oder substituiert mit 1 - 3 Substituenten ausgewählt aus der

Gruppe bestehend aus F, Cl, $-CF_3$, Methyl, Methoxy und -NR(30)R(31);
R(30) und R(31)

Wasserstoff oder Alkyl mit 1, 2, 3 oder 4 C-Atomen;

oder
R(23), R(25) und R(26)

Wasserstoff;

R(24) und R(27)

unabhängig voneinander Wasserstoff oder Alkyl mit 1, 2, 3 oder 4 C-Atomen;

oder
R(23) und R(24) sowie R(26) und R(27)

gemeinsam 5 oder 6 Methylengruppen, von denen eine $CH_2$-Gruppe durch Sauerstoff, -S-, -NH-, $-NCH_3$ oder -N-Benzyl ersetzt sein kann;

R(35) und R(36)

unabhängig voneinander Wasserstoff oder Alkyl mit 1, 2, 3, 4, 5 oder 6 C-Atomen;

oder
R(35) und R(36)

gemeinsam 4 - 7 Methylengruppen, von denen eine $CH_2$-Gruppe durch Sauerstoff, -S-, -NH-, $-NCH_3$ oder -N-Benzyl ersetzt sein kann;

oder
R(35) Phenyl,

welches nicht substituiert ist oder substituiert mit 1 - 3 Substituenten ausgewählt aus der Gruppe bestehend aus F, Cl, $-CF_3$, Methyl, Methoxy, $SO_2R(5)$, $SO_2NR(6)R(7)$ und -NR(32)R(33);
R(5) Alkyl mit 1, 2, 3, 4, 5 oder 6 C-Atomen
R(6) und R(7)

unabhängig voneinander Wasserstoff oder Alkyl mit 1, 2, 3 oder 4 C-Atomen;

R(32) und R(33)

unabhängig voneinander Wasserstoff oder Alkyl mit 1, 2, 3 oder 4 C-Atomen;

oder
R(35) $C_1$-$C_9$-Heteroaryl,

das unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;

und die jeweils anderen Substituenten R(1), R(2) und R(3)

unabhängig voneinander Alkyl mit 1, 2, 3, 4, 5, 6, 7 oder 8 C-Atomen, $(CH_2)_pR(10)$
p Null, 1, 2, 3 oder 4;
R(10) Phenyl,

welches nicht substituiert ist oder substituiert mit 1 - 3 Substituenten ausgewählt aus der Gruppe bestehend aus F, Cl, $-CF_3$, Methyl, Methoxy, $-SO_2NR(17)R(8)$ und $-SO_2R(9)$;

R(17) und R(8)

unabhängig voneinander Wasserstoff oder Alkyl mit 1, 2, 3 oder 4 C-Atomen;

R(9) Alkyl mit 1, 2, 3 oder 4 C-Atomen;

oder
der jeweils andere Rest R(1) und R(3)

Wasserstoff,

R(4) Wasserstoff oder Alkyl mit 1, 2, 3 oder 4 C-Atomen;
sowie deren pharmazeutisch verträgliche Salze;

(HOE 95/F 265 - NZ 299 739)
ap) Benzoylguanidine der Formel I

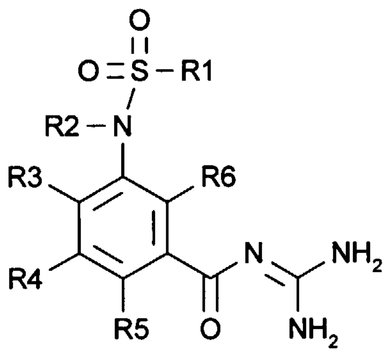

I

worin bedeuten:

R(1) Alkyl mit 1, 2, 3, 4, 5, 6, 7 oder 8 C-Atomen oder NR(7)R(8);

R(7) und R(8)

unabhängig voneinander Wasserstoff oder Alkyl mit 1, 2, 3, 4, 5, 6, 7 oder 8 C-Atomen

R(2) Wasserstoff, Alkyl mit 1, 2, 3, 4, 5, 6, 7 oder 8 C-Atomen oder - $SO_2R(9)$

R(9) unabhängig wie R(1) definiert;

R(3) Wasserstoff, -SR(25), -OR(25), -NR(25)R(26) oder -CR(25)R(26)R(27);

R(25) Wasserstoff, Alkyl mit 1, 2, 3, 4, 5, 6, 7 oder 8 C-Atomen, oder Phenyl,

das unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;

oder
R(25)

-($C_1$-$C_9$)-Heteroaryl,

das unsubstituiert oder substituiert ist mit 1 - 3 Substituenten ausgewählt aus der Gruppe bestehend aus der Gruppe F, Cl, $CF_3$, $CH_3$, Methoxy, Hydroxy, Amino, Methylamino und Dimethylamino;

R(26) und R(27)

unabhängig voneinander wie R(25) definiert oder Wasserstoff oder Alkyl mit 1, 2, 3, 4, 5, 6, 7 oder 8 C-Atomen;

R(4) Wasserstoff, F, Cl, Br, I, OH, -C≡N, $CF_3$, Alkyl mit 1, 2, 3, 4, 5, 6, 7 oder 8 C-Atomen, Alkenyl mit 2, 3, 4, 5, 6, 7 oder 8 C-Atomen oder $-(CH_2)_mR(14)$;

m Null, 1 oder 2;
R(14) $-(C_3-C_8)$-Cycloalkyl oder Phenyl,

welches nicht substituiert oder substituiert ist mit 1 - 3 Substituenten ausgewählt aus der Gruppe bestehend aus F und Cl, - $CF_3$, Methyl, Methoxy und -NR(15)R(16);
R(15) und R(16)

unabhängig voneinander Wasserstoff oder $-CH_3$;

R(5) und R(6)

unabhängig voneinander Wasserstoff, Alkyl mit 1, 2, 3 oder 4 C-Atomen, F, Cl, -OR(32), -NR(33)R(34) oder $CF_3$;
R(32), R(33) und R(34)

unabhängig voneinander Wasserstoff oder Alkyl mit 1, 2, 3 oder 4 C-Atomen;

sowie deren pharmazeutisch verträgliche Salze;

(HOE 95/F 269 K - EP 774 458)
aq) Benzoldicarbonsäure-diguanidide der Formel I

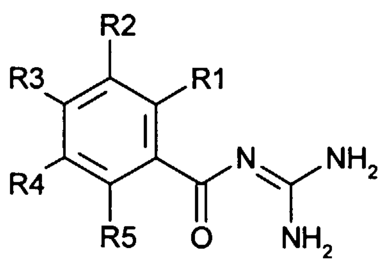

I

worin bedeuten:

einer der Reste R(1), R(2), R(3) und R(4)

$-CO-N=C(NH_2)_2$;

und die jeweils anderen Reste R(1), R(2), R(3) und R(4):
R(1) Wasserstoff, Alkyl mit 1, 2, 3 oder 4 C-Atomen, F, Cl, Br, I, - OR(32), -NR(33)R(34) oder $CF_3$;

R(32), R(33) und R(34)

unabhängig voneinander Wasserstoff oder Alkyl mit 1, 2, 3 oder 4 C-Atomen;

R(2) und R(4)

unabhängig voneinander Wasserstoff, F, Cl, Br, I, OH, -CN, $CF_3$, $-CO-N=C(NH_2)_2$, Alkyl mit 1, 2, 3, 4, 5, 6, 7 oder 8 C-Atomen, Alkenyl mit 2, 3, 4, 5, 6, 7 oder 8 C-Atomen oder $-(CH_2)_mR(14)$;

m Null, 1 oder 2;

R(14) -($C_3$-$C_8$)-Cycloalkyl oder Phenyl,

welches nicht substituiert oder substituiert ist mit 1 - 3 Substituenten ausgewählt aus der Gruppe bestehend aus F und Cl, - $CF_3$, Methyl, Methoxy und -NR(15)R(16);

R(15) und R(16)

Wasserstoff oder -$CH_3$;

oder

R(2) und R(4)

unabhängig voneinander Pyrrol-1-yl, Pyrrol-2-yl oder Pyrrol-3-yl,

welches nicht substituiert oder substituiert ist mit 1 - 4 Substituenten ausgewählt aus der Gruppe bestehend aus F, Cl, Br, I, -CN, ($C_2$-$C_8$)-Alkanoyl, ($C_2$-$C_8$)-Alkoxycarbonyl, Formyl, Carboxy, -$CF_3$, Methyl, Methoxy;

oder

R(2) und R(4)

unabhängig voneinander R(22)-$SO_2$-, R(23)R(24)N-CO-, R(28)-CO-oder R(29)R(30)N-$SO_2$;

R(22) und R(28)

unabhängig voneinander Methyl oder -$CF_3$;

R(23), R(24), R(29) und R(30)

unabhängig voneinander Wasserstoff oder Methyl;

oder

R(2) und R(4)

unabhängig voneinander -OR(35) oder -NR(35)R(36);

R(35) und R(36)

unabhängig voneinander Wasserstoff oder Alkyl mit 1, 2, 3, 4, 5 oder 6 C-Atomen;

oder

R(35) und R(36)

gemeinsam 4 - 7 Methylengruppen, von denen eine $CH_2$-Gruppe durch Sauerstoff, -S-, -NH-, -$NCH_3$ oder -N-Benzyl ersetzt sein kann;

R(3) Wasserstoff, -SR(25), -OR(25), -NR(25)R(26), -CR(25)R(26)R(27);

R(25) Wasserstoff, Alkyl mit 1, 2, 3, 4, 5, 6, 7 oder 8 C-Atomen oder Phenyl,

das unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;

oder

R(25) -($C_1$-$C_9$)-Heteroaryl,

das unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;

R(26) und R(27)

unabhängig voneinander wie R(25) definiert oder Wasserstoff oder Alkyl mit 1, 2, 3, 4, 5, 6, 7 oder 8 C-Atomen;

R(5) Alkyl mit 1, 2, 3 oder 4 C-Atomen, F, Cl, Br, I, X-$(CH_2)_y$-$CF_3$ oder Phenyl,

welches nicht substituiert oder substituiert ist mit 1 - 3 Substituenten ausgewählt aus der Gruppe bestehend aus F und Cl, -$CF_3$, Methyl, Methoxy und -NR(6)R(7);
R(6) und R(7)

unabhängig voneinander Wasserstoff oder -$CH_3$;

X eine Bindung oder Sauerstoff;
y Null, 1 oder 2;

sowie deren pharmazeutisch verträgliche Salze;

(HOE 95/F 269 BK - EP 774 457)
ar) Benzoldicarbonsäure-diguanidide der Formel I

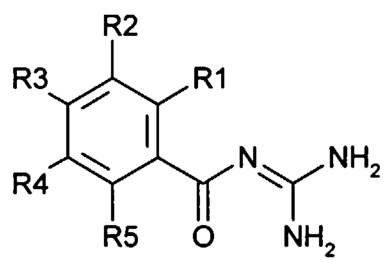

I

worin bedeuten:

einer der Reste R(1), R(2), R(3) und R(5)

-CO-N=$C(NH_2)_2$;

und die jeweils anderen Reste R(1), R(2), R(3) und R(5):
R(1) und R(5)

unabhängig voneinander Wasserstoff, Alkyl mit 1, 2, 3 oder 4 C-Atomen, F, Cl, -OR(32), -NR(33)R(34) oder $CF_3$;
R(32), R(33) und R(34)

unabhängig voneinander Wasserstoff oder Alkyl mit 1, 2, 3 oder 4 C-Atomen;

R(2) Wasserstoff, F, Cl, Br, I, OH, -C°N, $CF_3$, -CO-N=$C(NH_2)_2$, Alkyl mit 1, 2, 3, 4, 5, 6, 7 oder 8 C-Atomen, Alkenyl mit 2, 3, 4, 5, 6, 7 oder 8 C-Atomen oder -$(CH_2)_m$R(14);

m Null, 1 oder 2;
R(14) -($C_3$-$C_8$)-Cycloalkyl oder Phenyl,

welches nicht substituiert oder substituiert ist mit 1 - 3 Substituenten ausgewählt aus der Gruppe bestehend aus F und Cl, - $CF_3$, Methyl, Methoxy und -NR(15)R(16);
R(15) und R(16)

unabhängig voneinander Wasserstoff oder -$CH_3$;

oder
R(2) R(22)-$SO_2$-, R(23)R(24)N-CO-, R(28)-CO- oder R(29)R(30)N-$SO_2$;

R(22) und R(28)

unabhängig voneinander Methyl oder $-CF_3$;

R(23), R(24), R(29) und R(30)

unabhängig voneinander Wasserstoff oder Methyl;

oder
R(2) -OR(35) oder -NR(35)R(36);

R(35) und R(36)

unabhängig voneinander Wasserstoff oder Alkyl mit 1, 2, 3, 4, 5 oder 6 C-Atomen;

oder
R(35) und R(36)

gemeinsam 4 - 7 Methylengruppen, von denen eine $CH_2$-Gruppe durch Sauerstoff, -S-, -NH-, $-NCH_3$ oder -N-Benzyl ersetzt sein kann;

R(3) Wasserstoff, -SR(25), -OR(25), -NR(25)R(26), -CR(25)R(26)R(27);

R(25) Wasserstoff, Alkyl mit 1, 2, 3, 4, 5, 6, 7 oder 8 C-Atomen oder Phenyl,

das unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;

oder
R(25 $-(C_1-C_9)$-Heteroaryl,

das unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;

R(26) und R(27)

unabhängig voneinander wie R(25) definiert oder Wasserstoff oder Alkyl mit 1, 2, 3, 4, 5, 6, 7 oder 8 C-Atomen;

R(4) $CF_3$, Alkyl mit 1 , 2, 3, 4, 5, 6, 7 oder 8 C-Atomen, Alkenyl mit 2, 3, 4, 5, 6, 7 oder 8 C-Atomen, $-(C_3-C_8)$-Cycloalkyl oder $-(CH_2)_m$R(14);

m 1 oder 2;
R(14) $-(C_3-C_8)$-Cycloalkyl oder Phenyl,

welches nicht substituiert oder substituiert ist mit 1 - 3 Substituenten ausgewählt aus der Gruppe bestehend aus F und Cl, - $CF_3$, Methyl, Methoxy und -NR(15)R(16);
R(15) und R(16)

unabhängig voneinander Wasserstoff oder $-CH_3$;

oder
R(4) Phenyl,

welches substituiert ist mit 2, 3, 4 oder fünf Substituenten ausgewählt aus der Gruppe bestehend aus F, Cl, $CF_3$, Methyl, Methoxy und -NR(15)R(16);

R(15) und R(16)

unabhängig voneinander Wasserstoff oder $CH_3$;

sowie deren pharmazeutisch verträgliche Salze;

(HOE 96/F 013 - EP-OS 787 717)
as) Diaryldicarbonsäure-diguanidide der Formel I

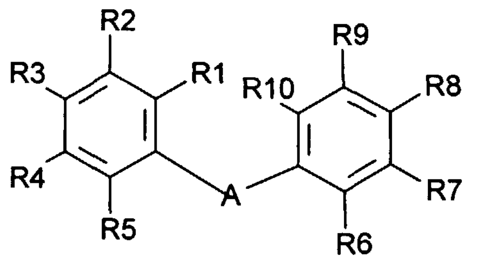

I

worin bedeuten:

einer der Reste R(1), R(2), R(3), R(4) und R(5)

$-CO-N=C(NH_2)_2$;

die jeweils anderen Reste R(1) und R(5)

unabhängig voneinander Wasserstoff, Alkyl mit 1, 2, 3 oder 4 C-Atomen, F, Cl, -OR(32), -NR(33)R(34) oder $CF_3$;
R(32), R(33) und R(34)

unabhängig voneinander Wasserstoff oder Alkyl mit 1, 2, 3 oder 4 C-Atomen;

die jeweils anderen Reste R(2) und R(4)

unabhängig voneinander Wasserstoff, F, Cl, Br, I, OH, -CN, $CF_3$, $-CO-N=C(NH_2)_2$, Alkyl mit 1, 2, 3, 4, 5, 6, 7 oder 8 C-Atomen, Alkenyl mit 2, 3, 4, 5, 6, 7 oder 8 C-Atomen oder $-(CH_2)_m R(14)$;
m Null, 1 oder 2;
R(14) $-(C_3-C_8)$-Cycloalkyl oder Phenyl,

welches nicht substituiert oder substituiert ist mit 1 - 3 Substituenten ausgewählt aus der Gruppe bestehend aus F und Cl, $CF_3$, Methyl, Methoxy und -NR(15)R(16);
R(15) und R(16)

Wasserstoff oder $-CH_3$;

oder
die jeweils anderen Reste R(2) und R(4)

unabhängig voneinander Pyrrol-1-yl, Pyrrol-2-yl oder Pyrrol-3-yl,

welches nicht substituiert oder substituiert ist mit 1 - 4 Substituenten ausgewählt aus der Gruppe bestehend aus F, Cl, Br, I, -CN, $(C_2-C_8)$-Alkanoyl, $(C_2-C_8)$-Alkoxycarbonyl, Formyl, Carboxy, $-CF_3$, Methyl, Methoxy;

oder
die jeweils anderen Reste R(2) und R(4)

$R(22)-SO_2-$, R(23)R(24)N-CO-, R(28)-CO- oder R(29)R(30)N-SO2;
R(22) und R(28)

unabhängig voneinander Methyl oder $-CF_3$;

R(23), R(24), R(29) und R(30)

unabhängig voneinander Wasserstoff oder Methyl;

oder
die jeweils anderen Reste R(2) und R(4)

unabhängig voneinander -OR(35) oder -NR(35)R(36);
R(35) und R(36)

unabhängig voneinander Wasserstoff oder Alkyl mit 1, 2, 3, 4, 5 oder 6 C-Atomen;

oder
R(35) und R(36)

gemeinsam 4 - 7 Methylengruppen, von denen eine $CH_2$-Gruppe durch Sauerstoff, -S-, -NH-, $-NCH_3$ oder -N-Benzyl ersetzt sein kann;

der jeweils andere Rest R(3)

Wasserstoff, -SR(25), -OR(25), -NR(25)R(26), -CR(25)R(26)R(27);
R(25) Wasserstoff, Alkyl mit 1, 2, 3, 4, 5, 6, 7 oder 8 C-Atomen oder Phenyl,

das unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;

oder
R(25) $-(C_1-C_9)$-Heteroaryl,

das unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;

R(26) und R(27)

unabhängig voneinander wie R(25) definiert oder Wasserstoff oder Alkyl mit 1, 2, 3, 4, 5, 6, 7 oder 8 C-Atomen;

einer der Reste R(6), R(7), R(8), R(9) und R(10)

$-CO-N=C(NH_2)_2$;

die jeweils anderen Reste R(6) und R(10)

unabhängig voneinander Wasserstoff, Alkyl mit 1 , 2, 3 oder 4 C-Atomen, F, Cl, -OR(132), -NR(133)R(134) oder $CF_3$;
R(132), R(133) und R(134)

unabhängig voneinander Wasserstoff oder Alkyl mit 1, 2, 3 oder 4 C-Atomen;

die jeweils anderen Reste R(7) und R(9)

unabhängig voneinander Wasserstoff, F, Cl, Br, I, OH, -CN, $CF_3$, $-CO-N=C(NH_2)_2$, Alkyl mit 1 , 2, 3, 4, 5, 6, 7 oder 8 C-Atomen, Alkenyl mit 2, 3, 4, 5, 6, 7 oder 8 C-Atomen oder $-(CH_2)_{mm}R(114)$;
mm Null, 1 oder 2;
R(114)

-($C_3$-$C_8$)-Cycloalkyl oder Phenyl,

welches nicht substituiert oder substituiert ist mit 1 - 3 Substituenten ausgewählt aus der Gruppe bestehend aus F und Cl, - $CF_3$, Methyl, Methoxy und -NR(115)R(116);
R(115) und R(116)

Wasserstoff oder -$CH_3$;

oder
die jeweils anderen Reste R(7) und R(9)

unabhängig voneinander Pyrrol-1-yl, Pyrrol-2-yl oder Pyrrol-3-yl,

welches nicht substituiert oder substituiert ist mit 1 - 4 Substituenten ausgewählt aus der Gruppe bestehend aus F, Cl, Br, I, -CN, ($C_2$-$C_8$)-Alkanoyl, ($C_2$-$C_8$)-Alkoxycarbonyl, Formyl, Carboxy, -$CF_3$, Methyl und Methoxy;

oder
die jeweils anderen Reste R(7) und R(9)

R(122)-$SO_2$-, R(123)R(124)N-CO-, R(128)-CO- oder R(129)R(130)N-$SO_2$;
R(122) und R(128)

unabhängig voneinander Methyl oder -$CF_3$;

R(123), R(124), R(129) und R(130)

unabhängig voneinander Wasserstoff oder Methyl;

oder
die jeweils anderen Reste R(7) und R(9)

unabhängig voneinander -OR(135) oder -NR(135)R(136);
R(135) und R(136)

unabhängig voneinander Wasserstoff oder Alkyl mit 1, 2, 3, 4, 5 oder 6 C-Atomen;

oder
R(135) und R(136)

gemeinsam 4 - 7 Methylengruppen, von denen eine $CH_2$-Gruppe durch Sauerstoff, -S-, -NH-, -$NCH_3$ oder -N-Benzyl ersetzt sein kann;

der jeweils andere Rest R(8)

Wasserstoff, -SR(125), -OR(125), -NR(125)R(126) oder -CR(125)R(126)R(127);
R(125)

Wasserstoff, Alkyl mit 1, 2, 3, 4, 5, 6, 7 oder 8 C-Atomen oder Phenyl,

das unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;

oder
R(125)

-($C_1$-$C_9$)-Heteroaryl,

das unsubstituiert oder substituiert ist mit 1 - 3 Substituenten ausgewählt aus der Gruppe bestehend aus der Gruppe F, Cl, $CF_3$, $CH_3$, Methoxy, Hydroxy, Amino, Methylamino und Dimethylamino;

R(126) und R(127)

unabhängig voneinander wie R(125) definiert oder Wasserstoff oder Alkyl mit 1, 2, 3, 4, 5, 6, 7 oder 8 C-Atomen;

A abwesend, -NR(11)-CO-, -NR(12)-CO-NR(13)-, -NR(17)-CO-NR(18)-$SO_2$-, - NR(19)-$SO_2$-, -$SO_2$-NR(19)-$SO_2$-, -$SO_2$-NR(19)-CO-, -O-CO-NR(19)-$SO_2$- oder -CR(20)=CR(21)-;

R(11), R(12), R(13), R(17), R(18), R(19), R(20) und R(21)

unabhängig voneinander Wasserstoff oder Alkyl mit 1, 2, 3, 4, 5, 6, 7 oder 8 C-Atomen

sowie deren pharmazeutisch verträgliche Salze;

(HOE 96/F 026 - EP 790 245)
at) Substituierte Thiophenylalkenylcarbonsäureguanidide der Formel I

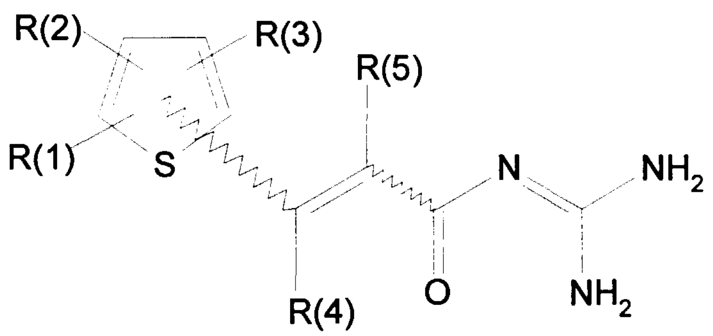

I

worin bedeuten:

mindestens einer der Substituenten R(1), R(2) und R(3)

-$O_p$-$(CH_2)_s$-$C_qF_{2q+1}$, R(40)CO- oder R(31)$SO_k$-;
p Null oder 1;
s Null, 1, 2, 3 oder 4;
q 1, 2, 3, 4, 5, 6, 7 oder 8;
k Null, 1 oder 2;
R(40) Alkyl mit 1 , 2, 3, 4, 5, 6, 7 oder 8 C-Atomen, Perfluoralkyl mit 1, 2, 3, 4, 5, 6, 7 oder 8 C-Atomen, Cycloalkyl mit 3, 4, 5, 6, 7 oder 8 C-Atomen, oder Phenyl,

das nicht substituiert oder substituiert ist mit 1 - 3 Substituenten ausgewählt aus der Gruppe bestehend aus F, Cl, $CF_3$, Methyl und Methoxy;

R(31) Alkyl mit 1 , 2, 3, 4, 5, 6, 7 oder 8 C-Atomen, Perfluoralkyl mit 1, 2, 3, 4, 5, 6, 7 oder 8 C-Atomen, Cycloalkyl mit 3, 4, 5, 6, 7 oder 8 C-Atomen, oder Phenyl,

das nicht substituiert oder substituiert ist mit 1 - 3 Substituenten ausgewählt aus der Gruppe bestehend aus F, Cl, $CF_3$, Methyl oder Methoxy;

oder
R(31) NR(41)R(42);

R(41) und R(42)

unabhängig voneinander Wasserstoff, Alkyl mit 1, 2, 3 oder 4 C-Atomen, Perfluoralkyl mit 1, 2, 3 oder 4 C-Atomen,

oder
R(41) und R(42)

gemeinsam 4 oder 5 Methylengruppen, von denen eine $CH_2$-Gruppe durch Sauerstoff, S, NH, N-$CH_3$ oder N-Benzyl ersetzt sein kann;

und die jeweils anderen Substituenten R(1), R(2) und R(3)

unabhängig voneinander H, F, Cl, Br, I, CN, $-O_{na}-C_{ma}H_{2ma+1}$ oder - $O_{ga}C_{ra}H_{2ra}R(10)$;
na Null oder 1;
ma Null, 1, 2, 3, 4, 5, 6, 7 oder 8;
ga Null oder 1;
ra Null, 1, 2, 3 oder 4;
R(10) Cycloalkyl mit 3, 4, 5, 6, 7 oder 8 C-Atomen oder Phenyl,

wobei das Phenyl nicht substituiert oder substituiert ist mit 1 - 3 Substituenten ausgewählt aus der Gruppe bestehend aus F, Cl, $CF_3$, Methyl und Methoxy;

R(4) und R(5)

unabhängig voneinander Wasserstoff, F, Cl, Br, I, CN, Alkyl mit 1, 2, 3, 4, 5, 6, 7 oder 8 C-Atomen, Perfluoralkyl mit 1, 2, 3, 4, 5, 6, 7 oder 8 C-Atomen, Cycloalkyl mit 3, 4, 5, 6, 7 oder 8 C-Atomen oder Phenyl,

das nicht substituiert oder substituiert ist mit 1 - 3 Substituenten ausgewählt aus der Gruppe bestehend aus F, Cl, $CF_3$, Methyl, Methoxy und NR(14)R(15);
R(14) und R(15)

unabhängig voneinander H, Alkyl mit 1, 2, 3 oder 4 C-Atomen oder Perfluoralkyl mit 1, 2, 3 oder 4 C-Atomen;

sowie deren pharmazeutisch verträgliche Salze;

(HOE 96/F 032 - EP 791 577)
au) Ortho-substituierte Benzoylguanidine der Formel I

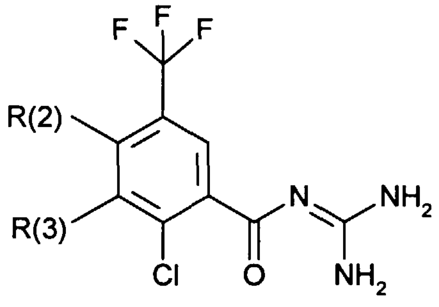

worin bedeuten:

R(2) und R(3)

unabhängig voneinander Wasserstoff, Cl, Br, I, $(C_1-C_8)$-Alkyl, $(C_3-C_8)$-Cycloalkyl oder -OR(5);
R(5) $(C_1-C_8)$-Alkyl oder $-C_dH_{2d}-(C_3-C_8)$-Cycloalkyl;

d Null, 1 oder 2;

wobei stets einer der beiden Substituenten R(2) und R(3) Wasserstoff ist, jedoch nicht beide Substituenten R(2) und R(3) gleichzeitig Wasserstoff sind, sowie deren pharmazeutisch verträgliche Salze;

(HOE 96/F 042 - EP 794 171)
av) Benzoylguanidine der Formel I

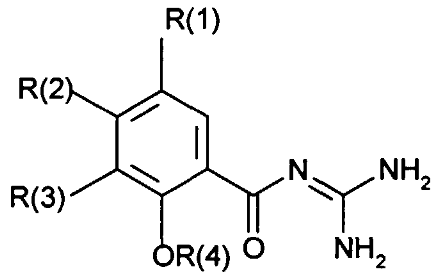

worin bedeuten:

R(1) H, F, Cl, Br, I, CN, $NO_2$, Alkyl mit 1, 2, 3, 4, 5, 6, 7 oder 8 C-Atomen, Alkoxy mit 1, 2, 3, 4, 5, 6, 7 oder 8 C-Atomen, Cycloalkyl mit 3, 4, 5, 6, 7 oder 8 C-Atomen, Cycloalkoxy mit 3, 4, 5, 6, 7 oder 8 C-Atomen oder $X_a$-$(CH_2)_b$-$(CF_2)_c$-$CF_3$;

X Sauerstoff, S, NR(5),
a Null oder 1;
b Null, 1 oder 2;
c Null, 1, 2 oder 3;

R (5) H, Alkyl mit 1, 2, 3 oder 4 C-Atomen oder -$C_dH_{2d}$R(6);

d Null, 1, 2, 3 oder 4;
R(6) Cycloalkyl mit 3, 4, 5, 6, 7 oder 8 C-Atomen, Phenyl, Biphenylyl oder Naphthyl,

wobei die Aromaten Phenyl, Biphenylyl oder Naphthyl nicht substituiert oder substituiert sind mit 1 - 3 Substituenten ausgewählt aus der Gruppe bestehend aus F, Cl, $CF_3$, Methyl, Methoxy und NR(7)R(8);
R(7) und R(8)

unabhängig H oder Alkyl mit 1, 2, 3 oder 4 C-Atomen;

oder
R(1) -SR(10), -OR(10) oder -CR(10)R(11)R(12);

R(10) -$C_fH_2$f-Cycloalkyl mit 3, 4, 5, 6, 7 oder 8 C-Atomen im Cycloalkylring, oder Phenyl,

wobei Phenyl unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;

f Null, 1 oder 2;
R(11) und R(12)

unabhängig voneinander wie R(10) definiert oder Wasserstoff oder Alkyl mit 1, 2, 3 oder 4 C-Atomen;

oder

R(1) Phenyl, Naphthyl, Biphenylyl oder Heteroaryl mit 1, 2, 3, 4, 5, 6, 7, 8 oder 9 C-Atomen, letzteres über ein C- oder ein N-Atom des Rings verknüpft,

die jeweils unsubstituiert oder substituiert sind mit 1 - 3 Substituenten ausgewählt aus der Gruppe bestehend aus F, Cl, $CF_3$, $CH_3$, Methoxy, Hydroxy, Amino, Methylamino und Dimethylamino,

oder
R(1) -SR(13), -OR(13), -NHR(13), -NR(13)R(14), -CHR(13)R(15), -C[R(15)R(16)OH], -C≡CR(18), -C[R(19)]=CHR(18), $-C[R(20)R(21)]_k-(CO)-[CR(22)R(23)]_l-R(24)$,

k Null, 1, 2, 3 oder 4;
l Null, 1, 2, 3 oder 4;
R(13) und R(14)

gleich oder verschieden $-(CH_2)_g-(CHOH)_h-(CH_2)_i-(CHOH)_j-R(17)$ oder $-(CH_2)_g-O-(CH_2-CH_2O)_h-R(24)$;
R(17) Wasserstoff oder Methyl,
g, h und i

gleich oder verschieden Null, 1, 2, 3 oder 4;

j 1, 2, 3 oder 4;

R(15) und R(16)

gleich oder verschieden Wasserstoff, Alkyl mit 1, 2, 3, 4, 5 oder 6 C-Atomen, oder zusammen mit dem sie tragenden Kohlenstoffatom Cycloalkyl mit 3, 4, 5, 6, 7 oder 8 C-Atomen;

R(18)

Phenyl,

das unsubstituiert oder substituiert ist mit 1 - 3 Substituenten ausgewählt aus der Gruppe bestehend aus F, Cl, $CF_3$, Methyl, Methoxy und NR(25)R(26);
R(25) und R(26)

H oder Alkyl mit 1, 2, 3 oder 4 C-Atomen;

oder
R(18) Heteroaryl mit 1, 2, 3, 4, 5, 6, 7, 8 oder 9 Kohlenstoffatomen,

das unsubstituiert oder wie Phenyl substituiert ist;

oder
R(18) Alkyl mit 1, 2, 3, 4, 5 oder 6 C-Atomen,

das unsubstituiert oder mit 1 - 3 OH substituiert ist;

oder
R (18)

Cycloalkyl mit 3, 4, 5, 6, 7 oder 8 C-Atomen;

R(19), R(20), R(21), R(22) und R(23)

gleich oder verschieden Wasserstoff oder Methyl;

R(24) H, Alkyl mit 1, 2, 3, 4, 5 oder 6 C-Atomen, Cycloalkyl mit 3, 4, 5, 6, 7 oder 8 C-Atomen oder $-C_mH_2m-$

R(18);

m 1, 2, 3 oder 4;

R(2) und R(3)

wie R(1) definiert;

R(4) Alkyl mit 1, 2, 3 oder 4 C-Atomen;
sowie deren pharmazeutisch verträgliche Salze;

(HOE 96/F 043 - EP 794 172)
aw) Ortho-substituierte Benzoylguanidine der Formel I

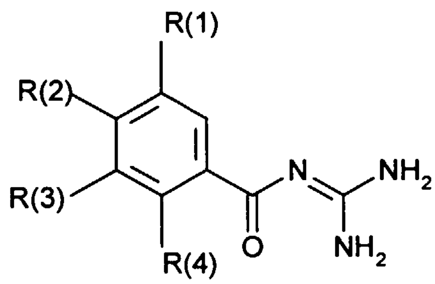

worin bedeuten:

R(1) H, F, Cl, Br, I, CN, $NO_2$, Alkyl mit 1, 2, 3, 4, 5, 6, 7 oder 8 C-Atomen, Alkoxy mit 1, 2, 3, 4, 5, 6, 7 oder 8 C-Atomen, Cycloalkyl mit 3, 4, 5, 6, 7 oder 8 C-Atomen, Cycloalkoxy mit 3, 4, 5, 6, 7 oder 8 C-Atomen oder $X_a$-$(CH_2)_b$-$(CF_2)_c$-$CF_3$;

X Sauerstoff, S, NR(5),
a Null oder 1;
b Null, 1 oder 2;
c Null, 1, 2 oder 3;

R (5) H, Alkyl mit 1, 2, 3 oder 4 C-Atomen oder -$C_dH_{2d}$R(6);

d Null, 1, 2, 3 oder 4;
R(6) Cycloalkyl mit 3, 4, 5, 6, 7 oder 8 C-Atomen, Phenyl, Biphenylyl oder Naphthyl,

wobei die Aromaten Phenyl, Biphenylyl oder Naphthyl nicht substituiert oder substituiert sind mit 1 - 3 Substituenten ausgewählt aus der Gruppe bestehend aus F, Cl, $CF_3$, Methyl, Methoxy und NR(7)R(8);
R(7) und R(8)

unabhängig H oder Alkyl mit 1, 2, 3 oder 4 C-Atomen;

oder
R(1) -SR(10), -OR(10) oder -CR(10)R(11)R(12);

R(10) -$C_fH_{2f}$-Cycloalkyl mit 3, 4, 5, 6, 7 oder 8 C-Atomen im Cycloalkylring, oder Phenyl,

wobei Phenyl unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;

f Null, 1 oder 2;
R(11) und R(12)

unabhängig voneinander wie R(10) definiert oder Wasserstoff oder Alkyl mit 1, 2, 3 oder 4 C-Atomen;

oder
R(1) Phenyl, Naphthyl, Biphenylyl oder Heteroaryl mit 1, 2, 3, 4, 5, 6, 7, 8 oder 9 C-Atomen, letzteres über ein C- oder ein N-Atom des Rings verknüpft,

die jeweils unsubstituiert oder substituiert sind mit 1 - 3 Substituenten ausgewählt aus der Gruppe bestehend aus F, Cl, $CF_3$, $CH_3$, Methoxy, Hydroxy, Amino, Methylamino und Dimethylamino,

oder
R(1) -SR(13), -OR(13), -NHR(13), -NR(13)R(14), -CHR(13)R(15), -C[R(15)R(16)OH], -C≡CR(18), -C[R(19)]=CHR(18), $-C[R(20)R(21)]_k-(CO)-[CR(22)R(23)]_l-R(24)$,

k Null, 1, 2, 3 oder 4;
l Null, 1, 2, 3 oder 4;
R(13) und R(14)

gleich oder verschieden $-(CH_2)_g-(CHOH)_h-(CH_2)_i-(CHOH)_j-R(17)$ oder $-(CH_2)_g-O-(CH_2-CH_2O)_h-R(24)$;
R(17) Wasserstoff oder Methyl,
g, h und i

gleich oder verschieden Null, 1, 2, 3 oder 4;

j 1, 2, 3 oder 4;

R(15) und R(16)

gleich oder verschieden Wasserstoff, Alkyl mit 1, 2, 3, 4, 5 oder 6 C-Atomen, oder zusammen mit dem sie tragenden Kohlenstoffatom Cycloalkyl mit 3, 4, 5, 6, 7 oder 8 C-Atomen;

R(18)

Phenyl,

das unsubstituiert oder substituiert ist mit 1 - 3 Substituenten ausgewählt aus der Gruppe bestehend aus F, Cl, $CF_3$, Methyl, Methoxy und NR(25)R(26);
R(25) und R(26)

H oder Alkyl mit 1, 2, 3 oder 4 C-Atomen;

oder
R(18) Heteroaryl mit 1, 2, 3, 4, 5, 6, 7, 8 oder 9 Kohlenstoffatomen,

das unsubstituiert oder wie Phenyl substituiert ist;

oder
R(18) Alkyl mit 1, 2, 3, 4, 5 oder 6 C-Atomen,

das unsubstituiert oder mit 1 - 3 OH substituiert ist;

oder
R (18)

Cycloalkyl mit 3, 4, 5, 6, 7 oder 8 C-Atomen;

R(19), R(20), R(21), R(22) und R(23)

gleich oder verschieden Wasserstoff oder Methyl;

R(24) H, Alkyl mit 1, 2, 3, 4, 5 oder 6 C-Atomen, Cycloalkyl mit 3, 4, 5, 6, 7 oder 8 C-Atomen oder $-C_mH_{2m}-$R(18);

m 1, 2, 3 oder 4;

einer der beiden Substituenten R(2) und R(3)

Hydroxyl;

und
der jeweils andere der Substituenten R(2) und R(3)

definiert ist wie R(1);

R(4) Alkyl mit 1, 2, 3 oder 4 C-Atomen; Alkoxy mit 1, 2, 3 oder 4 C-Atomen, F, Cl, Br, I oder $-(CH_2)_n-(CF_2)_o-CF_3$;

n Null oder 1;
o Null oder 1;

sowie deren pharmazeutisch verträgliche Salze.

(HOE 96/F 135 - EP 810 207)
ax) Bis-ortho-substituierte Benzoylguanidine der Formel I

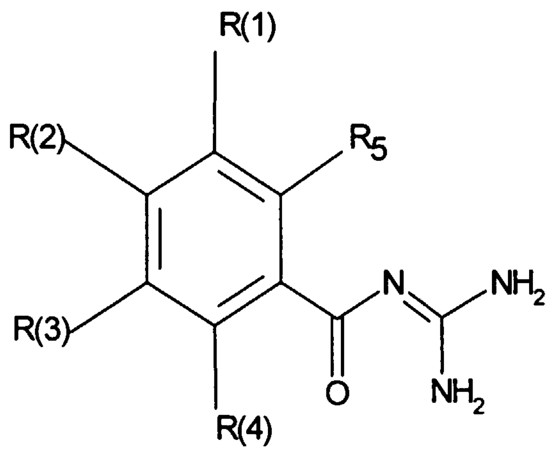

worin bedeuten:

R(1), R(2) und R(3)

unabhängig voneinander R(10)-$SO_a$- oder R(14)R(15)N-$SO_2$-;
a Null, 1 oder 2,
R(10), R(14) und R(15)

unabhängig voneinander Alkyl mit 1, 2, 3, 4, 5, 6, 7 oder 8 C-Atomen, Perfluoralkyl mit 1, 2, 3, 4, 5, 6, 7 oder 8 C-Atomen, Alkenyl mit 3, 4, 5 oder 6 C-Atomen oder $-C_{ab}H_{2ab}-$R(16);
ab Null, 1, 2, 3 oder 4;
R(16) Cycloalkyl mit 3, 4, 5, 6 oder 7 C-Atomen oder Phenyl,

welches nicht substituiert ist oder substituiert mit 1 - 3 Substituenten ausgewählt aus der Gruppe bestehend aus F, Cl, $CF_3$, Methyl, Methoxy und NR(17)R(18);
R(17) und R(18)

unabhängig voneinander Wasserstoff, $CF_3$ oder Alkyl mit 1, 2, 3 oder 4 C-Atomen;

oder
R(14) und R(15)

gemeinsam 4 oder 5 Methylengruppen, von denen eine $CH_2$-Gruppe durch Sauerstoff, Schwefel, NH, N-$CH_3$ oder N-Benzyl ersetzt sein kann;
oder
R(14) und R(15)

Wasserstoff;

oder
R(1), R(2) und R(3)

unabhängig voneinander SR(21), -OR(22), -NR(23)R(24) oder -CR(25)R(26)R(27);
R(21), R(22), R(23) und R(25)

unabhängig voneinander -$C_bH_{2b}$-($C_1$-$C_9$)-Heteroaryl,

das unsubstituiert oder substituiert ist mit 1 - 3 Substituenten ausgewählt aus der Gruppe bestehend aus F, Cl, $CF_3$, $CH_3$, Methoxy, Hydroxy, Amino, Methylamino und Dimethylamino;
b Null, 1 oder 2;

R(24), R(26) und R(27)

unabhängig voneinander Wasserstoff, Alkyl mit 1, 2, 3 oder 4 C-Atomen oder Perfluoralkyl mit 1, 2, 3 oder 4 C-Atomen;

oder
R(1), R(2) und R(3)

unabhängig voneinander Wasserstoff, F, Cl, Br, I, CN, -$(Xa)_{dg}$-$C_{da}H_{2da+1}$, - $(Xb)_{dh}$-$(CH_2)_{db}$-$C_{de}F_{2de+1}$, Alkenyl mit 3, 4, 5, 6, 7 oder 8 C-Atomen oder -$C_{df}H_{2df}$R(30);
(Xa) Sauerstoff, Schwefel oder NR(33);
R(33) Wasserstoff, Alkyl mit 1, 2, 3 oder 4 C-Atomen oder Perfluoralkyl mit 1, 2, 3 oder 4 C-Atomen;
dg Null oder 1;
(Xb) Sauerstoff, Schwefel oder NR(34);

R(34) Wasserstoff, Alkyl mit 1, 2, 3 oder 4 C-Atomen oder Perfluoralkyl mit 1, 2, 3 oder 4 C-Atomen;

dh Null oder 1;
da Null, 1, 2, 3, 4, 5, 6, 7 oder 8;
db Null, 1, 2, 3 oder 4;
de Null, 1, 2, 3, 4, 5, 6 oder 7;
df Null, 1, 2, 3 oder 4;
R(30) Cycloalkyl mit 3, 4, 5, 6, 7 oder 8 C-Atomen, Phenyl, Biphenylyl oder Naphthyl,

wobei die Aromaten Phenyl, Biphenylyl oder Naphthyl nicht substituiert oder substituiert sind mit 1 - 3 Substituenten ausgewählt aus der Gruppe bestehend aus F, Cl, $CF_3$, Methyl, Methoxy und NR(31)R(32);
R(31) und R(32)

Wasserstoff, Alkyl mit 1, 2, 3 oder 4 C-Atomen oder Perfluoralkyl mit 1, 2, 3 oder 4 C-Atomen;

oder
R(1), R(2) und R(3)

unabhängig voneinander NR(40)R(41) oder $-(Xe)-(CH_2)_{eb}R(45)$;
R(40) und R(41)

unabhängig voneinander Wasserstoff, Alkyl mit 1, 2, 3, 4, 5, 6, 7 oder 8 C-Atomen, Perfluoralkyl mit 1, 2, 3, 4, 5, 6, 7 oder 8 C-Atomen oder $(CH_2)_e$-R(42);
e Null, 1, 2, 3 oder 4;
R(42) Cycloalkyl mit 3, 4, 5, 6 oder 7 C-Atomen oder Phenyl,

welches nicht substituiert ist oder substituiert mit 1 - 3 Substituenten ausgewählt aus der Gruppe bestehend aus F, Cl, $CF_3$, Methyl, Methoxy und NR(43)R(44);
R(43) und R(44)

unabhängig voneinander Wasserstoff, $CF_3$ oder Alkyl mit 1, 2, 3 oder 4 C-Atomen;

oder
R(40) und R(41)

gemeinsam 4 oder 5 Methylengruppen, von denen eine $CH_2$-Gruppe durch Sauerstoff, Schwefel, NH, N-$CH_3$ oder N-Benzyl ersetzt sein kann;

(Xe) Sauerstoff, Schwefel oder NR(47);

R(47) Wasserstoff, Alkyl mit 1, 2, 3 oder 4 C-Atomen oder Perfluoralkyl mit 1, 2, 3 oder 4 C-Atomen;

eb Null, 1, 2, 3 oder 4;
R(45) Cycloalkyl mit 3, 4, 5, 6 oder 7 C-Atomen oder Phenyl,

welches nicht substituiert ist oder substituiert mit 1 - 3 Substituenten ausgewählt aus der Gruppe bestehend aus F, Cl, $CF_3$, Methyl, Methoxy, NR(50)R(51) und $-(Xfa)-(CH_2)_{ed}-(Xfb)R(46)$;
Xfa $CH_2$, Sauerstoff, Schwefel oder NR(48);
Xfb Sauerstoff, Schwefel oder NR(49);

R(48), R(49), R(50) und R(51)

unabhängig voneinander Wasserstoff, Alkyl mit 1, 2, 3 oder 4 C-Atomen oder Perfluoralkyl mit 1, 2, 3 oder 4 C-Atomen;

ed 1, 2, 3 oder 4;
R(46) Wasserstoff, Alkyl mit 1, 2, 3 oder 4 C-Atomen oder Perfluoralkyl mit 1, 2, 3 oder 4 C-Atomen;

oder
R(1), R(2) und R(3)

unabhängig voneinander -CHR(52)R(53);
R(52) $-(CH_2)_g-(CHOH)_h-(CH)_i-(CHOH)_k-R(54)$ oder $-(CH_2)_g-O-(CH_2-CH_2O)_h-R(54)$;

R(54) Wasserstoff oder Methyl;
g, h, i

gleich oder verschieden Null, 1, 2, 3 oder 4;

k 1, 2, 3 oder 4;

R(53) Wasserstoff oder Alkyl mit 1, 2, 3 oder 4 C-Atomen;

oder
R(1), R(2) und R(3)

unabhängig voneinander -C(OH)R(55)R(56);
R(55) und R(56)

gleich oder verschieden Wasserstoff oder Alkyl mit 1, 2, 3 oder 4 C-Atomen;

oder
R(55) und R(56)

gemeinsam Cycloalkyl mit 3, 4, 5 oder 6 C-Atomen;

oder
R(55) $-CH_2OH$;

und
R(4) und R(5)

unabhängig voneinander Alkyl mit 1, 2, 3 oder 4 C-Atomen, Alkoxy mit 1, 2, 3 oder 4 C-Atomen, OH, F, Cl, Br, I, CN, $-O_n-(CH_2)_o-(CF_2)_p-CF_3$;
n Null oder 1;
o Null, 1 oder 2;
p Null, 1 oder 2;

sowie deren pharmazeutisch verträgliche Salze.

(HOE 96/F 136 - EP 810 205)
ay) Substituierte 1-Naphthoylguanidine der Formel I

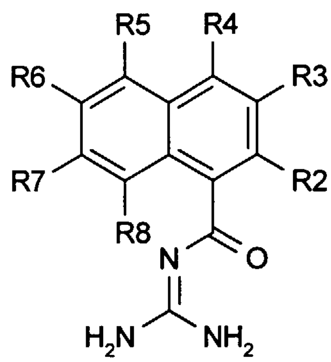

I

worin bedeuten

R2, R3, R4, R5, R6, R7 und R8

unabhängig voneinander H, F, Cl, Br, I, CN, $NO_2$, $CF_3$, $C_2F_5$ oder $X_aY_b$ Z;
X O, S, NR(10), CR(11)R(12), C=O, C(=O)NR(10), C(=O)O, SO, $SO_2$, $SO_2NR(10)$, OC=O, NR(10)C=O oder $NR(10)SO_2$,

wobei die Verknüpfung mit dem Naphthalinring jeweils über das links stehende Atom erfolgt;

R(10), R(11) und R(12)

unabhängig voneinander H, Alkyl mit 1, 2, 3, 4, 5 oder 6 C-Atomen, Perfluoralkyl mit 1, 2, 3 oder 4 C-Atomen oder Cycloalkyl mit 3, 4, 5, 6 oder 7 C-Atomen;

a Null oder 1;
Y Alkylen mit 1, 2, 3, 4, 5, 6, 7 oder 8 $CH_2$-Gruppen,

wobei eine dieser $CH_2$-Gruppen durch O, S, NR(13) oder o-, p-oder m-Phenylen ersetzt sein kann;
R(13) H, Alkyl mit 1, 2, 3, 4, 5 oder 6 C-Atomen, Perfluoralkyl mit 1, 2, 3 oder 4 C-Atomen oder Cycloalkyl mit 3, 4, 5 oder 6 C-Atomen;

b Null oder 1;
Z H, Alkyl mit 1, 2, 3, 4, 5, 6 oder 7 C-Atomen, Cycloalkyl mit 3, 4, 5, 6 oder 7 C-Atomen, C(=O)R(15), $SO_2$R(15), NR(16)R(17) oder Phenyl,

das nicht substituiert oder substituiert ist mit 1 - 3 Substituenten ausgewählt aus der Gruppe bestehend aus F, Cl, Br, $CF_3$, Methyl, Methoxy, NR(21)R(22);
R(21) und R(22)

unabhängig voneinander H oder Alkyl mit 1, 2, 3 oder 4 C-Atomen oder Perfluoralkyl mit 1, 2, 3 oder 4 C-Atomen;

R(15) N=C($NH_2$)$_2$, NR(18)R(19), N($CH_2$)$_c$NR(18)R(19) oder OR(20);

c 2 oder 3;
R(18) und R(19)

unabhängig voneinander H, Alkyl mit 1, 2, 3, 4, 5, 6, 7 oder 8 C-Atomen oder Perfluoralkyl mit 1, 2, 3 oder 4 C-Atomen;

oder
R(18) und R(19)

gemeinsam 4 oder 5 Methylengruppen,

von denen eine $CH_2$-Gruppe durch Sauerstoff, S, NH, N-$CH_3$, N-Benzyl oder N-(p-Chlorphenyl) ersetzt sein kann;

R(20) H, Alkyl mit 1, 2, 3, 4, 5 oder 6 C-Atomen, Perfluoralkyl mit 1, 2, 3 oder 4 C-Atomen oder Cycloalkyl mit 3, 4, 5, 6 oder 7 C-Atomen;

R(16) und R(17)

unabhängig voneinander H, Alkyl mit 1, 2, 3, 4, 5, 6, 7 oder 8 C-Atomen oder Perfluoralkyl mit 1, 2, 3 oder 4 C-Atomen;

oder
R(16) und R(17)

gemeinsam 4 oder 5 Methylengruppen, von denen eine $CH_2$-Gruppe durch Sauerstoff, S, NH, N-$CH_3$, N-Benzyl oder N-(p-Chlorphenyl) ersetzt sein kann;

oder
Z ein N-haltiger Heterocyclus mit 1, 2, 3, 4, 5, 6, 7, 8 oder 9 C-Atomen,

wobei der N-haltige Heterocyclus über N oder C verknüpft ist und nicht substituiert oder substituiert ist mit 1 - 3 Substituenten ausgewählt aus der Gruppe bestehend aus F, Cl, Br, $CF_3$, Methyl, Methoxy und NR(21)R(22);

wobei jedoch für den Fall, daß R(4) für einen Alkoxyrest steht, mindestens einer der Substituenten R(2), R(3), R(5), R(6), R(7) und R(8) ungleich Wasserstoff ist;
sowie deren pharmazeutisch verträgliche Salze.

(HOE96/F 137 - EP 810 206)
az) Substituierte 2-Naphthoylguanidine der Formel I

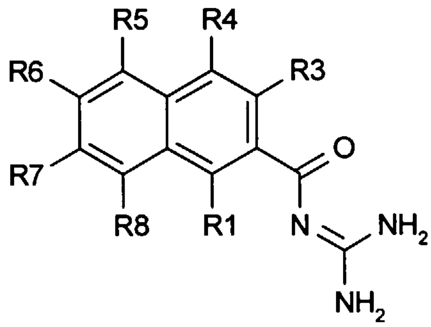

I

worin bedeuten:

mindestens einer der Substituenten R1, R3, R4, R5, R6, R7 und R8

$XY_a$ WZ oder $X'Y_aWZ'$;
X O, S, NR(10) oder CR(11)R(12);

R(10), R(11) und R(12)

unabhängig voneinander H, Alkyl mit 1, 2, 3, 4, 5 oder 6 C-Atomen, Perfluoralkyl mit 1, 2, 3 oder 4 C-Atomen oder Cycloalkyl mit 3, 4, 5, 6 oder 7 C-Atomen;

Y Alkylen mit 1, 2, 3, 4, 5, 6, 7 oder 8 $CH_2$-Gruppen,

wobei eine dieser $CH_2$-Gruppen durch O, S, NR(13) oder o-, p-oder m-Phenylen ersetzt sein kann;

R(13) H, Alkyl mit 1, 2, 3, 4, 5 oder 6 C-Atomen, Perfluoralkyl mit 1, 2, 3 oder 4 C-Atomen oder Cycloalkyl mit 3, 4, 5 oder 6 C-Atomen;
a Null oder 1;
W $CH_2$, $SO_2$, S(=O)(=NH) oder - falls W nicht unmittelbar auf ein Heteroatom der Gruppe $XY_a$ folgt - auch O oder NR(14);

R(14) H, Alkyl mit 1, 2, 3, 4, 5 oder 6 C-Atomen, Perfluoralkyl mit 1, 2, 3 oder 4 C-Atomen oder Cycloalkyl mit 3, 4, 5 oder 6 C-Atomen;

Z C(=O)R(15), $SO_2$R(15) oder - falls W nicht O oder NR(14) bedeutet - auch NR(16)R(17);

R(15)

N=C$(NH_2)_2$, NR(18)R(19), N$(CH_2)_b$NR(18)R(19) oder OR(20);
b 2 oder 3;
R(18) und R(19)

unabhängig voneinander H, Alkyl mit 1, 2, 3, 4, 5, 6, 7 oder 8 C-Atomen oder Perfluoralkyl mit 1, 2, 3 oder 4 C-Atomen;

oder
R(18) und R(19)

gemeinsam 4 oder 5 Methylengruppen,

von denen eine $CH_2$-Gruppe durch Sauerstoff, S, NH, N-$CH_3$, N-Benzyl oder N-(p-Chlorphenyl) ersetzt sein kann;

R(20)

H, Alkyl mit 1, 2, 3, 4, 5 oder 6 C-Atomen, Perfluoralkyl mit 1, 2, 3 oder 4 C-Atomen oder Cycloalkyl mit 3, 4, 5, 6 oder 7 C-Atomen;

R(16) und R(17)

unabhängig voneinander H, Alkyl mit 1, 2, 3, 4, 5, 6, 7 oder 8 C-Atomen oder Perfluoralkyl mit 1, 2, 3 oder 4 C-Atomen;

oder
R(16) und R(17)

gemeinsam 4 oder 5 Methylengruppen,

von denen eine $CH_2$-Gruppe durch Sauerstoff, S, NH, N-$CH_3$, N-Benzyl oder N-(p-Chlorphenyl) ersetzt sein kann;

X' C=O, C(=O)NR(30), C(=O)O, SO, $SO_2$, $SO_2$NR(30), OC=O, NR(30)C=O oder NR(30)$SO_2$,

wobei die Verknüpfung mit dem Naphthalinring jeweils über das links stehende Atom erfolgt;

R(30) H, Alkyl mit 1, 2, 3, 4, 5 oder 6 C-Atomen, Perfluoralkyl mit 1, 2, 3 oder 4 C-Atomen oder Cycloalkyl mit 3, 4, 5, 6 oder 7 C-Atomen;
Z' C(=O)R(15), $SO_2$R(15), einen N-haltigen Heterocyclus mit 1, 2, 3, 4, 5, 6, 7, 8 oder 9 C-Atomen,

wobei der N-haltige Heterocyclus über N oder C verknüpft ist und nicht substituiert oder substituiert ist mit 1 - 3 Substituenten ausgewählt aus der Gruppe bestehend aus F, Cl, Br, $CF_3$, Methyl, Methoxy und NR(21)R(22);
R(21) und R(22)

unabhängig voneinander H, Alkyl mit 1, 2, 3 oder 4 C-Atomen oder Perfluoralkyl mit 1, 2, 3 oder 4 C-Atomen;

R(15)

N=C($NH_2$)$_2$, NR(18)R(19), N($CH_2$)$_b$NR(18)R(19) oder OR(20); R(18) und R(19)

unabhängig voneinander H, Alkyl mit 1, 2, 3, 4, 5, 6, 7 oder 8 C-Atomen oder Perfluoralkyl mit 1, 2, 3 oder 4 C-Atomen;

oder
R(18) und R(19)

gemeinsam 4 oder 5 Methylengruppen,

von denen eine $CH_2$-Gruppe durch Sauerstoff, S, NH, N-$CH_3$, N-Benzyl oder N-(p-Chlorphenyl) ersetzt sein kann;

b 2 oder 3;
R(20) H, Alkyl mit 1, 2, 3, 4, 5 oder 6 C-Atomen, Perfluoralkyl mit 1, 2, 3 oder 4 C-Atomen oder Cycloalkyl mit 3, 4, 5, 6 oder 7 C-Atomen;

oder
Z' - falls W nicht O oder NR(14) bedeutet - NR(16)R(17);

R(16) und R(17)

unabhängig voneinander H, Alkyl mit 1, 2, 3, 4, 5, 6, 7 oder 8 C-Atomen oder Perfluoralkyl mit 1, 2, 3 oder 4 C-Atomen;

oder
R(16) und R(17)

gemeinsam 4 oder 5 Methylengruppen,

von denen eine $CH_2$-Gruppe durch Sauerstoff, S, NH, N-$CH_3$, N-Benzyl oder N-(p-Chlorphenyl) ersetzt sein kann;

und die jeweils übrigen Substituenten R1, R3, R4, R5, R6, R7 und R8, die noch nicht durch die zuvor gegebenen Definitionen vergeben sind,

unabhängig voneinander H, F, Cl, Br, I, CN, $NO_2$, $CF_3$, $C_2F_5$ oder $V_pQ_qU$;
V O, S, SO, $SO_2$, NR(60), OC=O, C=O, C(=O)NR(60), C(=O)O oder CR(66)R(67);

R(60), R(66) und R(67)

unabhängig voneinander H, Alkyl mit 1, 2, 3, 4, 5 oder 6 C-Atomen, Perfluoralkyl mit 1, 2, 3 oder 4 C-Atomen oder Cycloalkyl mit 3, 4, 5, 6 oder 7 C-Atomen;

p Null oder 1;
Q Alkylen mit 1, 2, 3, 4, 5, 6, 7 oder 8 $CH_2$-Gruppen,

wobei eine dieser $CH_2$-Gruppen durch O, S, NR(68) oder o-, p-oder m-Phenylen ersetzt sein kann;
R(68)

H, Alkyl mit 1, 2, 3, 4, 5 oder 6 C-Atomen, Perfluoralkyl mit 1, 2, 3 oder 4 C-Atomen oder Cycloalkyl mit 3, 4, 5 oder 6 C-Atomen;

q Null oder 1;
U H, Alkyl mit 1, 2, 3, 4, 5, 6 oder 7 C-Atomen, Cycloalkyl mit 3, 4, 5, 6 oder 7 C-Atomen, C(=O)R(65), $SO_2$R(65), NR(61)R(62) oder Phenyl,

das nicht substituiert oder substituiert ist mit 1 - 3 Substituenten ausgewählt aus der Gruppe bestehend aus F, Cl, Br, $CF_3$, Methyl, Methoxy und NR(63)R(64);
R(63) und R(64)

unabhängig voneinander H, Alkyl mit 1, 2, 3 oder 4 C-Atomen oder Perfluoralkyl mit 1, 2, 3 oder 4 C-Atomen;

R(65) N=C($NH_2$)$_2$, NR(61)R(62) oder OR(60);
R(61) und R(62)

unabhängig voneinander H, Alkyl mit 1, 2, 3, 4, 5, 6, 7 oder 8 C-Atomen oder Perfluoralkyl mit 1, 2, 3 oder 4 C-Atomen;

oder
R(61) und R(62)

gemeinsam 4 oder 5 Methylengruppen,

von denen eine $CH_2$-Gruppe durch Sauerstoff, S, NH, N-$CH_3$, N-Benzyl oder N-(p-Chlorphenyl) ersetzt sein kann;

oder
U einen N-haltigen Heterocyclus mit 1, 2, 3, 4, 5, 6, 7, 8 oder 9 C-Atomen,

wobei der N-haltige Heterocyclus über N oder C verknüpft ist und nicht substituiert oder substituiert ist mit 1 - 3 Substituenten ausgewählt aus der Gruppe bestehend aus F, Cl, Br, $CF_3$, Methyl, Methoxy und NR(63)R(64);

wobei jedoch mindestens einer der Substituenten R5, R6, R7 und R8 ungleich Wasserstoff ist; sowie deren pharmazeutisch verträgliche Salze.

(HOE 96/F 141 - EP 811 610))
ba) Ortho-substituierte Benzoylguanidine der Formel I

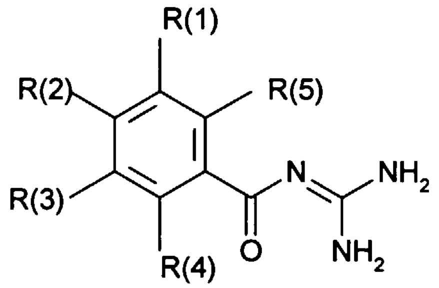

I

worin bedeuten:

R(1) H, F, Cl, Br, I, CN, $NO_2$, Alkyl mit 1, 2, 3, 4, 5, 6, 7 oder 8 C-Atomen, Alkoxy mit 1, 2, 3, 4, 5, 6, 7 oder 8 C-Atomen, Cycloalkyl mit 3, 4, 5, 6, 7 oder 8 C-Atomen, Cycloalkoxy mit 3, 4, 5, 6, 7 oder 8 C-Atomen oder $X_a$-$(CH_2)_b$-$(CF_2)_c$-$CF_3$;

X Sauerstoff, Schwefel oder NR(9),
a Null oder 1;
b Null, 1 oder 2;
c Null, 1, 2 oder 3;

R(9) H, Alkyl mit 1, 2, 3 oder 4 C-Atomen oder $-C_dH_{2d}R(6)$;

d Null, 1, 2, 3 oder 4;
R(6) Cycloalkyl mit 3, 4, 5, 6, 7 oder 8 C-Atomen, Phenyl, Biphenylyl oder Naphthyl,

wobei die Aromaten Phenyl, Biphenylyl oder Naphthyl nicht substituiert oder substituiert sind mit 1 - 3 Substituenten ausgewählt aus der Gruppe bestehend aus F, Cl, $CF_3$, Methyl, Methoxy und NR(7)R(8);
R(7) und R(8)

unabhängig H oder Alkyl mit 1, 2, 3 oder 4 C-Atomen;

oder
R(1) -SR(10), -OR(10) oder -CR(10)R(11)R(12);

R(10) $-C_fH_{2f}$-Cycloalkyl mit 3, 4, 5, 6, 7 oder 8 C-Atomen im Cycloalkylring, Heteroaryl mit 1, 2, 3, 4, 5, 6, 7, 8 oder 9 C-Atomen oder Phenyl,

wobei Heteroaryl und Phenyl unsubstituiert oder substituiert sind mit 1 - 3 Substituenten ausgewählt aus der Gruppe bestehend aus F, Cl, $CF_3$, $CH_3$, Methoxy, Hydroxy, Amino, Methylamino und Dimethylamino;

f Null, 1 oder 2;
R(11) und R(12)

unabhängig voneinander wie R(10) definiert oder Wasserstoff oder Alkyl mit 1, 2, 3 oder 4 C-Atomen;

oder
R(1) Phenyl, Naphthyl, Biphenylyl oder Heteroaryl mit 1, 2, 3, 4, 5, 6, 7, 8 oder 9 C-Atomen, letzteres über ein C- oder ein N-Atom des Rings verknüpft,

die jeweils unsubstituiert oder substituiert sind mit 1 - 3 Substituenten ausgewählt aus der Gruppe bestehend aus F, Cl, $CF_3$, $CH_3$, Methoxy, Hydroxy, Amino, Methylamino und Dimethylamino,

oder
R(1) -SR(13), -OR(13), -NHR(13), -NR(13)R(14), -CHR(13)R(15), -C[R(15)R(16)]OH, -C≡CR(18), -C[R(19)]=CHR(18), $-C[R(20)R(21)]_k-(CO)-[CR(22)R(23)]_l-R(24)$,

k Null, 1, 2, 3 oder 4;
l Null, 1, 2, 3 oder 4;
R(13) und R(14)

gleich oder verschieden $-(CH_2)_g-(CHOH)_h-(CH_2)_i-(CHOH)_{kk}-R(17)$ oder $-(CH_2)_g-O-(CH_2-CH_2O)_h-R(24)$;
R(17) Wasserstoff oder Methyl,
g, h und i

gleich oder verschieden Null, 1, 2, 3 oder 4;

kk 1, 2, 3 oder 4;

R(15) und R(16)

gleich oder verschieden Wasserstoff, Alkyl mit 1, 2, 3, 4, 5 oder 6 C-Atomen, oder zusammen mit dem sie tragenden Kohlenstoffatom Cycloalkyl mit 3, 4, 5, 6, 7 oder 8 C-Atomen;

R(18)

Phenyl,

das unsubstituiert oder substituiert ist mit 1 - 3 Substituenten ausgewählt aus der Gruppe bestehend aus F, Cl, $CF_3$, Methyl, Methoxy und NR(25)R(26);
R(25) und R(26)

H oder Alkyl mit 1, 2, 3 oder 4 C-Atomen;

oder
R(18) Heteroaryl mit 1, 2, 3, 4, 5, 6, 7, 8 oder 9 Kohlenstoffatomen,

das unsubstituiert oder wie Phenyl substituiert ist;

oder
R(18) Alkyl mit 1, 2, 3, 4, 5 oder 6 C-Atomen,

das unsubstituiert oder mit 1 - 3 OH substituiert ist;

oder
R (18)

Cycloalkyl mit 3, 4, 5, 6, 7 oder 8 C-Atomen;

R(19), R(20), R(21), R(22) und R(23)

gleich oder verschieden Wasserstoff oder Methyl;

R(24) H, Alkyl mit 1, 2, 3, 4, 5 oder 6 C-Atomen, Cycloalkyl mit 3, 4, 5, 6, 7 oder 8 C-Atomen oder $-C_mH_{2m}$-R(18);

m 1, 2, 3 oder 4;

einer der beiden Substituenten R(2) und R(3)

-O-CO-R(27);
R(27) Alkyl mit 1, 2, 3, 4, 5, 6, 7 oder 8 C-Atomen, Cycloalkyl mit 3, 4, 5, 6, 7 oder 8 C-Atomen, Phenyl, Biphenylyl, Naphthyl, Pyridyl oder Chinolinyl,

wobei Phenyl, Biphenylyl, Naphthyl, Pyridyl oder Chinolinyl unsubstituiert oder substituiert sind mit 1 - 3 Substituenten ausgewählt aus der Gruppe bestehend aus F, Cl, $CF_3$, Methyl, Methoxy und NR(7)R(8);
R(7) und R(8)

unabhängig voneinander Wasserstoff oder Alkyl mit 1, 2, 3 oder 4 C-Atomen;

wobei stets einer der Substituenten R(2) und R(3)

definiert ist wie R(1);

R(4) und R(5)

unabhängig voneinander Wasserstoff, Alkyl mit 1, 2, 3 oder 4 C-Atomen;
Alkoxy mit 1, 2, 3 oder 4 C-Atomen, F, Cl, Br, I, CN oder $-(CH_2)_n-(CF_2)_o-CF_3$;
n Null oder 1;
o Null oder 1;

sowie deren pharmazeutisch verträgliche Salze.

(HOE 96/F 154 - EP 814 077)
bb) Benzoylguanidine der Formel I

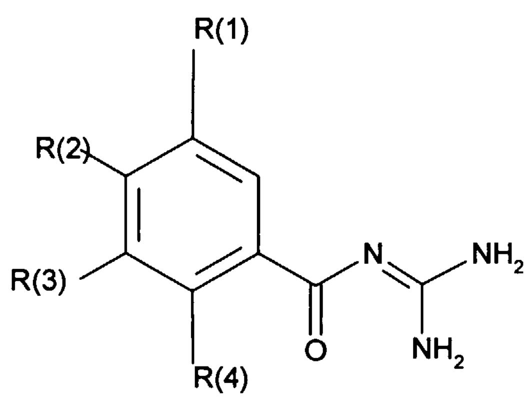

worin bedeuten:

R(1) R(13)-$SO_m$ oder R(14)R(15)N-$SO_2$-;

m 1 oder 2;
R(13) Alkyl mit 1, 2, 3, 4, 5, 6, 7 oder 8 C-Atomen, Perfluoralkyl mit 1, 2, 3, 4, 5, 6, 7 oder 8 C-Atomen, Alkenyl mit 3, 4, 5, 6, 7 oder 8 C-Atomen oder $-C_nH_{2n}$-R(16),

n Null, 1, 2, 3 oder 4;
R(16) Cycloalkyl mit 3, 4, 5, 6, 7 oder 8 C-Atomen, Phenyl, Biphenylyl oder Naphthyl,

wobei Phenyl, Biphenylyl und Naphthyl nicht substituiert oder substituiert sind mit 1 - 3 Substituenten ausgewählt aus der Gruppe bestehend aus F, Cl, $CF_3$, Methyl, Methoxy und NR(25)R(26);
R(25) und R(26)

unabhängig voneinander Wasserstoff, Alkyl mit 1, 2, 3 oder 4 C-Atomen oder Perfluoralkyl mit 1, 2, 3 oder 4 C-Atomen;

R(14) Wasserstoff, Alkyl mit 1, 2, 3, 4, 5, 6, 7 oder 8 C-Atomen, Perfluoralkyl mit 1, 2, 3, 4, 5, 6, 7 oder 8 C-Atomen, Alkenyl mit 3, 4, 5, 6, 7 oder 8 C-Atomen oder $-C_nH_{2n}$-R(27),

n Null, 1, 2, 3 oder 4;
R(27) Cycloalkyl mit 3, 4, 5, 6, 7 oder 8 C-Atomen, Phenyl, Biphenylyl oder Naphthyl,

wobei Phenyl, Biphenylyl und Naphthyl nicht substituiert oder substituiert sind mit 1 - 3 Substituenten ausgewählt aus der Gruppe bestehend aus F, Cl, $CF_3$, Methyl, Methoxy und NR(28)R(29);
R(28) und R(29)

unabhängig voneinander Wasserstoff, Alkyl mit 1, 2, 3 oder 4 C-Atomen oder Perfluoralkyl mit 1, 2, 3 oder 4 C-Atomen;

R(15) Wasserstoff, Alkyl mit 1, 2, 3 oder 4 C-Atomen oder Perfluoralkyl mit 1, 2, 3 oder 4 C-Atomen;
oder
R(14) und R(15)

gemeinsam 4 oder 5 Methylengruppen, von denen eine $CH_2$-Gruppe durch Sauerstoff, S, NH, N-$CH_3$ oder N-Benzyl ersetzt sein kann;

einer der Substituenten R(2) und R(3)

Wasserstoff;

und der jeweils andere Substituent R(2) und R(3)

-CHR(30)R(31);
R(30)

$-(CH_2)_g-(CHOH)_h-(CH_2)_i-(CHOH)_k-$R(32) oder $-(CH_2)_g-O-(CH_2-CH_2O)_h-$R(24);

R(24 und R(32)

unabhängig voneinander Wasserstoff oder Methyl;

g, h, i

gleich oder verschieden Null, 1, 2, 3 oder 4;

k 1, 2, 3 oder 4;

oder der jeweils andere Substituent R(2) und R(3)

-C(OH)R(33)R(34);
R(31), R(33) und R(34)

gleich oder verschieden Wasserstoff oder Alkyl mit 1, 2, 3 oder 4 C-Atomen

oder

R(33) und R(34)

gemeinsam Cycloalkyl mit 3, 4, 5 oder 6 C-Atomen;

oder
R(33) $-CH_2OH$;

R(4) Alkyl mit 1, 2, 3 oder 4 C-Atomen, Alkoxy mit 1, 2, 3 oder 4 C-Atomen, F, Cl, Br, I, CN, $-(CH_2)_n-(CF_2)_o-CF_3$;

n Null oder 1;
o Null, 1 oder 2;

sowie deren pharmazeutisch verträgliche Salze.

(HOE 96/F 202 - EP 837 055)
bc) Indanylidinacetylguanidine der Formel I

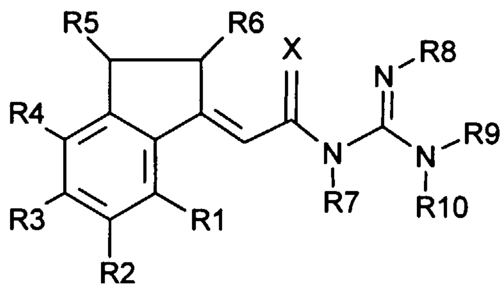

I

worin bedeuten:

R1, R2, R3, R4, R5 und R6

unabhängig voneinander H, $C_1$-$C_{10}$-Alkyl; Haloalkyl mit 1 - 6 Kohlenstoffatomen, O-$C_1$-$C_{10}$-Alkyl, Haloalkoxy mit 1 - 6 C-Atomen, F, Cl, Br, I, Aryl, substituiertes Aryl, Heteroaryl, substituiertes Heteroaryl, OH, O-Niederalkyl, O-Aryl, O-Niederalkylaryl, O-substituiertes Aryl, O-Niederalkyl-substituiertes Aryl, O-C(=O)-$C_1$-$C_4$-Alkyl-Aryl, O-C(=O)-NH-$C_1$-$C_4$-Alkyl, O-C(=O)-N($C_1$-$C_4$-Alkyl)$_2$, $NO_2$, CN, $CF_3$, $NH_2$, NH-C(=O)-$C_1$-$C_4$-Alkyl, NH-C(=O)-$NH_2$, COOH, C(=O)-O-$C_1$-$C_4$-Alkyl, C(=O)-$NH_2$, C(=O)-NH-$C_1$-$C_4$-Alkyl, C(=O)-N($C_1$-$C_4$-Alkyl)$_2$, $C_1$-$C_4$-COOH, $C_1$-$C_4$-Alkyl-C(=O)-O-$C_1$-$C_4$-Alkyl, $SO_3H$, $SO_2$-Alkyl; $SO_2$-Alkylaryl, $SO_2$-N-(Alkyl)$_2$, $SO_2$-N(Alkyl)(Alkylaryl), C(=O)-R11, $C_1$-$C_{10}$-Alkyl-C(=O)-R11, $C_2$-$C_{10}$-Alkenyl-C(=O)-R11, $C_2$-$C_{10}$-Alkinyl-C(=O)-R11, NH-C(=O)-$C_1$-$C_{10}$-Alkyl-C(=O)-R11 oder O-$C_1$-$C_{11}$-Alkyl-C(=O)-R11;
R11 $C_1$-$C_4$-Alkyl, $C_1$-$C_4$-Alkinyl, Aryl, substituiertes Aryl, $NH_2$, NH-$C_1$-$C_4$-Alkyl, N-($C_1$-$C_4$-Alkyl)$_2$, $SO_3H$, $SO_2$-Alkyl, $SO_2$-Alkylaryl, $SO_2$-N-(Alkyl)$_2$ oder $SO_2$-N(Alkyl)(Alkylaryl);

X O, S oder NH;

R7, R8, R9 und R10

unabhängig voneinander H, Alkyl, Cycloalkyl, Aryl, Alkylaryl,

oder
R8 und R9

zusammen Teil eines a 5-, 6- oder 7-gliedrigen heterocyclischen Rings;

oder ihre pharmazeutisch akzeptablen Salze.

(HOE 96/F 226 - EP 825 178)

bd) Phenylsubstituierte Alkenylcarbonsäure-guanidide der Formel I

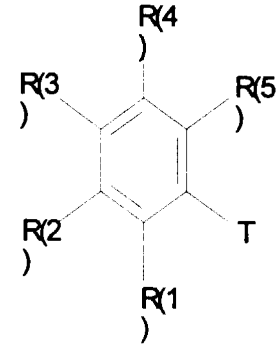

I

worin bedeuten:

T

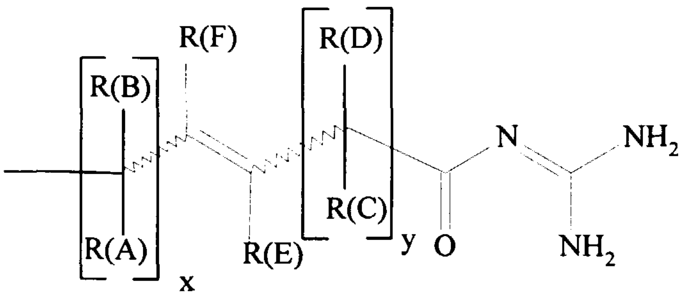

R(A) Wasserstoff, F, Cl, Br, I, CN, OH, OR(6), $(C_1-C_4)$-Alkyl, $O_r(CH_2)_aC_bF_{2b+1}$, $(C_3-C_8)$-Cycloalkyl oder NR(7)R(8)

r Null oder 1;
a Null, 1, 2, 3 oder 4;
b 1, 2, 3 oder 4;
R(6) $(C_1-C_4)$-Alkyl, $(C_1-C_4)$-Perfluoralkyl, $(C_3-C_6)$-Alkenyl, $(C_3-C_8)$-Cycloalkyl, Phenyl oder Benzyl,

wobei der Phenylkern nicht substituiert oder substituiert ist mit 1 - 3 Substituenten ausgewählt aus der Gruppe bestehend aus F, Cl, $CF_3$, Methyl, Methoxy und NR(9)R(10); R(9) und R(10)

H, $(C_1-C_4)$-Alkyl oder $(C_1-C_4)$-Perfluoralkyl;

R(7) und R(8)

unabhängig voneinander wie R(6) definiert;

oder
R(7) und R(8)

gemeinsam 4 oder 5 Methylengruppen, von denen eine $CH_2$-Gruppe durch Sauerstoff, Schwefel, NH, $N-CH_3$ oder N-Benzyl ersetzt sein kann;

R(B), R(C) und R(D)

unabhängig wie R(A) definiert;
x Null, 1 oder 2;
y Null, 1 oder 2;
R(F) Wasserstoff, F, Cl, Br, I, CN, OR(12), $(C_1-C_8)$-Alkyl, $O_p(CH_2)_fC_gF_{2g+1}$, $(C_3-C_8)$-Cycloalkyl oder $(C_1-C_9)$-Heteroaryl;

p Null oder 1;
f Null, 1, 2, 3 oder 4;
g 1, 2, 3, 4, 5, 6, 7 oder 8;
R(12) $(C_1-C_8)$-Alkyl, $(C_1-C_4)$-Perfluoralkyl, $(C_3-C_8)$-Alkenyl, $(C_3-C_8)$-Cycloalkyl, Phenyl oder Benzyl,

wobei der Phenylkern nicht substituiert ist oder substituiert 1 - 3 Substituenten ausgewählt aus der Gruppe bestehend aus F, Cl, $CF_3$, Methyl, Methoxy und NR(13)R(14);
R(13) und R(14)

H, $(C_1-C_4)$-Alkyl oder $(C_1-C_4)$-Perfluoralkyl;

R(E) unabhängig wie R(F) definiert;

R(1) unabhängig wie T definiert;
oder
R(1) Wasserstoff, $-O_kC_mH_{2m+1}$, $-O_n(CH_2)_pC_qF_{2q+1}$, F, Cl, Br, I, CN, - (C=O)-N=C$(NH_2)_2$, $-SO_rR(17)$, $-SO_{r2}NR(31)R(32)$; $-O_u(CH_2)_vC_6H_5$, $-O_{u2}-(C_1-C_9)$-Heteroaryl oder $-S_{u2}-(C_1-C_9)$-Heteroaryl;

k Null oder 1;
m Null, 1, 2, 3, 4, 5, 6, 7 oder 8;
n Null oder 1;
p Null, 1, 2, 3 oder 4;
q 1, 2, 3, 4, 5, 6, 7 oder 8;
r Null, 1, 2;
r2 Null, 1, 2;
R(31) und R(32)

unabhängig voneinander Wasserstoff, $(C_1-C_8)$-Alkyl oder $(C_1-C_8)$-Perfluoralkyl;

oder
R(31) und R(32)

gemeinsam 4 oder 5 Methylengruppen, von denen eine $CH_2$-Gruppe durch Sauerstoff, S, NH, N-$CH_3$ oder N-Benzyl ersetzt sein kann;

R(17) $(C_1-C_8)$-Alkyl;
u Null oder 1;
u2 Null oder 1;
v Null, 1, 2, 3 oder 4;

wobei der Phenylkern substituiert ist oder substituiert mit 1 - 3 Substituenten ausgewählt aus der Gruppe bestehend aus F, Cl, $CF_3$, Methyl, Methoxy, $-(CH_2)_wNR(21)R(22)$, NR(18)R(19) und $(C_1-C_9)$-Heteroaryl;
R(18), R(19), R(21) und R(22)

unabhängig voneinander $(C_1-C_4)$-Alkyl oder $(C_1-C_4)$-Perfluoralkyl;

w 1, 2, 3 oder 4;
wobei der Heterocyclus des $(C_1-C_9)$-Heteroaryl unsubstituiert oder substituiert ist mit 1 - 3 Substituenten ausgewählt aus der Gruppe bestehend aus F, Cl, $CF_3$, Methyl oder Methoxy;

R(2), R(3), R(4) und R(5)

unabhängig voneinander wie R(1) definiert,

oder
R(1) und R(2) oder R(2) und R(3)

jeweils gemeinsam -CH-CH=CH-CH-,

das nicht substituiert oder substituiert ist mit 1 - 3 Substituenten ausgewählt aus der Gruppe bestehend aus F, Cl, $CF_3$, Methyl, Methoxy, - $(CH_2)_{w2}$NR(24)R(25) und NR(26)R(27);
R(24), R(25), R(26) und R(27)

H, ($C_1$-$C_4$)-Alkyl oder ($C_1$-$C_4$)-Perfluoralkyl;

w2 1, 2, 3 oder 4;

wobei der Rest T im Molekül mindestens zweimal, jedoch nur höchstens dreimal vorhanden ist;
sowie deren pharmazeutisch verträgliche Salze.

(HOE 97/ F 082)
be) Benzoylguanidine der Formel I

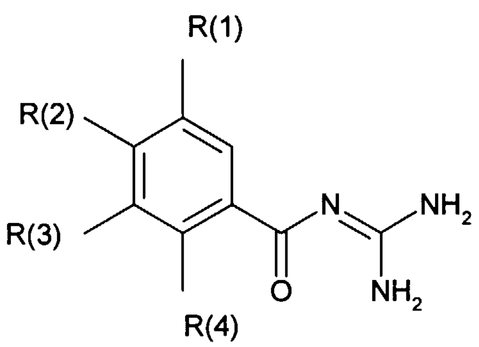

I

worin bedeuten:

R(1) $CF_3$;
einer der Substituenten R(2) und R(3)

Wasserstoff;

und der jeweils andere Substituent R(2) und R(3)

$-C(OH)(CH_3)-CH_2OH$, $-CH(CH_3)-CH_2OH$ oder $-C(OH)(CH_3)_2$;

R(4) Methyl, Methoxy, Cl oder $CF_3$;
sowie deren pharmazeutisch verträgliche Salze;

(DE 195 02 895, DE 44 30 212, EP 667 341, DE 44 04 183, EP 708 088, EP 723 963, EP 0 694 537, DE 44 21 495, EP 699 660, EP 699 663, EP 699 666, DE 43 37 611, EP 0719 766, WO 94/26709, WO 96 04 241, EP 726 254, US 4 251 545, DE 35 02 629, WO 84/00875, Kumamoto et al., Pharm. Bull. [1966], 7 - 13; US 3 780 027, JP 8225513; EP 743 301)
II. Und Verbindungen der Formel

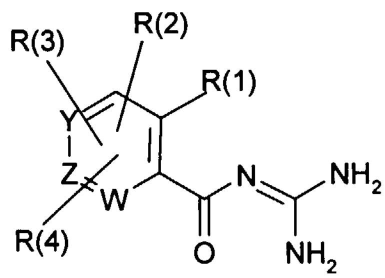

worin bedeuten:

W, Y und Z

ein Stickstoffatom oder ein mit R(2) oder R(3) oder R(4) substituiertes C-atom;

R(1) Wasserstoff, A, Hal, $-CF_3$, $-CH_2F$, $-CHF_2$, $-CH_2CF_3$, $-C_2F_5$, -CN, - $NO_2$, - Ethinyl, oder einen X-R';

A Alkyl mit 1 bis 6 C-Atomen;
Hal F, Cl, Br oder I;
X Sauerstoff, S oder NR'';

R'' Wasserstoff, A oder eine cyclische Methylenkette mit 3 bis 7 C-Atomen;

R' H, A, HO-A-, HOOC-A-, $(C_3$-$C_7)$-Cycloalkyl, $(C_6$-$C_8)$-Cycloalkyl-alkyl, $CF_3$, $CH_2F$, $CHF_2$, $CH_2$-$CF_3$, Ph, -$CH_2$-Ph oder Het;

Ph unsubstituiertes oder ein-, zwei-, oder dreifach durch A, OA, NR'R'', Hal, $CF_3$ substituiertes Phenyl, Naphthyl, oder Biphenylyl;
Het einen ein- oder zweikernigen gesättigten, ungesättigten oder aromatischen Heterocyclus mit 1 bis 4 N-, O-, und/oder S-Atomen,

der unsubstituiert oder ein-, zwei- oder dreifach substitiert ist durch Hal, $CF_3$, A, OH, OA, -X-R', -CN, -$NO_2$, und/oder Carbonylsauerstoff,

wobei Het über N oder oder eine Alkylenkette $C_mH_{2m}$ mit m = Null bis 6 gebunden ist;

oder
R' und R''

zusammen Alkylen mit 4 - 5 C-Atomen, worin eine $CH_2$-Gruppe auch durch Sauerstoff, S, NH, N-A, N-Ph und N-$CH_2$-Ph ersetzt sein kann;

R(2) und R(3)

unabhängig voneinander Wasserstoff, Hal, A, HO-A-, X-R', -C(=N-OH)-A, A-O-CO-$(C_1$-$C_4)$-Alkyl-, CN, $NO_2$, COOH, halogensubstituiertes A, insbesondere $CF_3$, $CH_2F$, $CHF_2$, $C_2F_5$, $CH_2CF_3$, oder $S(O)_nR'''$;
R''' A, Ph oder -Het;
n Null, 1 oder 2;

oder
R(2) und R(3)

unabhängig voneinander $SO_2NR'R''$, Ph oder -O-Ph, -O-$CH_2$-Ph, -CO-A, -CHO, -COOA, -CSNR'R'', CONR'R'', -CH=CH-COOH, -CH=CH-COOA, Indenyl, Indanyl, Decahydronaphthyl, Cyclopentenyl, Dihydrothienyl, Dihydrofuryl, Heterobicyclyl, Alkylthienyl, Halothienyl, Haloalkylthienyl, Acylthienyl, Halofuryl,

Haloalkylfuryl oder Pyrrolyl;

oder
R(2) und R(3)

unabhängig voneinander R(5)-O-;
R(5) Wasserstoff, A, ($C_1$-$C_6$)-Alkenyl oder ($C_3$-$C_7$)-Cycloalkyl;

R(4) Ph, Het, -O-Het; $CF_3$, $S(O)_nR'''$, $-SO_2NR'R''$, Alk;

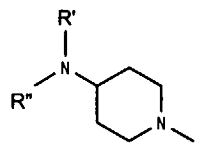 ;

oder
zwei der Substituenten R(1) bis R(4)

miteinander eine Gruppe -O-CR(6)R(7)-CO-NR(8)-,

oder 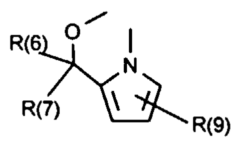 oder 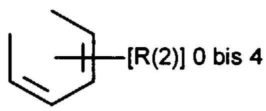 ;

mit R(2) in der angegebenen Bedeutung;

R(6), R(7), R(8) und R(9)

unabhängig voneinander H oder A;

oder
R(8) ($C_5$-$C_7$)-Cycloalkyl;
oder
R(9) Cyano;

Alk geradkettiges oder verzweigtes ($C_1$-$C_8$)-Alkyl oder ($C_3$-$C_8$)-Cycloalkyl, welches unsubstituiert oder durch A ein-, zwei- oder dreifach substituiert ist;
oder
Alk ein durch H, A, Ph oder Het substituierter Ethenyl oder Ethinylrest;

(EP 708 091, EP 622 356, JP 5-125085)
III. Und ebenfalls Indoloylguanidin-Derivate der Formel

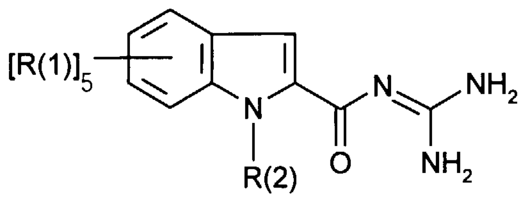

worin bedeuten

R(2) Wasserstoff, unsubstituiertes oder substituiertes ($C_1$-$C_8$)-Alkyl, ($C_3$-$C_7$)-Cycloalkyl, OH, ($C_1$-$C_6$)-Alkyl-O-, einen aromatischen Rest oder eine Gruppe -$CH_2$-R(20);

R(20) ($C_2$-$C_6$)-Alkenyl oder ($C_2$-$C_6$)-Alkinyl;

R(1) 1 bis 5 gleiche oder verschiedene Substituenten, die bedeuten:

Wasserstoff, unsubstituiertes oder substituiertes ($C_1$-$C_8$)-Alkyl, ($C_2$-$C_6$)-Alkenyl, ($C_2$-$C_6$)-Alkinyl, ($C_3$-$C_7$)-Cycloalkyl, Halogen, -$NO_2$, ($C_2$-$C_8$)-Alkanoyl, Arylalkanoyl mit bis zu 10 C-Atomen, Aroyl mit bis zu 11 C-Atomen, - COOH, ($C_2$-$C_6$)-Alkoxycarbonyl, eine aromatische Gruppe, eine der nachfolgend aufgeführten Gruppen: -OR(3), -NR(6)R(7) oder -S(O)$_n$R(40);
R(3) Wasserstoff, ($C_1$-$C_8$)-Alkyl, substituiertes ($C_1$-$C_8$)-Alkyl, ($C_3$-$C_7$)-Cycloalkyl, einen aromatischen Rest oder eine Gruppe -$CH_2$-R(30) R(30) Alkenyl oder Alkinyl;
R(6) und R(7)

unabhängig voneinander Wasserstoff, unsubstituiertes oder substituiertes ($C_1$-$C_8$)-Alkyl, ($C_3$-$C_7$)-Cycloalkyl, ($C_2$-$C_8$)-Alkanoyl, eine Arylalkanoylgruppe mit bis zu 10 C-Atomen, eine Aroylgruppe mit bis zu 11 C-Atomen, eine aromatische Gruppe oder -$CH_2$-R(60);
R(60) ($C_2$-$C_6$)-Alkenyl oder ($C_2$-$C_6$)-Alkinyl;

oder
R(6) und R(7)

gemeinsam mit dem N-Atom ein 5 - 7-gliedriges cyclisches Amin, das noch weitere Heteroatome im Ring enthalten kann;

n Null, 1 oder 2;
R(40) unsubstituiertes oder substituiertes ($C_1$-$C_8$)-Alkyl, oder eine aromatische Gruppe, oder eine Gruppe

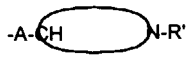

A Sauerstoff , -S(O)$_n$- oder -N(R50)-;

R(50) Wasserstoff oder ($C_1$-$C_8$)-Alkyl;

R' Wasserstoff, unsubstituiertes oder substituiertes ($C_1$-$C_8$)-Alkyl,
worin der Ring einen gesättigten 3 - 8 gliedrigen Heterocyclus mit einem N-atom repräsentiert,

besagtes substituiertes Alkyl eine oder mehrere Gruppen trägt ausgewählt aus der Gruppe bestehend aus Halogen, -OH, ($C_1$-$C_6$)-Alkoxy, -CN, - COOH, ($C_2$-$C_6$)-Alkoxycarbonyl, ($C_2$-$C_8$)-Alkanoyl, Arylalkanoyl mit bis zu 10 C-Atomen, Aroyl mit bis zu 11 C-Atomen, eine aromatische Gruppe, - CONR(4)(R5),
R(4) und R(5)

gleich oder verschieden Wasserstoff oder ($C_1$-$C_8$)-Alkyl;

oder
R(4) und R(5)

miteinander verbunden und bilden zusammen ein 5 - 7-gliedriges cyclisches Amin, das noch weitere Heteroatome im Ring enthalten kann,

oder besagtes substituiertes Alkyl eine Gruppe

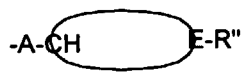

trägt, worin bedeuten:
E ein N-Atom oder eine CH-Gruppe;
R" Wasserstoff, unsubstituiertes oder mit OH substituiertes ($C_1$-$C_8$)-Alkyl oder substituiertes ($C_1$-$C_8$)-Alkyl, ($C_1$-$C_6$)-Alkoxy, -CN, -COOH, ($C_2$-$C_6$)-Alkoxycarbonyl, ($C_2$-$C_8$)-Alkanoyl, Aralkanoyl mit bis zu 10 C-Atomen, Aroyl mit bis zu 11 C-Atomen, eine aromatische Gruppe, -NR(6)R(7), -CONR(4)R(5);
R(4) und R(5)

unabhängig voneinander Wasserstoff oder ($C_1$-$C_8$)-Alkyl;

wobei das cyclische System der Forme

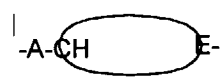

ein 3 - 8 gliedriges gesättigtes aliphatisches oder heterocyclisches Ringsystem mit einem N-Atom bedeutet,

und wobei die erwähnten aromatischen Gruppen einen Arylrest mit bis zu 10 C-Atomen, einen 5- oder 6-gliedrigen Heteroarylrest mit 1-4 N-Atomen, eine 5- oder 6-gliedrige Heteroarylgruppe enthaltend 1 oder 2 N-Atome und ein Heteroatom in der Bedeutung von Sauerstoff oder Schwefel, oder Furyl bedeuten,
und wobei die erwähnten Arylreste unsubstituiert oder substituiert sein können durch unsubstituiertes ($C_1$-$C_8$)-Alkyl oder substituiertes ($C_1$-$C_8$)-Alkyl, Halogen, - $NO_2$, ($C_2$-$C_6$)-Alkoxycarbonyl, COOH, -OR(3), NR(6)R(7), -CONR(4)R(5), -$SO_2$NR(6)R(7) oder $S(O)_n$R(40),
wobei R(1) und der Guanidinocarbonylrest an einer beliebigen Stelle des 5- oder 6-gliedrigen Rings des Indolsystems stehen können,
sowie die zugehörigen pharmazeutisch verträglichen Salze;

(WO 95 04052)
IV. Und geeignet heterocyclische Guanidin-Derivate der Formel

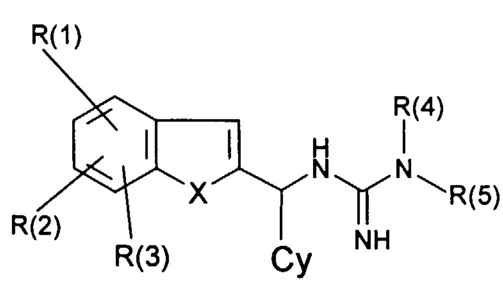

worin bedeuten:

X -O-, -S-, -NH-, -N[($C_1$-$C_4$)-Alkyl]- oder -N(Phenyl)-;
R(1), R(2) und R(3)

Wasserstoff, Halogen, ($C_1$-$C_4$)-Alkyl, ($C_1$-$C_4$)-Alkyl-O-, Phenyl, Benzyl;

oder
zwei der Substituenten R(1), R(2) und R(3)

zusammen mit einer Seite des Benzosystems einen 4 - 6-gliedrigen carbocyclischen Ring;

R(4) und R(5)

unabhängig voneinander Wasserstoff, ($C_1$-$C_{12}$)-Alkyl, Benzhydryl, Aralkyl,

das unsubstituiert oder substituiert ist durch einen oder mehrere Substituenten aus den Gruppen Halogen, ($C_1$-$C_4$)-Alkyl, ($C_1$-$C_4$)-Alkyl-O-oder -$CF_3$, -$(CH_2)_m$-$CH_2$-T,
m Null bis 3;
T -CO-O-T(1);

T(1) Wasserstoff oder (C1-C4)-Alkyl;

Cy einen benzokondensierten ungesättigten oder Dihydro-5-Ringheterocyclus

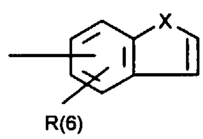 ,

einen Pyrazol- oder Imidazolring der Formel

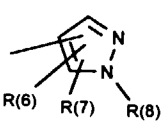 bzw. 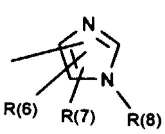

einen Naphthylrest oder ein Dihydro- oder Tetrahydronaphthylrest

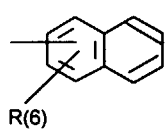

einen 2-, 3- oder 4-Pyridylrest

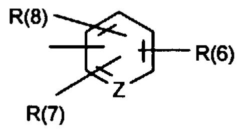

Z N- oder CH;

einen Thienylrest

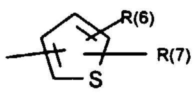

R(6) Wasserstoff, Halogen, Hydroxy, $(C_1-C_{10})$-Alkyl, $(C_1-C_{10})$-Alkyl-O-, Phenoxy, $(C_1-C_{10})$-Alkyloxymethyloxy- oder $-(O)_nS-R(9)$;

R(9) $(C_1-C_{10})$-Alkyl, Thienyl, Pyridyl-, Thiazolyl, Thiadiazolyl, Imidazolyl, Pyrazolyl oder Phenyl,

die unsubstituiert oder ein- oder zweifach substituiert sind mit Halogen, $(C_1-C_4)$-Alkyl oder $(C_1-C_4)$-Alkyl-O-;

R(7) und R(8)

Wasserstoff, Halogen, Hydroxy, $(C_1-C_{10})$-Alkyl, $(C_1-C_{10})$-Alkyl-O-, Phenyl, Phenoxy oder $(C_1-C_{10})$-Alkoxymethyloxy;

oder
Cy Phenyl,

das unsubstituiert ist oder ein- oder zweifach substituiert ist mit Halogen, $(C_1-C_4)$-Alkyl oder $(C_1-C_4)$-Alkyl-O-;

oder
Cy -Gr-Am;

Gr $-R(13)-R(12)-(CH_2)_q-C[W][W(1)]-(CH_2)_{q'}-$; R(13)R(14)- oder - R(15)-;

R(12) eine Einfachbindung, -O-, $-(O)_nS-$, -CO- oder -CONH-;
R(13) eine Einfachbindung, Phenyl, Thienyl, Pyridyl, Thiazolyl, Thiadiazolyl, Imidazolyl oder Pyrazolyl;
R(14) eine Einfachbindung $SO_2$-;
R(15) $(C_2 - C_{10})$-Alkenyl- oder $(C_2-C_{10})$-Alkinyl;
W und W(1)

unabhängig voneinander Wasserstoff, $(C_1-C_4)$-Alkyl;

oder
W und W(1)

miteinander cyclisch verbunden einen $(C_3-C_8)$-Kohlenwasserstoffring;

q und q'

Null bis 9;

Am -NR(10)R(11);

R(10) Wasserstoff, ($C_1$-$C_4$)-Alkyl oder Benzyl,
R(11) ($C_1$-$C_4$)-Alkyl, Phenyl oder Benzyl; oder
R(10) und R(11)

gemeinsam eine ($C_3$-$C_{10}$)-Alkylengruppe,

die unsubstituiert ist oder substituiert mit -COOH, ($C_1$-$C_5$)-Alkoxycarbonyl, ($C_2$-$C_4$)-Hydroxyalkylen oder Benzyl;

oder
Am Pyrrolyl, Pyridyl, Pyrazolyl, Morpholinyl, Dihydropyridyl, Tetrahydropyridyl, Chinuclidinyl, Imidazolyl, 3-Azabicyclo[3.2.1]octyl,

das unsubstituiert ist oder substituiert mit ($C_1$-$C_4$)-Alkyl,

oder
Am Azabicyclo[3.2.2]nonyl;
oder
Am eine Piperazingruppe der Formel

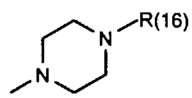

R(16) Wasserstoff, ($C_1$-$C_4$)-Alkyl, ($C_3$-$C_6$)-Cycloalkyl, Phenyl, Tolyl, Methoxyphenyl, Halophenyl, Diphenylmethylen, Benzyl oder Pyridyl;

oder
Am eine Azidogruppe $-(O)_t-(CH2)_q-C[W][W(1)]-(CH2)q'-N_3$;

t Null oder 1;
wobei W und W(1) die vorher angegebene Bedeutung besitzen;

sowie die optischen Enantiomeren und die pharmakologisch verträglichen Salze;

V. Und ferner die Guanidin-Verbindungen, wie sie beschrieben werden in
EP- 743 301 (DE 195 17 848), EP 758 644 (DE 195 29 612), EP 760 365 (DE 195 31 138)

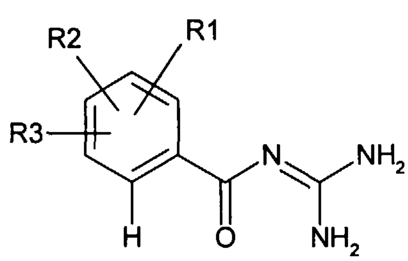

mit R1 = R2 gleich H, Halo, Alkyl, CN, $NO_2$, Perfluoralkyl, $SO_nCF_3$; R3 gleich $CH=CH_2$, $CH_2-CH=CH_2$, $CH_2-CH_2-$

$CH=CH_2$, Cycloalkenyl, Cycloalkenylalkyl; R4 gleich Alkyl, (substituiertes) Phenyl, oder wie beschrieben in DE 195 48 708, WO 97 25 310, WO 97 27 183, DE 196 01 303, EP 787 728, JP 82 25 513, JP 090 59 245, JP 090 67 332, JP 090 67 340, WO 97 11 055 und EP 743 301.

**13.** Verwendung nach Ansprüchen 1 - 11, dadurch gekennzeichnet, daß eine Verbindung benutzt wird, die ausgewählt ist aus der Gruppe bestehend aus den Verbindungen
I. (HOE 89/F 288 - US 5 292 755)
a) Benzoylguanidine der Formel I

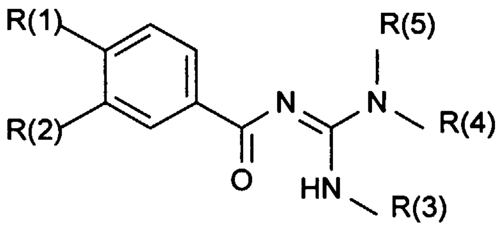

I

worin bedeuten:

R(1) oder R(2)

R(6)-S(O)$_n$- oder R(7)R(8)N-$O_2$S-;

und der jeweils andere Substituent R(1) oder R(2)

H, F, Cl, Br, ($C_1$-$C_4$)-Alkyl, ($C_1$-$C_4$)-Alkoxy oder Phenoxy,

das unsubstituiert ist oder substituiert mit 1 - 3 Substituenten ausgewählt aus der Gruppe bestehend aus Fluor, Chlor, Methyl und Methoxy;

oder der jeweils andere Substituent R(1) oder R(2)

R(6)-S(O)$_n$ oder R(7)R(8)N-;
n Null, 1 oder 2;
R(6) ($C_1$-$C_6$)-Alkyl, ($C_5$-$C_7$)-Cycloalkyl, Cyclopentylmethyl, Cyclohexylmethyl oder Phenyl,

das unsubstituiert ist oder substituiert mit 1 - 3 Substituenten ausgewählt aus der Gruppe bestehend aus Fluor, Chlor, Methyl und Methoxy;

R(7) und R(8)

gleich oder verschieden H oder ($C_1$-$C_6$)-Alkyl;

oder
R(7) Phenyl-$(CH_2)_m$;

m 1 - 4;

oder
R(7) Phenyl,

das unsubstituiert ist oder substituiert mit 1 - 2 Substituenten ausgewählt aus der Gruppe bestehend aus Fluor, Chlor, Methyl und Methoxy;

oder
R(7) und R(8)

gemeinsam eine geradkettige oder verzweigte ($C_4$-$C_7$)-Kette, wobei die Kette zusätzlich durch O, S oder NR(9) unterbrochen sein kann; R(9) H oder Methyl;

oder
R(7) und R(8)

gemeinsam mit dem Stickstoffatom, an das sie gebunden sind, ein Dihydroindol-, Tetrahydrochinolin- oder Tetrahydroisochinolin-System;

R(3), R(4) und R(5)

unabhängig voneinander H oder ($C_1$-$C_2$)-Alkyl,

oder
R(3) und R(4)

gemeinsam eine ($C_2$-$C_4$)-Alkylenkette;

oder
R(4) und R(5)

gemeinsam eine ($C_4$-$C_7$)-Alkylenkette;

sowie deren pharmazeutisch verträgliche Salze;

(HOE 92/F 34 - US 5 373 924)
b) Benzoylguanidine der Formel I

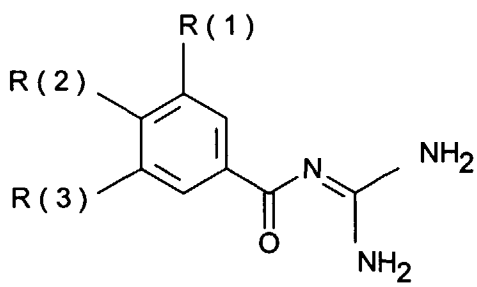

(I)

worin bedeuten:

R(1) R(4)-$SO_m$ oder R(5)R(6)N-$SO_2$-;

m Null, 1 oder 2;
R(4) und R(5)

$C_1$-$C_8$-Alkyl, $C_3$-$C_6$-Alkenyl oder -$C_nH_{2n}$-R(7);
n Null, 1, 2, 3 oder 4;
R(7) $C_5$-$C_7$-Cycloalkyl oder Phenyl,

welches unsubstituiert ist oder substituiert mit 1 - 3 Substituenten ausgewählt aus der Gruppe bestehend aus F, Cl, $CF_3$, Methyl, Methoxy und NR(8)R(9);
R(8) und R(9)

H oder $C_1$-$C_4$-Alkyl;

oder
R(5) H;

R(6) H oder $C_1$-$C_4$-Alkyl,
oder
R(5) und R(6)

gemeinsam 4 oder 5 Methylengruppen, von denen eine $CH_2$-Gruppe durch ein O, S, NH, N-$CH_3$ oder N-Benzyl ersetzt sein kann;

R(2) Wasserstoff, F, Cl, Br, ($C_1$-$C_4$)-Alkyl-, O-$(CH_2)_mC_pF_{2p+1}$ oder - X-R(10);

m Null oder 1;
p 1, 2 oder 3;
X O, S oder NR(11);
R(10) H, $C_1$-$C_6$-Alkyl, $C_5$-$C_7$-Cycloalkyl, Cyclohexylmethyl, Cyclopentylmethyl oder -$C_nH_{2n}$-R(12);

n Null, 1, 2, 3 oder 4;
R(12) Phenyl,

das unsubstituiert oder substituiert ist mit 1 - 3 Substituenten ausgewählt aus der Gruppe bestehend aus F, Cl, $CF_3$, Methyl, Methoxy und NR(8)R(9);
R(8) und R(9)

gleich H oder $C_1$-$C_4$-Alkyl;

R(11) Wasserstoff oder $C_1$-$C_3$-Alkyl;
oder
R(10) und R(11)

gemeinsam 4 oder 5 Methylengruppen, von denen eine $CH_2$-Gruppe durch O, S, NH, N-$CH_3$ oder N-Benzyl ersetzt sein kann;

R(3) wie R(1) definiert, oder $C_1$-$C_6$-Alkyl, Nitro, Cyano, Trifluormethyl, F, Cl, Br, J oder-X-R(10);

X O, S oder NR(11);

R(10) gleich H, $C_1$-$C_6$-Alkyl, $C_5$-$C_7$-Cycloalkyl, Cyclohexylmethyl, Cyclopentylmethyl oder -$C_nH_{2n}$-R(12);
n null bis 4;
R(12) Phenyl,

das unsubstituiert ist oder substituiert mit 1 - 3 Substituenten ausgewählt aus der Gruppe bestehend aus F, Cl, $CF_3$, Methyl, Methoxy und NR(8)R(9);
R(8) und R(9)

H oder $C_1$-$C_4$-Alkyl;

R(11) $C_1$-$C_3$-Alkyl,
oder
R(10) und R(11)

gemeinsam 4 oder 5 Methylengruppen, von denen eine $CH_2$-Gruppe durch O, S, NH, N-$CH_3$ oder N-Benzyl ersetzt sein kann;

sowie deren pharmazeutisch verträgliche Salze;

(HOE 92/F 303 K - EP-OS589 336, NZ 248 703)
f) Benzoylguanidine der Formel I

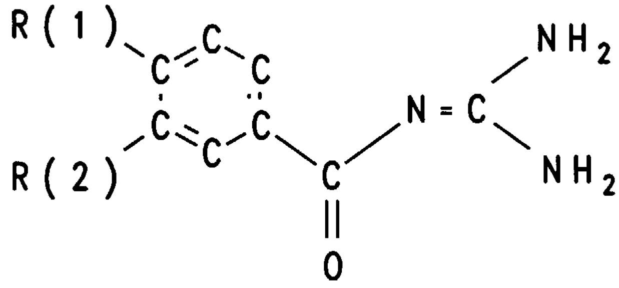

I

worin bedeuten:

R(1) oder R(2)

R(3)-S(O)$_n$- oder R(4)R(5)N-SO$_2$-

der jeweils andere Substituent R(1) oder R(2)

H, OH, F, Cl, Br, J, $C_1$-$C_4$-Alkyl, $C_1$-$C_4$-Alkoxy, Benzyloxy oder Phenoxy,

das unsubstituiert ist oder einen bis drei Substituenten ausgewählt aus der Gruppe bestehend aus Fluor, Chlor, Methyl, Methoxy, Hydroxy oder Benzyloxy trägt,

R(3)-S(O)$_n$, -NR4)R(5) oder 3,4-Dehydropiperidin
R(3) $C_1$-$C_6$-Alkyl, $C_5$-$C_7$-Cycloalkyl, Cyclopentylmethyl, Cyclohexylmethyl oder Phenyl,

das unsubstituiert ist oder einen bis drei Substituenten ausgewählt aus der Gruppe bestehend aus Fluor, Chlor, Methyl und Methoxy trägt;

R(4) und R(5)

gleich oder verschieden, H oder $C_1$-$C_6$-Alkyl;

oder
R(4) Phenyl-($CH_2$)$_m$-;

m 1, 2, 3 oder 4;

oder
R(4) Phenyl,

das unsubstituiert ist oder einen bis zwei Substituenten ausgewählt aus der Gruppe bestehend aus Fluor, Chlor, Methyl und Methoxy trägt;

oder
R(4) und R(5)
gemeinsam eine geradkettige oder verzweigte $C_4$-$C_7$-Kette, wobei die Kette zusätzlich durch O, S oder NR(6) unterbrochen sein kann,
R(6) H oder Methyl;
oder
R(4) und R(5)

gemeinsam mit dem Stickstoffatom, an das sie gebunden sind, ein Dihydroindol, Tetrahydrochinolin oder Tetrahydroisochinolin-System;
n Null, 1 oder 2;

sowie deren pharmazeutisch verträgliche Salze.

(HOE 92/F 411 - NZ 250 450, EP 603 650)
k) Benzoylguanidine der Formel I

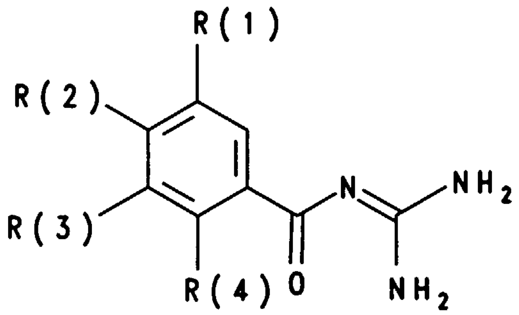

(I)

worin bedeuten:

einer der Substituenten R(1), R(2), R(3) oder R(4):

eine Aminogruppe

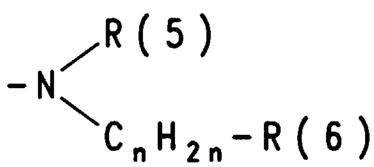

;

R(5) Wasserstoff oder $C_{(1-6)}$-Alkyl;
n Null, 1, 2, 3 oder 4;
R(6) H oder $C_{(1-4)}$-Alkyl;
worin eine $CH_2$-Gruppe durch 1 S-Atom oder eine Gruppe NR(7) ersetzt sein kann;
R(7) Wasserstoff, Methyl oder Ethyl;
oder
R(6) $C_{(3-8)}$-Cycloalkyl oder Phenyl,

das unsubstituiert ist oder 1, 2 oder 3 Substituenten ausgewählt aus der Gruppe bestehend aus F, Cl, Br, Methyl, Methoxy, -NR(8)R(9) trägt;
R(8) und R(9)

H, Methyl oder Ethyl;

oder
R(5) und R(6)

gemeinsam mit dem Stickstoffatom einen 5-, 6- oder 7-gliedrigen Ring, in welchem 1 C-Atom durch Sauerstoff, S oder NR(10) ersetzt sein kann;
R(10) H, $C_{(1-3)}$-Alkyl oder Benzyl;

und die jeweils anderen Substituenten R(1), R(2), R(3), R(4):

Wasserstoff, F, Cl, Br, I, CN, $CF_3$, $NO_2$, $CF_3$-O-, $C_mF_{2m+1}$-$CH_2$-O- oder R(11)-$C_qH_{2q}$-$X_p$-;
m 1, 2 oder 3;
q Null, 1, 2, 3 oder 4;

p Null oder 1;
X Sauerstoff oder NR(12);

R(12) H oder $C_{(1-3)}$-Alkyl;

R(11) Wasserstoff, $C_{(1-6)}$-Alkyl, $C_{(3-8)}$-Cycloalkyl oder Phenyl,

das unsubstituiert oder substituiert ist mit 1, 2 oder 3 Substituenten ausgewählt aus der Gruppe bestehend aus F, Cl, $CH_3$, $CH_3$-O-und NR(13)R(14);
R(13), R(14)

H, Methyl oder Ethyl;

sowie deren pharmazeutisch verträgliche Salze;

(93/F 054 - NZ 250 919, EP-OS 612 723)
m) Benzoylguanidine der Formel I

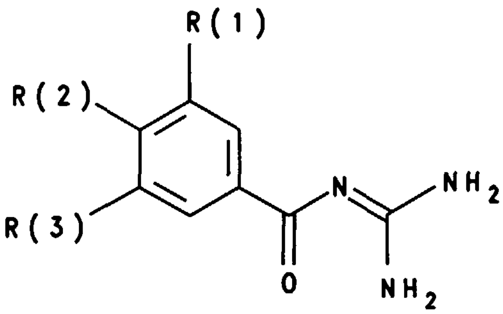

(I)

worin bedeuten:

R(1), R(2), R(3)

Wasserstoff, F, Cl, Br, I oder ($C_1$-$C_{12}$)-Alkyl;

einer der Substituenten R(1), R(2) oder R(3)

$N_3$, CN, OH oder ($C_1$-$C_{10}$)-Alkyloxy, wenn mindestens einer der restlichen Substituenten R(1), R(2) oder R(3) einen hinreichend lipophilen Alkylrest mit 3 bis 12 Kohlenstoff-Atomen bedeutet;

oder
einer der Substituenten R(1), R(2) oder R(3)

R(4)-$C_nH_{2n}$-$O_m$-;
m Null oder 1;
n Null, 1, 2 oder 3;
R(4) $C_pF_{2p+1}$;

p 1, 2 oder 3, sofern n gleich Null oder 1 ist;

oder
R(4) ($C_3$-$C_{12}$)-Cycloalkyl, Phenyl, Pyridyl, Chinolyl oder Isochinolyl, wobei die aromatischen und heteroaromatischen Ringsysteme unsubstituiert sind oder substituiert mit einem Substituenten ausgewählt aus der Gruppe bestehend aus F, Cl, $CF_3$, Methyl, Methoxy und NR(5)R(6);
R(5) und R(6)

Wasserstoff oder ($C_1$-$C_4$)-Alkyl;

oder einer der Substituenten R(1), R(2) oder R(3)

-C≡CR(5) oder -C[R(6)]=CR(5);
R(5) Phenyl,

das unsubstituiert oder substituiert ist mit 1 - 3 Substituenten ausgewählt aus der Gruppe bestehend aus F, Cl, $CF_3$, Methyl, Methoxy, Hydroxy, Amino, Methylamino und Dimethylamino, ($C_1$-$C_9$)-Heteroaryl,

das unsubstituiert oder wie Phenyl substituiert ist,

oder
R(5) ($C_1$-$C_6$)-Alkyl,

das unsubstituiert oder mit 1 - 3 OH substituiert ist;

oder
R(5) ($C_3$-$C_8$)-Cycloalkyl,
R(6) Wasserstoff oder Methyl;

sowie deren pharmakologisch akzeptable Salze;

(93/F 153 - EP-OS 627 413, NZ 260 660)
o) Benzoylguanidine der Formel I

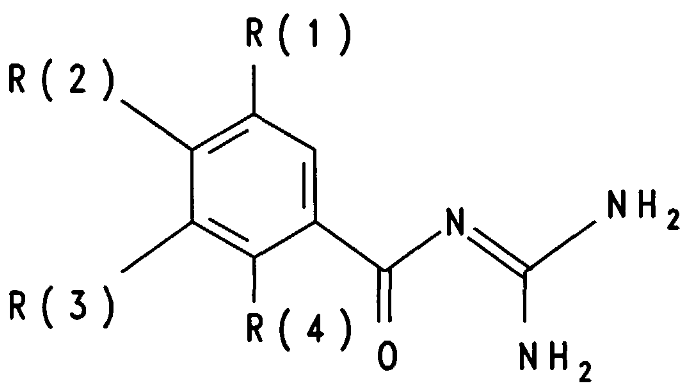

(I)

worin bedeuten:

R(1) Wasserstoff, F, Cl, Br, I, $-NO_2$, -C≡N, $X_o$-$(CH_2)_p$-$(CF_2)_q$-$CF_3$, R(5)-$SO_m$, R(6)-CO- oder R(6)R(7)N-$SO_2$-, mit

X Sauerstoff, S oder NR(14);
m Null, 1 oder 2;
o Null oder 1;
p Null, 1 oder 2;
q Null, 1, 2, 3, 4, 5 oder 6;
R(5) und R(6)

($C_1$-$C_8$)-Alkyl, ($C_3$-$C_6$)-Alkenyl, $-C_nH_{2n}$-R(8) oder $CF_3$;
n Null, 1, 2, 3 oder 4;
R(8) ($C_3$-$C_7$)-Cycloalkyl oder Phenyl,

welches nicht substituiert ist oder substituiert mit 1 - 3 Substituenten ausgewählt aus der Gruppe bestehend aus F, Cl, $CF_3$, Methyl, Methoxy und NR(9)R(10);
R(9) und R(10)

H oder $C_1$-$C_4$-Alkyl;

oder
R(6) H;
R(7) H oder ($C_1$-$C_4$)-Alkyl;
oder
R(6) und R(7)

gemeinsam 4 oder 5 Methylengruppen, von denen eine $CH_2$-Gruppe durch Sauerstoff, S, NH, N-$CH_3$ oder N-Benzyl ersetzt sein kann;

R(2)

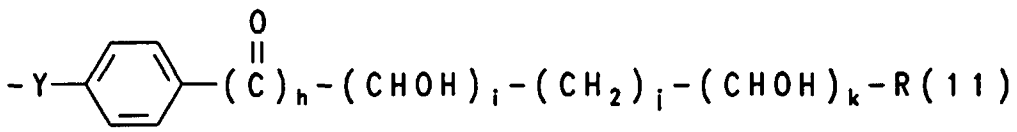

oder

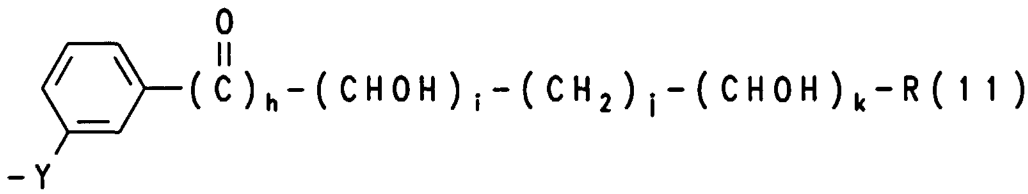

oder

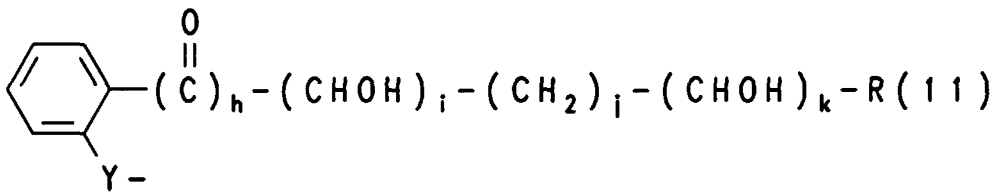

Y Sauerstoff, -S- oder -NR(12)-;
R(11) und R(12)

Wasserstoff oder ($C_1$-$C_3$)-Alkyl;

h Null oder 1;
i, j und k

unabhängig Null, 1, 2, 3 oder 4;

wobei jedoch nicht h, i und k gleichzeitig Null sind,

R(3) wie R(1) definiert, oder ($C_1$-$C_6$)-Alkyl oder -X-R(13);

X Sauerstoff, S oder NR(14);

R(14) H oder ($C_1$-$C_3$)-Alkyl;
R(13) H, ($C_1$-$C_6$)-Alkyl, ($C_3$-$C_8$)-Cycloalkyl oder -$C_bH_{2b}$-R(15);
b Null, 1, 2, 3 oder 4;
oder
R(13) und R(14)

gemeinsam 4 oder 5 Methylengruppen, wobei eine $CH_2$-Gruppe durch Sauerstoff, S, NH, N-$CH_3$ oder N-Benzyl ersetzt sein kann; R(15) Phenyl,

das unsubstituiert oder substituiert ist mit 1 - 3 Substituenten ausgewählt aus der Gruppe bestehend aus F, Cl, $CF_3$, Methyl, Methoxy und NR(9)R(10);
R(9) und R(10)

H oder ($C_1$-$C_4$)-Alkyl;

R(4) Wasserstoff, -OR(16) oder -NR(16)R(17);

R(16) und R(17)

unabhängig Wasserstoff oder ($C_1$-$C_3$)-Alkyl;

sowie deren pharmazeutisch verträgliche Salze;

(HOE 93/F 223 K - EP 639 573, NZ 264 130)
r) Benzokondensierte 5-Ringheterocyclen der Formel I

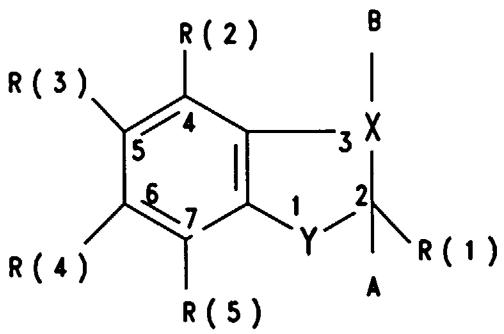

(I),

worin bedeuten:

X N oder CR(6);
Y Sauerstoff, S oder NR(7);
A, B gemeinsam eine Bindung
oder
A, B beide Wasserstoff, sofern gleichzeitig X gleich CR(6) und Y gleich NR(7) sind;
einer der Substituenten R(1) bis R(6) eine -CO-N=C($NH_2$)$_2$-Gruppe;
die jeweils anderen Substituenten R(1) bis R(6)

Wasserstoff, F, Cl, Br, I oder ($C_1$-$C_6$)-Alkyl;

bis zu zwei der anderen Substituenten R(1) bis R(6)

CN, $NO_2$, $N_3$, ($C_1$-$C_4$)-Alkyloxy oder $CF_3$;

bis zu einem der anderen Substituenten

R(8)-$C_nH_{2n}$-Z-;

n Null bis 10;

wobei die Alkylenkette $-C_nH_{2n}-$ geradkettig oder verzweigt ist und wobei ein C-Atom durch ein Sauerstoff- oder S-Atom oder durch ein N-Atom ersetzt sein kann;

R(8) Wasserstoff, $(C_2-C_6)$-Alkenyl oder $(C_3-C_{10})$-Cycloalkyl,

das unsubstituiert oder durch 1 bis 4 Methylgruppen oder eine OH-Gruppe substituiert ist, oder eine Ethylengruppe -CH=CH- enthalten kann, und worin eine Methylengruppe durch ein Sauerstoff- oder S-Atom oder durch ein N-Atom ersetzt sein kann;

oder
R(8) Phenyl,

unsubstituiert oder substituiert durch 1 bis 3 Substituenten ausgewählt aus der Gruppe bestehend aus F, Cl, Br, I, $CF_3$, $CH_3-S(O)_s-$ oder $R(9)-W_y-$;
s Null, 1 oder 2;
R(9) H, Methyl, Ethyl,
W Sauerstoff oder NR(10);

R(10) H oder Methyl;

y Null oder 1;

oder
R(8) $C_mF_{2m+1}$;

m 1 bis 3;

oder
R(8) 1 - oder 2-Naphthyl, Pyridyl, Chinolyl oder Isochinolyl;

Z -CO-, $-CH_2-$ oder $-[CR(11)(OH)_q-$;

q 1, 2 oder 3;
R(11) H oder Methyl;

oder
Z Sauerstoff oder -NR(12)-;

R(12) H oder Methyl;

oder
Z $-S(O)_s-$;

s Null, 1 oder 2;

oder
Z $-SO_2-NR(13)-$;

R(13) H oder $(C_1-C_4)$-Alkyl;

R(7) Wasserstoff, $(C_1-C_{10})$-Alkyl, $(C_2-C_{10})$-Alkenyl oder $R(8)-C_nH_{2n}-$;

sowie deren pharmazeutisch verträgliche Salze;

(HOE 94/F 134 - EP-OS 686 627, NZ 272 103)
z) Benzoylguanidine der Formel I

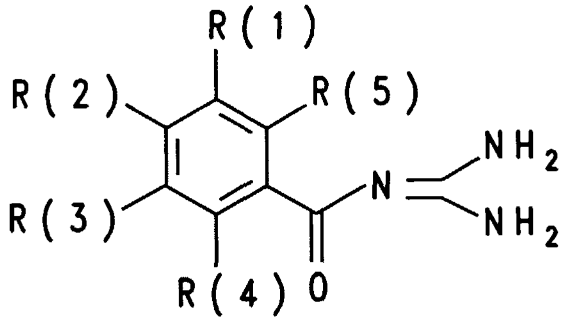

I

worin bedeuten:

R(1) R(6)-$SO_m$;

m Null, 1 oder 2;
R(6) Perfluoralkyl mit 1, 2, 3, 4, 5 oder 6 C-Atomen, das geradkettig oder verzweigt ist;

R(2) und R(3)

unabhängig voneinander Wasserstoff, F, Cl, Br, I, Alkyl mit 1, 2, 3 oder 4 C-Atomen, Alkoxy mit 1, 2, 3 oder 4 C-Atomen oder Phenoxy,

das unsubstituiert ist oder substituiert ist mit 1 - 3 Substituenten ausgewählt aus der Gruppe bestehend aus F, Cl, Methyl und Methoxy;

oder
R(2) und R(3)

unabhängig voneinander Pyrrol-1-yl, Pyrrol-2-yl- oder Pyrrol-3-yl,

welches nicht substituiert ist oder substituiert mit 1 bis 4 Substituenten, ausgewählt aus der Gruppe bestehend aus F, Cl, Br, I, CN, Alkanoyl mit 2, 3, 4, 5, 6, 7 oder 8 C-Atomen, Alkoxycarbonyl mit 2, 3, 4, 5, 6, 7 oder 8 C-Atomen, Formyl, Carboxy, $CF_3$, Methyl und Methoxy;

R(4) und R(5)

unabhängig voneinander Wasserstoff, Alkyl mit 1, 2 oder 3-C-Atomen, F, Cl, Br, I, CN, OR(7), NR(8)R(9) oder $-(CH_2)_n-(CF_2)_o-CF_3$;
R(7), R(8) und R(9)

unabhängig voneinander Wasserstoff oder Alkyl mit 1, 2, 3 oder 4 C-Atomen;

n Null oder 1;
o Null, 1 oder 2;

sowie deren pharmakologisch akzeptable Salze;

(HOE 95/F 007 K - EP-OS 723 956, NZ 280 887)
ag) Benzoylguanidine der Formel I

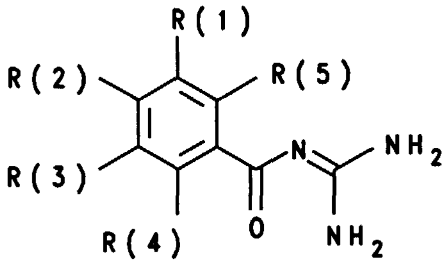

I

worin bedeuten:

einer der drei Substituenten R(1), R(2) und R(3)

R(6)-A-B-D-;
R(6) ein basischer protonierbarer Rest, d.h. eine Aminogruppe -NR(7)R(8), eine Amidinogruppe R(7)R(8)N-C[=N-R(9)]- oder eine Guanidinogruppe

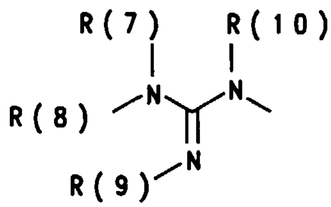

R(7), R(8), R(9) und R(10)

unabhängig voneinander Wasserstoff oder Alkyl mit 1, 2, 3 oder 4 C-Atomen;

oder
R(7) und R(8)

gemeinsam $C_aH_{2a}$;
a 4, 5, 6 oder 7;
wobei im Falle von a = 5, 6 oder 7 eine Methylengruppe der Gruppe $C_aH_{2a}$ durch eine Heteroatomgruppe O, $SO_m$ oder NR(11) ersetzt sein kann,

oder
R(8) und R(9) oder R(9) und R(10) oder R(7) und R(10)

eine Gruppe $C_aH_{2a}$;
a 2, 3, 4 oder 5;

wobei im Falle von a = 3, 4 oder 5 eine Methylengruppe der Gruppe $C_aH_{2a}$ durch eine Heteroatomgruppe O, $SO_m$ oder NR(11) ersetzt sein kann;

m Null, 1 oder 2;
R(11) Wasserstoff oder Methyl;

oder
R(6) ein basisches heteroaromatisches Ringsystem mit 1 - 9 C-Atomen;
A $C_bH_{2b}$;

b 1, 2, 3, 4, 5, 6, 7, 8, 9 oder 10;

wobei in der Gruppe $C_bH_{2b}$ ein oder zwei Methylengruppen durch eine der Gruppierungen ausgewählt aus der Gruppe bestehend aus -O-,-CO-, -CH[OR(20)]-, $-SO_m-$, -NR(20)-, -NR(20)-CO-, - NR(20)-CO-NH-, -NR(20)-CO-NH-$SO_2$-und

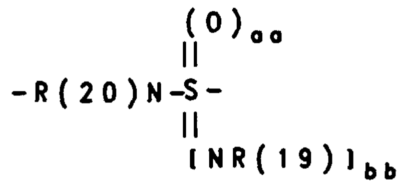

$-SO_{aa}[NR(19)]_{bb}-$ ersetzt sein können;
und wobei in der Gruppe $C_bH_{2b}$ eine Methylengruppe durch -CH-R(99) ersetzt sein kann, wobei R(99) gemeinsam mit R(7) einen Pyrrolidin- oder Piperidin-Ring bildet;
aa 1 oder 2;
bb 0 oder 1;
aa + bb = 2 ;
R(19) Wasserstoff oder Alkyl mit 1, 2, 3 oder 4 C-Atomen
R(20) Wasserstoff oder Methyl;

B einen Phenylen- oder Naphthylenrest,

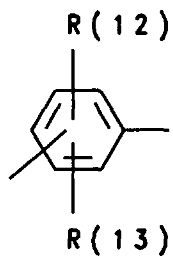

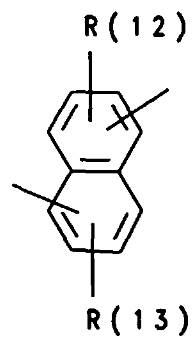

R(12) und R(13)

unabhängig voneinander Wasserstoff, Methyl, F, Cl, Br, J, $CF_3$ oder $-SO_w-R(14)$;
R(14) Methyl oder NR(15)R(16);
R(15) und R(16)

unabhängig voneinander Wasserstoff oder Alkyl mit 1, 2, 3 oder 4 C-Atomen;

w Null, 1 oder 2;

D $-C_dH_{2d}-X_f-$;

d Null, 1, 2, 3 oder 4;
X -O-, -CO-, -CH[OR(21)]-, $-SO_m-$ oder -NR(21)-;
f Null oder 1;

R(21) Wasserstoff oder Methyl;
m Null, 1 oder 2;

und die jeweils anderen Substituenten R(1) und R(2) und R(3)

unabhängig voneinander Wasserstoff, F, Cl, Br, J, -CN, -($C_1$-$C_8$)-Alkyl, -($C_2$-$C_8$)-Alkenyl, -NR(35)R(36) oder R(17)-$C_gH_{2g}$-$Z_h$-;
g Null, 1, 2, 3 oder 4;
h Null oder 1;
R(35) und R(36)

unabhängig voneinander Wasserstoff oder Alkyl mit 1, 2, 3, 4, 5 oder 6 C-Atomen;

oder
R(35) und R(36)

gemeinsam 4 - 7 Methylengruppen, von denen eine $CH_2$-Gruppe durch Sauerstoff, -S-, -NH-, -$NCH_3$ oder -N-Benzyl ersetzt sein kann;

Z -O-, -CO-, -$SO_v$-, -NR(18)-, -NR(18)-CO-, -NR(18)-CO-NH- oder -NR(18)-$SO_2$-;

R(18) Wasserstoff oder Methyl;
v Null, 1 oder 2;

R(17) Wasserstoff, Cycloalkyl mit 3, 5 oder 6 C-Atomen oder $C_kF_{2k+1}$-;

k 1, 2 oder 3,

oder
R(17) Pyrrol-1-yl, Pyrrol-2-yl oder Pyrrol-3-yl,

welches nicht substituiert oder substituiert ist mit 1 - 4 Substituenten ausgewählt aus der Gruppe bestehend aus F, Cl, Br, I, -CN, ($C_2$-$C_8$)-Alkanoyl, ($C_2$-$C_8$)-Alkoxycarbonyl, Formyl, Carboxy, - $CF_3$, Methyl und Methoxy;

oder
R(17) -($C_3$-$C_8$)-Cycloalkyl oder Phenyl,

welches nicht substituiert oder substituiert ist mit 1 - 3 Substituenten ausgewählt aus der Gruppe bestehend aus F und Cl, - $CF_3$, Methyl, Hydroxy, Methoxy, -NR(37)R(38), $CH_3SO_2$- und $H_2NO_2S$-;
R(37) und R(38)

Wasserstoff oder -$CH_3$;

R(4) und R(5)

unabhängig voneinander Wasserstoff, Alkyl mit 1, 2, 3 oder 4 C-Atomen, F, Cl, -OR(32), -NR(33)R(34) oder -$C_rF_{2r+1}$;
R(32), R(33) und R(34)

unabhängig voneinander Wasserstoff oder Alkyl mit 1, 2 oder 3 C-Atomen;

r 1, 2, 3 oder 4;

sowie deren pharmakologisch verträgliche Salze;

(HOE 95/F 173 - NZ 299 052)
am) Substituierte Zimtsäureguanidide der Formel I

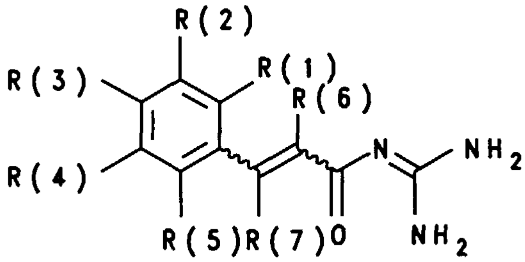

I

worin bedeuten:

mindestens einer der Substituenten R(1), R(2), R(3), R(4) und R(5) $-X_a-Y_b-L_n-U$;

X CR(16)R(17), O, S oder NR(18);

R(16), R(17) und R(18)

unabhängig voneinander H, Alkyl mit 1, 2, 3 oder 4 C-Atomen oder Perfluoralkyl mit 1, 2, 3 oder 4 C-Atomen;

a Null oder 1;

Y Alkylen mit 1, 2, 3, 4, 5, 6, 7 oder 8 C-Atomen, Alkylen-T mit 1, 2, 3, 4, 5, 6, 7 oder 8 C-Atomen in der Alkylengruppe, T, T-Alkylen mit 1, 2, 3, 4, 5, 6, 7 oder 8 C-Atomen in der Alkylengruppe;

T NR(20), O, S oder Phenylen,

wobei das Phenylen nicht substituiert oder substituiert ist mit 1 - 3 Substituenten ausgewählt aus der Gruppe bestehend aus F, Cl, $CF_3$, Methyl, Methoxy und NR(21)R(22);
R(20), R(21) und R(22)

unabhängig voneinander H, Alkyl mit 1, 2, 3 oder 4 C-Atomen oder Perfluoralkyl mit 1, 2, 3 oder 4 C-Atomen;

b Null oder 1;

L O, S, NR(23) oder $C_kH_{2k}$;

k 1, 2, 3, 4, 5, 6, 7, 8;

n Null oder 1;

U NR(24)R(25) oder einen N-haltigen Heterocyclus mit 1, 2, 3, 4, 5, 6, 7, 8 oder 9 C-Atomen;

R(24) und R(25)

unabhängig voneinander Wasserstoff, Alkyl mit 1, 2, 3, 4, 5, 6, 7 oder 8 C-Atomen, Perfluoralkyl mit 1, 2, 3, 4, 5, 6, 7 oder 8 C-Atomen;

oder
R(24) und R(25)

gemeinsam 4 oder 5 Methylenguppen, von denen eine $CH_2$-Gruppe durch Sauerstoff, S, NH, N-$CH_3$ oder N-Benzyl ersetzt sein kann;

wobei die N-haltigen Heterocyclen N- oder C-verbrückt sind und nicht substituiert oder substituiert sind mit 1 - 3 Substituenten ausgewählt aus der Gruppe bestehend aus F, Cl, $CF_3$, Methyl, Methoxy und NR(27)R(28);

R(23), R(27) und R(28)

unabhängig voneinander H, Alkyl mit 1, 2, 3 oder 4 C-Atomen oder Perfluoralkyl mit 1, 2, 3 oder 4 C-Atomen;

und die jeweils anderen Substituenten R(1), R(2), R(3), R(4) und R(5)

unabhängig voneinander H, F, Cl, Br, I, CN, $-O_n-C_mH_{2m+1}$, $-O_p-(CH_2)_s-C_qF_{2q+1}$ oder $-C_rH_{2r}R(10)$;
n Null oder 1;
m Null 1, 2, 3, 4, 5, 6, 7 oder 8;
p Null oder 1;
q 1, 2, 3, 4, 5, 6, 7 oder 8;
s Null, 1, 2, 3 oder 4;
r Null, 1, 2, 3 oder 4;
R(10)

Cycloalkyl mit 3, 4, 5, 6, 7 oder 8 C-Atomen, oder Phenyl,

wobei das Phenyl nicht substituiert oder substituiert ist mit 1 - 3 Substituenten ausgewählt aus der Gruppe bestehend aus F, Cl, $CF_3$, Methyl, Methoxy und NR(11)R(12);
R(11) und R(12)

unabhängig voneinander H, Alkyl mit 1, 2, 3 oder 4 C-Atomen oder Perfluoralkyl mit 1, 2, 3 oder 4 C-Atomen;

R(6) und R(7)

unabhängig voneinander Wasserstoff, F, Cl, Br, I, CN, Alkyl mit 1, 2, 3, 4, 5, 6, 7 oder 8 C-Atomen, Perfluoralkyl mit 1, 2, 3, 4, 5, 6, 7 oder 8 C-Atomen, Cycloalkyl mit 3, 4, 5, 6, 7 oder 8 C-Atomen, oder Phenyl,

das nicht substituiert oder substituiert ist mit 1 - 3 Substituenten ausgewählt aus der Gruppe bestehend aus F, Cl, $CF_3$, Methyl, Methoxy und NR(14)R(15);
R(14) und R(15)

unabhängig voneinander H, Alkyl mit 1, 2, 3 oder 4 C-Atomen oder Perfluoralkyl mit 1, 2, 3 oder 4 C-Atomen;

sowie deren pharmazeutisch verträgliche Salze;

(HOE 96/F 226 - EP 825 178)
bd) Phenylsubstituierte Alkenylcarbonsäure-guanidide der Formel I

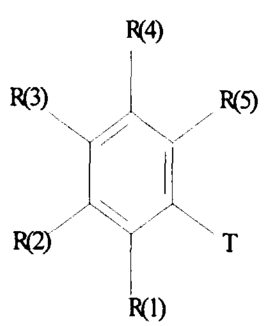

I

worin bedeuten:

T

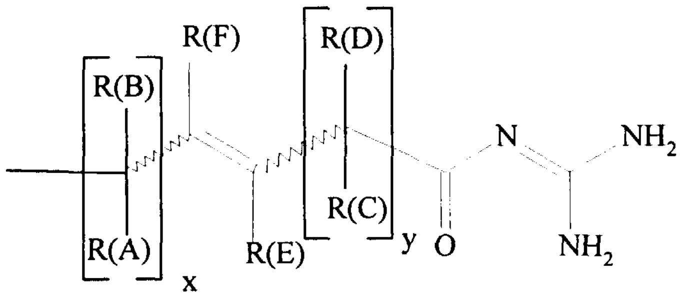

R(A) Wasserstoff, F, Cl, Br, I, CN, OH, OR(6), $(C_1-C_4)$-Alkyl, $O_r(CH_2)_aC_bF_{2b+1}$, $(C_3-C_8)$-Cycloalkyl oder NR(7)R(8)

r Null oder 1;
a Null, 1, 2, 3 oder 4;
b 1, 2, 3 oder 4;
R(6) $(C_1-C_4)$-Alkyl, $(C_1-C_4)$-Perfluoralkyl, $(C_3-C_6)$-Alkenyl, $(C_3-C_8)$-Cycloalkyl, Phenyl oder Benzyl,

wobei der Phenylkern nicht substituiert oder substituiert ist mit 1 - 3 Substituenten ausgewählt aus der Gruppe bestehend aus F, Cl, $CF_3$, Methyl, Methoxy und NR(9)R(10);
R(9) und R(10)

H, $(C_1-C_4)$-Alkyl oder $(C_1-C_4)$-Perfluoralkyl;

R(7) und R(8)

unabhängig voneinander wie R(6) definiert;

oder
R(7) und R(8)

gemeinsam 4 oder 5 Methylengruppen, von denen eine $CH_2$-Gruppe durch Sauerstoff, Schwefel, NH, $N-CH_3$ oder N-Benzyl ersetzt sein kann;

R(B), R(C) und R(D)

unabhängig wie R(A) definiert;

x Null, 1 oder 2;

y Null, 1 oder 2;

R(F) Wasserstoff, F, Cl, Br, I, CN, OR(12), $(C_1-C_8)$-Alkyl, $O_p(CH_2)_fC_gF_{2g+1}$, $(C_3-C_8)$-Cycloalkyl oder $(C_1-C_9)$-Heteroaryl;

p Null oder 1;
f Null, 1, 2, 3 oder 4;
g 1, 2, 3, 4, 5, 6, 7 oder 8;
R(12) $(C_1-C_8)$-Alkyl, $(C_1-C_4)$-Perfluoralkyl, $(C_3-C_8)$-Alkenyl, $(C_3-C_8)$-Cycloalkyl, Phenyl oder Benzyl,

wobei der Phenylkern nicht substituiert ist oder substituiert 1 - 3 Substituenten ausgewählt aus der Gruppe bestehend aus F, Cl, $CF_3$, Methyl, Methoxy und NR(13)R(14);
R(13) und R(14)

H, $(C_1-C_4)$-Alkyl oder $(C_1-C_4)$-Perfluoralkyl;

R(E) unabhängig wie R(F) definiert;

R(1) unabhängig wie T definiert;
oder
R(1) Wasserstoff, $-O_kC_mH_{2m+1}$, $-O_n(CH_2)_pC_qF_{2q+1}$, F, Cl, Br, I, CN, - (C=O)-N=C$(NH_2)_2$, $-SO_rR(17)$, $-SO_{r2}NR(31)R(32)$; $-O_u(CH_2)_vC_6H_5$, $-O_{u2}-(C_1-C_9)$-Heteroaryl oder $-S_{u2}-(C_1-C_9)$-Heteroaryl;

k Null oder 1;
m Null, 1, 2, 3, 4, 5, 6, 7 oder 8;
n Null oder 1;
p Null, 1, 2, 3 oder 4;
q 1, 2, 3, 4, 5, 6, 7 oder 8;
r Null, 1, 2;
r2 Null, 1, 2;
R(31) und R(32)

unabhängig voneinander Wasserstoff, $(C_1-C_8)$-Alkyl oder $(C_1-C_8)$-Perfluoralkyl;

oder
R(31) und R(32)

gemeinsam 4 oder 5 Methylengruppen, von denen eine $CH_2$-Gruppe durch Sauerstoff, S, NH, $N-CH_3$ oder N-Benzyl ersetzt sein kann;

R(17) $(C_1-C_8)$-Alkyl;
u Null oder 1;
u2 Null oder 1;
v Null, 1, 2, 3 oder 4;

wobei der Phenylkern substituiert ist oder substituiert mit 1 - 3 Substituenten ausgewählt aus der Gruppe bestehend aus F, Cl, $CF_3$, Methyl, Methoxy, $-(CH_2)_wNR(21)R(22)$, NR(18)R(19) und $(C_1-C_9)$-Heteroaryl;
R(18), R(19), R(21) und R(22)

unabhängig voneinander $(C_1-C_4)$-Alkyl oder $(C_1-C_4)$-Perfluoralkyl;

w 1, 2, 3 oder 4;
wobei der Heterocyclus des $(C_1-C_9)$-Heteroaryl unsubstituiert oder substituiert ist mit 1 - 3 Substituenten ausgewählt aus der Gruppe bestehend aus F, Cl, $CF_3$, Methyl oder Methoxy;

R(2), R(3), R(4) und R(5)

unabhängig voneinander wie R(1) definiert,

oder
R(1) und R(2) oder R(2) und R(3)

jeweils gemeinsam -CH-CH=CH-CH-,

das nicht substituiert oder substituiert ist mit 1 - 3 Substituenten ausgewählt aus der Gruppe bestehend aus F, Cl, $CF_3$, Methyl, Methoxy, - $(CH_2)_{w2}NR(24)R(25)$ und NR(26)R(27);
R(24), R(25), R(26) und R(27)

H, $(C_1-C_4)$-Alkyl oder $(C_1-C_4)$-Perfluoralkyl;

w2 1, 2, 3 oder 4;

wobei der Rest T im Molekül mindestens zweimal, jedoch nur höchstens dreimal vorhanden ist; sowie deren pharmazeutisch verträgliche Salze.

**14.** Verwendung nach Ansprüchen 1 -11, dadurchgekennzeichnet, daß eine Verbindung benutzt wird, die ausgewählt ist aus der Gruppe bestehend aus

3-Dimethylamino-5-trifluormethylbenzoylguanidin,
3-Phenyl-5-trifluormethylbenzoylguanidin,
3-Isopropyl-4-fluor-5-trifluormethylbenzoylguanidin,
3-Chlor-5-(1-pyrrolyl)benzoylguanidin,
3-Chlor-5-trifluormethylbenzoylguanidin,
3-Dimethylamino-5-(1-pyrrolyl)benzoylguanidin,
3-Dimethylamino-4-tert.butylbenzoylguanidin,
4-(3-Chloranilino)-3-methylsulfonylbenzoylguanidin,
4-(4-Aminoethylphenoxy)-3-methylsulfonylbenzoylguanidin,
4-(4-Aminoethylsulfonylmethylphenoxy)-3-trifluormethylbenzoylguanidin,
4-(4-Aminoethylthiophenoxy)-3-trifluormethylbenzoylguanidin,
3,5-Di-tert.butylbenzoylguanidin,
3,5-Di-tert.butyl-4-hydroxybenzoylguanidin,
5-Chlor-2-indoloylguanidin,
3,5-Dichlor-2-indoloylguanidin,
5-Chlor-3-phenylthio-2-indoloylguanidin,
5-Chlor-3-phenylsulfonyl-2-indoloylguanidin,
5-Methoxy-3-isopropyl-2-indoloylguanidin,
4-Benzyloxy-2-indoloylguanidin,
5-Chlor-1-(4-picolyl)-2-indoloylguanidin,
2-(4-Hydroxyphenyl)-4-benzimidazoloylguanidin,
N-{3-[3-(3-Guanidino-2-methyl-3-oxopropenyl)-phenyl]2-methyl-acryloyl}-guanidin,
und deren pharmakologisch verträglichen Salzen.